(19) Europäisches Patentamt
European Patent Office
Office européen des brevets

(11) EP 4 237 506 B1

(12) **EUROPÄISCHE PATENTSCHRIFT**

(45) Veröffentlichungstag und Bekanntmachung des Hinweises auf die Patenterteilung:
**17.07.2024 Patentblatt 2024/29**

(21) Anmeldenummer: **21799240.3**

(22) Anmeldetag: **25.10.2021**

(51) Internationale Patentklassifikation (IPC):
***C09K 11/06*** *(2006.01)* ***C07D 491/048*** *(2006.01)*
***C07D 495/04*** *(2006.01)* ***H10K 85/60*** *(2023.01)*

(52) Gemeinsame Patentklassifikation (CPC):
**C09K 11/06; C07D 491/048; C07D 495/04; H10K 85/657; H10K 85/6572; H10K 85/6574;**
H10K 50/11; H10K 85/342; H10K 2101/10; H10K 2101/90

(86) Internationale Anmeldenummer:
**PCT/EP2021/079450**

(87) Internationale Veröffentlichungsnummer:
**WO 2022/090108 (05.05.2022 Gazette 2022/18)**

# (54) ORGANISCHE ELEKTROLUMINESZIERENDE VORRICHTUNG

ORGANIC ELECTROLUMINESCENT DEVICE

DISPOSITIF ÉLECTROLUMINESCENT ORGANIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.10.2020 EP 20204008**

(43) Veröffentlichungstag der Anmeldung:
**06.09.2023 Patentblatt 2023/36**

(73) Patentinhaber: **Merck Patent GmbH**
**64293 Darmstadt (DE)**

(72) Erfinder:
- **PARHAM, Amir Hossain**
  **64293 Darmstadt (DE)**
- **EHRENREICH, Christian**
  **64293 Darmstadt (DE)**

(74) Vertreter: **Merck Patent Association**
**Merck Patent GmbH**
**64271 Darmstadt (DE)**

(56) Entgegenhaltungen:
**KR-A- 20170 113 320** **KR-A- 20180 010 165**
**US-A1- 2020 010 476**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 4 237 506 B1

## Beschreibung

### Gegenstand der Erfindung

Die vorliegende Erfindung betrifft eine organische elektrolumineszierende Vorrichtung enthaltend eine lichtemittierende Schicht, die ein elektronentransportierendes Hostmaterial und ein lochtransportierendes Hostmaterial umfasst, sowie eine Formulierung enthaltend eine Mischung der Hostmaterialien und eine Mischung enthaltend die Hostmaterialien. Das elektronentransportierende Hostmaterial entspricht einer Verbindung der Formel (1) enthaltend Diazadibenzofuran- oder Diazadibenzothiophen-Einheiten. Das lochtransportierende Hostmaterial entspricht einer Verbindung der Formel (2) aus der Klasse der Biscarbazole oder deren Derivate.

### Hintergrund der Erfindung

Der Aufbau organischer Elektrolumineszenzvorrichtungen (z.B. OLEDs - organic light emitting diodes oder OLECs - organic light emitting electrochemical cells), in denen organische Halbleiter als funktionelle Materialien eingesetzt werden, ist seit Langem bekannt. Als emittierende Materialien werden hierbei neben fluoreszierenden Emittern zunehmend metallorganische Komplexe eingesetzt, die Phosphoreszenz statt Fluoreszenz zeigen. Generell gibt es bei OLEDs, insbesondere auch bei OLEDs die Triplettemission (Phosphoreszenz) zeigen, jedoch immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung, und davon insbesondere die Host bzw. Matrixmaterialien. Verbesserungen dieser Materialien können zu deutlichen Verbesserungen elektrolumineszierender Vorrichtungen führen.

Hostmaterialien zur Verwendung in organischen elektronischen Vorrichtungen sind dem Fachmann gut bekannt. Im Stand der Technik wird häufig auch der Begriff Matrixmaterial verwendet, wenn ein Hostmaterial für phosphoreszierende Emitter gemeint ist. Diese Verwendung des Begriffs gilt auch für die vorliegende Erfindung. Mittlerweile wurde eine Vielzahl von Hostmaterialien sowohl für fluoreszierende als auch für phosphoreszierende elektronische Vorrichtungen entwickelt.

Eine weitere Möglichkeit, die Leistungsdaten elektronischer Vorrichtungen, insbesondere von organischen Elektrolumineszenzvorrichtungen, zu verbessern, besteht darin, Kombinationen aus zwei oder mehr Materialien, insbesondere Hostmaterialien bzw. Matrixmaterialien, zu verwenden.

In US 6,392,250 B1 wird die Verwendung einer Mischung bestehend aus einem Elektronentransportmaterial, einem Lochtransportmaterial und einem fluoreszierenden Emitter in der Emissionsschicht einer OLED offenbart. Mit Hilfe dieser Mischung konnte die Lebensdauer der OLED gegenüber dem Stand der Technik verbessert werden.

In US 6,803,720 B1 wird die Verwendung einer Mischung enthaltend einen phosphoreszierenden Emitter sowie ein Loch- und ein Elektronentransportmaterial in der Emissionsschicht einer OLED offenbart. Dabei sind sowohl das Loch- und das Elektrontransportmaterial kleine organische Moleküle.

In WO2015169412 werden spezielle Azadibenzofuranverbindungen bzw.

Azadibenzothiophenverbindungen als Hostmaterial für organische Elektrolumineszenzvorrichtungen beschrieben.

In WO2015105315 und WO2015105316 werden Heterocyclen enthaltend zwei N-Atome und deren Verwendung in organischen Elektrolumineszenzvorrichtungen als Hostmaterial beschrieben, gegebenenfalls in Kombination mit einem weiteren Hostmaterial.

In US2016013421 werden Benzothienopyrimidinverbindungen und deren Verwendung in einer organischen elektrolumineszierenden Vorrichtung als Hostmaterial beschrieben.

In US2016072078 werden elektronentransportierende Hostmaterialien enthaltend Carbazoleinheiten beschrieben.

In US2017200903 werden Diazadibenzofuranverbindungen bzw. Diazadibenzothiophenverbindungen und deren Verwendung in einer organischen elektrolumineszierenden Vorrichtung offenbart, insbesondere als Elektronentransportmaterial.

In KR20160046077 und KR20160046078 wird eine organische lichtemittierende Vorrichtung enthaltend eine lichtemittierende Schicht enthaltend spezielle Emitter in Kombination mit verschiedenen Hostmaterialien beschrieben.

In KR20170113320 werden speziell substituierte Dibenzofuranverbindungen sowie deren Verwendung als Hostmaterial zusammen mit einem Biscarbazol in einer organischen elektrolumineszierenden Vorrichtung beschrieben.

In WO17109637 werden Benzothienopyrimidinverbindungen und Benzofuropyrimidinverbindungen und deren Verwendung in einer organischen elektrolumineszierenden Vorrichtung als Hostmaterial beschrieben, wobei die Benzothienopyrimidinverbindungen und Benzofuropyrimidinverbindungen jeweils zwei Substituenten tragen, die eine Furan-, Thiophen- oder Pyrrol-Einheit enthalten.

In WO17186760 werden Diazacarbazolverbindungen und deren Verwendung in einer organischen elektrolumineszierenden Vorrichtung als Hostmaterial, Elektronentransportmaterial und Lochblockiermaterial beschrieben.

In WO18060218 werden Diazadibenzofuranverbindungen bzw. Diazadibenzothiophenverbindungen und deren Verwendung in einer organischen elektrolumineszierenden Vorrichtung offenbart, wobei die Benzothienopyrimidinverbindungen und Benzofuropyrimidinverbindungen jeweils mindestens einen Substituenten tragen, der eine über N gebundene Carbazoleinheit enthält.

In KR20180010165 werden Diazadibenzofuranverbindungen bzw. Diazadibenzothiophenverbindungen und deren Verwendung in einer organischen elektrolumineszierenden Vorrichtung offenbart.

In WO18060307 werden Diazadibenzofuranverbindungen bzw. Diazadibenzothiophenverbindungen und deren Verwendung in einer organischen elektrolumineszierenden Vorrichtung offenbart.

In WO18234932, WO19059577, WO19229583 und WO19229584 werden Diazadibenzofuran- und Diazadibenzothiophenderivate beschrieben, die als Hostmaterialien in einer elektrolumineszierenden Vorrichtung eingesetzt werden können.

In US2020161564 werden Diazadibenzofuranverbindungen bzw. Diazadibenzothiophenverbindungen und deren Verwendung in einer organischen elektrolumineszierenden Vorrichtung offenbart.

In US20200010476 werden heterocyclische Verbindungen offenbart sowie deren Verwendung in organischen elektrolumineszierenden Vorrichtungen beschrieben.

In WO20067657 wird eine Zusammensetzung von Materialien und deren Verwendung in optoelektronischen Vorrichtungen beschrieben.

Allerdings besteht bei Verwendung dieser Materialien oder bei der Verwendung von Mischungen der Materialien noch Verbesserungsbedarf, insbesondere in Bezug auf Effizienz, Betriebsspannung und/oder Lebensdauer der organischen elektrolumineszierenden Vorrichtung.

Aufgabe der vorliegenden Erfindung ist daher die Bereitstellung einer Kombination von Hostmaterialien, welche sich für den Einsatz in einer organischen elektrolumineszierenden Vorrichtung, insbesondere in einer fluoreszierenden oder phosphoreszierenden OLED eignen und zu guten Device-Eigenschaften insbesondere im Hinblick auf eine verbesserte Lebensdauer, führen, sowie die Bereitstellung der entsprechenden elektrolumineszierenden Vorrichtung.

## Zusammenfassung der Erfindung

Es wurde nun gefunden, dass die Kombination mindestens einer Verbindung der Formel (1) als erstes Hostmaterial und mindestens einer lochtransportierenden Verbindung der Formel (2) als zweites Hostmaterial in einer lichtemittierenden Schicht einer organischen elektrolumineszierenden Vorrichtung, diese Aufgabe löst und die Nachteile aus dem Stand der Technik beseitigen. Die Verwendung einer derartigen Materialkombination zur Herstellung der lichtemittierenden Schicht in einer organischen elektrolumineszierenden Vorrichtung führt zu sehr guten Eigenschaften dieser Vorrichtungen, insbesondere hinsichtlich der Lebensdauer, insbesondere bei gleicher oder verbesserter Effizienz und/oder Betriebsspannung. Die Vorteile zeigen sich insbesondere auch bei Anwesenheit einer lichtemittierenden Komponente in der Emissionsschicht, insbesondere bei Kombination mit Emittern der Formel (3) oder Emittern der Formeln (I) bis (VIII) bei Konzentrationen zwischen 2 und 15 Gew.-%, oder in Kombination mit Monoaminen der Formel (4) in der Lochinjektionsschicht und/oder Lochtransportschicht.

Ein erster Gegenstand der vorliegenden Erfindung ist daher eine organische elektrolumineszierende Vorrichtung umfassend eine Anode, eine Kathode und mindestens eine organische Schicht, enthaltend mindestens eine lichtemittierende Schicht, wobei die mindestens eine lichtemittierende Schicht mindestens eine Verbindung der Formel (1) als Hostmaterial 1 und mindestens eine Verbindung der Formel (2) als Hostmaterial 2 enthält,

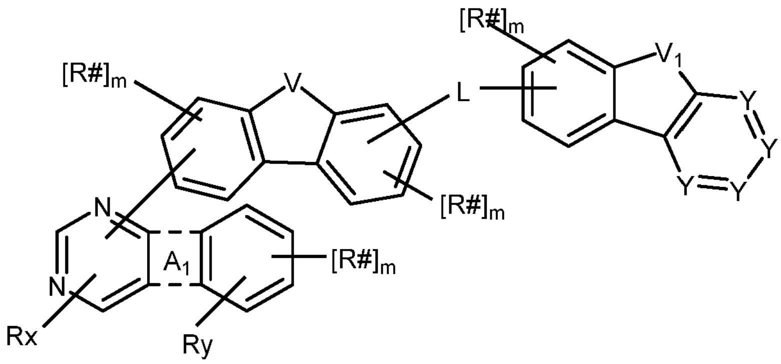

Formel (1)

,

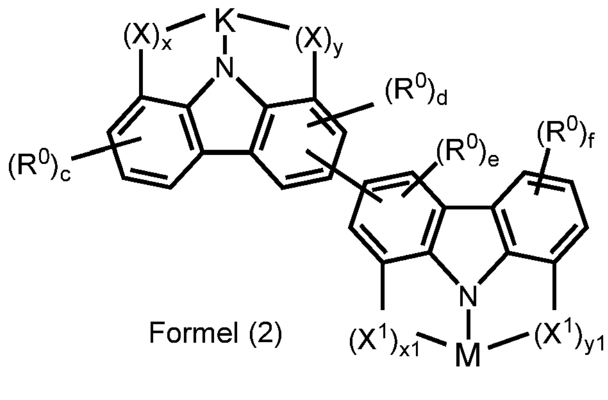

Formel (2)

,

wobei für die verwendeten Symbole und Indizes gilt:

Ring $A_1$ in Formel (1) entspricht der Formel (1A)

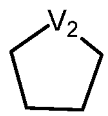

Formel (1A)

| | |
|---|---|
| V | ist O oder S; |
| $V_1$ | ist O, S, $C(R^1)_2$ oder N-A; |
| $V_2$ | ist O oder S; |
| Y | ist bei jedem Auftreten unabhängig voneinander gleich oder verschieden CH, CR oder CA, oder zwei benachbarte Gruppen Y bilden zusammen eine Gruppe der Formel (1B) |

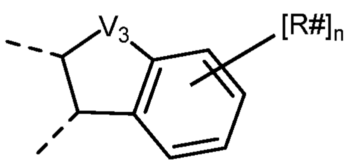

Formel (1B)

und die restlichen Gruppen Y sind unabhängig voneinander CH oder CR, wobei $V_3$ O, S, $C(R^1)_2$ oder N-A bedeutet und die gestrichelten Bindungen die Verknüpfung dieser Gruppe an den Rest der Formel (1) zeigen;

| | |
|---|---|
| Rx | ist $L_1$-R*; |
| Ry | ist $L_2$-R*; |
| R* | ist bei jedem Auftreten unabhängig ein aromatisches oder heteroaromatisches Ringsystem mit 6 bis 14 Ringatomen, das mit einem oder mehreren Resten R substituiert sein kann; |
| L, $L_1$, $L_2$ | sind jeweils unabhängig voneinander eine Bindung oder eine Phenylengruppe, die jeweils mit einem oder mehreren Resten R substituiert sein kann; |

A ist bei jedem Auftreten gleich oder verschieden ein aromatisches oder heteroaromatisches Ringsystem mit 6 bis 30 Ringatomen, das mit einem oder mehreren Resten R substituiert sein kann;

n ist 0, 1, 2, 3 oder 4;

m ist 0, 1, 2 oder 3;

R# ist D oder eine Arylgruppe mit 6 bis 12 C-Atomen, die mit einem oder mehreren Resten R substituiert sein kann;

$R^1$ ist bei jedem Auftreten gleich oder verschieden eine Alkylgruppe mit 1 bis 20 C-Atomen, Phenyl, das mit einem oder mehreren Resten R substituiert sein kann oder die zwei $R^1$ bilden zusammen mit dem C-Atom an das sie binden eine Spiroverbindung;

R ist bei jedem Auftreten gleich oder verschieden ausgewählt aus D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nichtbenachbarte $CH_2$-Gruppen durch $R^2C=CR^2$, O oder S ersetzt sein können und wobei ein oder mehrere H-Atome durch D, F, oder CN ersetzt sein können;

$R^2$ ist bei jedem Auftreten gleich oder verschieden ausgewählt aus der Gruppe bestehend aus H, D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch O oder S ersetzt sein können und wobei ein oder mehrere H-Atome durch D, F, oder CN ersetzt sein können;

K, M sind jeweils unabhängig voneinander ein unsubstituiertes oder teilweise oder vollständig deuteriertes oder einfach durch R* substituiertes aromatisches Ringsystem mit 6 bis 40 Ringatomen, wenn x und y 0 bedeuten und wenn x1 und y1 0 bedeuten, oder

K, M bilden jeweils unabhängig voneinander zusammen mit X oder $X^1$ ein heteroaromatisches Ringsystem mit 14 bis 40 Ringatomen, sobald der Wert von x, x1, y und/oder y1 1 bedeutet;

x, x1 sind jeweils unabhängig bei jedem Auftreten 0 oder 1;

y, y1 sind jeweils unabhängig bei jedem Auftreten 0 oder 1;

X und $X^1$ sind jeweils unabhängig voneinander bei jedem Auftreten eine Bindung oder $C(R^+)_2$;

$R^0$ ist bei jedem Auftreten unabhängig voneinander ein unsubstituiertes oder teilweise oder vollständig deuteriertes aromatisches Ringsystem mit 6 bis 18 C-Atomen;

$R^+$ ist bei jedem Auftreten unabhängig voneinander eine geradkettige oder verzweigte Alkylgruppe mit 1 bis 4 C-Atomen und

c, d, e und f sind unabhängig voneinander 0 oder 1.

Weitere Gegenstände der Erfindung umfassen ein Verfahren zur Herstellung der organischen elektrolumineszierenden Vorrichtungen sowie Mischungen enthaltend mindestens eine Verbindung der Formel (1) und mindestens eine Verbindung der Formel (2), spezielle Materialkombinationen und Formulierungen, die derartige Mischungen bzw. Materialkombinationen enthalten. Die entsprechenden bevorzugten Ausführungsformen, wie nachfolgend beschrieben, sind ebenfalls Gegenstand der vorliegenden Erfindung. Die überraschenden und vorteilhaften Effekte werden durch spezifische Selektion der Verbindungen der Formel (1) und der Verbindungen der Formel (2) erreicht. Die überraschenden und vorteilhaften Effekte werden durch spezifische Selektion der Verbindungen der Formel (1) und der Verbindungen der Formel (2) erreicht bevorzugt zusammen mit speziellen Emittern in der lichtemittierenden Schicht und/oder mit speziellen Monoaminen in der Lochinjektions- und/oder Lochtransportschicht erreicht.

## Detaillierte Beschreibung der Erfindung

Die erfindungsgemäße organische elektrolumineszierende Vorrichtung ist beispielsweise ein organischer lichtemittierender Transistor (OLET), ein organisches Feld-Quench-Device (OFQD), eine organische lichtemittierende elektrochemische Zelle (OLEC, LEC, LEEC), eine organische Laserdiode (O-Laser) oder eine organische lichtemittierende Diode (OLED). Die erfindungsgemäße organische elektrolumineszierende Vorrichtung ist insbesondere eine organische lichtemittierende Diode oder eine organische lichtemittierende elektrochemische Zelle. Besonders bevorzugt ist die erfindungsgemäße Vorrichtung eine OLED.

Die organische Schicht der erfindungsgemäßen Vorrichtung, die die lichtemittierende Schicht enthaltend die Materialkombination aus mindestens einer Verbindung der Formel (1) und mindestens einer Verbindung der Formel (2) enthält, wie zuvor beschrieben oder nachfolgend beschrieben, bevorzugt neben dieser lichtemittierenden Schicht (EML), eine Lochinjektionsschicht (HIL), eine Lochtransportschicht (HTL), eine Elektronentransportschicht (ETL), eine Elektroneninjektionsschicht (EIL) und/oder eine Lochblockierschicht (HBL). Es können in der erfindungsgemäßen Vorrichtung auch mehrere Schichten dieser Gruppe ausgewählt aus EML, HIL, HTL, ETL, EIL und HBL enthalten sein.

Die Vorrichtung kann aber auch anorganische Materialien enthalten oder auch Schichten, welche vollständig aus anorganischen Materialien aufgebaut sind.

Es ist bevorzugt, dass die organische Schicht der erfindungsgemäßen Vorrichtung eine Lochinjektionsschicht

und/oder die Lochtransportschicht enthält, deren lochinjizierendes Material und lochtransportierendes Material ein Monoamin ist, welches keine Carbazoleinheit enthält. Eine geeignete Auswahl an Monoaminverbindungen und bevorzugte Monoamine werden nachfolgend beschrieben.

Es ist bevorzugt, dass die lichtemittierende Schicht enthaltend mindestens eine Verbindung der Formel (1) und mindestens eine Verbindung der Formel (2) eine phosphoreszierende Schicht ist, die dadurch gekennzeichnet ist, dass sie zusätzlich zu der Hostmaterialienkombination Verbindungen der Formel (1) und Formel (2), wie zuvor beschrieben, mindestens einen phosphoreszierenden Emitter enthält. Eine geeignete Auswahl an Emittern und bevorzugte Emitter werden nachfolgend beschrieben.

Eine Arylgruppe im Sinne dieser Erfindung enthält 6 bis 40 aromatische Ringatome, bevorzugt C-Atome. Eine Heteroarylgruppe im Sinne dieser Erfindung enthält 5 bis 40 aromatische Ringatome, wobei die Ringatome C-Atome und mindestens ein Heteroatom umfassen, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Phenyl, abgeleitet von Benzol, bzw. ein einfacher heteroaromatischer Cyclus, beispielsweise abgeleitet von Pyridin, Pyrimidin oder Thiophen, oder eine kondensierte Aryl- oder Heteroarylgruppe, beispielsweise abgeleitet von Naphthalin, Anthracen, Phenanthren, Chinolin oder Isochinolin, verstanden. Eine Arylgruppe mit 6 bis 18 C-Atomen ist daher vorzugsweise Phenyl, Naphthyl oder Phenanthryl, wobei die Anbindung der Arylgruppe als Substituent dabei nicht eingeschränkt ist. Die Aryl- oder Heteroarylgruppe im Sinne dieser Erfindung kann einen oder mehrere Reste R tragen, wobei der Substituent R nachfolgend beschrieben wird.

Ein aromatisches Ringsystem im Sinne dieser Erfindung enthält 6 bis 40 Ringatome. Das aromatisches Ringsystem umfasst auch Arylgruppen, wie zuvor beschrieben.

Ein heteroaromatisches Ringsystem im Sinne dieser Erfindung enthält 5 bis 40 Ringatome und mindestens ein Heteroatom. Ein bevorzugtes heteroaromatisches Ringsystem hat 10 bis 40 Ringatome und mindestens ein Heteroatom. Das heteroaromatische Ringsystem umfasst auch Heteroarylgruppen, wie zuvor beschrieben. Die Heteroatome im heteroaromatischen Ringsystem sind bevorzugt ausgewählt aus N, O und/oder S.

Unter einem aromatischen oder heteroaromatischen Ringsystem im Sinne dieser Erfindung wird ein System verstanden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische bzw. heteroaromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sind Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls von der Definition des aromatischen bzw. heteroaromatischen Ringsystems umfasst.

Unter einem aromatischen oder heteroaromatischen Ringsystem mit 5 - 40 Ringatomen, welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem mit 6 bis 18 C-Atomen als Ringatome wird vorzugsweise aus Phenyl, 1,2-Biphenyl, 1,3-Biphenyl, 1,4-Biphenyl, Fluorenyl, Naphthyl, Phenanthryl und Triphenylenyl ausgewählt, welche mit einem oder mehreren Resten R substituiert sein können, wobei der Substituent R nachfolgend beschrieben wird.

Ein bevorzugtes heteroaromatisches Ringsystem mit 6 bis 18 Ringatomen wird vorzugsweise aus Dibenzofuranyl und Dibenzothiophenyl ausgewählt, welches mit einem oder mehreren Resten R substituiert sein kann, wobei der Substituent R nachfolgend beschrieben wird.

Die Abkürzung A bedeutet bei jedem Auftreten jeweils unabhängig voneinander gleich oder verschieden ein aromatisches oder heteroaromatisches Ringsystem mit 6 bis 30 Ringatomen, das mit einem oder mehreren Resten R substituiert sein kann, wobei der Rest R eine Bedeutung hat, wie zuvor oder nachfolgend beschrieben.

Unter einer cyclischen Alkylgruppe im Sinne dieser Erfindung wird eine monocyclische, eine bicyclische oder eine polycyclische Gruppe verstanden.

Im Rahmen der vorliegenden Erfindung werden unter einer geradkettigen, verzweigten oder cyclischen $C_1$- bis $C_{20}$-Alkylgruppe beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl, 1,1-Dimethyl-n-hept-1-yl, 1,1-Dimethyl-n-oct-1-yl, 1,1-Dimethyl-n-dec-1-yl, 1,1-Dimethyl-n-dodec-1-yl, 1,1-Dimethyl-n-tetradec-1-yl, 1,1-Dimethyl-n-hexadec-1-yl, 1,1-Dimethyl-n-octadec-1-yl, 1,1-Diethyl-n-hex-1-yl, 1,1-Diethyl-n-hept-1-yl, 1,1-Diethyl-n-oct-1-yl, 1,1-Diethyl-n-dec-1-yl, 1,1-Diethyl-n-dodec-1-yl, 1,1-Diethyl-n-tetradec-1-yl, 1,1-Diethyln-n-hexadec-1-yl, 1,1-Diethyl-n-octadec-1-yl, 1-(n-Propyl)-cyclohex-1-yl, 1-(n-Butyl)-cyclohex-1-yl, 1-(n-Hexyl)-cyclohex-1-yl, 1-(n-Octyl)-cyclohex-1-yl und 1-(n-Decyl)-cyclohex-1-yl verstanden.

Wenn die Hostmaterialien der lichtemittierenden Schicht umfassend mindestens eine Verbindung der Formel (1), wie zuvor beschrieben oder nachfolgend bevorzugt beschrieben, und mindestens eine Verbindung der Formel (2), wie zuvor beschrieben oder nachfolgend beschrieben, für einen phosphoreszierenden Emitter eingesetzt wird, ist es bevorzugt, wenn deren Triplettenergie nicht wesentlich kleiner als die Triplettenergie des phosphoreszierenden Emitters ist. Dabei gilt bevorzugt für das Triplettniveau $T_1$ (Emitter) -$T_1$ (Matrix) $\leq$ 0.2 eV, besonders bevorzugt $\leq$ 0.15 eV, ganz besonders bevorzugt $\leq$ 0.1 eV. Dabei ist $T_1$ (Matrix) das Triplettniveau des Matrixmaterials in der Emissionsschicht, wobei diese Bedingung für jedes der beiden Matrixmaterialien gilt, und $T_1$(Emitter) ist das Triplettniveau des phosphoreszierenden Emitters. Enthält die Emissionsschicht mehr als zwei Matrixmaterialien, so gilt die oben genannte Beziehung bevorzugt auch für jedes weitere Matrixmaterial.

Im Folgenden wird das Hostmaterial 1 und dessen bevorzugte Ausführungsformen beschrieben, welches/welche in der erfindungsgemäßen Vorrichtung enthalten ist. Die bevorzugten Ausführungsformen des Hostmaterials 1 der Formel (1) gelten auch für die erfindungsgemäße Mischung und/oder Formulierung.

In Verbindungen der Formel (1) ist die Anbindung der Diazagruppe in jeder der Positionen 1, 2, 3 oder 4 des zentralen Heterocyclus möglich. Bevorzugt ist die Anbindung der Diazagruppe in Position 1 des zentralen Heterocyclus.

Eine bevorzugte Ausführungsform der Verbindungen der Formel (1) sind daher Verbindungen der Formel (1a),

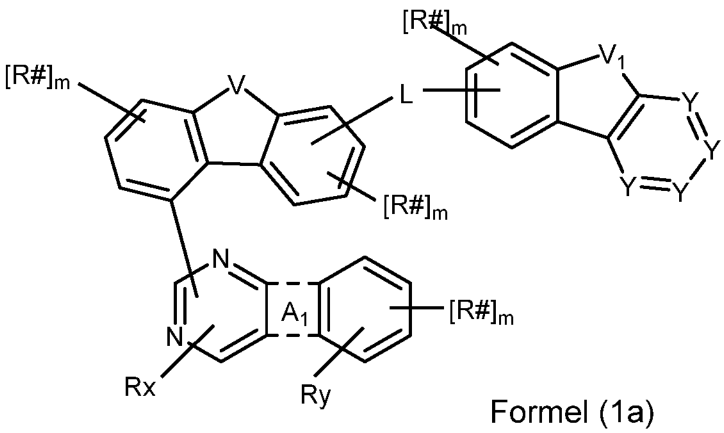

Formel (1a)

,

wobei die verwendeten Symbole Y, V, $V_1$, R#, m, Rx, Ry, L und Ring $A_1$ eine zuvor genannte oder nachfolgend genannt bevorzugte Bedeutung haben.

Ein weiterer Gegenstand der Erfindung ist die organische elektrolumineszierende Vorrichtung, wie zuvor beschrieben, wobei das Hostmaterial 1 der Formel (1a) entspricht, wie zuvor beschrieben.

In Verbindungen der Formeln (1) oder (1a) steht $V_1$ für O, S, $C(R^1)_2$ oder N-A, wobei $R^1$ und A eine zuvor angegebene oder nachfolgend angegebene bevorzugte Bedeutung hat.

Eine bevorzugte Ausführungsform der Verbindungen der Formeln (1) oder (1a) sind daher Verbindungen, bei denen $V_1$ NA bedeutet, die durch die Formel (1b) beschrieben werden,

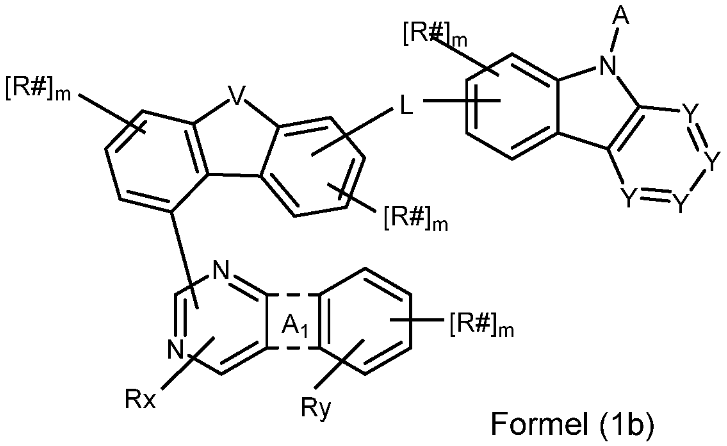

Formel (1b) ,

wobei die verwendeten Symbole Y, V, R#, m, Rx, Ry, L und Ring $A_1$ eine zuvor genannte oder nachfolgend genannt bevorzugte Bedeutung haben.

Eine bevorzugte Ausführungsform der Verbindungen der Formeln (1) oder (1a) sind daher Verbindungen, bei denen $V_1$ O bedeutet, die durch die Formel (1c) beschrieben werden,

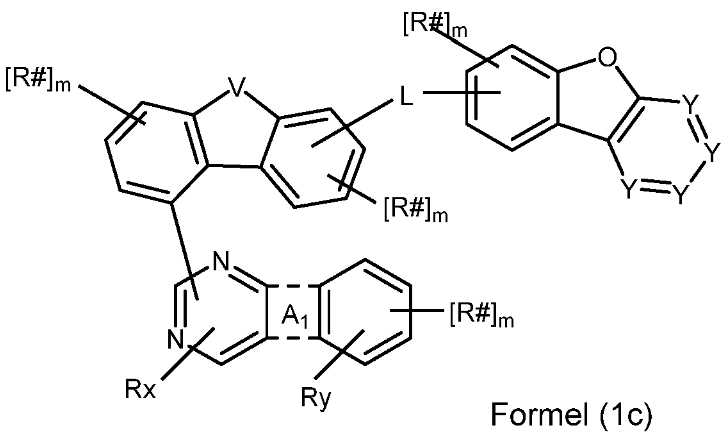

Formel (1c) ,

wobei die verwendeten Symbole Y, V, R#, m, Rx, Ry, L und Ring $A_1$ eine zuvor genannte oder nachfolgend genannt bevorzugte Bedeutung haben.

Eine bevorzugte Ausführungsform der Verbindungen der Formeln (1) oder (1a) sind daher Verbindungen, bei denen $V_1$ S bedeutet, die durch die Formel (1d) beschrieben werden,

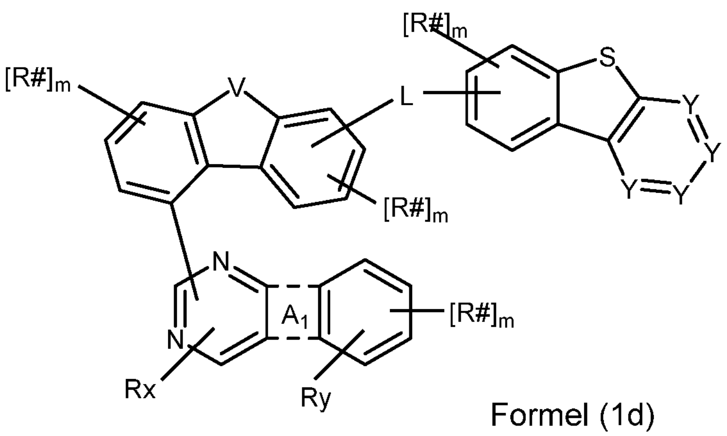

Formel (1d) ,

wobei die verwendeten Symbole Y, V, R#, m, Rx, Ry, L und Ring $A_1$ eine zuvor genannte oder nachfolgend genannt

bevorzugte Bedeutung haben.

Eine bevorzugte Ausführungsform der Verbindungen der Formeln (1) oder (1a) sind daher Verbindungen, bei denen $V_1$ $C(R^1)_2$ bedeutet, die durch die Formel (1e) beschrieben werden,

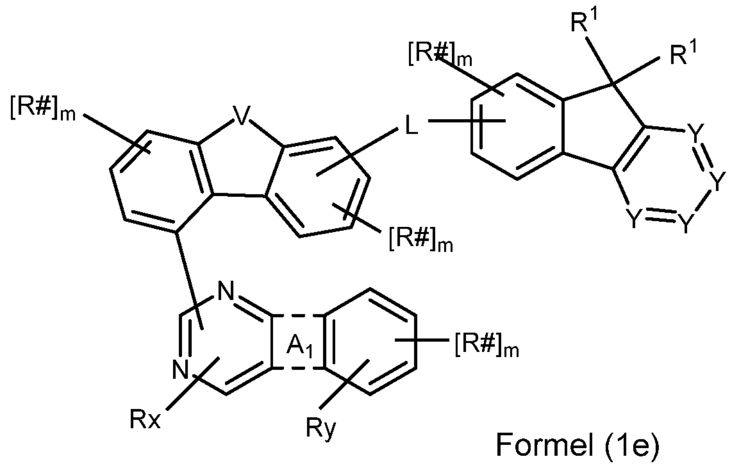

Formel (1e)

,

wobei die verwendeten Symbole Y, V, R#, m, Rx, Ry, L und Ring $A_1$ eine zuvor genannte oder nachfolgend genannt bevorzugte Bedeutung haben.

In Verbindungen der Formeln (1), (1a) und (1b) steht Y bei jedem Auftreten unabhängig voneinander gleich oder verschieden für CH, CR oder CA, oder zwei benachbarte Gruppen Y bilden zusammen eine Gruppe der Formel (1 B), wie zuvor beschrieben.

Bevorzugt steht $V_3$ für $C(R^1)_2$ oder N-A in Formel (1 B), wobei $R^1$ und A eine zuvor genannte oder besonders bevorzugt genannte Bedeutung haben. Besonders bevorzugt steht $V_3$ für $C(R^1)_2$ in Formel (1B), wobei $R^1$ eine zuvor genannte oder besonders bevorzugt genannte Bedeutung hat. Ist $V_3$ $C(R^1)_2$, so ist $R^1$ bevorzugt gleich und eine Alkylgruppe mit 1 bis 10 C-Atomen oder Phenyl. Ist $V_3$ $C(R^1)_2$, so ist $R^1$ besonders bevorzugt gleich und eine Methylgruppe. Ist $V_3$ N-A, so ist A bevorzugt ein aromatisches Ringsystem mit 6 bis 18 Ringatomen, das mit einem oder mehreren Resten R substituiert sein kann. Ist $V_3$ N-A, so ist A besonders bevorzugt Phenyl, das mit einem oder mehreren Resten R substituiert sein kann. In Formel (1B) steht R# bevorzugt für D oder Phenyl. Steth R# in der Formel (1B) für D, so ist n bevorzugt 1, 2, 3 oder 4, besonders bevorzugt 4. Steht R# in der Formel (1B) für Phenyl, so ist n bevorzugt 1. In Formel (1B) ist n ganz besonders bevorzugt 0.

In Verbindungen der Formeln (1), (1a), (1b), (1c), (1d) und (1e) steht Y bei jedem Auftreten unabhängig voneinander gleich oder verschieden bevorzugt für CH, CR oder CA.

In Verbindungen der Formeln (1), (1a), (1b), (1c), (1d) und (1e) steht Y bei jedem Auftreten unabhängig voneinander gleich oder verschieden besonders bevorzugt für CH oder CA.

Steht Y in Verbindungen der Formeln (1), (1a), (1b), (1c), (1d) und (1e) für CA, so ist A bevorzugt ein aromatisches oder heteroaromatisches Ringsystem mit 6 bis 24 Ringatomen, das mit einem oder mehreren Resten R substituiert sein kann. Steht Y in Verbindungen der Formeln (1), (1a), (1b), (1c), (1d) und (1e) für CA, so ist A besonders bevorzugt Cabazolyl, Phenyl, 1,2-Bipihenyl, 1,4-Bipenyl, Triphenylenyl, Phenylen-Triphenylen, Dibenzofuranyl oder Dibenzothiophenyl, die mit einem oder mehreren Resten R substituiert sein können. Bevorzugt steht in Verbindungen der Formeln (1), (1a), (1b), (1c), (1d) und (1e) ein Y für CA und die restlichen Y stehen für CH oder CR, bevorzugt für CH. Bevorzugt ist Y in Verbindungen der Formeln (1), (1a), (1b), (1c), (1d) und (1e) gleich und steht für CH oder CR, bevorzugt für CH.

In Verbindungen der Formeln (1), (1a) und (1e) steht $R^1$ bei jedem Auftreten unabhängig voneinander gleich oder verschieden für eine Alkylgruppe mit 1 bis 20 C-Atomen, Phenyl, das mit einem oder mehreren Resten R substituiert sein kann oder die zwei Gruppen $R^1$ bilden zusammen mit dem C-Atom an das sie binden eine Spiroverbindung. In Verbindungen der Formeln (1), (1a) und (1e) ist $R^1$ bevorzugt gleich und eine Alkylgruppe mit 1 bis 10 C-Atomen oder die zwei Gruppen $R^1$ bilden zusammen mit dem C-Atom an das sie binden eine Spiroverbindung. $R^1$ ist besonders bevorzugt gleich und eine Methylgruppe oder die zwei Gruppen $R^1$ bilden zusammen mit dem C-Atom an das sie binden eine Spiroverbindung. $R^1$ ist ganz besonders bevorzugt Methyl.

Eine besonders bevorzugte Ausführungsform der Verbindungen der Formeln (1) oder (1a) sind Verbindungen der Formeln (1b) und (1c), wie zuvor beschrieben, wobei die verwendeten Symbole Y, V, R#, m, Rx, Ry, L und Ring $A_1$ eine zuvor genannte oder nachfolgend genannt bevorzugte Bedeutung haben

Ein weiterer Gegenstand der Erfindung ist daher eine organische elektrolumineszierende Vorrichtung, wie zuvor beschrieben, wobei in Hostmaterial 1 $V_1$ O oder NA bedeutet und A eine zuvor genannte oder bevorzugt genannte Bedeutung hat.

In Verbindungen der Formeln (1), (1a), (1b), (1c), (1d) und (1e), wie zuvor beschrieben oder bevorzugt beschrieben, ist L eine Bindung oder ist eine Phenylengruppe der Formeln L-1, L-2 oder L-3,

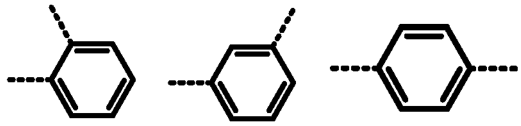

L-1 L-2 L-3

wobei die Phenylengruppen L-1, L-2 und L-3 mit einem oder mehreren Resten R substituiert sein kann. Bevorzugt sind die Phenylengruppen L-1, L-2 und L-3 nicht substituiert. Eine bevorzugte Phenylgruppe ist L-2. In Verbindungen der Formeln (1), (1a), (1b), (1c), (1d) und (1e), wie zuvor beschrieben oder bevorzugt beschrieben, ist L bevorzugt eine Bindung.

Der Substituent R# in Verbindungen der Formeln (1), (1a), (1b), (1c), (1d) und (1e) oder bevorzugt beschriebenen Verbindungen der Formeln (1), (1a), (1b), (1c), (1d) und (1e) steht bei jedem Auftreten unabhängig voneinander für D oder eine Arylgruppe mit 6 bis 12 C-Atomen, die mit einem oder mehreren Resten R substituiert sein kann.

Steht R# für D in Verbindungen der Formeln (1), (1a), (1b), (1c), (1d) und (1e), so ist es bevorzugt dass m bei jedem Auftreten gleich 3 bedeutet.

Steht R# für eine Arylgruppe mit 6 bis 12 C-Atomen, die mit einem oder mehreren Resten R substituiert sein kann, in Verbindungen der Formeln (1), (1a), (1b), (1c), (1d) und (1e), so ist m bei jedem Auftreten unabhängig voneinander bevorzugt 0, 1 oder 2, besonders bevorzugt 0 oder 1. Die Arylgruppe mit 6 bis 12 C-Atomen, die mit einem oder mehreren Resten R substituiert sein kann ist bevorzugt Phenyl, das mit einem oder mehreren Resten R substituiert sein kann, wobei R eine zuvor angegebene oder bevorzugt angegebene Bedeutung hat. R# ist bevorzugt unsubstituiertes Phenyl oder vollständig oder teilweise deuteriertes Phenyl. R# ist besonders bevorzugt unsubstituiertes Phenyl. In Verbindungen der Formeln (1), (1a), (1b), (1c), (1d) und (1e) steht m bevorzugt ein Mal für 1, wobei R# eine Arylgruppe mit 6 bis 12 C-Atomen bedeutet, die mit einem oder mehreren Resten R substituiert sein kann und alle anderen Indizes m stehen für 0. In Verbindungen der Formeln (1), (1a), (1b), (1c), (1d) und (1e) steht m bevorzugt bei jedem Auftreten für 0.

Der Substituent R in Verbindungen der Formeln (1), (1a), (1b), (1c), (1d), (1e) oder der Gruppe (1B) oder bevorzugt beschriebenen Verbindungen der Formeln (1), (1a), (1b), (1c), (1 d), (1e) oder einer bevorzugten Gruppe (1B) ist jeweils unabhängig bei jedem Auftreten ausgewählt aus D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch $R^2C=CR^2$, O oder S ersetzt sein können und wobei ein oder mehrere H-Atome durch D, F, oder CN ersetzt sein können. Der Substituent R wird bei Auftreten unabhängig voneinander bevorzugt aus D, F, CN, einer geradkettigen Alkylgruppe mit 1 bis 10 C-Atomen oder einer verzweigten oder cyclischen Alkylgruppe mit 3 bis 10 C-Atomen ausgewählt, wobei ein oder mehrere H-Atome der Alkylgruppe durch D, F, oder CN ersetzt sein können.

Der Substituent $R^2$ ist bei jedem Auftreten gleich oder verschieden ausgewählt aus der Gruppe bestehend aus H, D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch O oder S ersetzt sein können und wobei ein oder mehrere H-Atome durch D, F, oder CN ersetzt sein können und ist bevorzugt H oder D.

Der Ring $A_1$ im Diazasubstituenten der Formel (1C) des Hostmaterials 1 der Formeln (1), (1a), (1b), (1c), (1d) und (1e), wie zuvor beschrieben oder bevorzugt beschrieben,

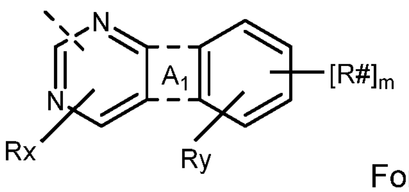

Formel (1C),

entspricht der Formel (1A),

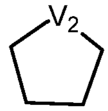 Formel (1A) ,

wobei $V_2$ O oder S bedeutet und die gestrichelte Linie in Formel (1C) die Anbindung an den Rest der Formel (1) oder den Rest der Formel (1a) des Hostmaterials 1 bedeutet.

Bevorzugte Ausführungsformen des Diazasubstituenten der Formel (1C) des Hostmaterials 1 der Formeln (1), (1a), (1b), (1c), (1d) und (1e), wie zuvor beschrieben oder bevorzugt beschrieben, entsprechen den Formeln (1C-1), (1C-2), (1C-3) und (1C-4),

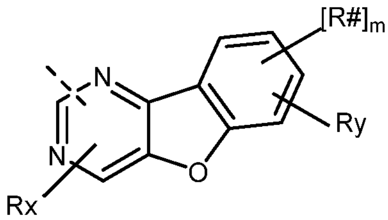
Formel (1C-1) ,

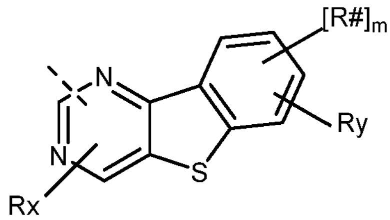
Formel (1C-2) ,

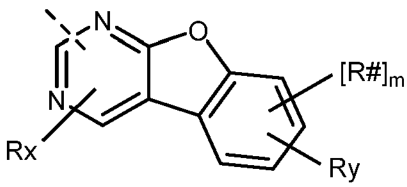
Formel (1C-3) ,

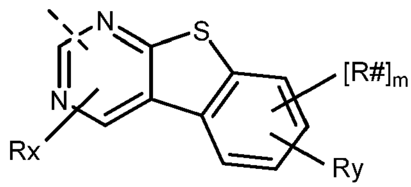
Formel (1C-4) ,

wobei die gestrichelte Linie, Rx, Ry, R# und m eine zuvor angegebene oder nachfolgend angegebene Bedeutung haben.

Die Anbindung des Substituenten Rx an den Diazasubstituenten der Formeln (1C), (1C-1), (1C-2), (1C-3) oder (1C-4) des Hostmaterials 1 der Formeln (1), (1a), (1b), (1c), (1d) und (1e), wie zuvor beschrieben oder bevorzugt beschrieben, sind nicht eingeschränkt, sondern an beiden Positionen möglich.

Bevorzugte Ausführungsformen des Diazasubstituenten der Formel (1C-1) entsprechen den Formeln (1C-5) und (1C-6)

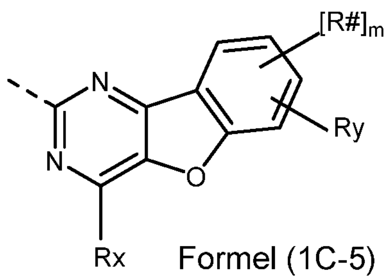
Formel (1C-5) ,

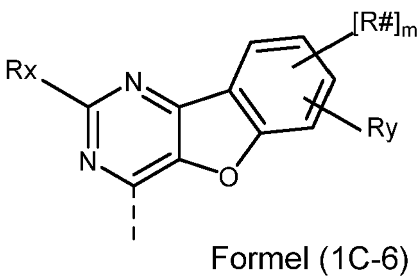
Formel (1C-6) ,

wobei die gestrichelte Linie, Rx, Ry, R# und m eine zuvor angegebene oder nachfolgend angegebene Bedeutung haben.

Bevorzugte Ausführungsformen des Diazasubstituenten der Formel (1C-2) entsprechen den Formeln (1C-7) und (1C-8)

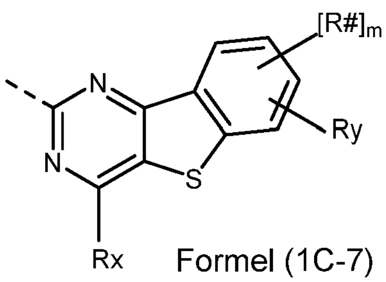

Formel (1C-7),

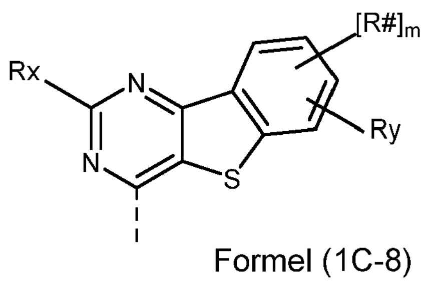

Formel (1C-8),

wobei die gestrichelte Linie, Rx, Ry, R# und m eine zuvor angegebene oder nachfolgend angegebene Bedeutung haben.

Bevorzugte Ausführungsformen des Diazasubstituenten der Formel (1C-3) entsprechen den Formeln (1C-9) und (1C-10)

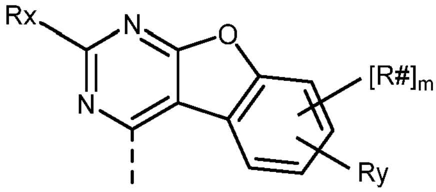

Formel (1C-9).

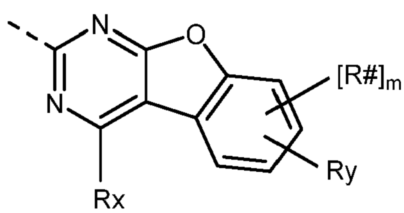

Formel (1C-10).

wobei die gestrichelte Linie, Rx, Ry, R# und m eine zuvor angegebene oder nachfolgend angegebene Bedeutung haben.

Bevorzugte Ausführungsformen des Diazasubstituenten der Formel (1C-4) entsprechen den Formeln (1C-11) und (1C-12)

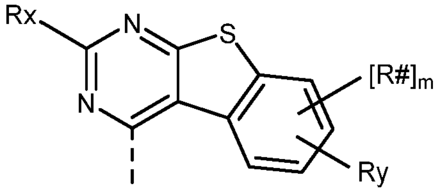

Formel (1C-11),

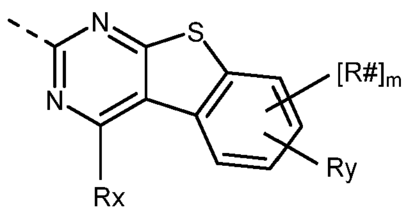

Formel (1C-12),

wobei die gestrichelte Linie, Rx, Ry, R# und m eine zuvor angegebene oder nachfolgend angegebene Bedeutung haben.

Ein weiterer Gegenstand der Erfindung ist die organische elektrolumineszierende Vorrichtung, wie zuvor beschrieben, wobei das Hostmaterial 1 einer der Formeln (1), (1a), (1b), (1c), (1d) und (1e) entspricht, wie zuvor beschrieben oder bevorzugt beschrieben, und der Diazasubstituent der Formel (1C) aus einer der Formeln (1C-1), (1C-2), (1C-3), (1C-4), (1C-5), (1C-6), (1C-7), (1C-8), (1C-9), (1C-10), (1C-11) oder (1C-12) ausgewählt wird.

In einer bevorzugten Ausführungsform des Hostmaterials 1 der Formeln (1), (1a), (1b), (1c), (1d) oder (1e), wie zuvor beschrieben oder bevorzugt beschrieben, wird der Diazasubstituent der Formel (1C) aus einer der Formeln (1C-5), (1C-7), (1C-9) oder (1C-11) ausgewählt.

In einer bevorzugten Ausführungsform des Hostmaterials 1 der Formeln (1), (1a), (1b), (1c), (1d) oder (1e), wie zuvor beschrieben oder bevorzugt beschrieben, wird der Diazasubstituent der Formel (1C) aus einer der Formeln (1C-5), (1C-6), (1C-9) oder (1C-10) ausgewählt; besonders bevorzugt aus der Formel (1C-5) oder (1C-9).

In einer bevorzugten Ausführungsform des Hostmaterials 1 der Formeln (1), (1a), (1b), (1c), (1d) oder (1e), wie zuvor beschrieben oder bevorzugt beschrieben, wird der Diazasubstituent der Formel (1C) aus einer der Formeln (1C-7), (1C-8), (1C-11) oder (1C-12) ausgewählt; besonders bevorzugt aus der Formel (1C-7) oder (1C-11).

Die Anbindung des Substituenten Ry an den Diazasubstituenten der Formeln (1C), (1C-1), (1C-2), (1C-3) oder (1C-4) des Hostmaterials 1 der Formeln (1), (1a), (1b), (1c), (1d) oder (1e), wie zuvor beschrieben oder bevorzugt beschrieben, ist nicht eingeschränkt, sondern an allen vier Positionen möglich.

Bevorzugt steht Ry an diesen beiden Positionen, wie in der Formel (1C-13) und (1C-14) abgebildet

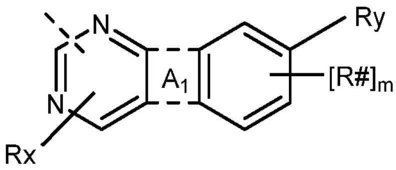

Formel (1C-13) ,

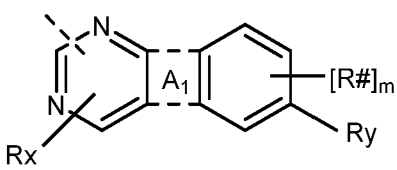

Formel (1C-14) ,

wobei die gestrichelte Linie, Ring $A_1$, Rx, Ry, R# und m eine zuvor angegebene oder nachfolgend angegebene Bedeutung haben.

Besonders bevorzugte Ausführungsformen des Diazasubstituenten der Formel (1C) des Hostmaterials 1 der Formeln (1), (1a), (1b), (1c), (1d) oder (1e), wie zuvor beschrieben oder bevorzugt beschrieben, entsprechen den Formeln (1C-15) bis (1C-30)

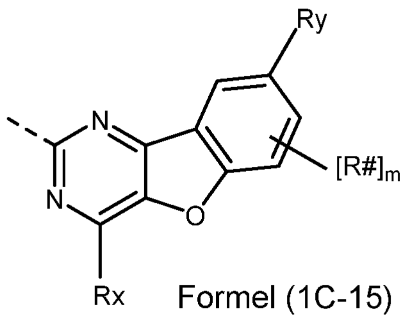

Formel (1C-15) ,

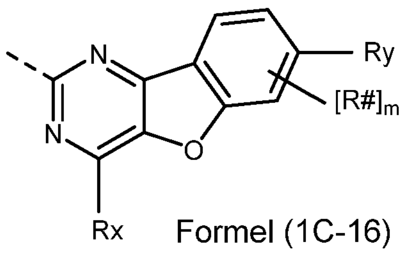

Formel (1C-16) ,

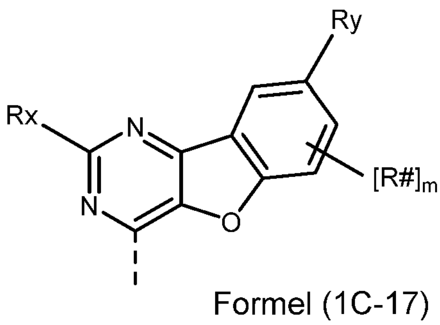

Formel (1C-17) ,

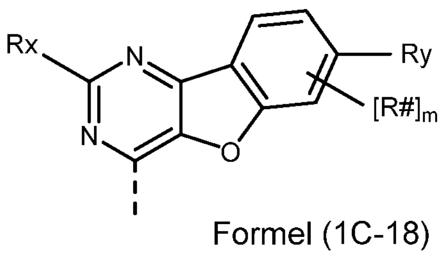

Formel (1C-18) ,

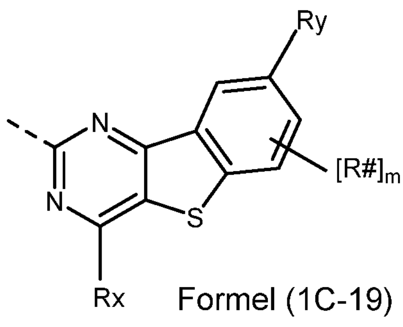

Formel (1C-19) ,

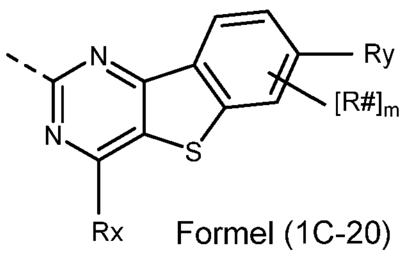

Formel (1C-20) ,

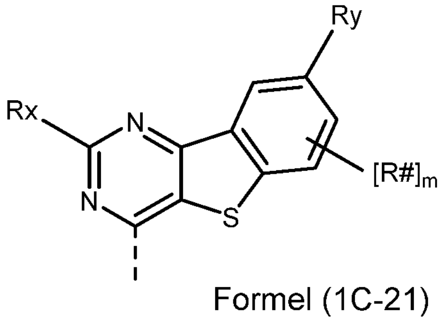

Formel (1C-21) ,

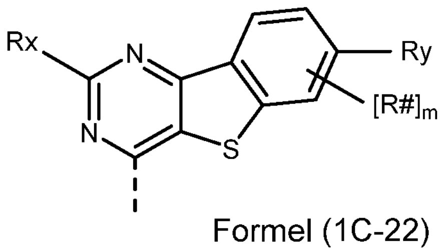

Formel (1C-22) ,

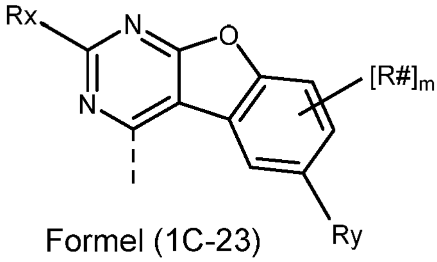

Formel (1C-23) ,

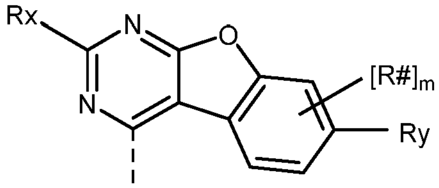

Formel (1C-24) ,

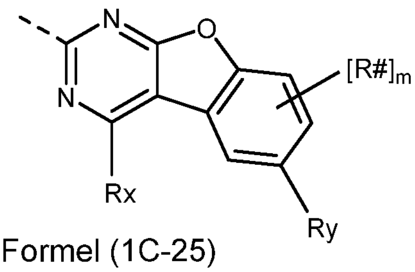

Formel (1C-25) ,

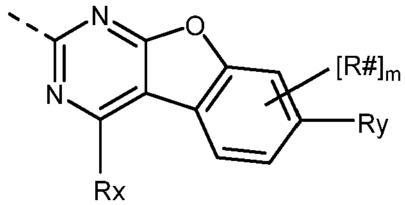

Formel (1C-26) ,

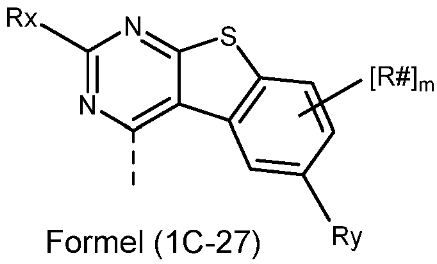

Formel (1C-27) ,

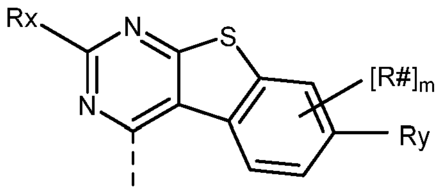

Formel (1C-28) ,

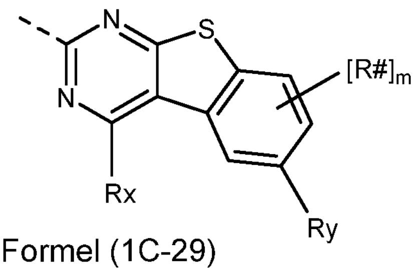

Formel (1C-29) ,

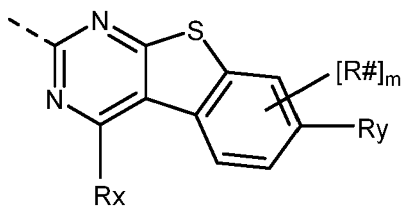

Formel (1C-30) ,

wobei die gestrichelte Linie, Rx, Ry, R# und m eine zuvor angegebene oder nachfolgend angegebene Bedeutung haben.

Besonders bevorzugte Ausführungsformen des Diazasubstituenten der Formel (1C) des Hostmaterials 1 der Formeln (1), (1a), (1b), (1c), (1d) oder (1e), wie zuvor beschrieben oder bevorzugt beschrieben, entsprechen den Formeln (1C-15), (1C-17), (1C-19), (1C-21), (1C-23), (1C-25), (1C-27) und (1C-29), wie zuvor beschrieben.

Ganz besonders bevorzugte Ausführungsformen des Diazasubstituenten der Formel (1C) des Hostmaterials 1 der Formeln (1), (1a), (1b), (1c), (1d) oder (1e), wie zuvor beschrieben oder bevorzugt beschrieben, entsprechen den

Formeln (1C-15), (1C-16), (1C-19), (1C-20), (1C-23), (1C-24), (1C-27) und (1C-28), wie zuvor beschrieben. Ganz besonders bevorzugte Ausführungsformen des Diazasubstituenten der Formel (1C) des Hostmaterials 1 der Formeln (1), (1a), (1b), (1c), (1d) oder (1e), wie zuvor beschrieben oder bevorzugt beschrieben, entsprechen den Formeln (1C-15), (1C-16), (1C-23) und (1C-24), wie zuvor beschrieben. Ganz besonders bevorzugte Ausführungsformen des Diazasubstituenten der Formel (1C) des Hostmaterials 1 der Formeln (1), (1a), (1b), (1c), (1d) oder (1e), wie zuvor beschrieben oder bevorzugt beschrieben, entsprechen den Formeln (1C-19), (1C-20), (1C-27) und (1C-28), wie zuvor beschrieben.

Im Hostmaterial 1 der Formeln (1), (1a), (1b), (1c), (1d) oder (1e) mit einem Diazasubstituenten der Formeln (1C), (1C-1), (1C-2), (1C-3), (1C-4), (1C-5), (1C-6), (1C-7), (1C-8), (1C-9), (1C-10), (1C-11), (1C-12), (1C-13), (1C-14), (1C-15), (1C-16), (1C-17), (1C-18), (1C-19), (1C-20), (1C-21), (1C-22), (1C-23), (1C-24), (1C-25), (1C-26), (1C-27), (1C-28), (1C-29) oder (1C-30), wie zuvor beschrieben oder bevorzugt beschrieben, steht der Substituent Rx für $L_1$-R* und der Substituent Ry für $L_2$-R*, wobei R* bei jedem Auftreten unabhängig ein aromatisches oder heteroaromatisches Ringsystem mit 6 bis 14 Ringatomen bedeutet, das mit einem oder mehreren Resten R substituiert sein kann, wobei R eine zuvor genannte Bedeutung hat und $L_1$ und $L_2$ eine zuvor und nachfolgend angegebene Bedeutung haben.

In Verbindungen der Formeln (1), (1a), (1b), (1c), (1d) oder (1e) mit einem Diazasubstituenten der Formeln (1C), (1C-1), (1C-2), (1C-3), (1C-4), (1C-5), (1C-6), (1C-7), (1C-8), (1C-9), (1C-10), (1C-11), (1C-12), (1C-13), (1C-14), (1C-15), (1C-16), (1C-17), (1C-18), (1C-19), (1C-20), (1C-21), (1C-22), (1C-23), (1C-24), (1C-25), (1C-26), (1C-27), (1C-28), (1C-29) oder (1C-30), wie zuvor beschrieben oder bevorzugt beschrieben, ist $L_1$ eine Bindung oder ist eine Phenylengruppe der Formeln L-1, L-2 oder L-3, wobei die Phenylengruppen L-1, L-2 und L-3 mit einem oder mehreren Resten R substituiert sein kann. Bevorzugt sind die Phenylengruppen L-1, L-2 und L-3 nicht substituiert. Bevorzugte Phenylgruppen für $L_1$ sind die Gruppen L-2 und L-3. In Verbindungen der Formeln (1), (1a), (1b), (1c), (1d) und (1e), wie zuvor beschrieben oder bevorzugt beschrieben, ist $L_1$ bevorzugt eine Bindung.

In Verbindungen der Formeln (1), (1a), (1b), (1c), (1d) oder (1e) mit einem Diazasubstituenten der Formeln (1C), (1C-1), (1C-2), (1C-3), (1C-4), (1C-5), (1C-6), (1C-7), (1C-8), (1C-9), (1C-10), (1C-11), (1C-12), (1C-13), (1C-14), (1C-15), (1C-16), (1C-17), (1C-18), (1C-19), (1C-20), (1C-21), (1C-22), (1C-23), (1C-24), (1C-25), (1C-26), (1C-27), (1C-28), (1C-29) oder (1C-30), wie zuvor beschrieben oder bevorzugt beschrieben, ist $L_2$ eine Bindung oder ist eine Phenylengruppe der Formeln L-1, L-2 oder L-3, wobei die Phenylengruppen L-1, L-2 und L-3 mit einem oder mehreren Resten R substituiert sein kann. Bevorzugt sind die Phenylengruppen L-1, L-2 und L-3 nicht substituiert. Bevorzugte Phenylgruppen für $L_1$ sind die Gruppen L-2 und L-3. In Verbindungen der Formeln (1), (1a), (1b), (1c), (1d) und (1e), wie zuvor beschrieben oder bevorzugt beschrieben, ist $L_2$ bevorzugt eine Bindung.

In Verbindungen der Formeln (1), (1a), (1b), (1c), (1d) oder (1e) mit einem Diazasubstituenten der Formeln (1C), (1C-1), (1C-2), (1C-3), (1C-4), (1C-5), (1C-6), (1C-7), (1C-8), (1C-9), (1C-10), (1C-11), (1C-12), (1C-13), (1C-14), (1C-15), (1C-16), (1C-17), (1C-18), (1C-19), (1C-20), (1C-21), (1C-22), (1C-23), (1C-24), (1C-25), (1C-26), (1C-27), (1C-28), (1C-29) oder (1C-30), wie zuvor beschrieben oder bevorzugt beschrieben, ist R* bei jedem Auftreten unabhängig bevorzugt Phenyl, 1,2-Biphenyl, 1,3-Biphenyl, 1,4-Biphenyl, Fluorenyl, Dibenzofuranyl oder Dibenzothiophenyl, das mit einem oder mehreren Resten R substituiert sein kann. Bevorzugt ist R* nicht substituiert.

In Verbindungen der Formeln (1), (1a), (1b), (1c), (1d) oder (1e) mit einem Diazasubstituenten der Formeln (1C), (1C-1), (1C-2), (1C-3), (1C-4), (1C-5), (1C-6), (1C-7), (1C-8), (1C-9), (1C-10), (1C-11), (1C-12), (1C-13), (1C-14), (1C-15), (1C-16), (1C-17), (1C-18), (1C-19), (1C-20), (1C-21), (1C-22), (1C-23), (1C-24), (1C-25), (1C-26), (1C-27), (1C-28), (1C-29) oder (1C-30), wie zuvor beschrieben oder bevorzugt beschrieben, ist R* in Ry besonders bevorzugt Phenyl, Fluorenyl, Dibenzofuranyl oder Dibenzothiophenyl, das mit einem oder mehreren Resten R substituiert sein kann. Bevorzugt ist R* in Ry nicht substituiert.

In Verbindungen der Formeln (1), (1a), (1b), (1c), (1d) oder (1e) mit einem Diazasubstituenten der Formeln (1C), (1C-1), (1C-2), (1C-3), (1C-4), (1C-5), (1C-6), (1C-7), (1C-8), (1C-9), (1C-10), (1C-11), (1C-12), (1C-13), (1C-14), (1C-15), (1C-16), (1C-17), (1C-18), (1C-19), (1C-20), (1C-21), (1C-22), (1C-23), (1C-24), (1C-25), (1C-26), (1C-27), (1C-28), (1C-29) oder (1C-30), wie zuvor beschrieben oder bevorzugt beschrieben, ist R* in Rx besonders bevorzugt Phenyl, 1,4-Biphenyl, Fluorenyl, Dibenzofuranyl oder Dibenzothiophenyl, das mit einem oder mehreren Resten R substituiert sein kann. Bevorzugt ist R* in Rx nicht substituiert.

In Verbindungen der Formeln (1), (1a), (1b), (1c), (1d) oder (1e) mit einem Diazasubstituenten der Formeln (1C), (1C-1), (1C-2), (1C-3), (1C-4), (1C-5), (1C-6), (1C-7), (1C-8), (1C-9), (1C-10), (1C-11), (1C-12), (1C-13), (1C-14), (1C-15), (1C-16), (1C-17), (1C-18), (1C-19), (1C-20), (1C-21), (1C-22), (1C-23), (1C-24), (1C-25), (1C-26), (1C-27), (1C-28), (1C-29) oder (1C-30), wie zuvor beschrieben oder bevorzugt beschrieben, steht V bevorzugt für O.

Demzufolge ist ein weiterer Gegenstand der Erfindung die organische elektrolumineszierende Vorrichtung, wie zuvor beschrieben, wobei im Hostmaterial 1 der Formeln (1), (1a), (1b), (1c), (1d) oder (1e) mit einem Diazasubstituenten der Formeln (1C), (1C-1), (1C-2), (1C-3), (1C-4), (1C-5), (1C-6), (1C-7), (1C-8), (1C-9), (1C-10), (1C-11), (1C-12), (1C-13), (1C-14), (1C-15), (1C-16), (1C-17), (1C-18), (1C-19), (1C-20), (1C-21), (1C-22), (1C-23), (1C-24), (1C-25), (1C-26), (1C-27), (1C-28), (1C-29) oder (1C-30), wie zuvor beschrieben oder bevorzugt beschrieben, V für O steht.

Beispiele für geeignete Hostmaterialien der Formel (1), wie zuvor beschrieben oder bevorzugt beschrieben, die erfindungsgemäß ausgewählt werden, und bevorzugt in Kombination mit mindestens einer Verbindung der Formel

(2) in der erfindungsgemäßen elektrolumineszierenden Vorrichtung verwendet werden, sind die nachstehend genannten Strukturen der Tabelle 1.

Tabelle 1:

| | | |
|---|---|---|
| 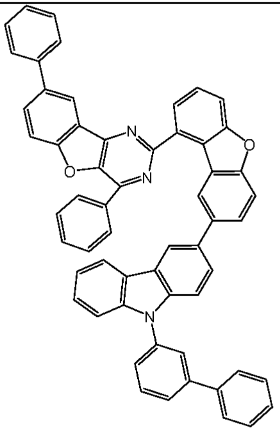 | 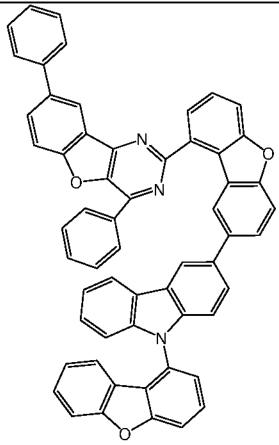 | 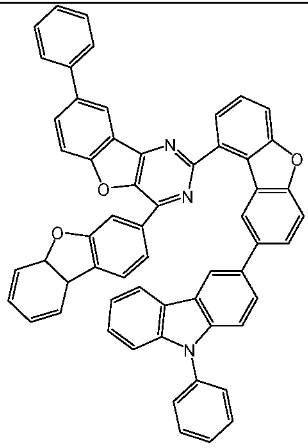 |
| 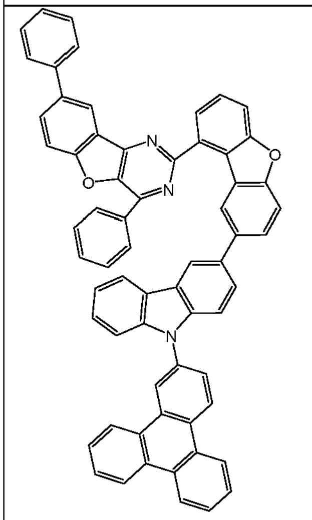 | 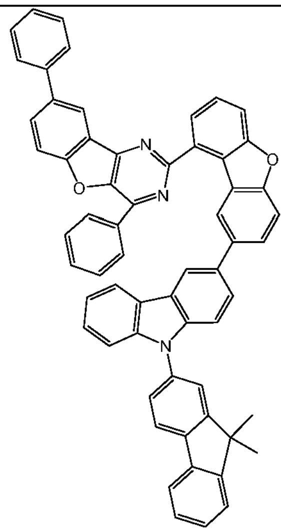 | 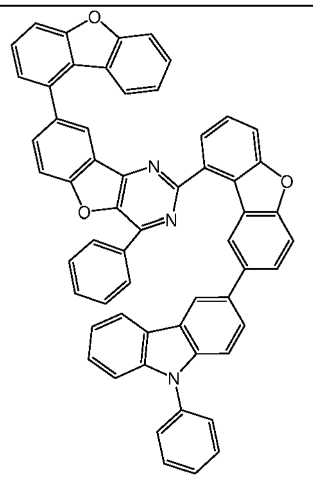 |
| 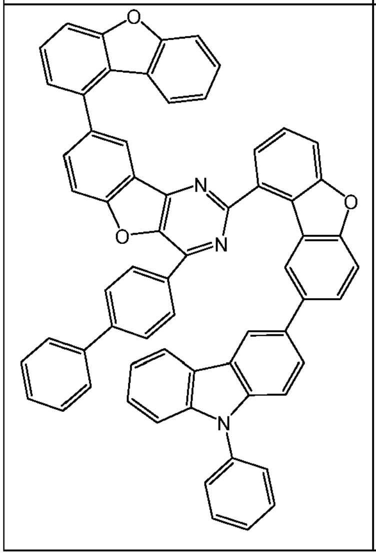 | 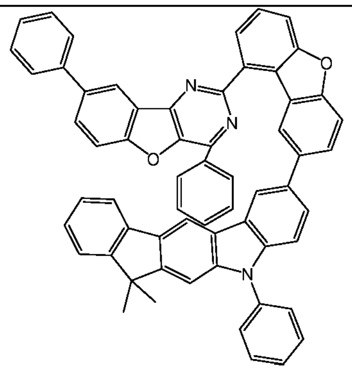 | 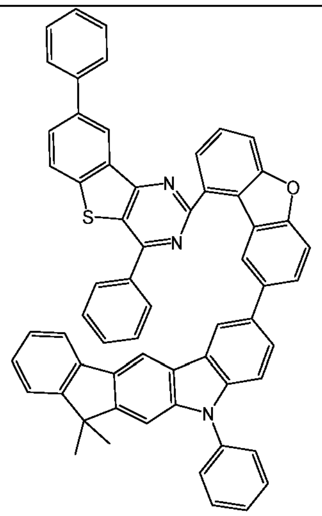 |

| | | |
|---|---|---|
| 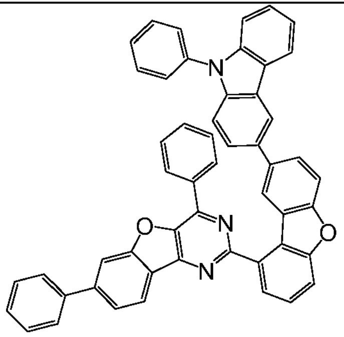 | 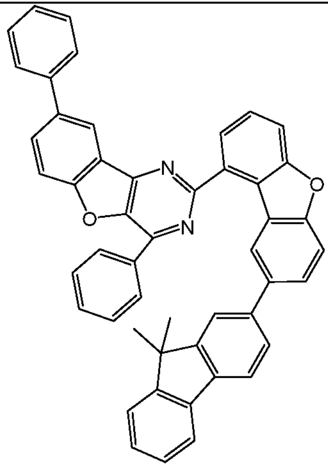 | 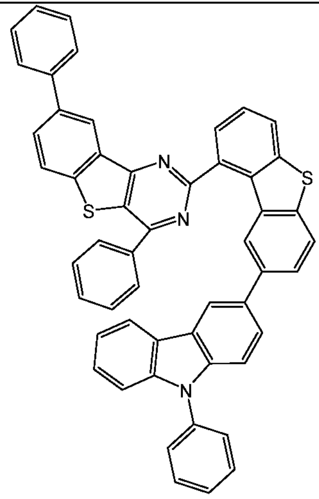 |
| 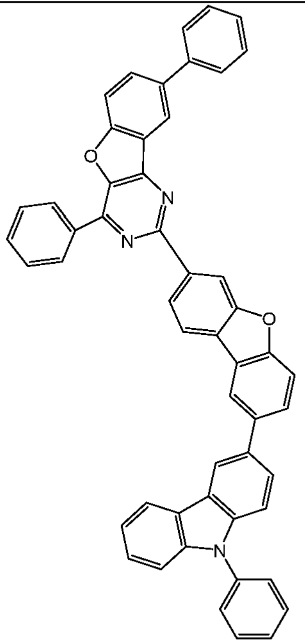 | 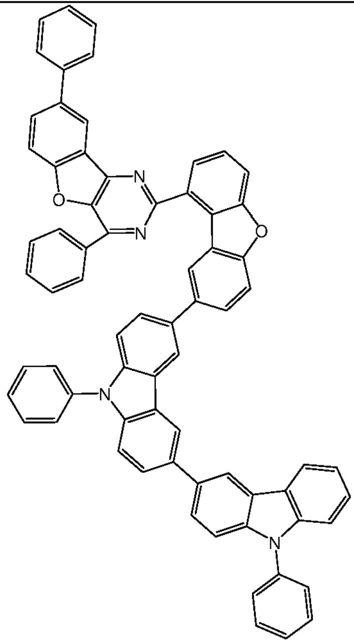 | 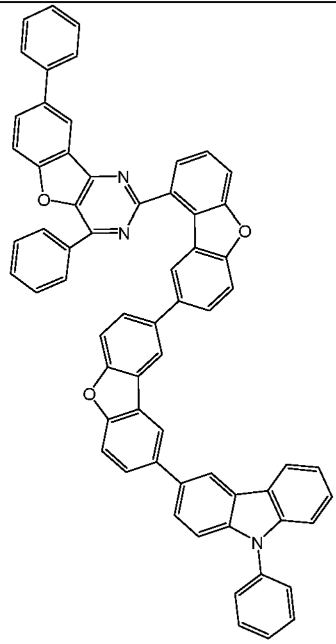 |
| 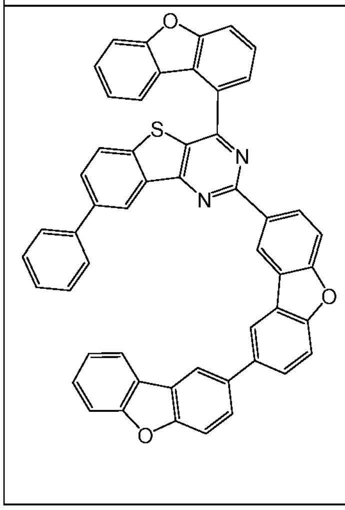 | 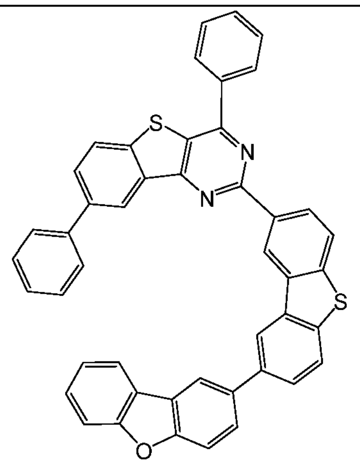 | 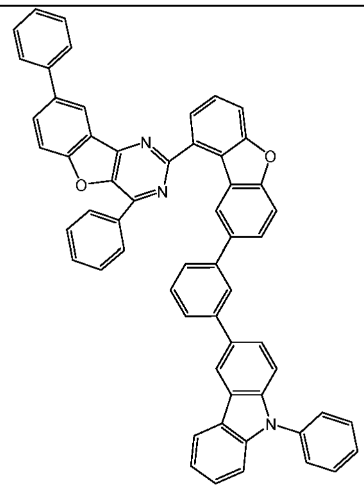 |

| | | |
|---|---|---|
| 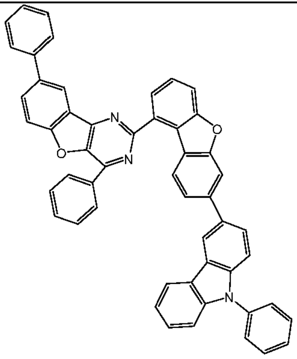 | 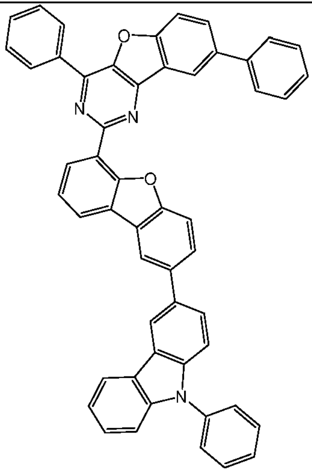 | 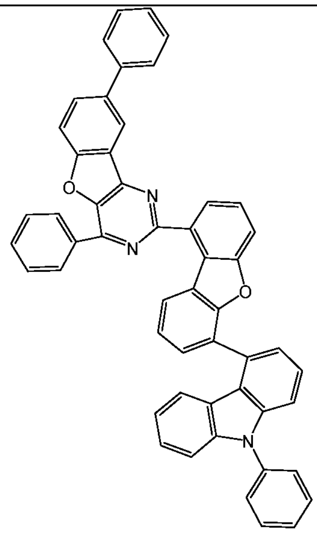 |
| 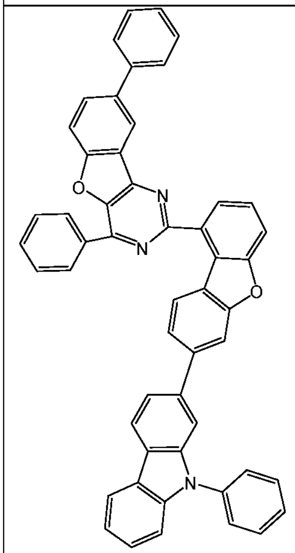 | 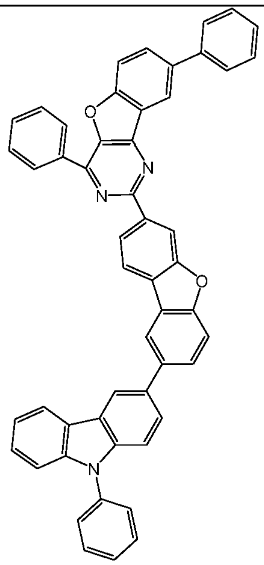 | 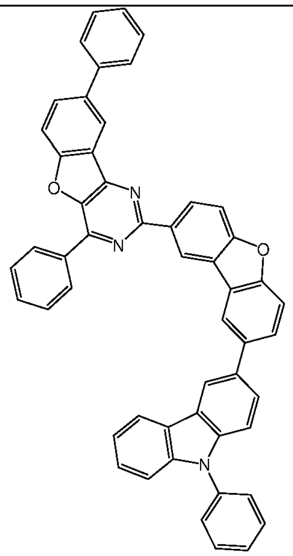 |
| 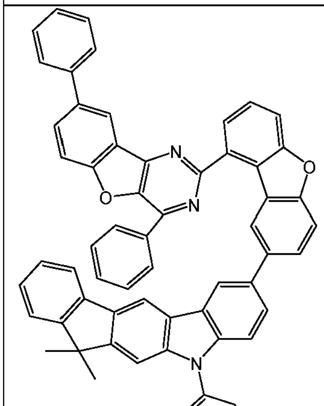 | 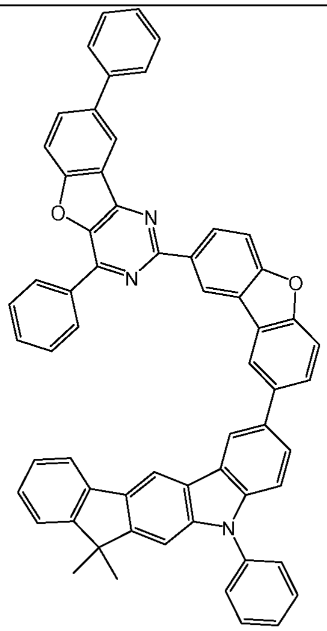 | 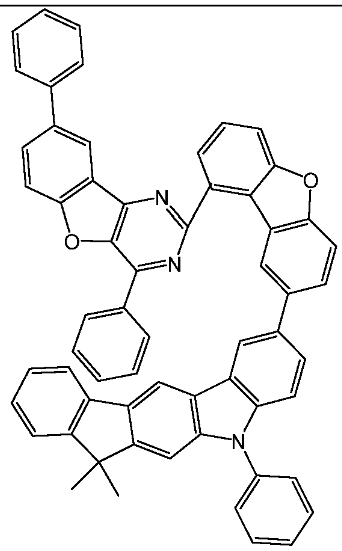 |

| | | |
|---|---|---|
| 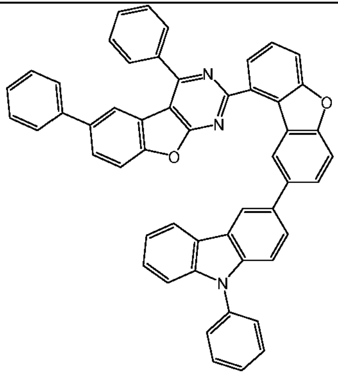 | 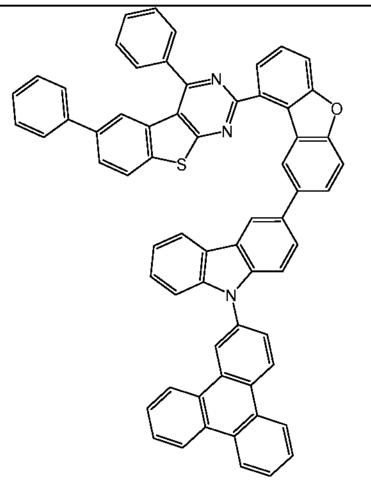 | 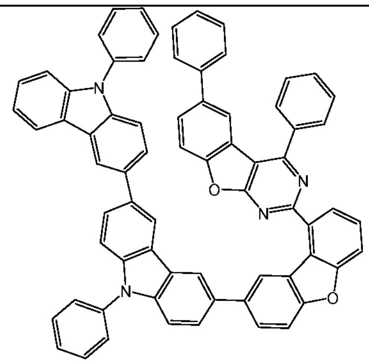 |
| 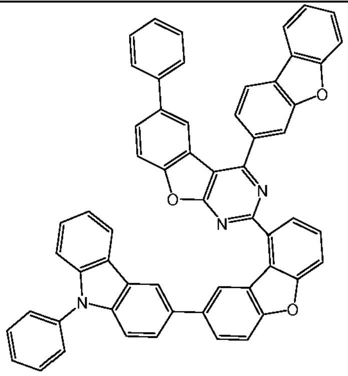 | 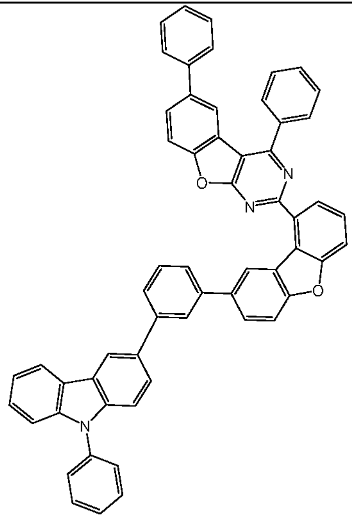 | 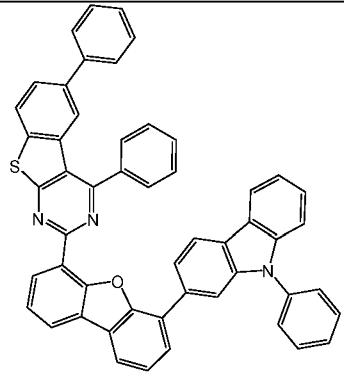 |
| 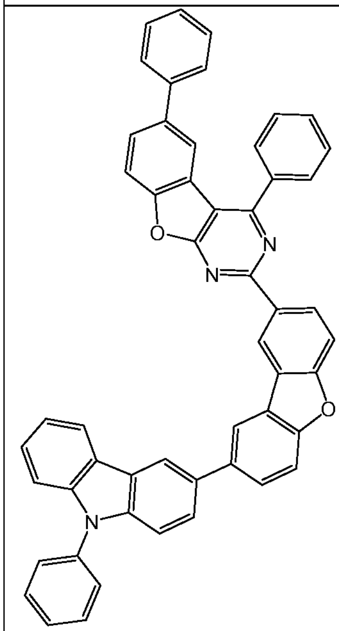 | 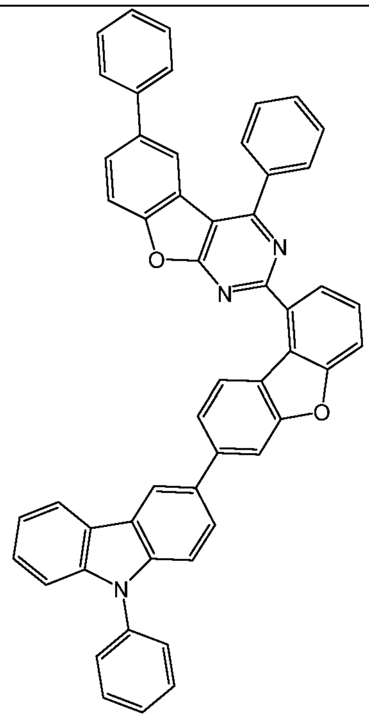 | 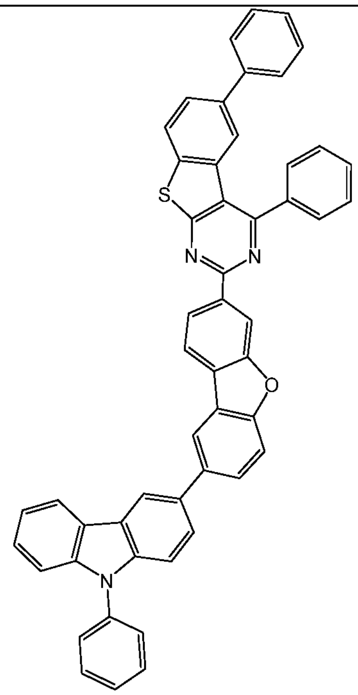 |

| | | |
|---|---|---|
| 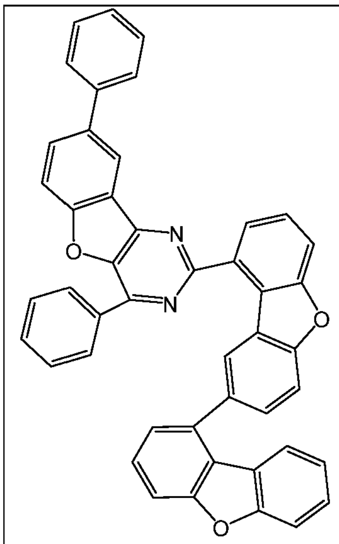 | 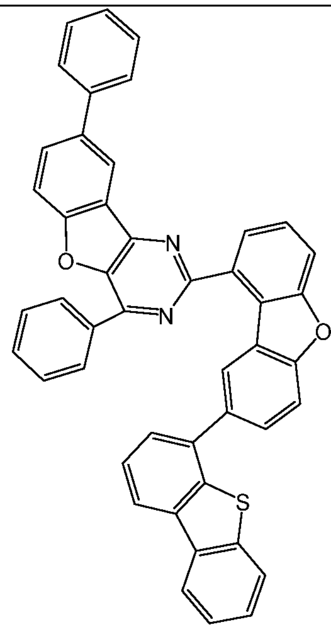 | 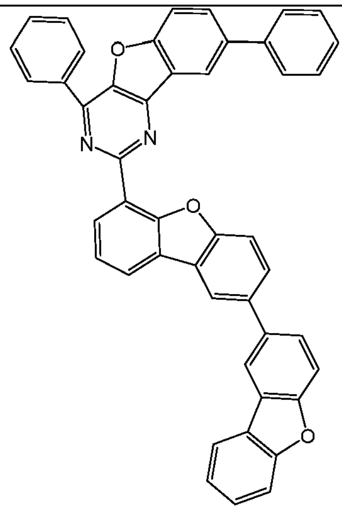 |
| 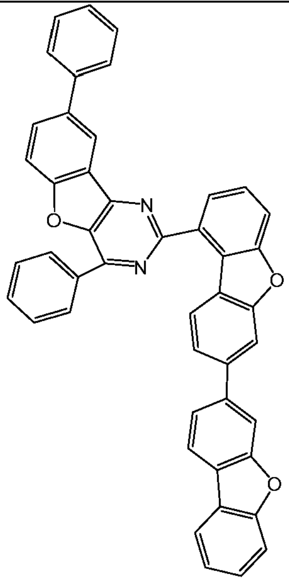 | 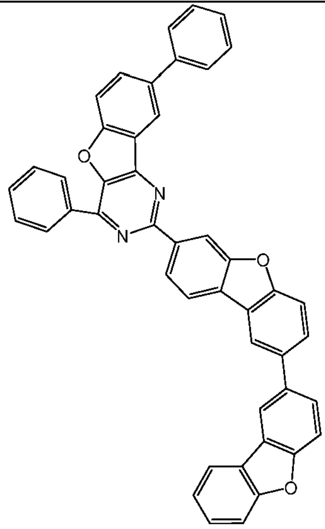 | 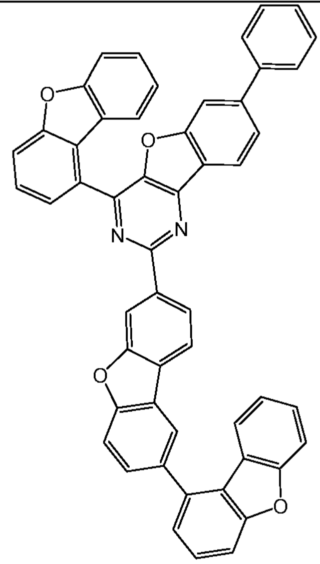 |
| 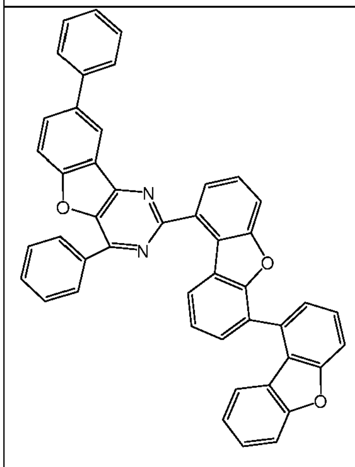 | 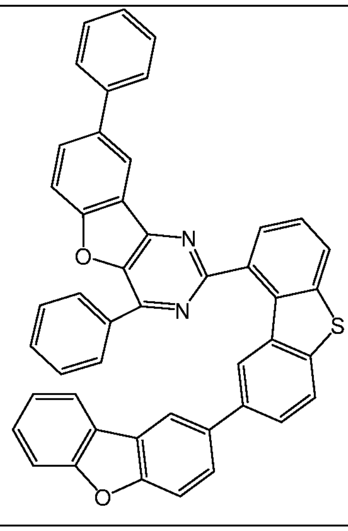 | 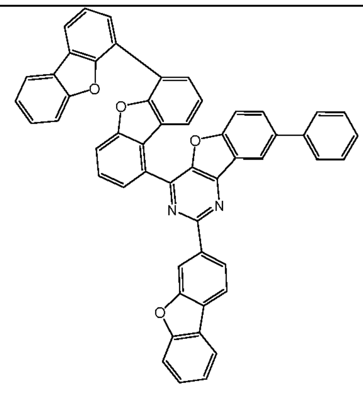 |

| | | |
|---|---|---|
| 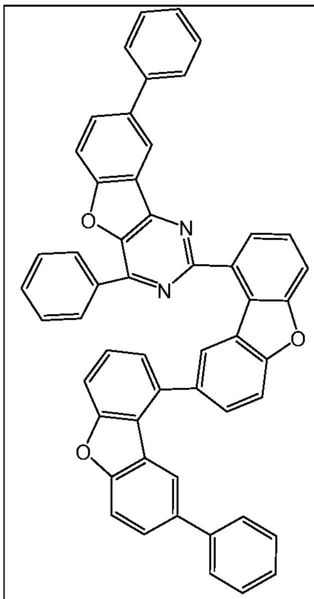 | 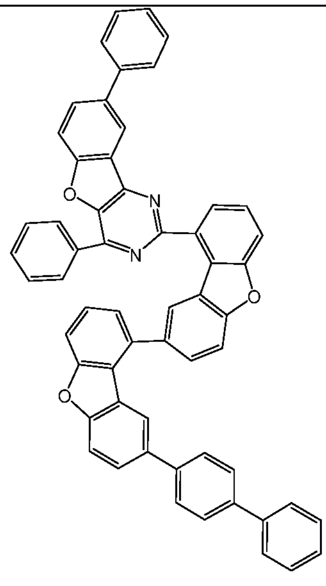 | 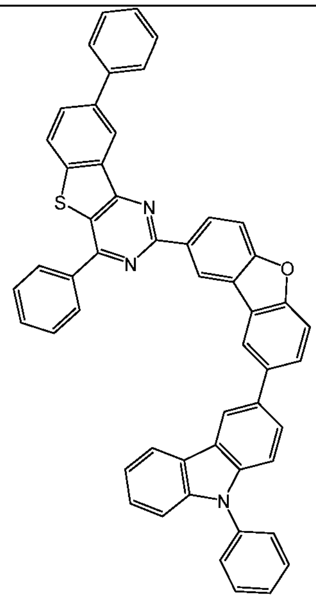 |
| 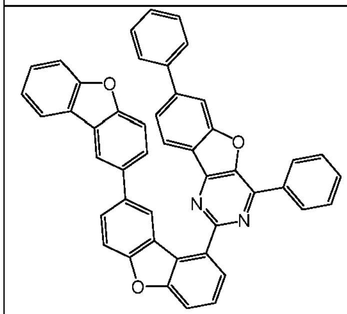 | 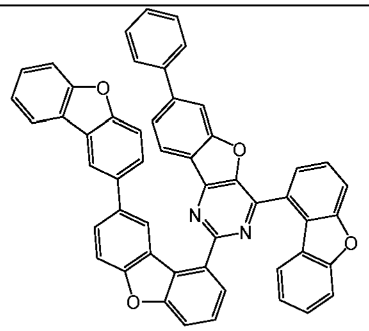 | 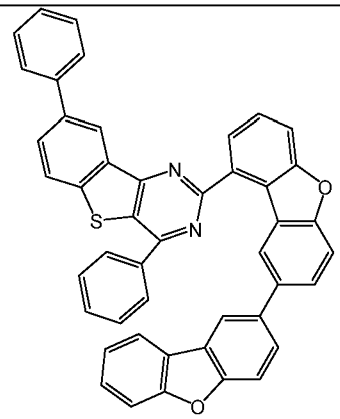 |
| 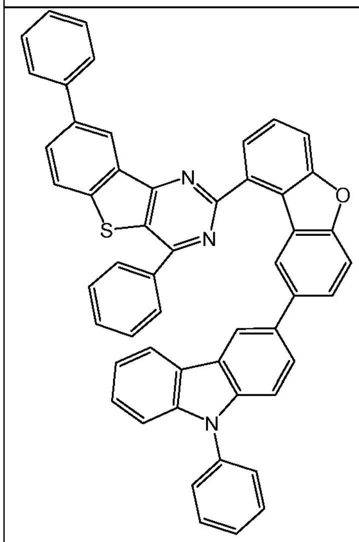 | 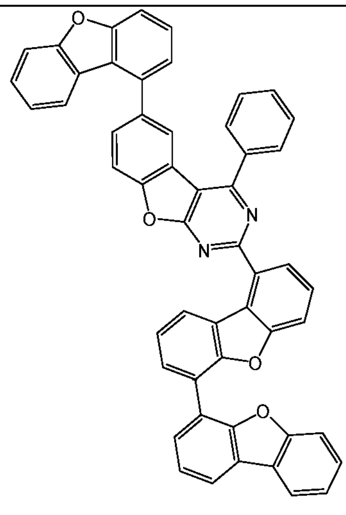 | 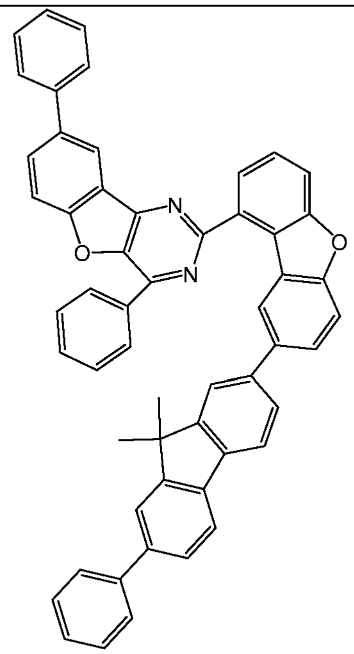 |

| | | |
|---|---|---|
| 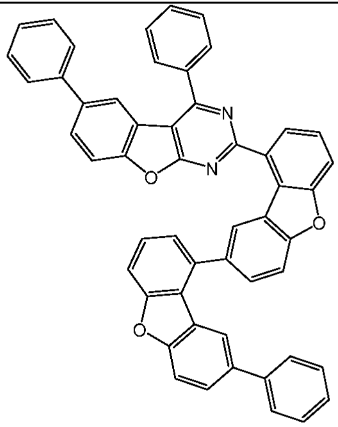 | 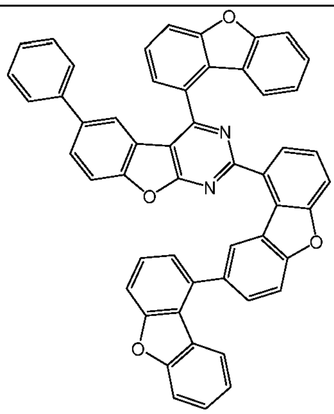 | 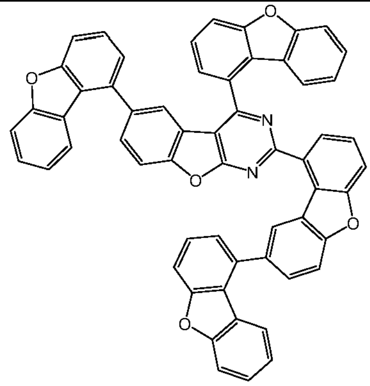 |
| 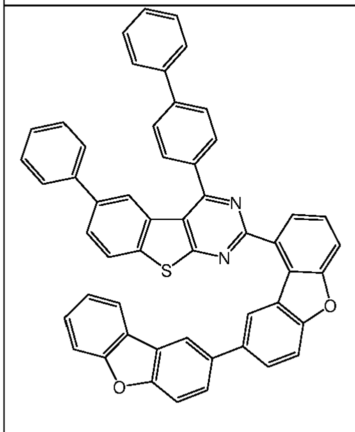 | 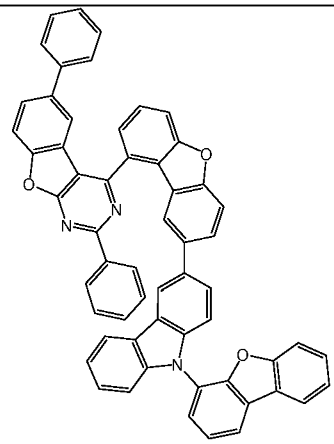 | 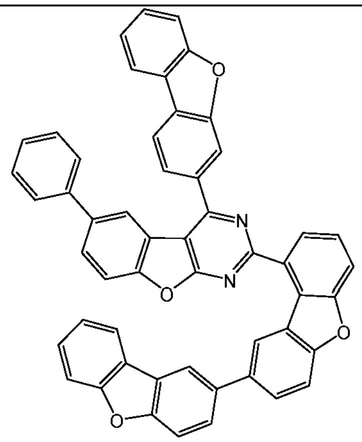 |
| 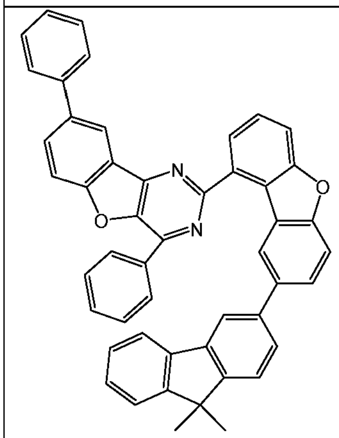 | 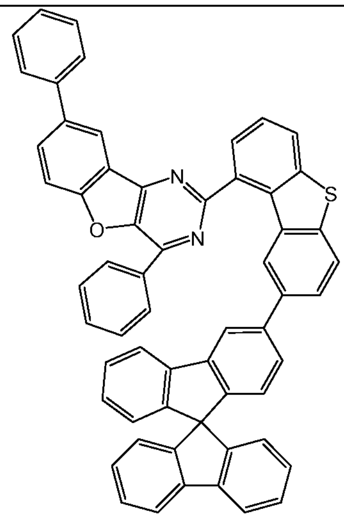 | 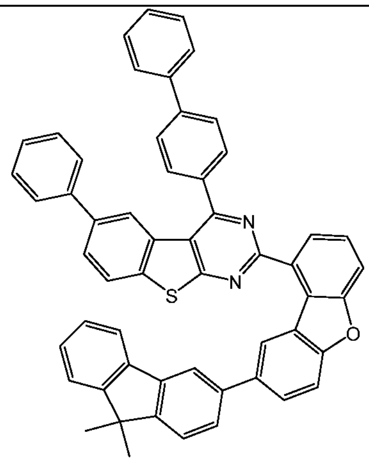 |

| | | |
|---|---|---|
| 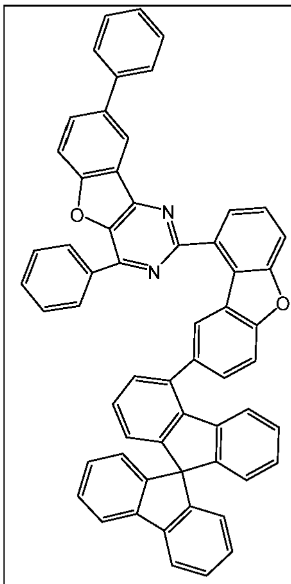 | 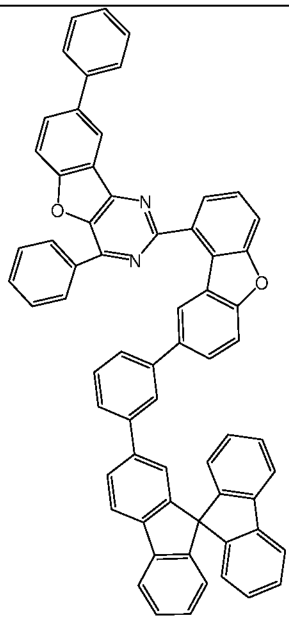 | 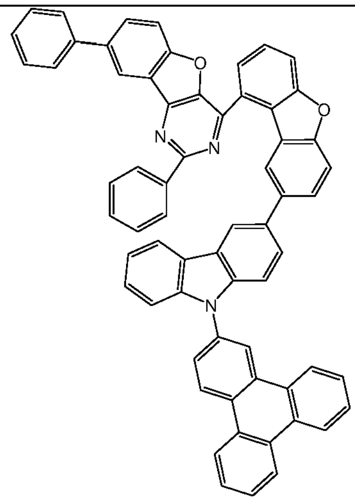 |
| 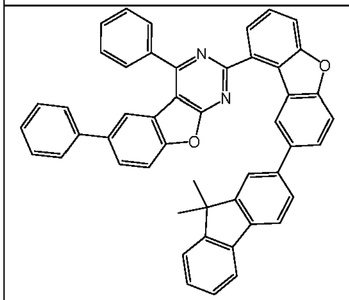 | 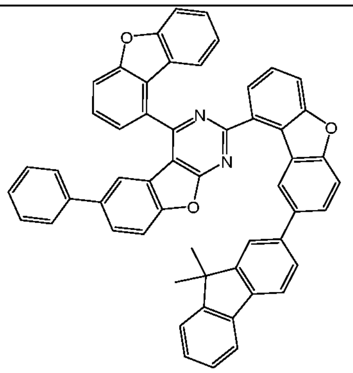 | 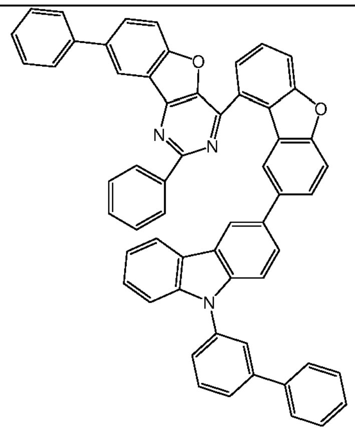 |
| 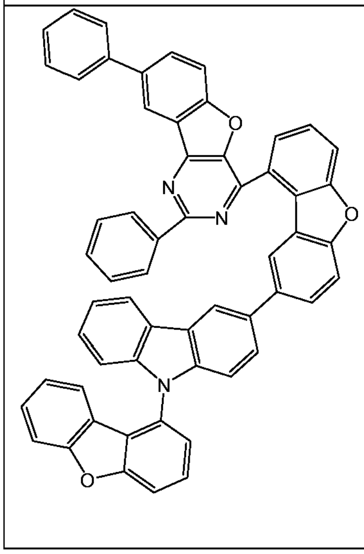 | 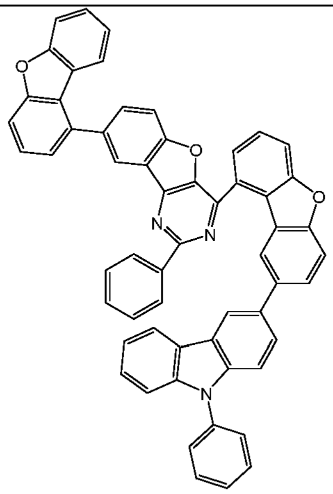 | 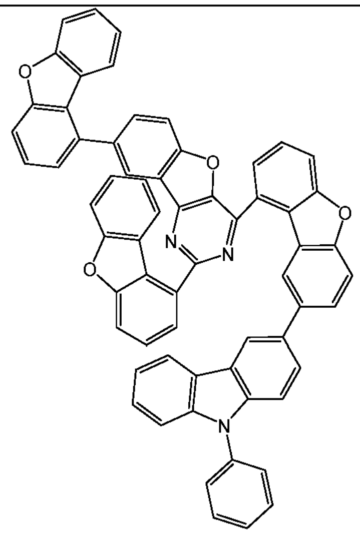 |

| | | |
|---|---|---|
| 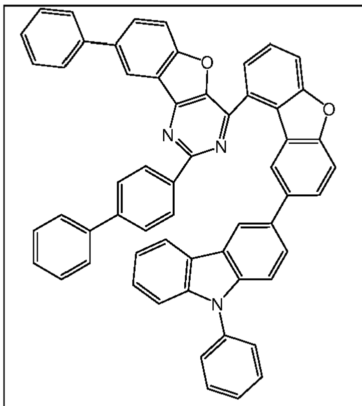 | 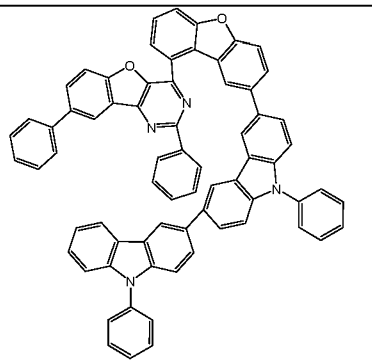 | 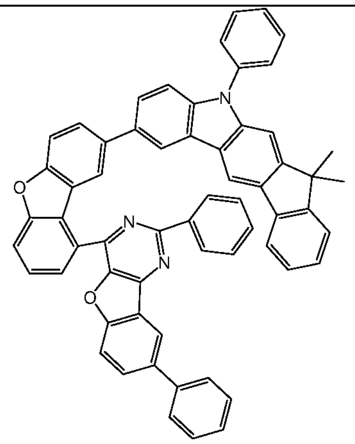 |
| 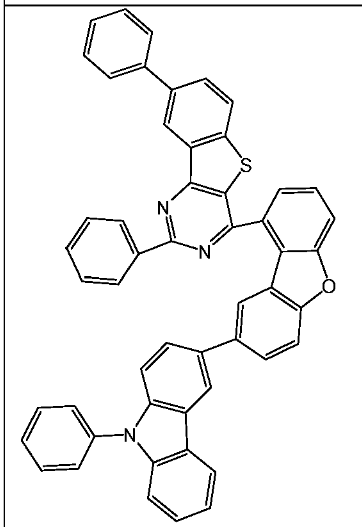 | 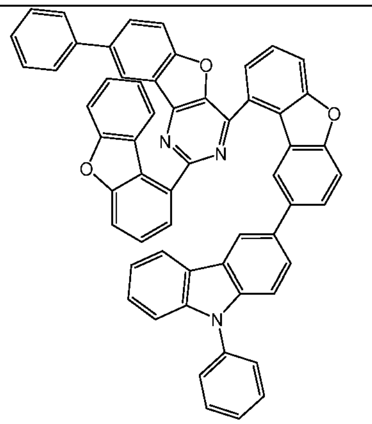 | 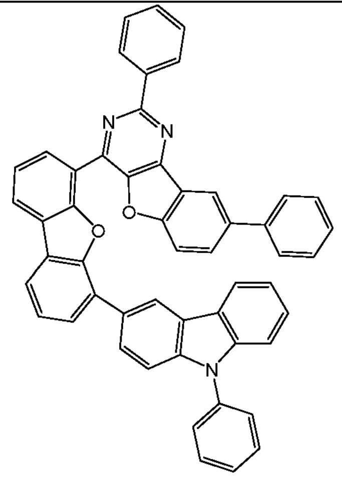 |
| 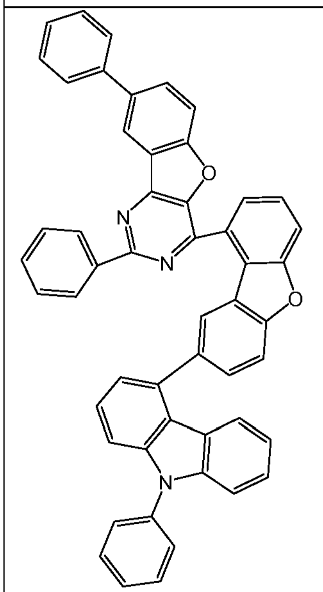 | 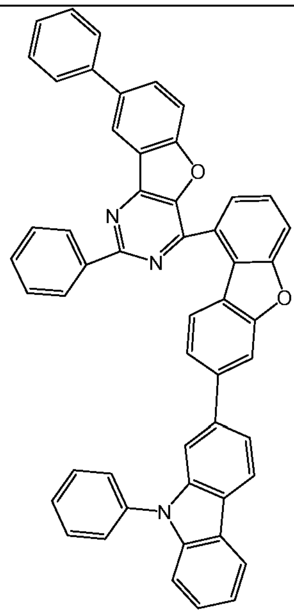 | 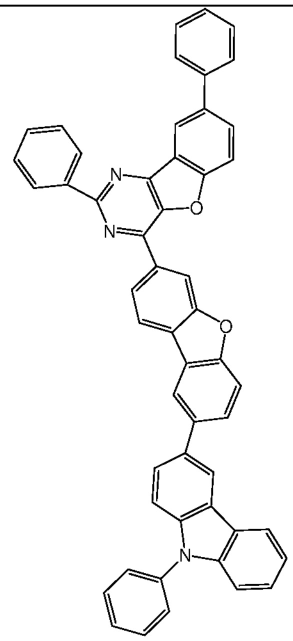 |

| | | |
|---|---|---|
| 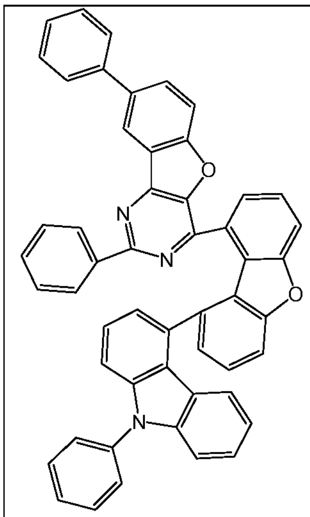 | 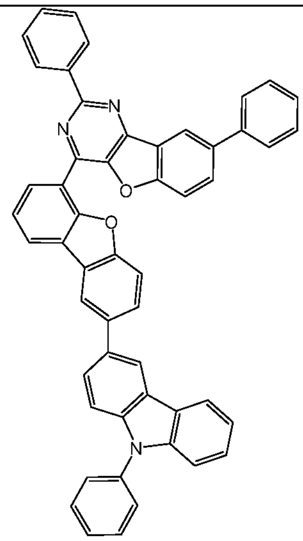 | 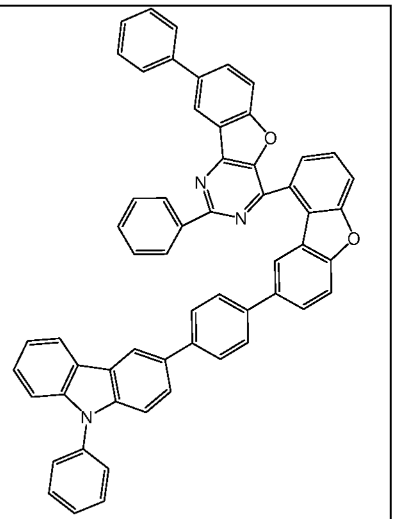 |
| 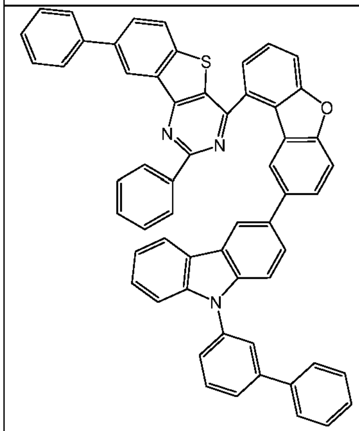 | 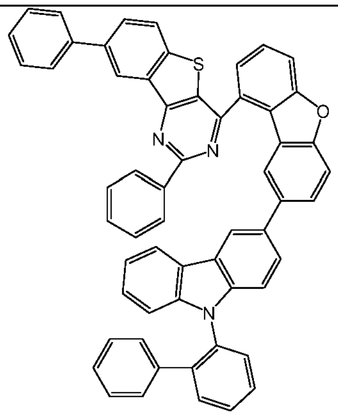 | 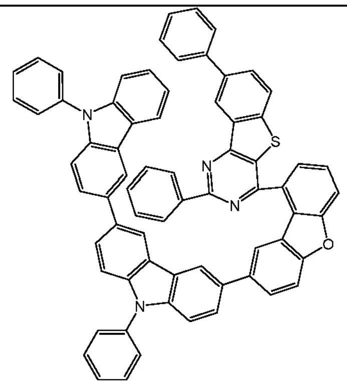 |
| 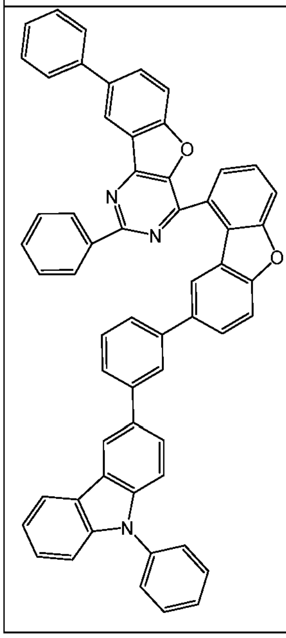 | 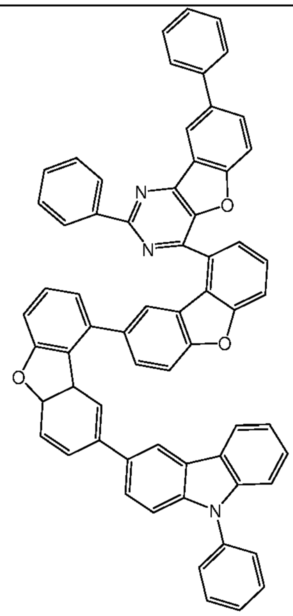 | 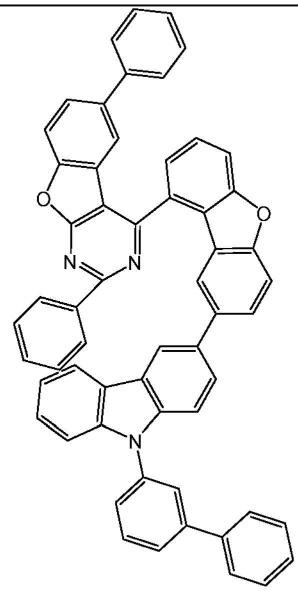 |

| | | |
|---|---|---|
| 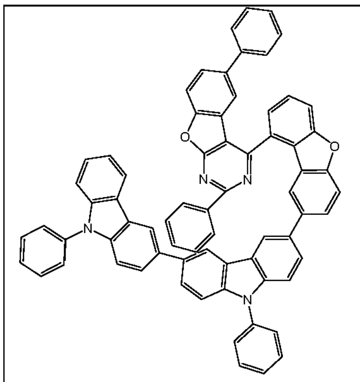 | 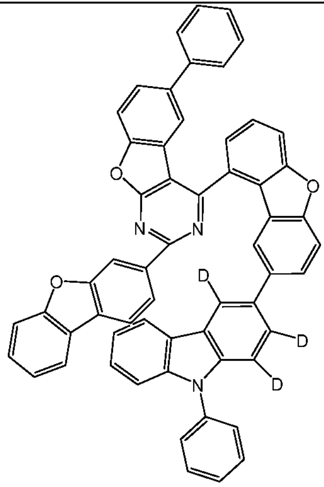 | 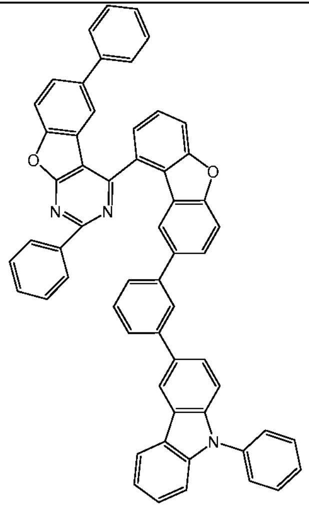 |
| 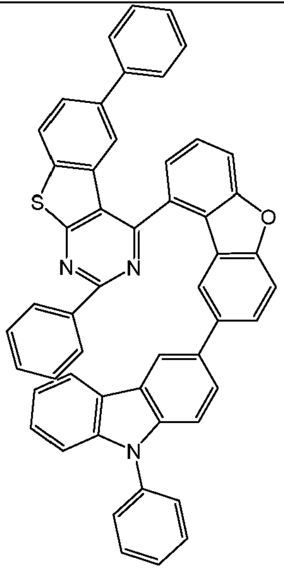 | 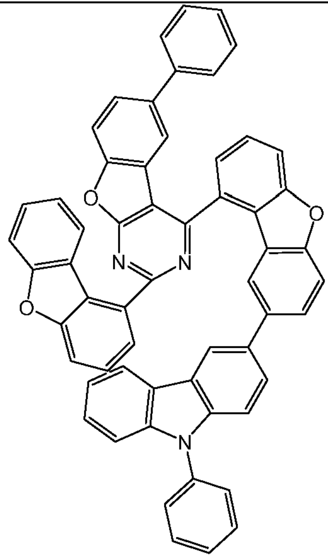 | 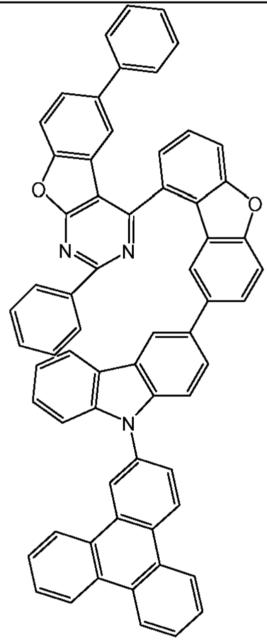 |
| 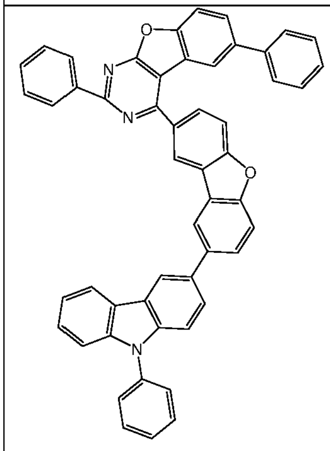 | 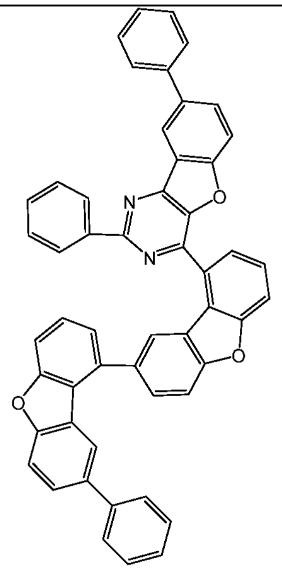 | 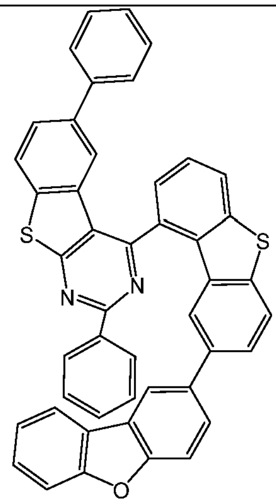 |

| | | |
|---|---|---|
| 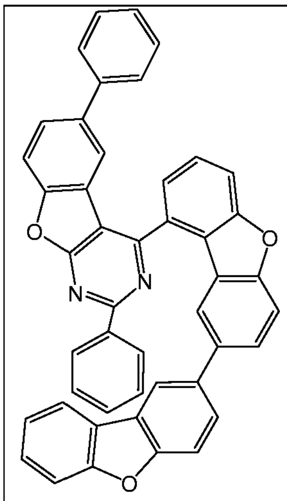 | 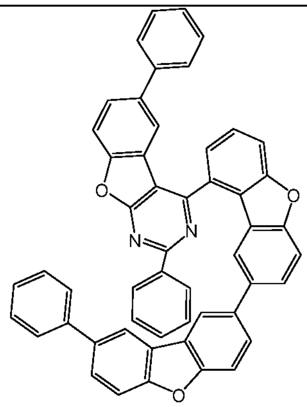 | 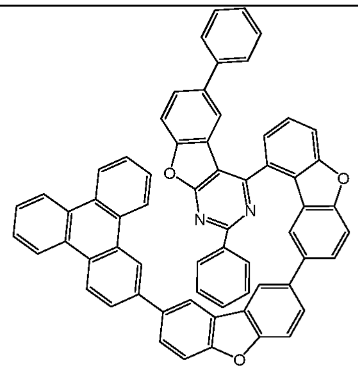 |
| 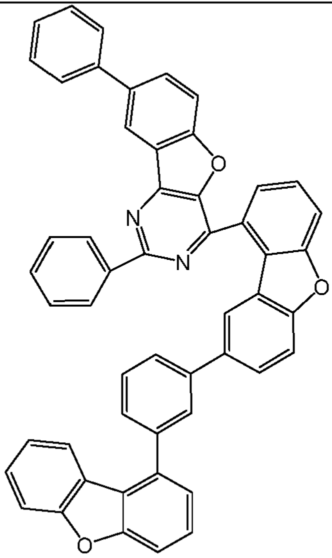 | 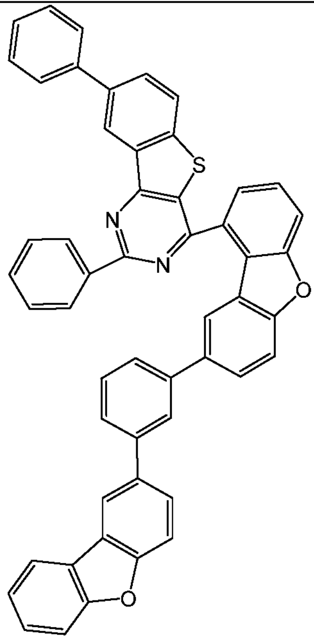 | 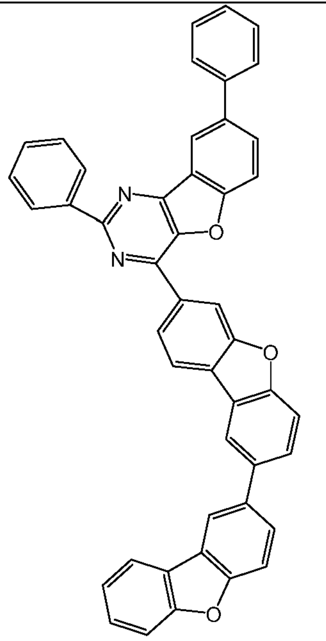 |
| 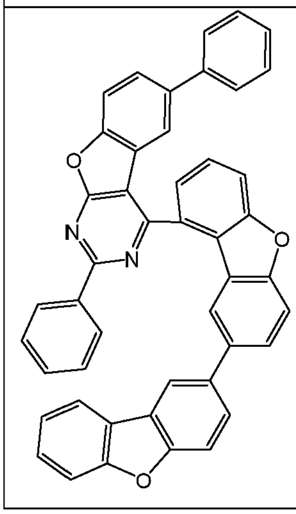 | 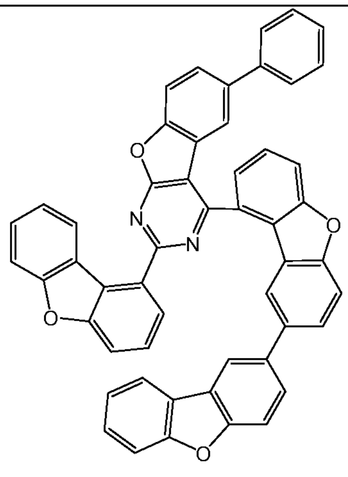 | 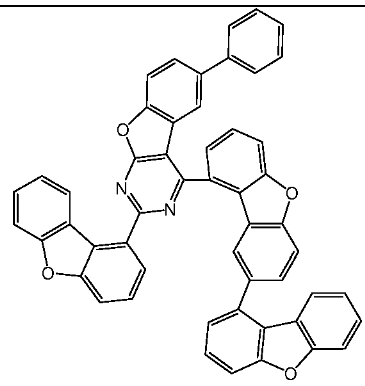 |

| | | |
|---|---|---|
| 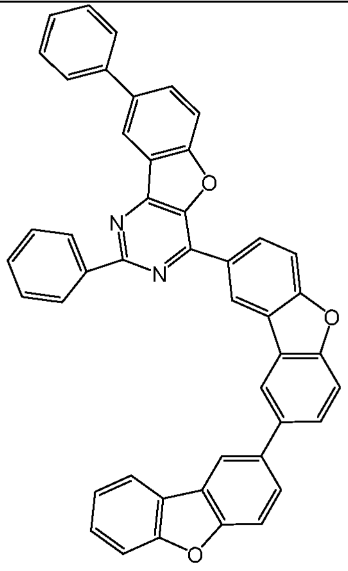 | 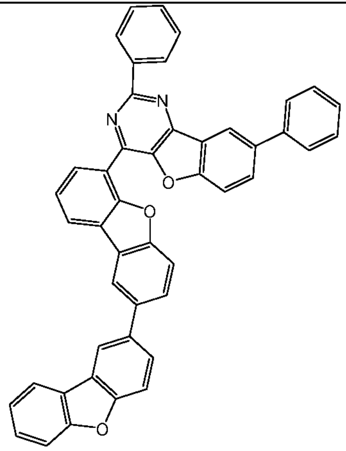 | 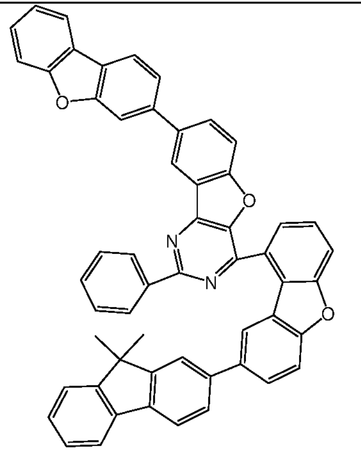 |
| 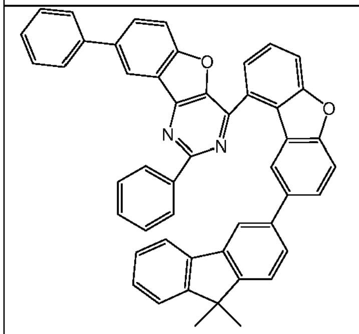 | 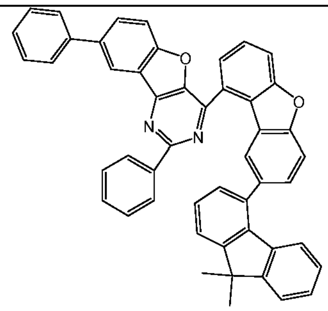 | 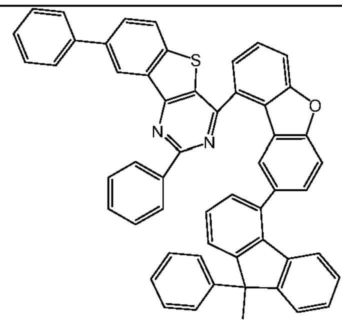 |
| 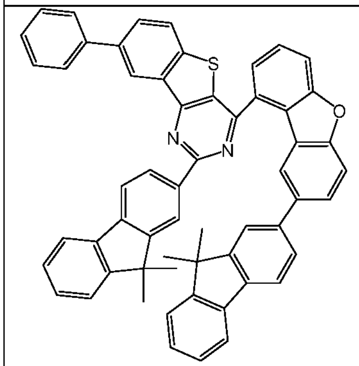 | 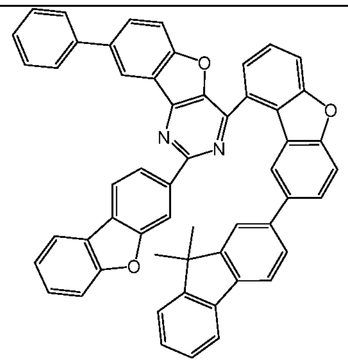 | 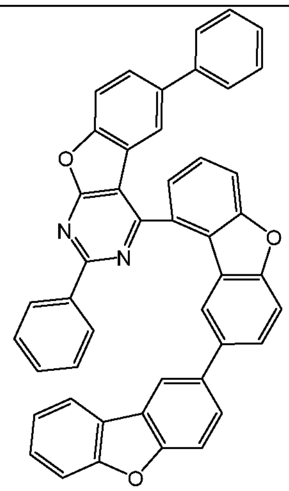 |

| | | |
|---|---|---|
| 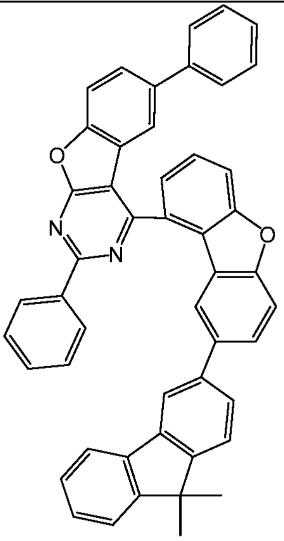 | 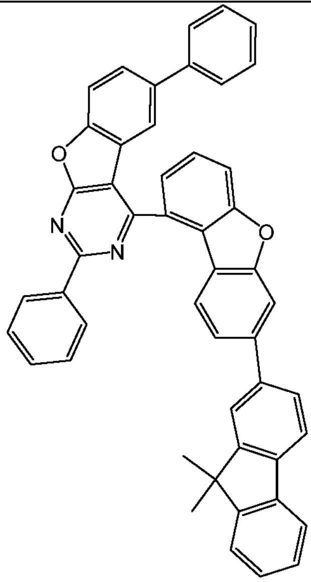 | 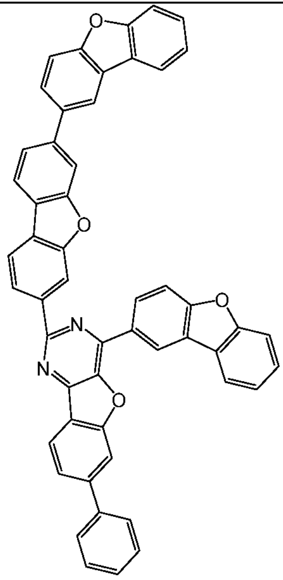 |
| 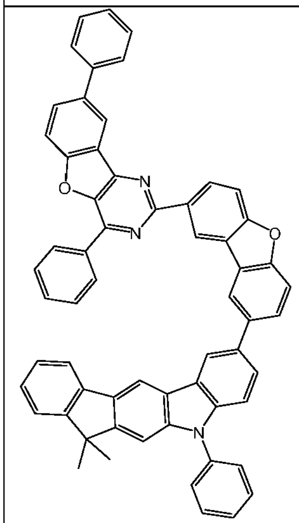 | 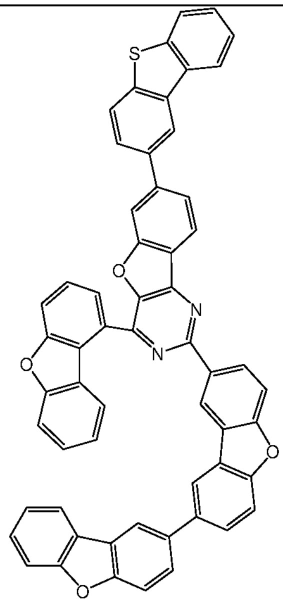 | 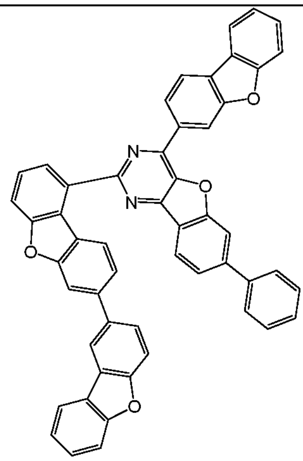 |
| 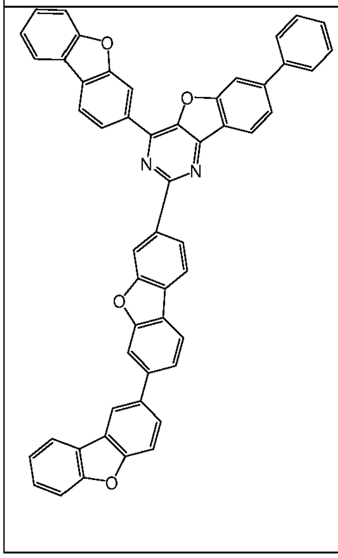 | 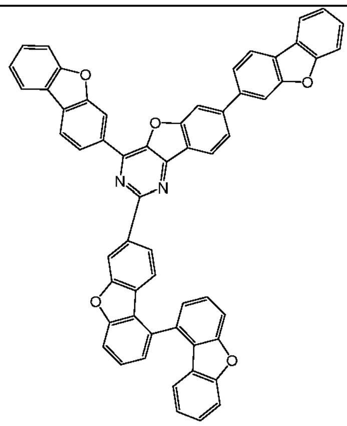 | 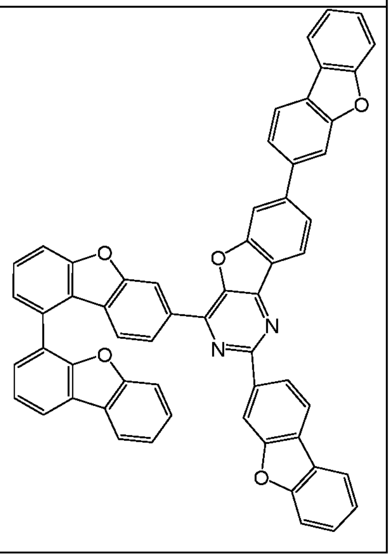 |

| | | |
|---|---|---|
| 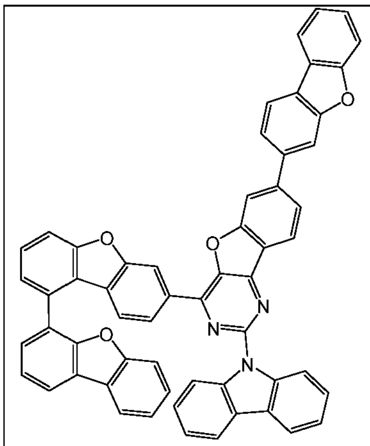 | 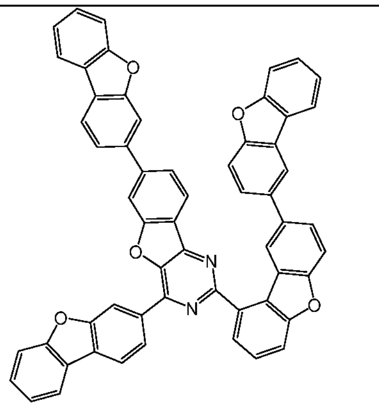 | 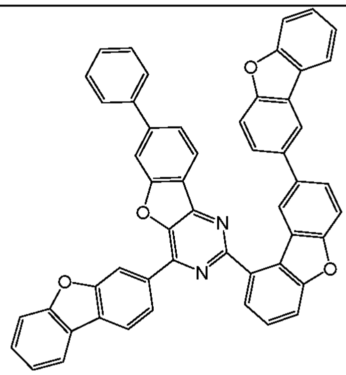 |
| 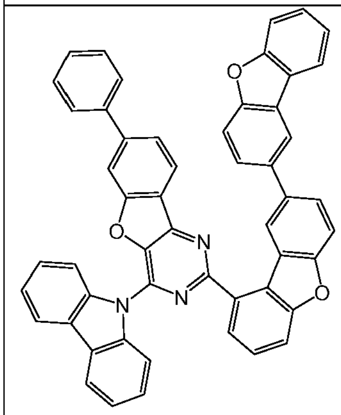 | 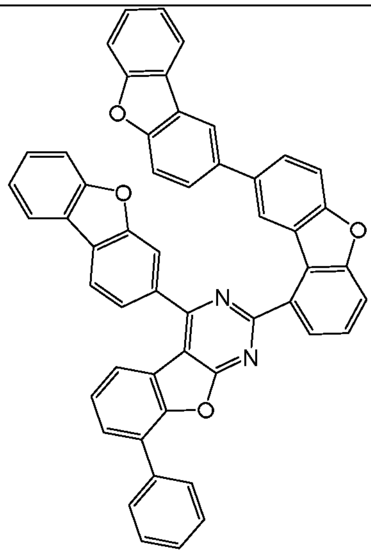 | 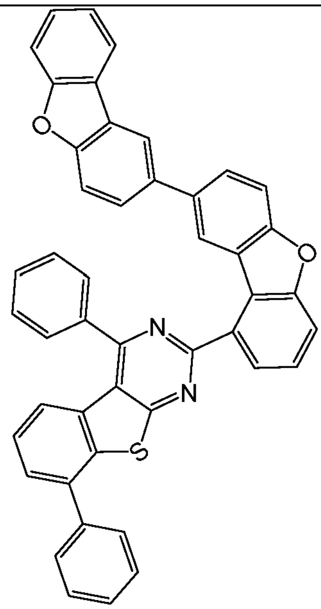 |

Weitere Beispiele von Verbindungen der Formel (1) sind im Ausführungsteil beschrieben.

Besonders geeignete Verbindungen der Formel (1), die bevorzugt in Kombination mit mindestens einer Verbindung der Formel (2) in der erfindungsgemäßen elektrolumineszierenden Vorrichtung verwendet werden, sind die Verbindungen E1 bis E27:

| | | |
|---|---|---|
| 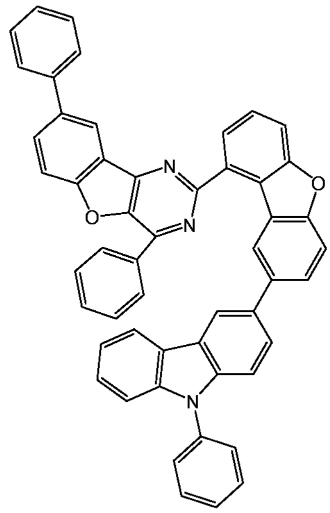 | 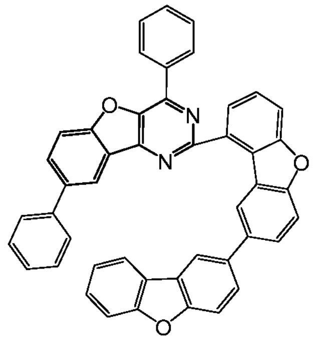 | 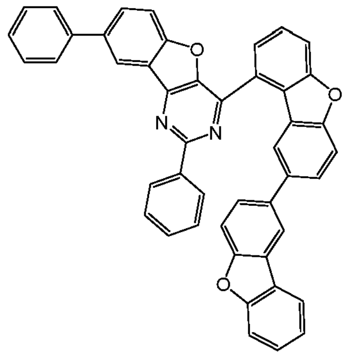 |
| E1 | E2 | E3 |
| 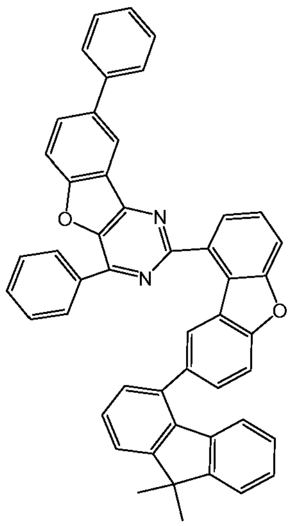 | 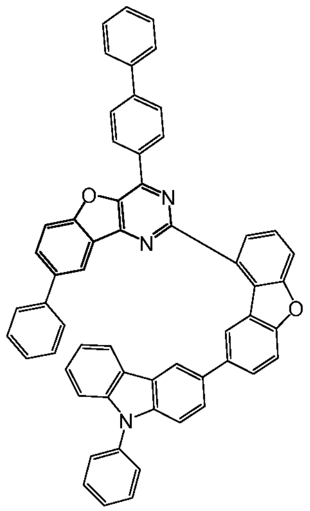 | 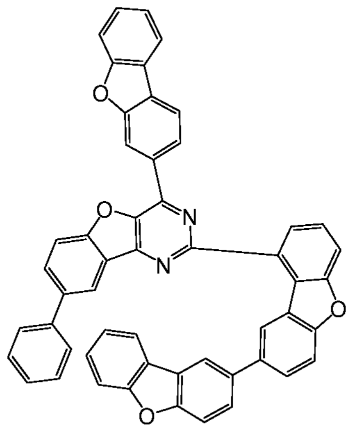 |
| E4 | E5 | E6 |
| 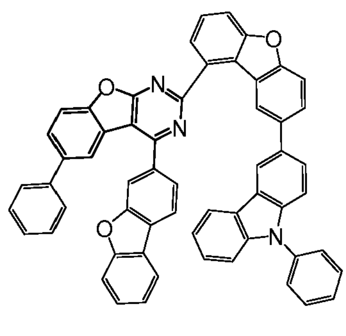 | 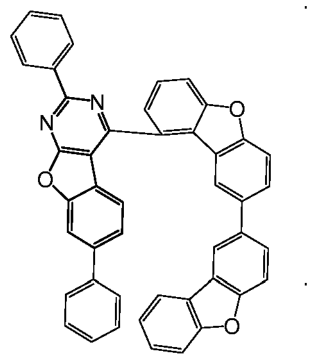 | 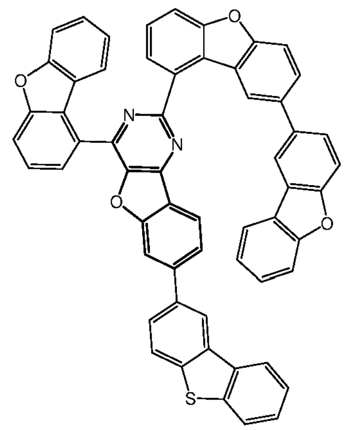 |
| E7 | E8 | E9 |

(fortgesetzt)

| | | |
|---|---|---|
| 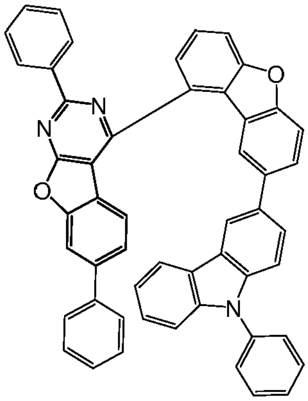 | 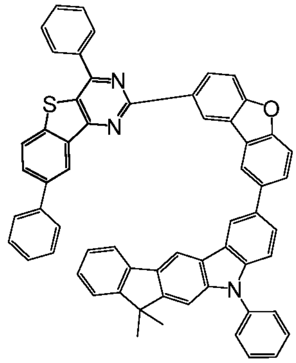 | 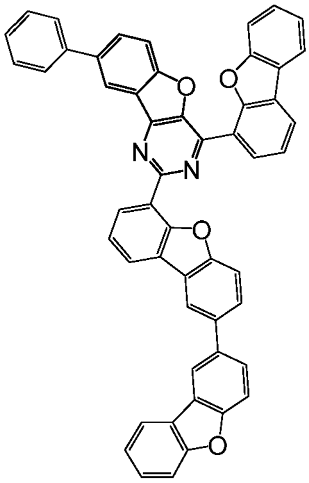 |
| E10 | E11 | E12 |
| 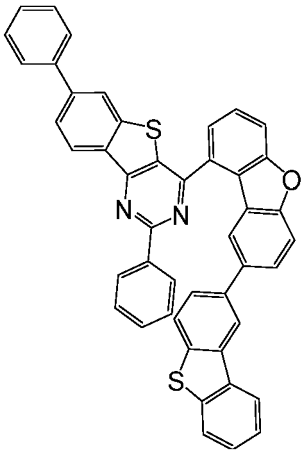 | 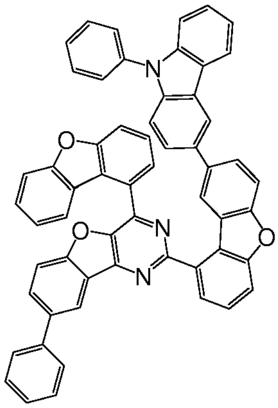 | 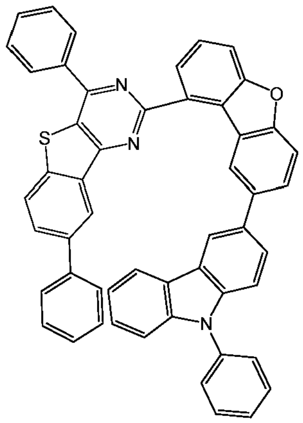 |
| E13 | E14 | E15 |
| 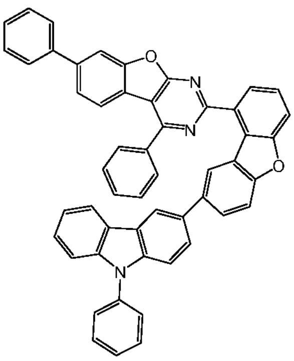 | 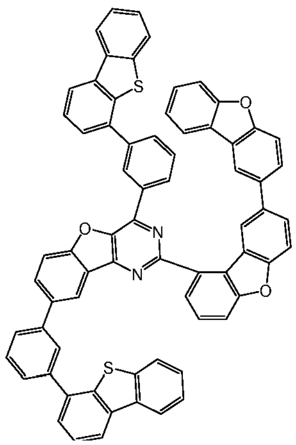 | 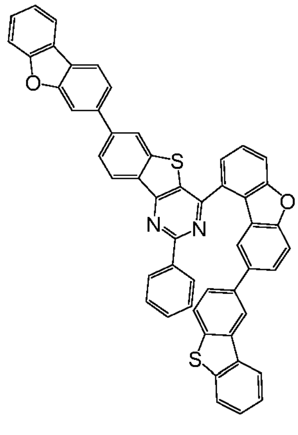 |
| E16 | E17 | E18 |

(fortgesetzt)

| | | |
|---|---|---|
| 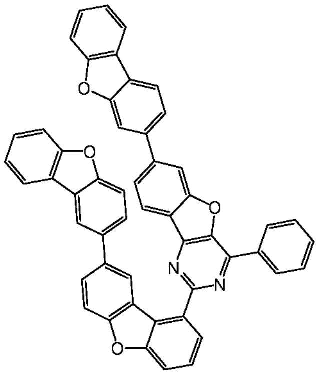 | 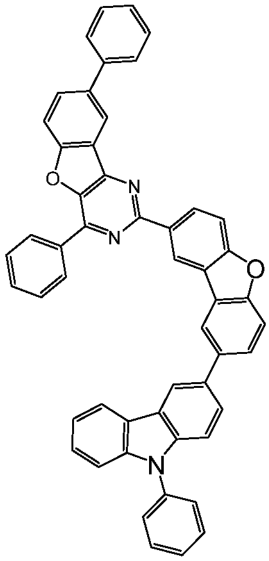 | 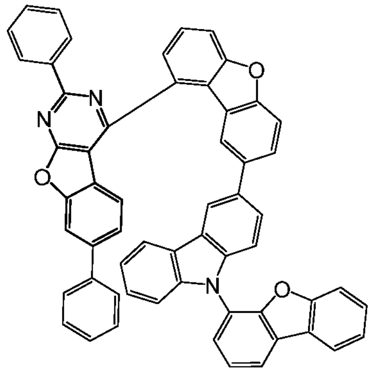 |
| E19 | E20 | E21 |
| 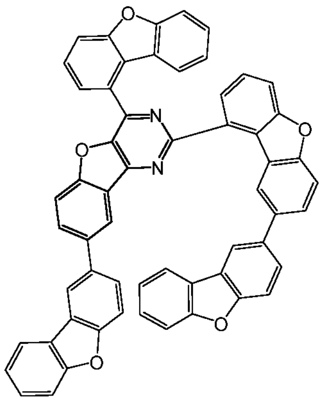 | 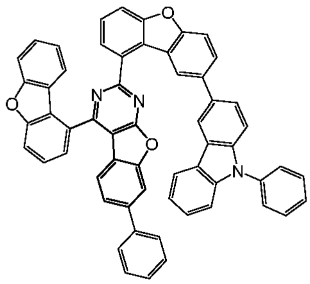 | 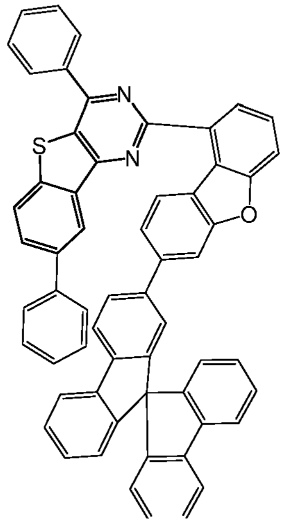 |
| E22 | E23 | E24 |
| 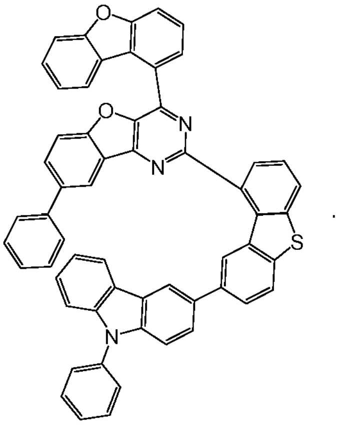 | 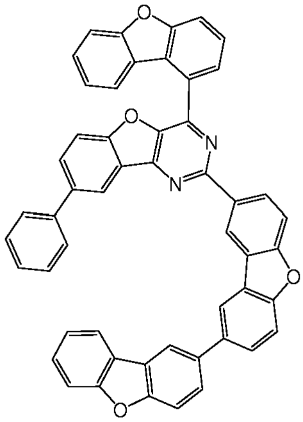 | 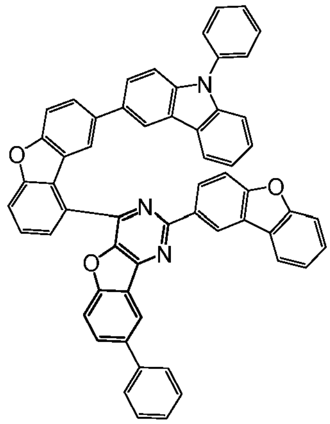 |
| E25 | E26 | E27. |

Die Herstellung der Verbindungen der Formel (1) oder der bevorzugten Verbindungen der Tabelle 1 sowie der Verbindungen E1 bis E27 ist dem Fachmann bekannt. Die Verbindungen können nach dem Fachmann bekannten Syntheseschritten, wie z.B.

Halogenierung, bevorzugt Bromierung, und einer sich anschließenden metallorganischen Kupplungsreaktion, z.B. Suzuki-Kupplung, Heck-Kupplung oder Hartwig-Buchwald-Kupplung, hergestellt werden.

Die Herstellung von Vorstufen für Verbindungen der Formel (1) kann beispielsweise nach folgendem Schema 1 erfolgen, wobei die Symbole und Indizes eine der zuvor genannten Bedeutungen haben und L eine Bindung bedeutet, m und R# eine der zuvor angegebenen oder bevorzugt angegebenen Bedeutungen haben.

Schema 1:

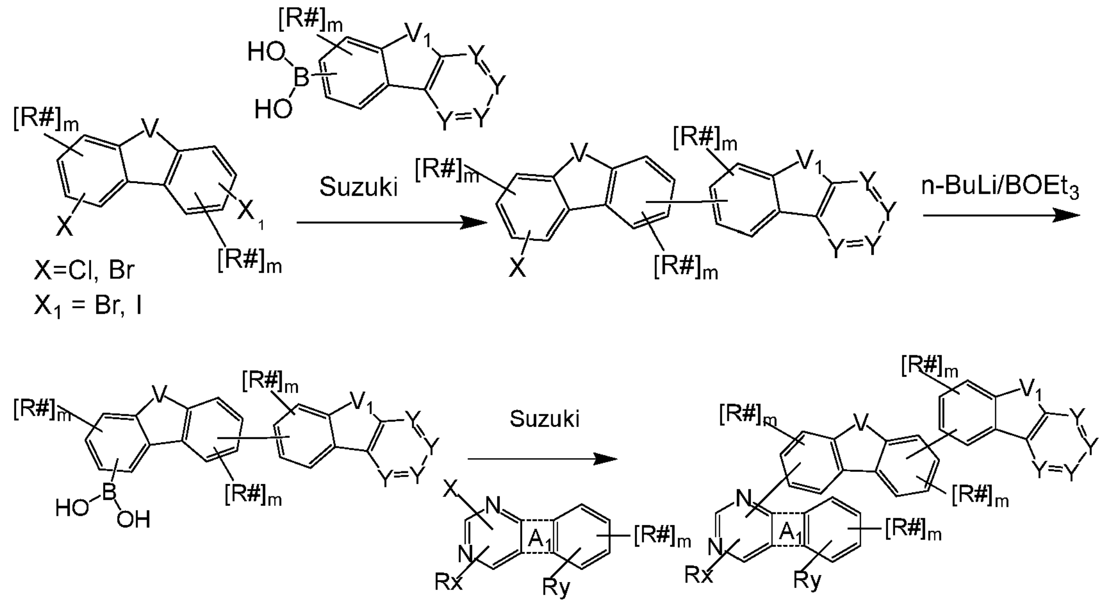


Die Anpassung der Herstellung des Hostmaterials 1 enthaltend einen Linker L ist für den Fachmann hinlänglich bekannt und im Ausführungsteil beschrieben. Die Bedingungen der Herstellung, wie im Ausführungsteil beschrieben, gilt auch für die Synthese des Hostmaterials 1 gemäß Schema 1.

Im Folgenden wird das Hostmaterial 2 und dessen bevorzugte Ausführungsformen beschrieben, welches/welche in der erfindungsgemäßen Vorrichtung enthalten ist. Die bevorzugten Ausführungsformen des Hostmaterials 1 der Formel (1) gelten auch für die erfindungsgemäße Mischung und/oder Formulierung.

Hostmaterial 2 ist mindestens eine Verbindung der Formel (2),

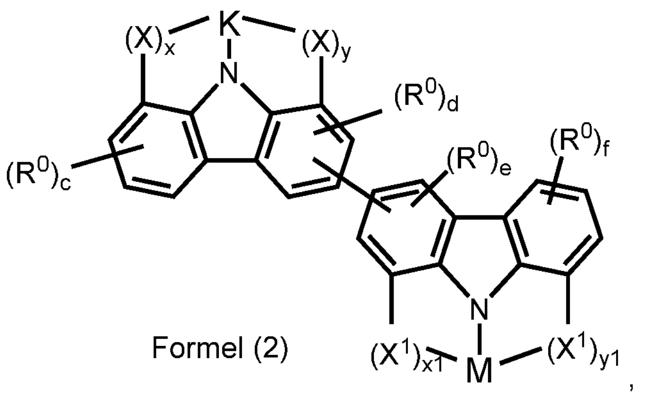
Formel (2)

,

wobei für die verwendeten Symbole und Indizes gilt:

K, M sind jeweils unabhängig voneinander ein unsubstituiertes oder teilweise oder vollständig deuteriertes oder einfach durch R* substituiertes aromatisches Ringsystem mit 6 bis 40 Ringatomen, wenn x und y 0 bedeuten und wenn x1 und y1 0 bedeuten, oder

K, M bilden jeweils unabhängig voneinander zusammen mit X oder $X^1$ ein heteroaromatisches Ringsystem mit 14 bis 40 Ringatomen, sobald der Wert von x, x1, y und/oder y1 1 bedeutet;

x, x1 sind jeweils unabhängig bei jedem Auftreten 0 oder 1;

y, y1 sind jeweils unabhängig bei jedem Auftreten 0 oder 1;

X und $X^1$ sind jeweils unabhängig voneinander bei jedem Auftreten eine Bindung oder $C(R^+)_2$;

$R^0$ ist bei jedem Auftreten unabhängig voneinander ein unsubstituiertes oder teilweise oder vollständig deuteriertes aromatisches Ringsystem mit 6 bis 18 C-Atomen;

$R^+$ ist bei jedem Auftreten unabhängig voneinander eine geradkettige oder verzweigte Alkylgruppe mit 1 bis 4 C-Atomen und

c, d, e und f sind unabhängig voneinander 0 oder 1.

In einer Ausführungsform der Erfindung werden für die erfindungsgemäße Vorrichtung Verbindungen der Formel (2) ausgewählt, wie zuvor beschrieben, die mit Verbindungen der Formel (1), wie zuvor beschrieben oder bevorzugt beschrieben, oder mit den Verbindungen der Tabelle 1 oder den Verbindungen E1 bis E27, in der lichtemittierenden Schicht verwendet werden.

In einer bevorzugten Ausführungsform der erfindungsgemäßen Vorrichtung werden Verbindungen der Formel (2) als Hostmaterial 2 verwendet, in denen x, y, x1 und y1 0 bedeuten. Verbindungen der Formel (2), in denen x, x1, y und y1 bei jedem Auftreten 0 bedeuten, können durch die folgende Formel (2a) dargestellt werden,

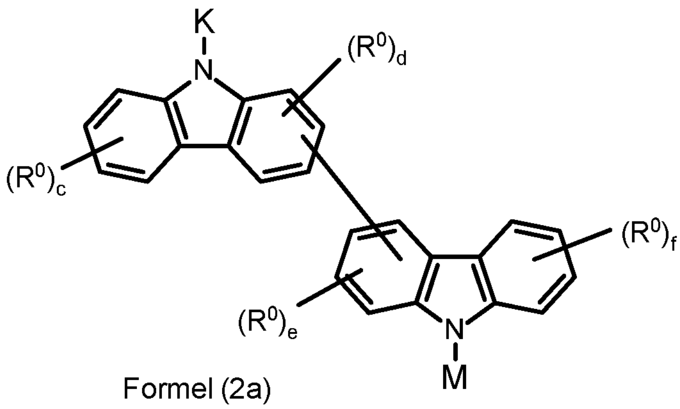

Formel (2a) ,

wobei $R^0$, c, d, e und f eine zuvor genannte oder nachfolgend genannte Bedeutung haben und

K und M jeweils unabhängig voneinander ein unsubstituiertes oder teilweise oder vollständig deuteriertes oder einfach durch R* substituiertes aromatisches Ringsystem mit 6 bis 40 Ringatomen bedeuten.

In bevorzugten Verbindungen der Formel (2a) ist die Summe der Indizes c+d+e+f bevorzugt 0 oder 1 und $R^0$ hat eine zuvor oder nachfolgend bevorzugt angegebene Bedeutung.

In Verbindungen der Formel (2) oder (2a) ist $R^0$ ist bei jedem Auftreten unabhängig voneinander bevorzugt ein unsubstituiertes aromatisches Ringsystem mit 6 bis 18 C-Atomen. $R^0$ ist bei jedem Auftreten unabhängig voneinander bevorzugt Phenyl, 1,3-Biphenyl, 1,4-Biphenyl, Naphthyl oder Triphenylenyl. $R^0$ ist bei jedem Auftreten unabhängig voneinander besonders bevorzugt Phenyl.

In Verbindungen der Formel (2) oder (2a) sind die Indizes c, d, e und f besonders bevorzugt 0.

In Verbindungen der Formel (2) oder (2a), sind K und M bei jedem Auftreten unabhängig voneinander bevorzugt ein unsubstituiertes oder teilweise deuteriertes aromatisches Ringsystem mit 6 bis 40 Ringatomen, wie zuvor beschrieben. K und M in Verbindungen der Formel (2) oder (2a) sind bei jedem Auftreten unabhängig voneinander besonders bevorzugt Phenyl, deuteriertes Phenyl, 1,3-Biphenyl, 1,4-Biphenyl, Terphenyl, teilweise deuteriertes Terphenyl, Quaterphenyl, Naphthyl, Fluorenyl, 9,9-Diphenyl-fluorenyl, Bispirofluorenyl oder Triphenylenyl.

Ein weiterer Gegenstand der Erfindung ist demzufolge eine organische elektrolumineszierende Vorrichtung, wie zuvor beschrieben oder bevorzugt beschrieben, wobei die mindestens eine Verbindung der Formel (2) einer Verbindung der Formel (2a) oder einer bevorzugten Ausführungsform der Verbindung der Formel (2a) entspricht.

In einer bevorzugten Ausführungsform der erfindungsgemäßen Vorrichtung werden Verbindungen der Formel (2) als Hostmaterial 2 verwendet, in denen x1 und y1 0 bedeuten, x und y 0 oder 1 bedeuten und die Summe von x und y 1 oder 2 bedeutet. Verbindungen der Formel (2), in denen x1 und y1 0 bedeuten, x und y 0 oder 1 bedeuten und die Summe von x und y 1 oder 2 bedeutet, können durch die folgende Formel (2b) dargestellt werden,

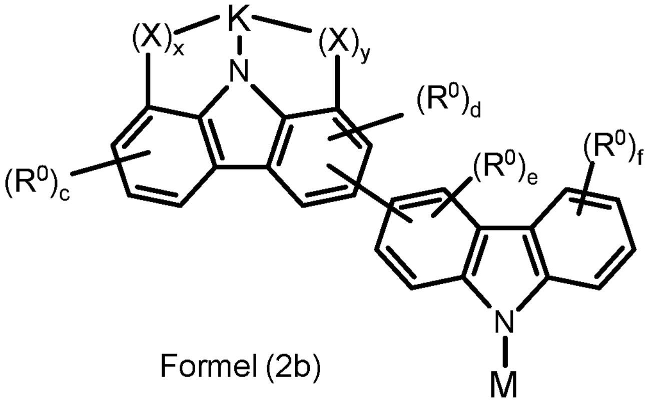

Formel (2b)

,

wobei X, x, y, $R^0$, c, d, e und f eine zuvor genannte oder nachfolgend genannte Bedeutung haben,
M ein unsubstituiertes oder teilweise oder vollständig deuteriertes oder einfach durch R* substituiertes aromatisches Ringsystem mit 6 bis 40 Ringatomen ist und
K zusammen mit X ein heteroaromatisches Ringsystem mit 14 bis 40 Ringatomen bildet, sobald der Wert von x oder y 1 bedeutet oder beide Werte x und y 1 bedeuten.

In bevorzugten Verbindungen der Formel (2b) ist die Summe der Indizes c+d+e+f bevorzugt 0, 1 oder 2 und $R^0$ hat eine zuvor angegebene oder bevorzugt angegebene Bedeutung.

In Verbindungen der Formel (2) oder (2b) sind die Indizes c, d, e und f besonders bevorzugt 0 oder 1. In Verbindungen der Formel (2) oder (2b) sind die Indizes c, d, e und f ganz besonders bevorzugt 0. In Verbindungen der Formel (2) oder (2b) sind die Indizes c, d, e und f ganz besonders bevorzugt 1. In Verbindungen der Formel (2) oder (2b) sind die Indizes c, d, e und f ganz besonders bevorzugt 2.

In Verbindungen der Formel (2) oder (2b) bildet K bevorzugt ein heteroaromatisches Ringsystem, wenn die Summe von x+y 1 oder 2 bedeutet. X ist in Verbindungen der Formel (2) oder (2b) bevorzugt eine direkte Bindung oder $C(CH_3)_2$.

Bevorzugte Verbindungen der Formel (2) oder (2b) können durch die Formeln (2b-1) bis (2b-6) dargestellt werden,

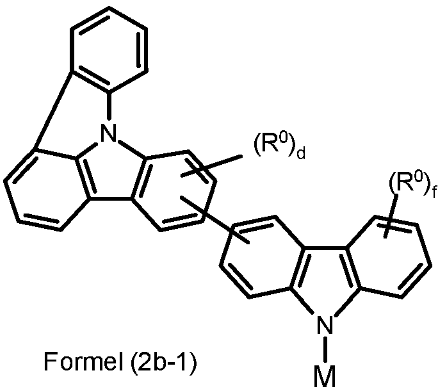

Formel (2b-1)

,

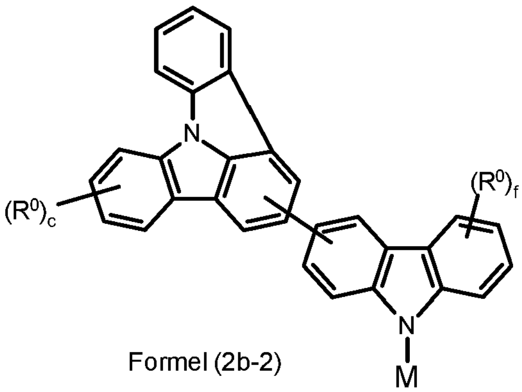

Formel (2b-2)

,

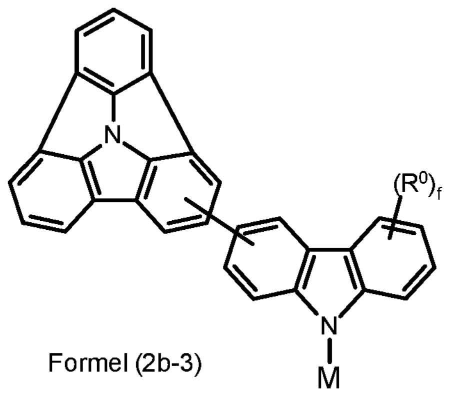

Formel (2b-3) ,

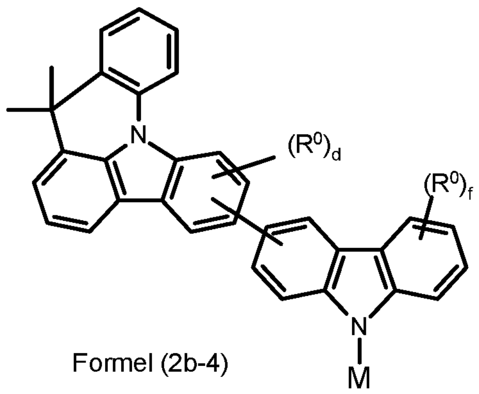

Formel (2b-4) ,

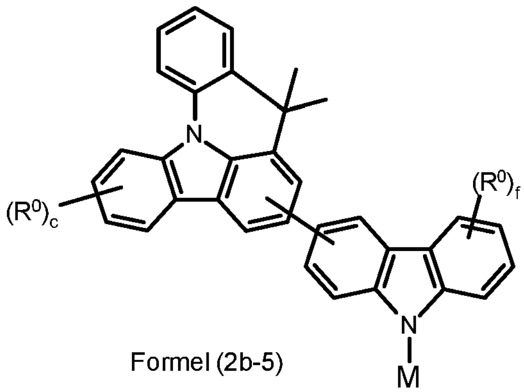

Formel (2b-5) ,

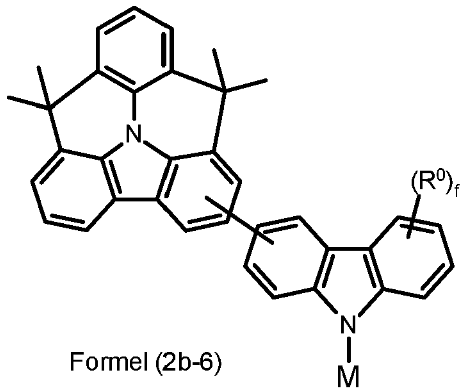

Formel (2b-6) ,

wobei M, $R^0$, c, d, e und f eine zuvor genannte oder bevorzugt genannte Bedeutung haben.

In Verbindungen der Formeln (2), (2b), (2b-1), (2b-2), (2b-3), (2b-4, (2b-5) oder (2b-6) ist M bevorzugt ein unsubstituiertes oder teilweise deuteriertes aromatisches Ringsystem mit 6 bis 40 Ringatomen, wie zuvor beschrieben. M in Verbindungen der Formeln (2), (2b), (2b-1), (2b-2), (2b-3), (2b-4, (2b-5) oder (2b-6) ist besonders bevorzugt Phenyl, deuteriertes Phenyl, 1,3-Biphenyl, 1,4-Biphenyl, Terphenyl, teilweise deuteriertes Terphenyl, Quaterphenyl, Naphthyl, Fluorenyl, 9,9-Diphenyl-fluorenyl, Bispirofluorenyl oder Triphenylenyl.

In Verbindungen der Formeln (2b-1), (2b-2), (2b-3), (2b-4, (2b-5) oder (2b-6) ist c, d, e und f bevorzugt 0 oder 1.

Ein weiterer Gegenstand der Erfindung ist demzufolge eine organische elektrolumineszierende Vorrichtung, wie zuvor beschrieben oder bevorzugt beschrieben, wobei die mindestens eine Verbindung der Formel (2) einer Verbindung der Formel (2b), (2b), (2b-1), (2b-2), (2b-3), (2b-4, (2b-5) oder (2b-6) oder einer bevorzugten Ausführungsform dieser Verbindungen entspricht.

In einer bevorzugten Ausführungsform der erfindungsgemäßen Vorrichtung werden Verbindungen der Formel (2) als Hostmaterial 2 verwendet, in denen c und f 0 oder 1 bedeuten, d und e 0 bedeuten und x, x1, y und y1 bei jedem Auftreten unabhängig voneinander 0 oder 1 bedeuten, wobei jedoch die Summe von x und y mindestens 1 und die Summe von x1 und y1 mindestens 1 bedeutet. Solche Verbindungen der Formel (2), wie zuvor beschrieben, können bevorzugt durch die folgende Formel (2c) dargestellt werden,

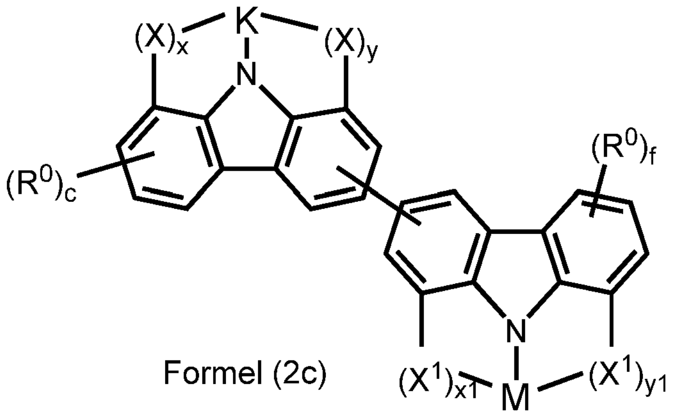

Formel (2c)

,

wobei X und $X^1$ eine zuvor genannte oder nachfolgend genannte Bedeutung haben, K und M jeweils unabhängig voneinander zusammen mit X oder $X^1$ ein heteroaromatisches Ringsystem mit 14 bis 40 Ringatomen bilden,

x, x1, y und/oder y1 0 oder 1 bedeuten und die Summe von x und y mindestens 1 und die Summe von x1 und y1 mindestens 1 bedeutet.

In bevorzugten Verbindungen der Formel (2c) ist die Summe von x und y 1 oder 2 und die Summe von x1 und y1 ist 1. In besonders bevorzugten Verbindungen der Formel (2c) sind die Summe von x und y 1 und die Summe von x1 und y1 jeweils 1.

In Verbindungen der Formel (2) oder (2c) bilden demzufolge K und M bevorzugt ein heteroaromatisches Ringsystem. X und $X^1$ sind in Verbindungen der Formel (2) oder (2c) bevorzugt eine direkte Bindung oder $C(CH_3)_2$.

Bevorzugte Verbindungen der Formel (2) oder (2c) können durch die Formeln (2c-1) bis (2c-8) dargestellt werden,

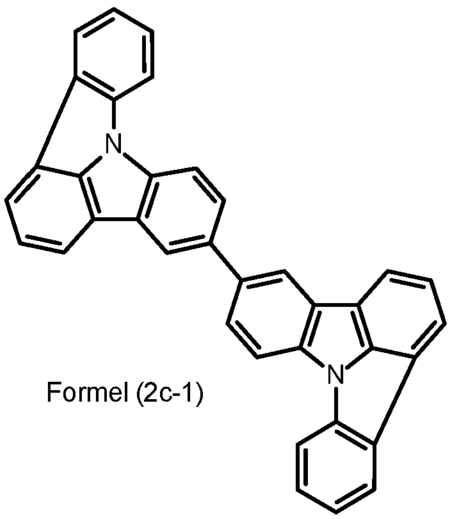

Formel (2c-1)

,

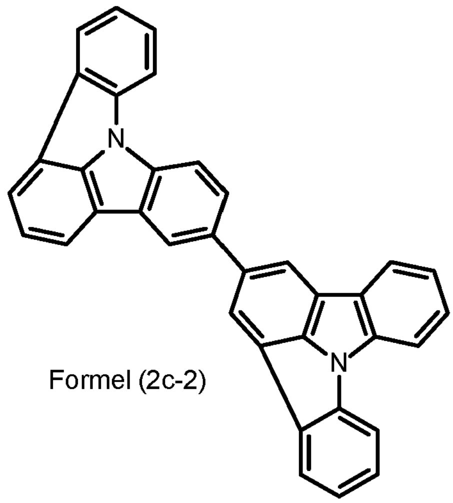

Formel (2c-2)

,

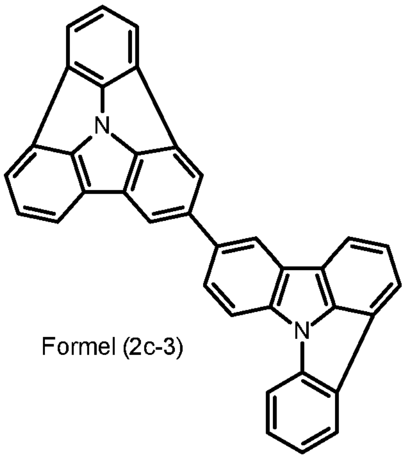

Formel (2c-3)

,

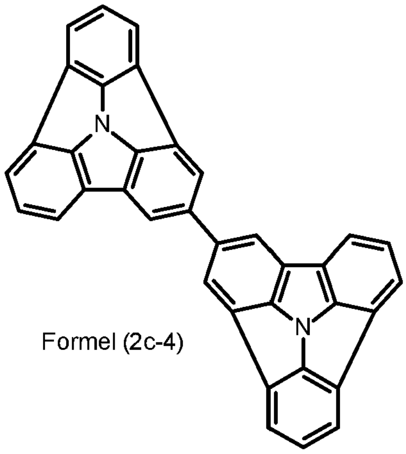

Formel (2c-4)

,

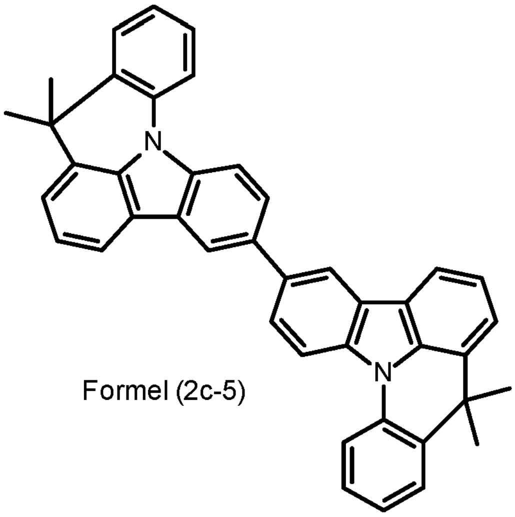

Formel (2c-5)

,

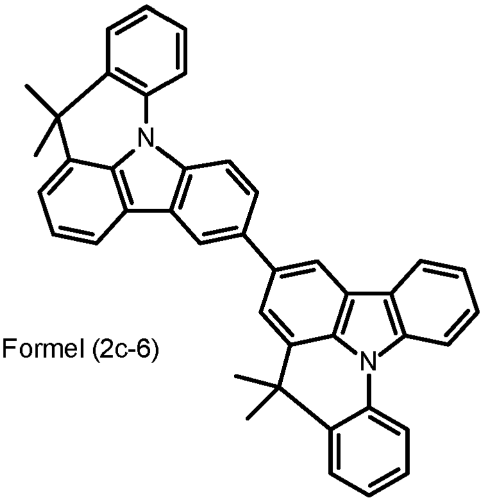

Formel (2c-6)

,

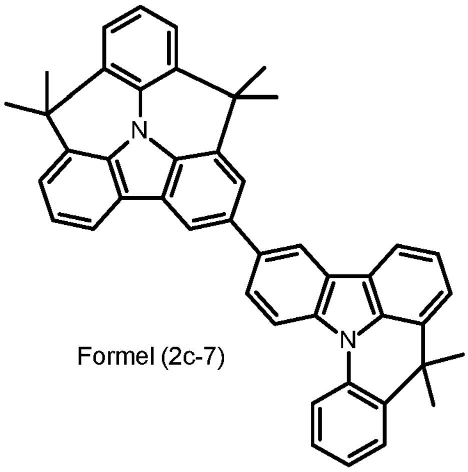

Formel (2c-7)

,

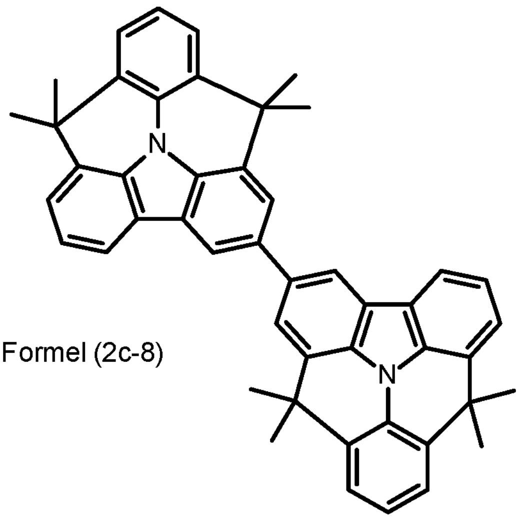

Formel (2c-8)

.

Bevorzugte Verbindungen der Formel (2c) sind auch die Verbindungen H9, H11, H12, H13, H14, H15, H19 und H20, wie nachfolgend beschrieben.

Ein weiterer Gegenstand der Erfindung ist demzufolge eine organische elektrolumineszierende Vorrichtung,

wie zuvor beschrieben oder bevorzugt beschrieben, wobei die mindestens eine Verbindung der Formel (2) einer Verbindung der Formeln (2c), (2c-1), (2c-2), (2c-3), (2c-4, (2c-5), (2c-6), (2c-7) oder (2c-8) entspricht.

In einer bevorzugten Ausführungsform der Verbindungen der Formeln (2), (2a), (2b), (2b-1), (2b-2), (2b-3), (2b-4, (2b-5) oder (2b-6) sind das Carbazol und das verbrückte Carbazol jeweils in 3-Position miteinander verknüpft.

In einer bevorzugten Ausführungsform der Verbindungen der Formel (2c) sind die beiden verbrückten Carbazole jeweils in 3-Position miteinander verknüpft.

Beispiele für geeignete Hostmaterialien der Formeln (2), (2a), (2b), (2b-1), (2b-2), (2b-3), (2b-4, (2b-5) und (2c), die erfindungsgemäß ausgewählt werden, und bevorzugt in Kombination mit mindestens einer Verbindung der Formel (1) in der erfindungsgemäßen elektrolumineszierenden Vorrichtung verwendet werden, sind die nachstehend genannten Strukturen der Tabelle 2.

Tabelle 2:

| | | |
|---|---|---|
| 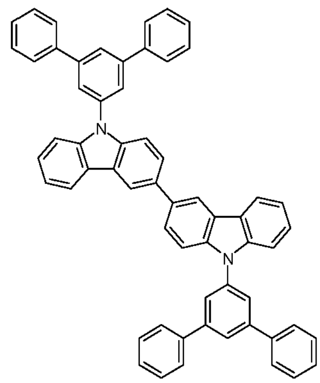 | 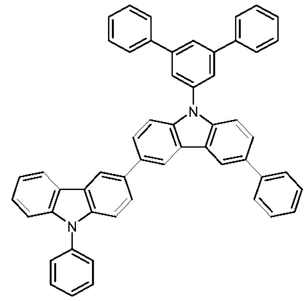 | 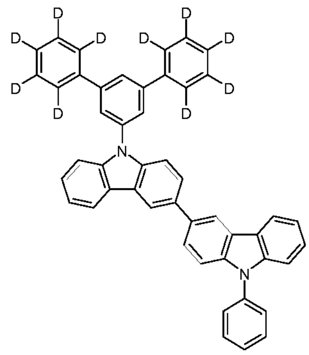 |
| 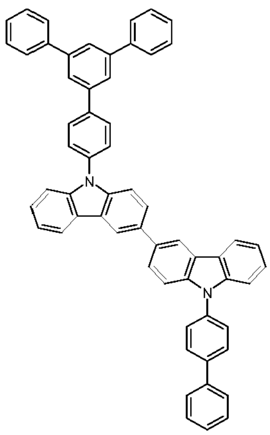 | 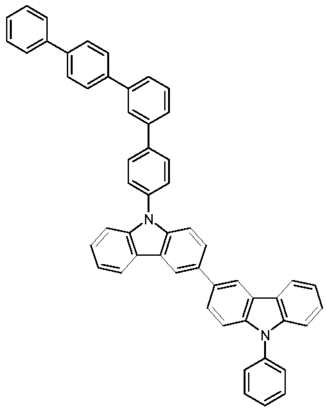 | 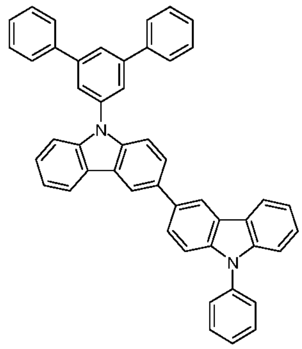 |
| 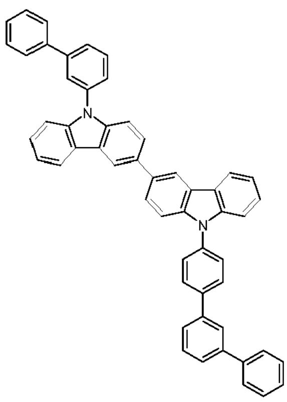 | 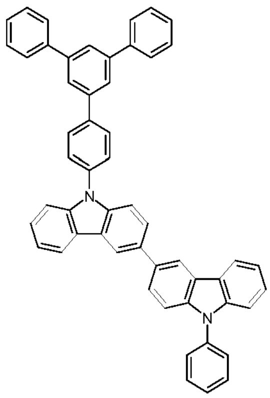 | 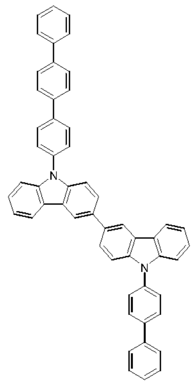 |

| | | |
|---|---|---|
| 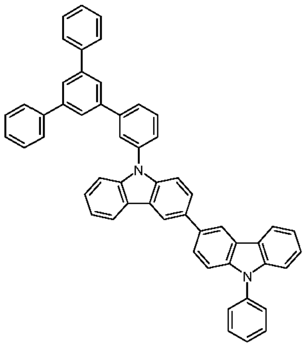 | 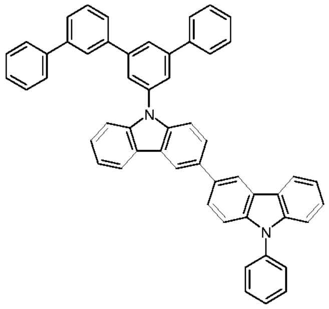 | 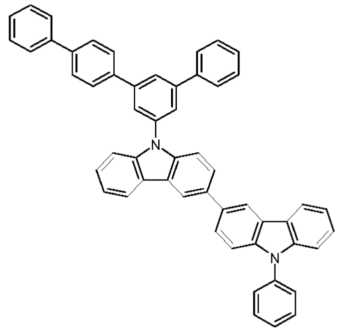 |
| 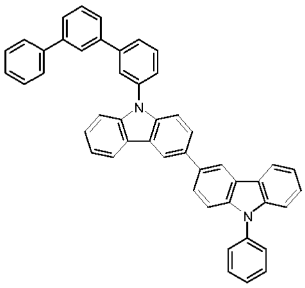 | 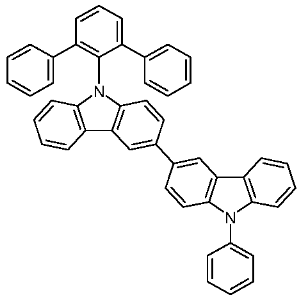 | 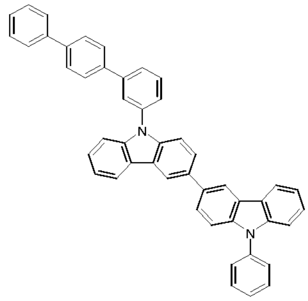 |
| 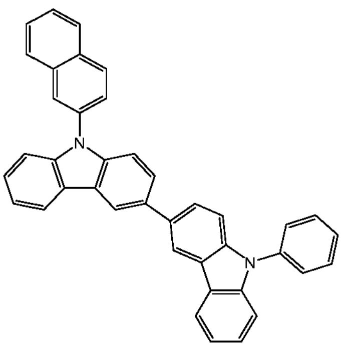 | 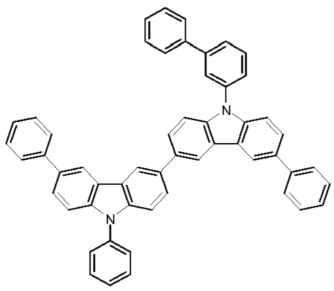 | 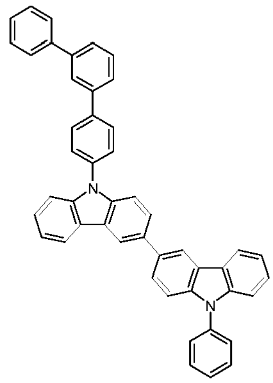 |
| 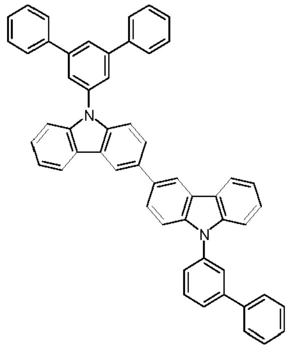 | 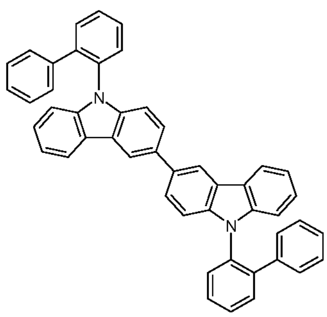 | 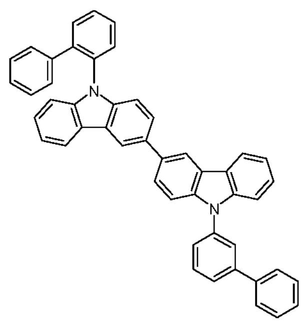 |

| | | |
|---|---|---|
| 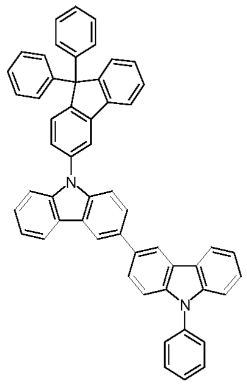 | 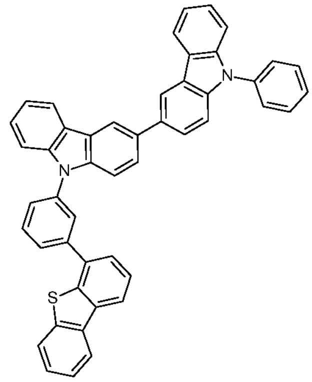 | 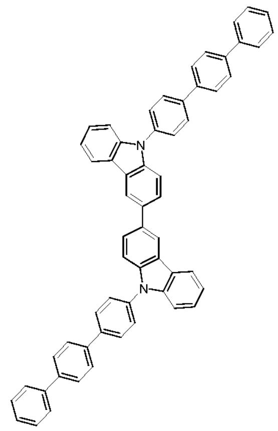 |
| 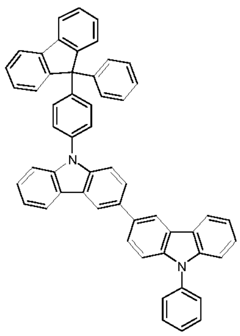 | 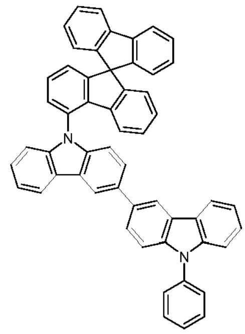 | 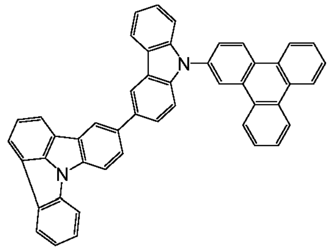 |
| 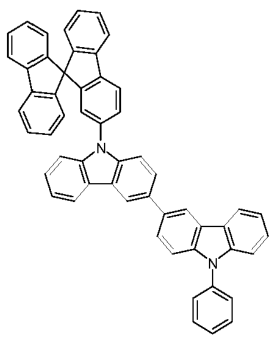 | 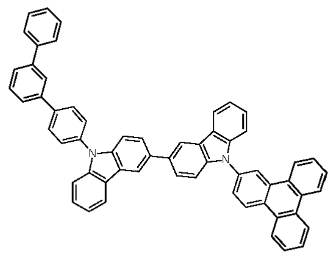 | 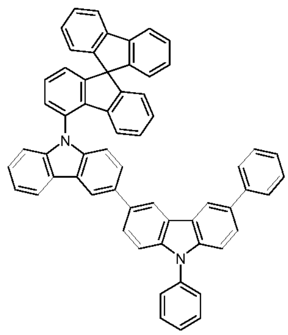 |
| 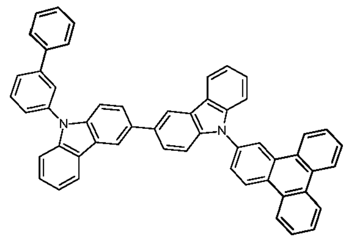 | 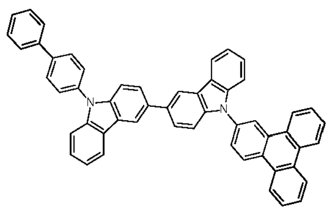 | 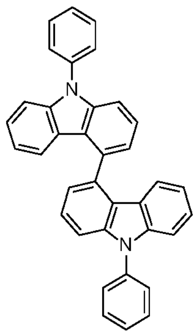 |
| 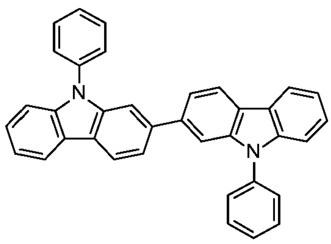 | 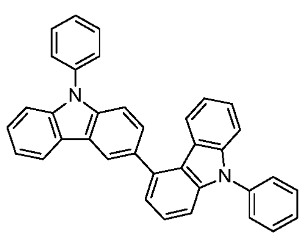 | 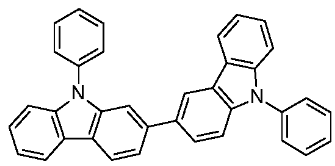 |

| | | |
|---|---|---|
| 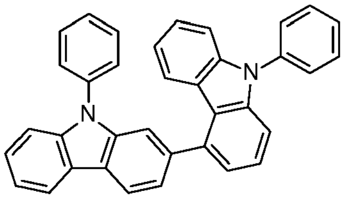 | 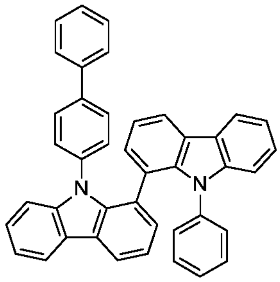 | 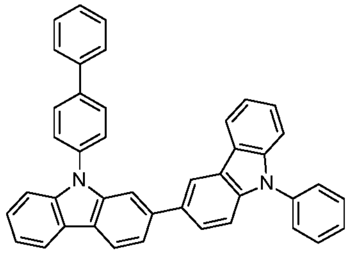 |
| 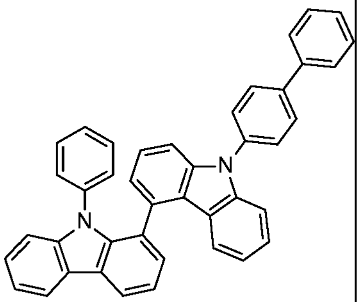 | 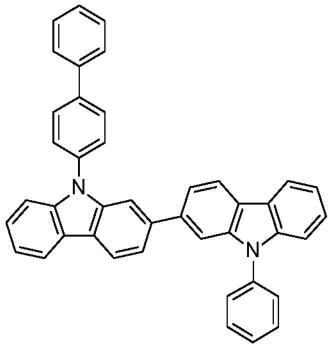 | 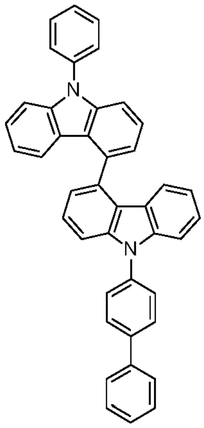 |
| 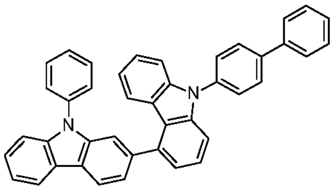 | 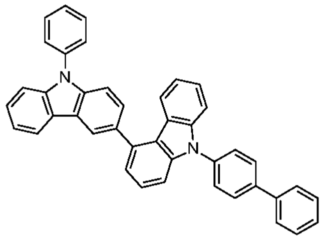 | 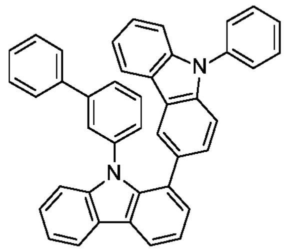 |
| 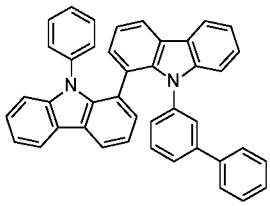 | 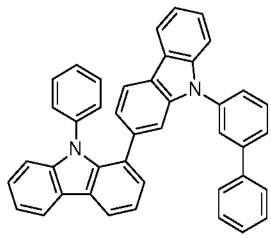 | 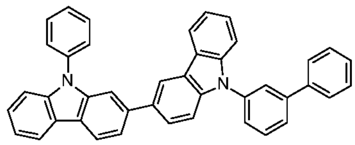 |
| 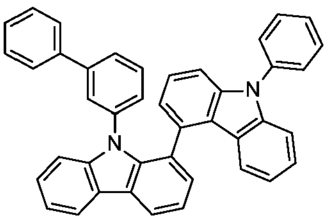 | 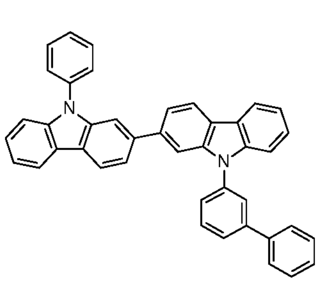 | 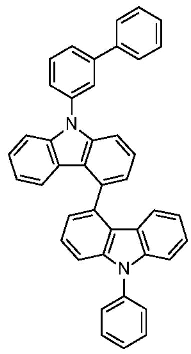 |

| | | |
|---|---|---|
| 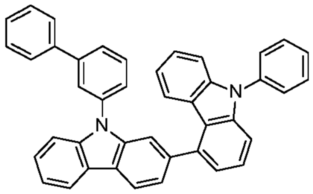 | 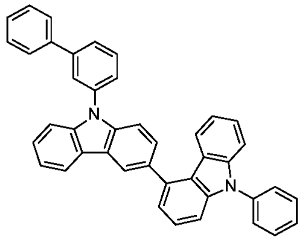 | 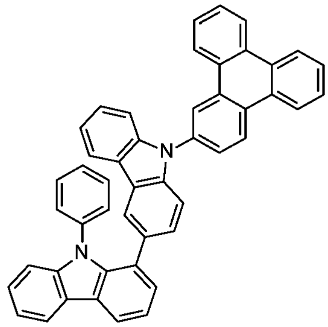 |
| 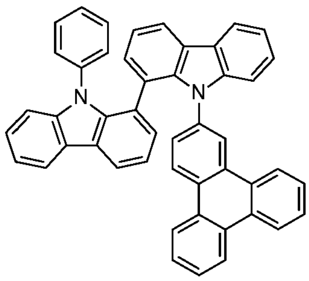 | 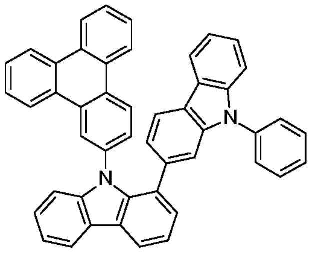 | 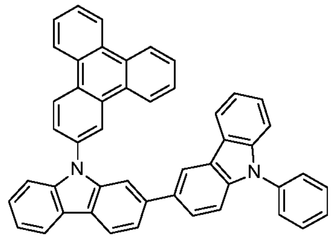 |
| 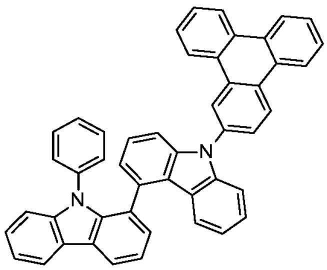 | 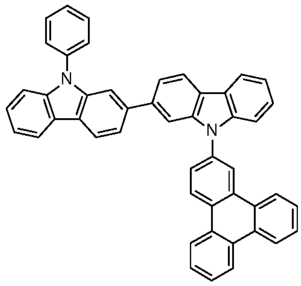 | 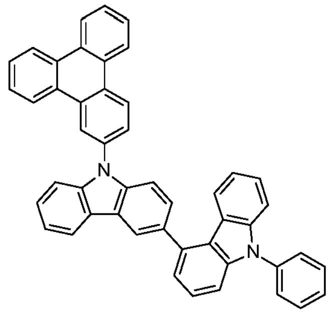 |
| 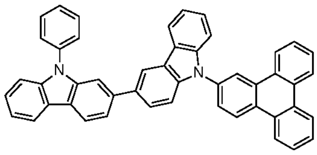 | 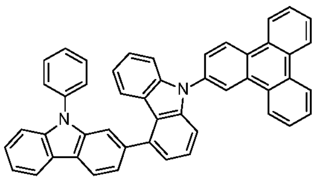 | 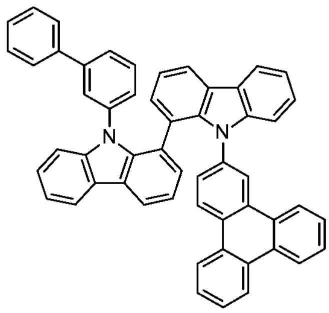 |
| 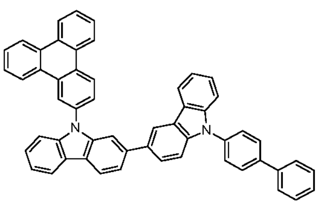 | 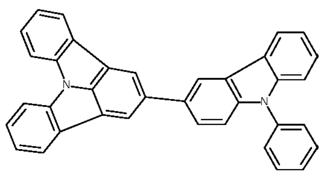 | 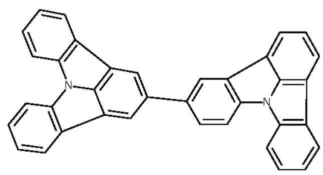 |
| 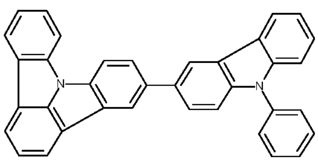 | 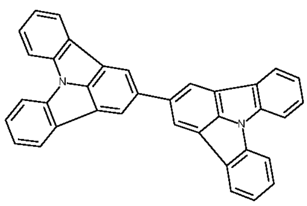 | 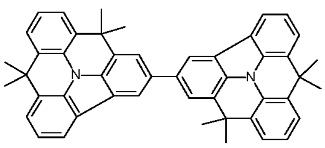 |

| | | |
|---|---|---|
| 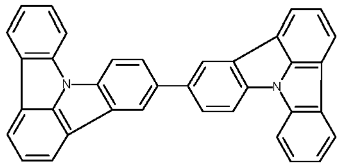 | 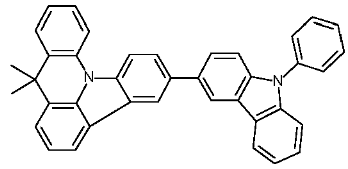 | 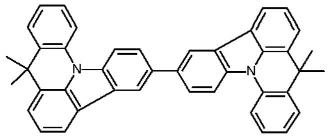 |

Besonders geeignete Verbindungen der Formel (2), die bevorzugt in Kombination mit mindestens einer Verbindung der Formel (1) in der erfindungsgemäßen elektrolumineszierenden Vorrichtung verwendet werden, sind die Verbindungen H1 bis H27:

| | | |
|---|---|---|
| 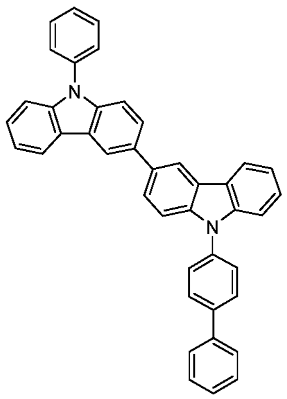 | 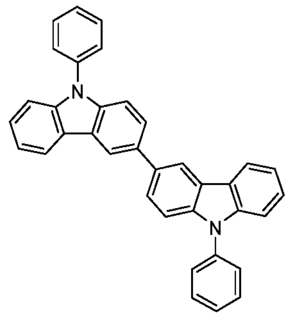 | 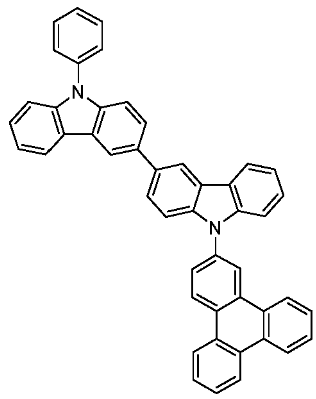 |
| H1 | H2 | H3 |
| 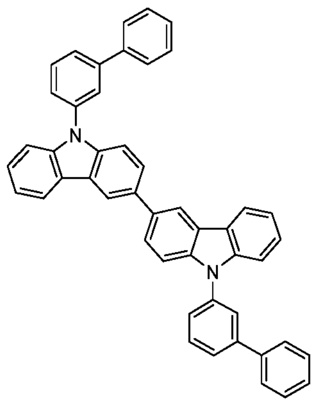 | 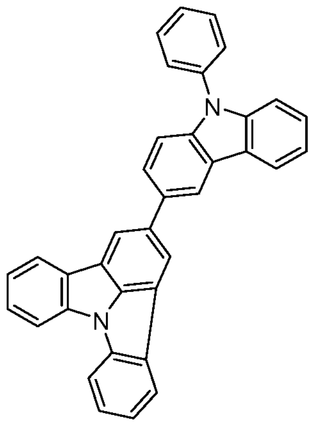 | 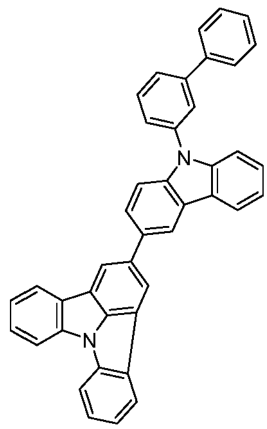 |
| **H4** | **H5** | **H6** |
| 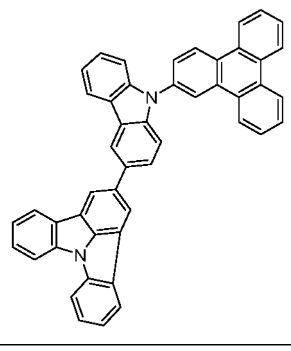 | 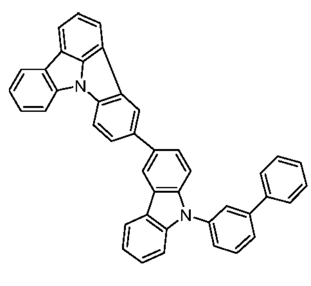 | 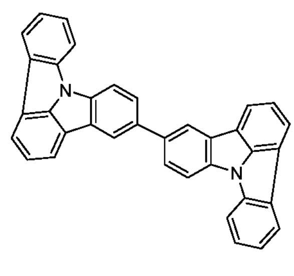 |
| H7 | H8 | H9 |

(fortgesetzt)

| | | |
|---|---|---|
| 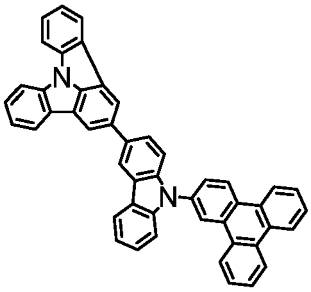 | 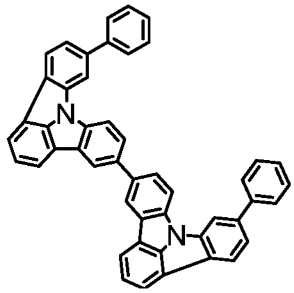 | 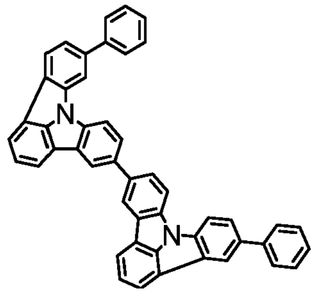 |
| H10 | H11 | H12 |
| 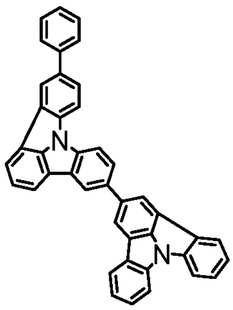 | 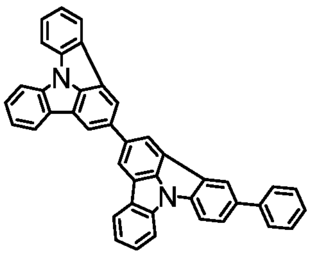 | 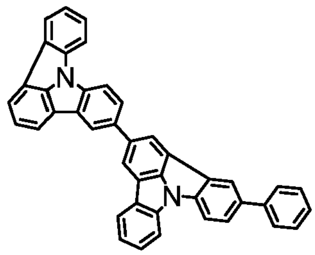 |
| H13 | H14 | H15 |
| 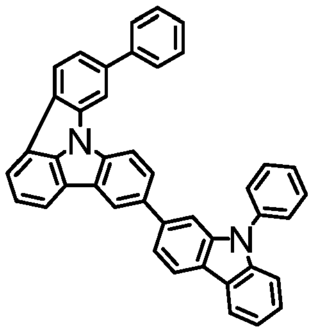 | 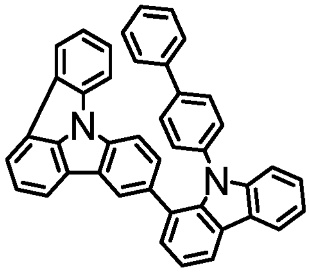 | 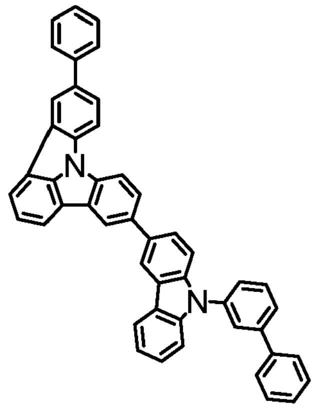 |
| H16 | H17 | H18 |
| 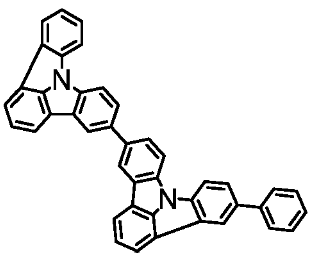 | 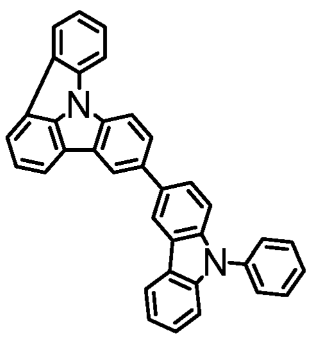 | 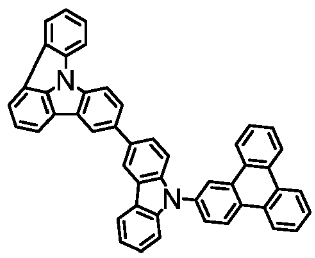 |
| H19 | H20 | H21 |

(fortgesetzt)

| | | |
|---|---|---|
| 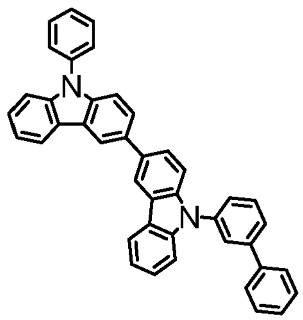 | 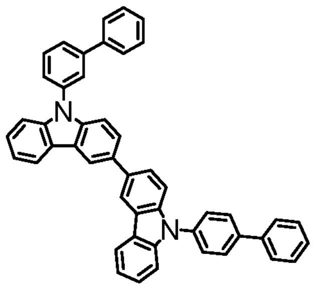 | 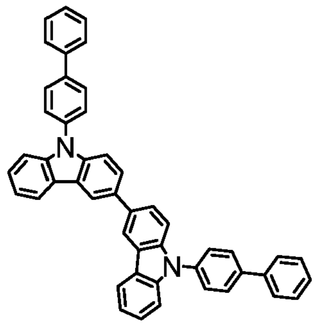 |
| H22 | H23 | H24 |
| 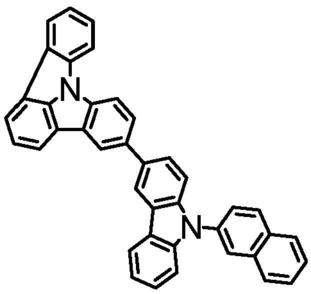 | 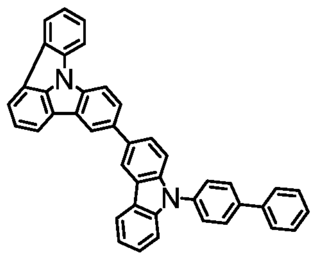 | 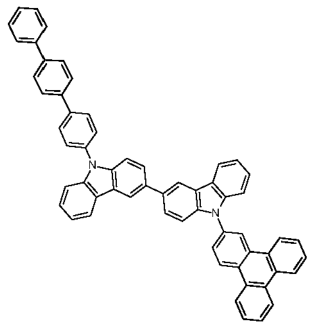 |
| H25 | H26 | H27. |

Die Herstellung der Verbindungen der Formel (2) oder der bevorzugten Verbindungen der Formeln (2), (2a), (2b), (2b-1), (2b-2), (2b-3), (2b-4, (2b-5) und (2c), sowie der Verbindungen der Tabelle 2 und H1 bis H27 ist dem Fachmann bekannt. Die Verbindungen können nach dem Fachmann bekannten Syntheseschritten, wie z.B. Halogenierung, bevorzugt Bromierung, und einer sich anschließenden metallorganischen Kupplungsreaktion, z.B. Suzuki-Kupplung, Heck-Kupplung oder Hartwig-Buchwald-Kupplung, hergestellt werden. Zum Teil sind die Verbindungen der Formel (2) kommerziell erhältlich.

Die vorstehend genannten Hostmaterialien der Formel (1) sowie deren bevorzugt beschriebene Ausführungsformen oder die Verbindungen der Tabelle 1 und der Verbindungen E1 bis E27 können in der erfindungsgemäßen Vorrichtung beliebig mit den genannten Hostmaterialien der Formeln (2), (2a), (2b), (2), (2a), (2b), (2b-1), (2b-2), (2b-3), (2b-4, (2b-5), (2c), (2c-1), (2c-2), (2c-3), (2c-4), (2c-5), (2c-6), (2c-7) und (2c-8) sowie deren bevorzugt beschriebene Ausführungsformen oder den Verbindungen der Tabelle 2 oder den Verbindungen H1 bis H27 kombiniert werden.

Zuvor genannte spezielle Kombinationen von Hostmaterialien der Formel (1) mit Hostmaterialien der Formel (2) sind bevorzugt, wie zuvor beschrieben. Bevorzugte Kombinationen von Hostmaterialien sind ebenfalls nachfolgend beschrieben.

Ein weiterer Gegenstand der Erfindung sind ebenfalls Mischungen enthaltend mindestens eine Verbindung der Formel (1) und mindestens eine Verbindung der Formel (2),

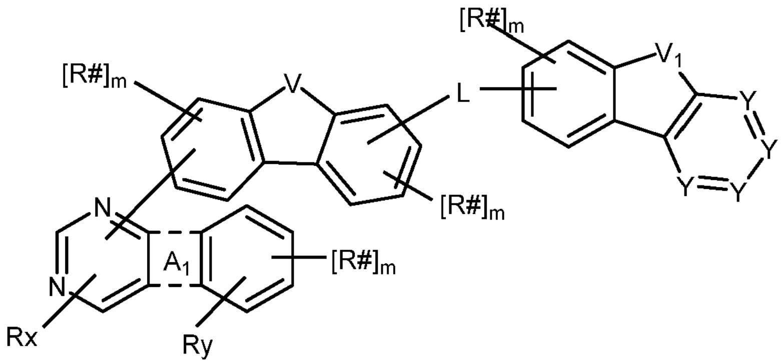

Formel (1)

,

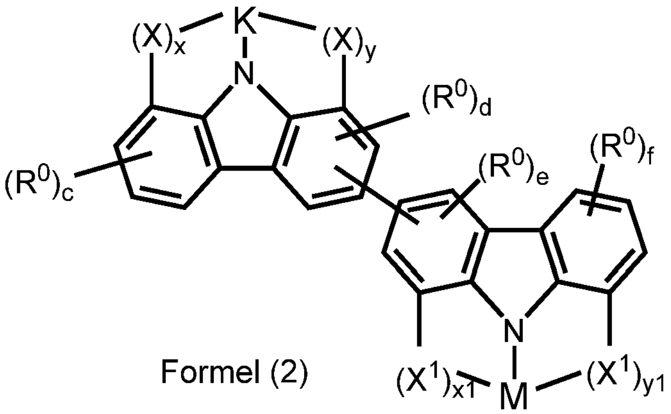

Formel (2)

,

wobei für die verwendeten Symbole und Indizes gilt:
Ring $A_1$ in Formel (1) entspricht der Formel (1A)

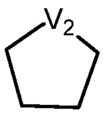

Formel (1A)

V ist O oder S;

$V_1$ ist O, S, $C(R^1)_2$ oder N-A;

$V_2$ ist O oder S;

Y ist bei jedem Auftreten unabhängig voneinander gleich oder verschieden CH, CR oder CA, oder zwei benachbarte Gruppen Y bilden zusammen eine Gruppe der Formel (1B)

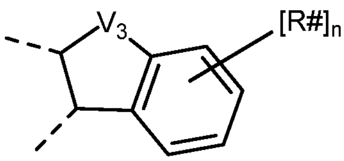

Formel (1B)

und die restlichen Gruppen Y sind unabhängig voneinander CH oder CR, wobei $V_3$ O, S, $C(R^1)_2$ oder N-A bedeutet und die gestrichelten Bindungen die Verknüpfung dieser Gruppe an den Rest der Formel (1) zeigen;

Rx ist $L_1$-R*;

Ry ist $L_2$-R*;

R* ist bei jedem Auftreten unabhängig ein aromatisches oder heteroaromatisches Ringsystem mit 6 bis 14 Ringatomen, das mit einem oder mehreren Resten R substituiert sein kann;

L, $L_1$, $L_2$ sind jeweils unabhängig voneinander eine Bindung oder eine Phenylengruppe, die jeweils mit einem

oder mehreren Resten R substituiert sein kann;

A ist bei jedem Auftreten gleich oder verschieden ein aromatisches oder heteroaromatisches Ringsystem mit 6 bis 30 Ringatomen, das mit einem oder mehreren Resten R substituiert sein kann;

n ist 0, 1, 2, 3 oder 4;

m ist 0, 1, 2 oder 3;

R# ist D oder eine Arylgruppe mit 6 bis 12 C-Atomen, die mit einem oder mehreren Resten R substituiert sein kann;

$R^1$ ist bei jedem Auftreten gleich oder verschieden eine Alkylgruppe mit 1 bis 20 C-Atomen, Phenyl, das mit einem oder mehreren Resten R substituiert sein kann oder die zwei $R^1$ bilden zusammen mit dem C-Atom an das sie binden eine Spiroverbindung;

R ist bei jedem Auftreten gleich oder verschieden ausgewählt aus D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch $R^2C=CR^2$, O oder S ersetzt sein können und wobei ein oder mehrere H-Atome durch D, F, oder CN ersetzt sein können;

$R^2$ ist bei jedem Auftreten gleich oder verschieden ausgewählt aus der Gruppe bestehend aus H, D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch O oder S ersetzt sein können und wobei ein oder mehrere H-Atome durch D, F, oder CN ersetzt sein können;

K, M sind jeweils unabhängig voneinander ein unsubstituiertes oder teilweise oder vollständig deuteriertes oder einfach durch R* substituiertes aromatisches Ringsystem mit 6 bis 40 Ringatomen, wenn x und y 0 bedeuten und wenn x1 und y1 0 bedeuten, oder

K, M bilden jeweils unabhängig voneinander zusammen mit X oder $X^1$ ein heteroaromatisches Ringsystem mit 14 bis 40 Ringatomen, sobald der Wert von x, x1, y und/oder y1 1 bedeutet;

x, x1 sind jeweils unabhängig bei jedem Auftreten 0 oder 1;

y, y1 sind jeweils unabhängig bei jedem Auftreten 0 oder 1;

X und $X^1$ sind jeweils unabhängig voneinander bei jedem Auftreten eine Bindung oder $C(R^+)_2$;

$R^0$ ist bei jedem Auftreten unabhängig voneinander ein unsubstituiertes oder teilweise oder vollständig deuteriertes aromatisches Ringsystem mit 6 bis 18 C-Atomen;

$R^+$ ist bei jedem Auftreten unabhängig voneinander eine geradkettige oder verzweigte Alkylgruppe mit 1 bis 4 C-Atomen und

c, d, e und f sind unabhängig voneinander 0 oder 1.

Die Ausführungen hinsichtlich der Hostmaterialien der Formeln (1) und (2) sowie deren bevorzugten Ausführungsformen und deren Kombination gilt entsprechend auch für die erfindungsgemäße Mischung.

Besonders bevorzugte Mischungen der Hostmaterialien der Formel (1) mit den Hostmaterialien der Formel (2) für die erfindungsgemäße Vorrichtung erhält man durch Kombination der Verbindungen E1 bis E27 mit den Verbindungen der Tabelle 2.

Ganz besonders bevorzugte Mischungen der Hostmaterialien der Formel (1) mit den Hostmaterialien der Formel (2) für die erfindungsgemäße Vorrichtung erhält man durch Kombination der Verbindungen E1 bis E27 mit den Verbindungen H1 bis H27 wie im Folgenden in Tabelle 3 gezeigt.

Tabelle 3:

| | | | | | | | | |
|---|---|---|---|---|---|---|---|---|
| M1 | E1 | H1 | M2 | E2 | H1 | M3 | E3 | H1 |
| M4 | E4 | H1 | M5 | E5 | H1 | M6 | E6 | H1 |
| M7 | E7 | H1 | M8 | E8 | H1 | M9 | E9 | H1 |
| M10 | E10 | H1 | M11 | E11 | H1 | M12 | E12 | H1 |
| M13 | E13 | H1 | M14 | E14 | H1 | M15 | E15 | H1 |
| M16 | E16 | H1 | M17 | E17 | H1 | M18 | E18 | H1 |
| M19 | E19 | H1 | M20 | E20 | H1 | M21 | E21 | H1 |
| M22 | E22 | H1 | M23 | E23 | H1 | M24 | E24 | H1 |
| M25 | E25 | H1 | M26 | E26 | H1 | M27 | E27 | H1 |
| M28 | E1 | H2 | M29 | E2 | H2 | M30 | E3 | H2 |
| M31 | E4 | H2 | M32 | E5 | H2 | M33 | E6 | H2 |

(fortgesetzt)

| | | | | | | | | |
|---|---|---|---|---|---|---|---|---|
| M34 | E7 | H2 | M35 | E8 | H2 | M36 | E9 | H2 |
| M37 | E10 | H2 | M38 | E11 | H2 | M39 | E12 | H2 |
| M40 | E13 | H2 | M41 | E14 | H2 | M42 | E15 | H2 |
| M43 | E16 | H2 | M44 | E17 | H2 | M45 | E18 | H2 |
| M46 | E19 | H2 | M47 | E20 | H2 | M48 | E21 | H2 |
| M49 | E22 | H2 | M50 | E23 | H2 | M51 | E24 | H2 |
| M52 | E25 | H2 | M53 | E26 | H2 | M54 | E27 | H2 |
| M55 | E1 | H3 | M56 | E2 | H3 | M57 | E3 | H3 |
| M58 | E4 | H3 | M59 | E5 | H3 | M60 | E6 | H3 |
| M61 | E7 | H3 | M62 | E8 | H3 | M63 | E9 | H3 |
| M64 | E10 | H3 | M65 | E11 | H3 | M66 | E12 | H3 |
| M67 | E13 | H3 | M68 | E14 | H3 | M69 | E15 | H3 |
| M70 | E16 | H3 | M71 | E17 | H3 | M72 | E18 | H3 |
| M73 | E19 | H3 | M74 | E20 | H3 | M75 | E21 | H3 |
| M76 | E22 | H3 | M77 | E23 | H3 | M78 | E24 | H3 |
| M79 | E25 | H3 | M80 | E26 | H3 | M81 | E27 | H3 |
| M82 | E1 | H4 | M83 | E2 | H4 | M84 | E3 | H4 |
| M85 | E4 | H4 | M86 | E5 | H4 | M87 | E6 | H4 |
| M88 | E7 | H4 | M89 | E8 | H4 | M90 | E9 | H4 |
| M91 | E10 | H4 | M92 | E11 | H4 | M93 | E12 | H4 |
| M94 | E13 | H4 | M95 | E14 | H4 | M96 | E15 | H4 |
| M97 | E16 | H4 | M98 | E17 | H4 | M99 | E18 | H4 |
| M100 | E19 | H4 | M101 | E20 | H4 | M102 | E21 | H4 |
| M103 | E22 | H4 | M104 | E23 | H4 | M105 | E24 | H4 |
| M106 | E25 | H4 | M107 | E26 | H4 | M108 | E27 | H4 |
| M109 | E1 | H5 | M110 | E2 | H5 | M111 | E3 | H5 |
| M112 | E4 | H5 | M113 | E5 | H5 | M114 | E6 | H5 |
| M115 | E7 | H5 | M116 | E8 | H5 | M117 | E9 | H5 |
| M118 | E10 | H5 | M119 | E11 | H5 | M120 | E12 | H5 |
| M121 | E13 | H5 | M122 | E14 | H5 | M123 | E15 | H5 |
| M124 | E16 | H5 | M125 | E17 | H5 | M126 | E18 | H5 |
| M127 | E19 | H5 | M128 | E20 | H5 | M129 | E21 | H5 |
| M130 | E22 | H5 | M131 | E23 | H5 | M132 | E24 | H5 |
| M133 | E25 | H5 | M134 | E26 | H5 | M135 | E27 | H5 |
| M136 | E1 | H6 | M137 | E2 | H6 | M138 | E3 | H6 |
| M139 | E4 | H6 | M140 | E5 | H6 | M141 | E6 | H6 |
| M142 | E7 | H6 | M143 | E8 | H6 | M144 | E9 | H6 |
| M145 | E10 | H6 | M146 | E11 | H6 | M147 | E12 | H6 |
| M148 | E13 | H6 | M149 | E14 | H6 | M150 | E15 | H6 |

(fortgesetzt)

| | | | | | | | | |
|---|---|---|---|---|---|---|---|---|
| M151 | E16 | H6 | M152 | E17 | H6 | M153 | E18 | H6 |
| M154 | E19 | H6 | M155 | E20 | H6 | M156 | E21 | H6 |
| M157 | E22 | H6 | M158 | E23 | H6 | M159 | E24 | H6 |
| M160 | E25 | H6 | M161 | E26 | H6 | M162 | E27 | H6 |
| M163 | E1 | H7 | M164 | E2 | H7 | M165 | E3 | H7 |
| M166 | E4 | H7 | M167 | E5 | H7 | M168 | E6 | H7 |
| M169 | E7 | H7 | M170 | E8 | H7 | M171 | E9 | H7 |
| M172 | E10 | H7 | M173 | E11 | H7 | M174 | E12 | H7 |
| M175 | E13 | H7 | M176 | E14 | H7 | M177 | E15 | H7 |
| M178 | E16 | H7 | M179 | E17 | H7 | M180 | E18 | H7 |
| M181 | E19 | H7 | M182 | E20 | H7 | M183 | E21 | H7 |
| M184 | E22 | H7 | M185 | E23 | H7 | M186 | E24 | H7 |
| M187 | E25 | H7 | M188 | E26 | H7 | M189 | E27 | H7 |
| M190 | E1 | H8 | M191 | E2 | H8 | M192 | E3 | H8 |
| M193 | E4 | H8 | M194 | E5 | H8 | M195 | E6 | H8 |
| M196 | E7 | H8 | M197 | E8 | H8 | M198 | E9 | H8 |
| M199 | E10 | H8 | M200 | E11 | H8 | M201 | E12 | H8 |
| M202 | E13 | H8 | M203 | E14 | H8 | M204 | E15 | H8 |
| M205 | E16 | H8 | M206 | E17 | H8 | M207 | E18 | H8 |
| M208 | E19 | H8 | M209 | E20 | H8 | M210 | E21 | H8 |
| M211 | E22 | H8 | M212 | E23 | H8 | M213 | E24 | H8 |
| M214 | E25 | H8 | M215 | E26 | H8 | M216 | E27 | H8 |
| M217 | E1 | H9 | M218 | E2 | H9 | M219 | E3 | H9 |
| M220 | E4 | H9 | M221 | E5 | H9 | M222 | E6 | H9 |
| M223 | E7 | H9 | M224 | E8 | H9 | M225 | E9 | H9 |
| M226 | E10 | H9 | M227 | E11 | H9 | M228 | E12 | H9 |
| M229 | E13 | H9 | M230 | E14 | H9 | M231 | E15 | H9 |
| M232 | E16 | H9 | M233 | E17 | H9 | M234 | E18 | H9 |
| M235 | E19 | H9 | M236 | E20 | H9 | M237 | E21 | H9 |
| M238 | E22 | H9 | M239 | E23 | H9 | M240 | E24 | H9 |
| M241 | E25 | H9 | M242 | E26 | H9 | M243 | E27 | H9 |
| M244 | E1 | H10 | M245 | E2 | H10 | M246 | E3 | H10 |
| M247 | E4 | H10 | M248 | E5 | H10 | M249 | E6 | H10 |
| M250 | E7 | H10 | M251 | E8 | H10 | M252 | E9 | H10 |
| M253 | E10 | H10 | M254 | E11 | H10 | M255 | E12 | H10 |
| M256 | E13 | H10 | M257 | E14 | H10 | M258 | E15 | H10 |
| M259 | E16 | H10 | M260 | E17 | H10 | M261 | E18 | H10 |
| M262 | E19 | H10 | M263 | E20 | H10 | M264 | E21 | H10 |
| M265 | E22 | H10 | M266 | E23 | H10 | M267 | E24 | H10 |

(fortgesetzt)

| | | | | | | | | |
|---|---|---|---|---|---|---|---|---|
| M268 | E25 | H10 | M269 | E26 | H10 | M270 | E27 | H10 |
| M271 | E1 | H11 | M272 | E2 | H11 | M273 | E3 | H11 |
| M274 | E4 | H11 | M275 | E5 | H11 | M276 | E6 | H11 |
| M277 | E7 | H11 | M278 | E8 | H11 | M279 | E9 | H11 |
| M280 | E10 | H11 | M281 | E11 | H11 | M282 | E12 | H11 |
| M283 | E13 | H11 | M284 | E14 | H11 | M285 | E15 | H11 |
| M286 | E16 | H11 | M287 | E17 | H11 | M288 | E18 | H11 |
| M289 | E19 | H11 | M290 | E20 | H11 | M291 | E21 | H11 |
| M292 | E22 | H11 | M293 | E23 | H11 | M294 | E24 | H11 |
| M295 | E25 | H11 | M296 | E26 | H11 | M297 | E27 | H11 |
| M298 | E1 | H12 | M299 | E2 | H12 | M300 | E3 | H12 |
| M301 | E4 | H12 | M302 | E5 | H12 | M303 | E6 | H12 |
| M304 | E7 | H12 | M305 | E8 | H12 | M306 | E9 | H12 |
| M307 | E10 | H12 | M308 | E11 | H12 | M309 | E12 | H12 |
| M310 | E13 | H12 | M311 | E14 | H12 | M312 | E15 | H12 |
| M313 | E16 | H12 | M314 | E17 | H12 | M315 | E18 | H12 |
| M316 | E19 | H12 | M317 | E20 | H12 | M318 | E21 | H12 |
| M319 | E22 | H12 | M320 | E23 | H12 | M321 | E24 | H12 |
| M322 | E25 | H12 | M323 | E26 | H12 | M324 | E27 | H12 |
| M325 | E1 | H13 | M326 | E2 | H13 | M327 | E3 | H13 |
| M328 | E4 | H13 | M329 | E5 | H13 | M330 | E6 | H13 |
| M331 | E7 | H13 | M332 | E8 | H13 | M333 | E9 | H13 |
| M334 | E10 | H13 | M335 | E11 | H13 | M336 | E12 | H13 |
| M337 | E13 | H13 | M338 | E14 | H13 | M339 | E15 | H13 |
| M340 | E16 | H13 | M341 | E17 | H13 | M342 | E18 | H13 |
| M343 | E19 | H13 | M344 | E20 | H13 | M345 | E21 | H13 |
| M346 | E22 | H13 | M347 | E23 | H13 | M348 | E24 | H13 |
| M349 | E25 | H13 | M350 | E26 | H13 | M351 | E27 | H13 |
| M352 | E1 | H14 | M353 | E2 | H14 | M354 | E3 | H14 |
| M355 | E4 | H14 | M356 | E5 | H14 | M357 | E6 | H14 |
| M358 | E7 | H14 | M359 | E8 | H14 | M360 | E9 | H14 |
| M361 | E10 | H14 | M362 | E11 | H14 | M363 | E12 | H14 |
| M364 | E13 | H14 | M365 | E14 | H14 | M366 | E15 | H14 |
| M367 | E16 | H14 | M368 | E17 | H14 | M369 | E18 | H14 |
| M370 | E19 | H14 | M371 | E20 | H14 | M372 | E21 | H14 |
| M373 | E22 | H14 | M374 | E23 | H14 | M375 | E24 | H14 |
| M376 | E25 | H14 | M377 | E26 | H14 | M378 | E27 | H14 |
| M379 | E1 | H15 | M380 | E2 | H15 | M381 | E3 | H15 |
| M382 | E4 | H15 | M383 | E5 | H15 | M384 | E6 | H15 |

(fortgesetzt)

| | | | | | | | | |
|---|---|---|---|---|---|---|---|---|
| M385 | E7 | H15 | M386 | E8 | H15 | M387 | E9 | H15 |
| M388 | E10 | H15 | M389 | E11 | H15 | M390 | E12 | H15 |
| M391 | E13 | H15 | M392 | E14 | H15 | M393 | E15 | H15 |
| M394 | E16 | H15 | M395 | E17 | H15 | M396 | E18 | H15 |
| M397 | E19 | H15 | M398 | E20 | H15 | M399 | E21 | H15 |
| M400 | E22 | H15 | M401 | E23 | H15 | M402 | E24 | H15 |
| M403 | E25 | H15 | M404 | E26 | H15 | M405 | E27 | H15 |
| M406 | E1 | H16 | M407 | E2 | H16 | M408 | E3 | H16 |
| M409 | E4 | H16 | M410 | E5 | H16 | M411 | E6 | H16 |
| M412 | E7 | H16 | M413 | E8 | H16 | M414 | E9 | H16 |
| M415 | E10 | H16 | M416 | E11 | H16 | M417 | E12 | H16 |
| M418 | E13 | H16 | M419 | E14 | H16 | M420 | E15 | H16 |
| M421 | E16 | H16 | M422 | E17 | H16 | M423 | E18 | H16 |
| M424 | E19 | H16 | M425 | E20 | H16 | M426 | E21 | H16 |
| M427 | E22 | H16 | M428 | E23 | H16 | M429 | E24 | H16 |
| M430 | E25 | H16 | M431 | E26 | H16 | M432 | E27 | H16 |
| M433 | E1 | H17 | M434 | E2 | H17 | M435 | E3 | H17 |
| M436 | E4 | H17 | M437 | E5 | H17 | M438 | E6 | H17 |
| M439 | E7 | H17 | M440 | E8 | H17 | M441 | E9 | H17 |
| M442 | E10 | H17 | M443 | E11 | H17 | M444 | E12 | H17 |
| M445 | E13 | H17 | M446 | E14 | H17 | M447 | E15 | H17 |
| M448 | E16 | H17 | M449 | E17 | H17 | M450 | E18 | H17 |
| M451 | E19 | H17 | M452 | E20 | H17 | M453 | E21 | H17 |
| M454 | E22 | H17 | M455 | E23 | H17 | M456 | E24 | H17 |
| M457 | E25 | H17 | M458 | E26 | H17 | M459 | E27 | H17 |
| M460 | E1 | H18 | M461 | E2 | H18 | M462 | E3 | H18 |
| M463 | E4 | H18 | M464 | E5 | H18 | M465 | E6 | H18 |
| M466 | E7 | H18 | M467 | E8 | H18 | M468 | E9 | H18 |
| M469 | E10 | H18 | M470 | E11 | H18 | M471 | E12 | H18 |
| M472 | E13 | H18 | M473 | E14 | H18 | M474 | E15 | H18 |
| M475 | E16 | H18 | M476 | E17 | H18 | M477 | E18 | H18 |
| M478 | E19 | H18 | M479 | E20 | H18 | M480 | E21 | H18 |
| M481 | E22 | H18 | M482 | E23 | H18 | M483 | E24 | H18 |
| M484 | E25 | H18 | M485 | E26 | H18 | M486 | E27 | H18 |
| M487 | E1 | H19 | M488 | E2 | H19 | M489 | E3 | H19 |
| M490 | E4 | H19 | M491 | E5 | H19 | M492 | E6 | H19 |
| M493 | E7 | H19 | M494 | E8 | H19 | M495 | E9 | H19 |
| M496 | E10 | H19 | M497 | E11 | H19 | M498 | E12 | H19 |
| M499 | E13 | H19 | M500 | E14 | H19 | M501 | E15 | H19 |

(fortgesetzt)

| | | | | | | | | |
|---|---|---|---|---|---|---|---|---|
| M502 | E16 | H19 | M503 | E17 | H19 | M504 | E18 | H19 |
| M505 | E19 | H19 | M506 | E20 | H19 | M507 | E21 | H19 |
| M508 | E22 | H19 | M509 | E23 | H19 | M510 | E24 | H19 |
| M511 | E25 | H19 | M512 | E26 | H19 | M513 | E27 | H19 |
| M514 | E1 | H20 | M515 | E2 | H20 | M516 | E3 | H20 |
| M517 | E4 | H20 | M518 | E5 | H20 | M519 | E6 | H20 |
| M520 | E7 | H20 | M521 | E8 | H20 | M522 | E9 | H20 |
| M523 | E10 | H20 | M524 | E11 | H20 | M525 | E12 | H20 |
| M526 | E13 | H20 | M527 | E14 | H20 | M528 | E15 | H20 |
| M529 | E16 | H20 | M530 | E17 | H20 | M531 | E18 | H20 |
| M532 | E19 | H20 | M533 | E20 | H20 | M534 | E21 | H20 |
| M535 | E22 | H20 | M536 | E23 | H20 | M537 | E24 | H20 |
| M538 | E25 | H20 | M539 | E26 | H20 | M540 | E27 | H20 |
| M541 | E1 | H21 | M542 | E2 | H21 | M543 | E3 | H21 |
| M544 | E4 | H21 | M545 | E5 | H21 | M546 | E6 | H21 |
| M547 | E7 | H21 | M548 | E8 | H21 | M549 | E9 | H21 |
| M550 | E10 | H21 | M551 | E11 | H21 | M552 | E12 | H21 |
| M553 | E13 | H21 | M554 | E14 | H21 | M555 | E15 | H21 |
| M556 | E16 | H21 | M557 | E17 | H21 | M558 | E18 | H21 |
| M559 | E19 | H21 | M560 | E20 | H21 | M561 | E21 | H21 |
| M562 | E22 | H21 | M563 | E23 | H21 | M564 | E24 | H21 |
| M565 | E25 | H21 | M566 | E26 | H21 | M567 | E27 | H21 |
| M568 | E1 | H22 | M569 | E2 | H22 | M570 | E3 | H22 |
| M571 | E4 | H22 | M572 | E5 | H22 | M573 | E6 | H22 |
| M574 | E7 | H22 | M575 | E8 | H22 | M576 | E9 | H22 |
| M577 | E10 | H22 | M578 | E11 | H22 | M579 | E12 | H22 |
| M580 | E13 | H22 | M581 | E14 | H22 | M582 | E15 | H22 |
| M583 | E16 | H22 | M584 | E17 | H22 | M585 | E18 | H22 |
| M586 | E19 | H22 | M587 | E20 | H22 | M588 | E21 | H22 |
| M589 | E22 | H22 | M590 | E23 | H22 | M591 | E24 | H22 |
| M592 | E25 | H22 | M593 | E26 | H22 | M594 | E27 | H22 |
| M595 | E1 | H23 | M596 | E2 | H23 | M597 | E3 | H23 |
| M598 | E4 | H23 | M599 | E5 | H23 | M600 | E6 | H23 |
| M601 | E7 | H23 | M602 | E8 | H23 | M603 | E9 | H23 |
| M604 | E10 | H23 | M605 | E11 | H23 | M606 | E12 | H23 |
| M607 | E13 | H23 | M608 | E14 | H23 | M609 | E15 | H23 |
| M610 | E16 | H23 | M611 | E17 | H23 | M612 | E18 | H23 |
| M613 | E19 | H23 | M614 | E20 | H23 | M615 | E21 | H23 |
| M616 | E22 | H23 | M617 | E23 | H23 | M618 | E24 | H23 |

(fortgesetzt)

| | | | | | | | | |
|---|---|---|---|---|---|---|---|---|
| M619 | E25 | H23 | M620 | E26 | H23 | M621 | E27 | H23 |
| M622 | E1 | H24 | M623 | E2 | H24 | M624 | E3 | H24 |
| M625 | E4 | H24 | M626 | E5 | H24 | M627 | E6 | H24 |
| M628 | E7 | H24 | M629 | E8 | H24 | M630 | E9 | H24 |
| M631 | E10 | H24 | M632 | E11 | H24 | M633 | E12 | H24 |
| M634 | E13 | H24 | M635 | E14 | H24 | M636 | E15 | H24 |
| M637 | E16 | H24 | M638 | E17 | H24 | M639 | E18 | H24 |
| M640 | E19 | H24 | M641 | E20 | H24 | M642 | E21 | H24 |
| M643 | E22 | H24 | M644 | E23 | H24 | M645 | E24 | H24 |
| M646 | E25 | H24 | M647 | E26 | H24 | M648 | E27 | H24 |
| M649 | E1 | H25 | M650 | E2 | H25 | M651 | E3 | H25 |
| M652 | E4 | H25 | M653 | E5 | H25 | M654 | E6 | H25 |
| M655 | E7 | H25 | M656 | E8 | H25 | M657 | E9 | H25 |
| M658 | E10 | H25 | M659 | E11 | H25 | M660 | E12 | H25 |
| M661 | E13 | H25 | M662 | E14 | H25 | M663 | E15 | H25 |
| M664 | E16 | H25 | M665 | E17 | H25 | M666 | E18 | H25 |
| M667 | E19 | H25 | M668 | E20 | H25 | M669 | E21 | H25 |
| M670 | E22 | H25 | M671 | E23 | H25 | M672 | E24 | H25 |
| M673 | E25 | H25 | M674 | E26 | H25 | M675 | E27 | H25 |
| M676 | E1 | H26 | M677 | E2 | H26 | M678 | E3 | H26 |
| M679 | E4 | H26 | M680 | E5 | H26 | M681 | E6 | H26 |
| M682 | E7 | H26 | M683 | E8 | H26 | M684 | E9 | H26 |
| M685 | E10 | H26 | M686 | E11 | H26 | M687 | E12 | H26 |
| M688 | E13 | H26 | M689 | E14 | H26 | M690 | E15 | H26 |
| M691 | E16 | H26 | M692 | E17 | H26 | M693 | E18 | H26 |
| M694 | E19 | H26 | M695 | E20 | H26 | M696 | E21 | H26 |
| M697 | E22 | H26 | M698 | E23 | H26 | M699 | E24 | H26 |
| M700 | E25 | H26 | M701 | E26 | H26 | M702 | E27 | H26 |
| M703 | E1 | H27 | M704 | E2 | H27 | M705 | E3 | H27 |
| M706 | E4 | H27 | M707 | E5 | H27 | M708 | E6 | H27 |
| M709 | E7 | H27 | M710 | E8 | H27 | M711 | E9 | H27 |
| M712 | E10 | H27 | M713 | E11 | H27 | M714 | E12 | H27 |
| M715 | E13 | H27 | M716 | E14 | H27 | M717 | E15 | H27 |
| M718 | E16 | H27 | M719 | E17 | H27 | M720 | E18 | H27 |
| M721 | E19 | H27 | M722 | E20 | H27 | M723 | E21 | H27 |
| M724 | E22 | H27 | M725 | E23 | H27 | M726 | E24 | H27 |
| M727 | E25 | H27 | M728 | E26 | H27 | M729 | E27 | H27 |

Die Konzentration des elektronentransportierenden Hostmaterials der Formel (1), wie zuvor beschrieben oder

bevorzugt beschrieben, in der erfindungsgemäßen Mischung oder in der lichtemittierenden Schicht der erfindungsgemäßen Vorrichtung liegt im Bereich von 5 Gew.-% bis 90 Gew.-%, bevorzugt im Bereich von 10 Gew.-% bis 85 Gew.-%, mehr bevorzugt im Bereich von 20 Gew.-% bis 85 Gew.-%, noch mehr bevorzugt im Bereich von 30 Gew.-% bis 80 Gew.-%, ganz besonders bevorzugt im Bereich von 20 Gew.-% bis 60 Gew.-% und am meisten bevorzugt im Bereich von 30 Gew.-% bis 50 Gew.-%, bezogen auf die gesamte Mischung oder bezogen auf die gesamte Zusammensetzung der lichtemittierenden Schicht.

Die Konzentration des lochtransportierenden Hostmaterials der Formel (2), wie zuvor beschrieben oder als bevorzugt beschrieben, in der erfindungsgemäßen Mischung oder in der lichtemittierenden Schicht der erfindungsgemäßen Vorrichtung liegt im Bereich von 10 Gew.-% bis 95 Gew.-%, bevorzugt im Bereich von 15 Gew.-% bis 90 Gew.-%, mehr bevorzugt im Bereich von 15 Gew.-% bis 80 Gew.-%, noch mehr bevorzugt im Bereich von 20 Gew.-% bis 70 Gew.-%, ganz besonders bevorzugt im Bereich von 40 Gew.-% bis 80 Gew.-% und am meisten bevorzugt im Bereich von 50 Gew.-% bis 70 Gew.-%, bezogen auf die gesamte Mischung oder bezogen auf die gesamte Zusammensetzung der lichtemittierenden Schicht.

Die vorliegende Erfindung betrifft auch eine Mischung, die neben den vorstehend genannten Hostmaterialien 1 und 2, wie zuvor beschrieben oder bevorzugt beschrieben, insbesondere Mischungen M1 bis M729, mindestens noch einen phosphoreszierenden Emitter enthält.

Die vorliegende Erfindung betrifft auch eine organische elektrolumineszierende Vorrichtung, wie zuvor beschrieben oder bevorzugt beschrieben, wobei die lichtemittierende Schicht neben den vorstehend genannten Hostmaterialien 1 und 2, wie zuvor beschrieben oder bevorzugt beschrieben, insbesondere den Materialkombinationen M1 bis M729, mindestens noch einen phosphoreszierenden Emitter enthält.

Vom Begriff phosphoreszierende Emitter sind typischerweise Verbindungen umfasst, bei denen die Lichtemission durch einen spin-verbotenen Übergang aus einem angeregten Zustand mit höherer Spinmultiplizität, also einem Spinzustand > 1, erfolgt, beispielsweise durch einen Übergang aus einem Triplett-Zustand oder einem Zustand mit einer noch höheren Spinquantenzahl, beispielsweise einem Quintett-Zustand. Bevorzugt wird hierbei ein Übergang aus einem Triplett-Zustand verstanden.

Als phosphoreszierende Emitter (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Emitter angesehen.

Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind.

Bevorzugte phosphoreszierende Emitter gemäß der vorliegenden Erfindung entsprechen der Formel (3),

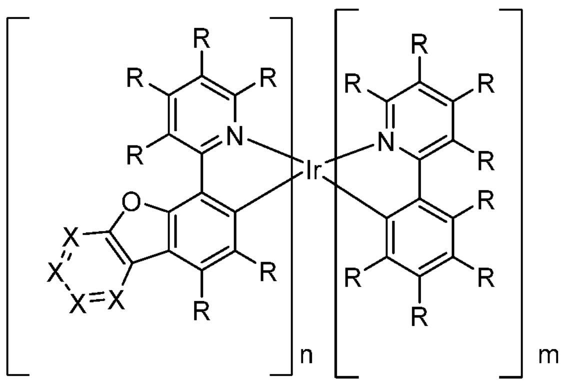

Formel (3)

,

wobei die Symbole und Indizes für diese Formel (3) die Bedeutung haben:

n+m ist 3, n ist 1 oder 2, m ist 2 oder 1,
X ist N oder CR,

R ist H, D oder eine verzweigte oder lineare Alkylgruppe mit 1 bis 10 C-Atomen oder eine teilweise oder vollständig deuterierte verzweigte oder lineare Alkylgruppe mit 1 bis 10 C-Atomen oder eine Cycloalkylgruppe mit 4 bis 7 C-Atomen, die teilweise oder vollständig mit Deuterium substituiert sein kann.

Ein weiterer Gegenstand der Erfindung ist demzufolge eine organische elektrolumineszierende Vorrichtung, wie zuvor beschrieben oder bevorzugt beschrieben, dadurch gekennzeichnet, dass die lichtemittierende Schicht neben den Hostmaterialien 1 und 2 mindestens einen phosphoreszierenden Emitter enthält, der der Formel (3) entspricht, wie zuvor beschrieben.

In Emittern der Formel (3) ist n bevorzugt 1 und m ist bevorzugt 2.

In Emittern der Formel (3) ist bevorzugt ein X ausgewählt aus N und die anderen X bedeuten CR.

In Emittern der Formel (3) ist mindestens ein R bevorzugt unterschiedlich von H. In Emittern der Formel (3) sind bevorzugt zwei R unterschiedlich von H und haben eine der sonst zuvor für die Emitter der Formel (3) angegebenen Bedeutungen.

Bevorzugte phosphoreszierende Emitter gemäß der vorliegenden Erfindung entsprechen den Formeln (I), (II), (III), (IV) oder (V),

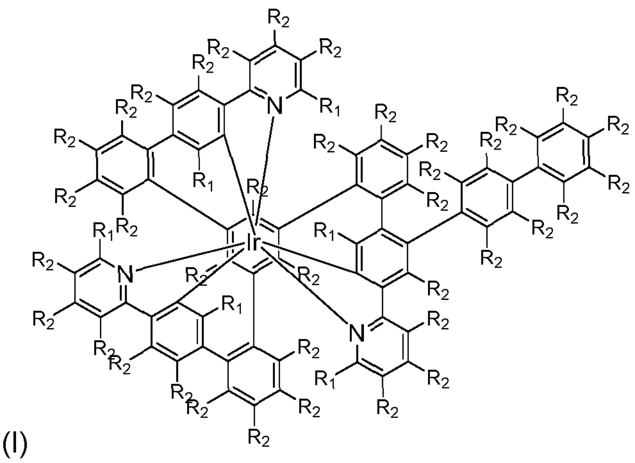

Formel (I) ,

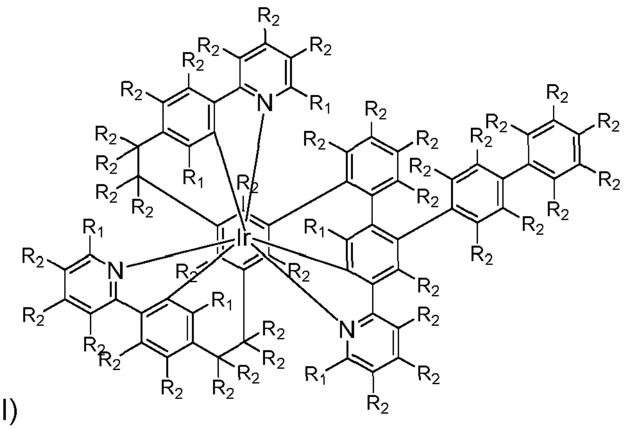

Formel (II) ,

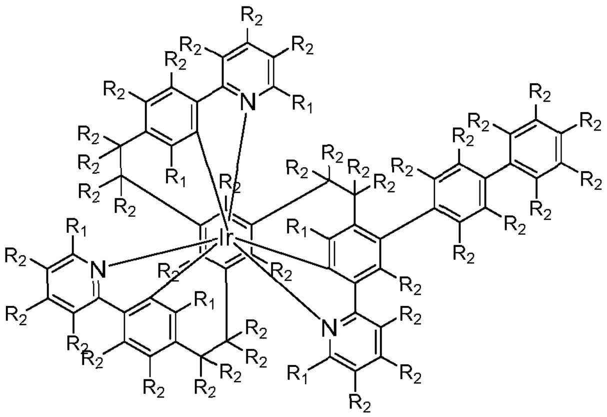

Formel (III) ,

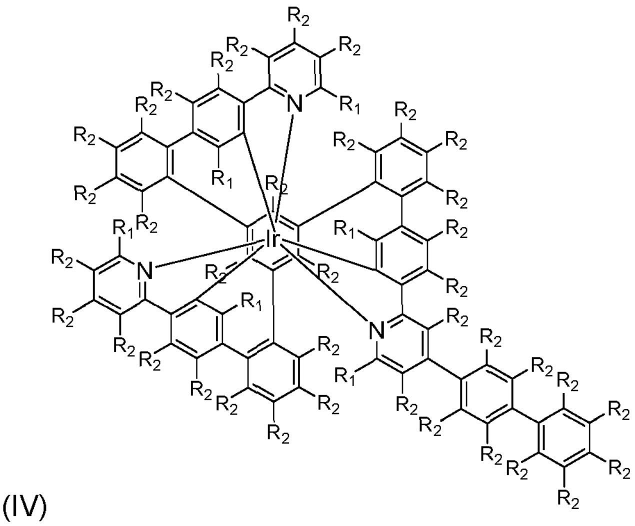

Formel (IV) ,

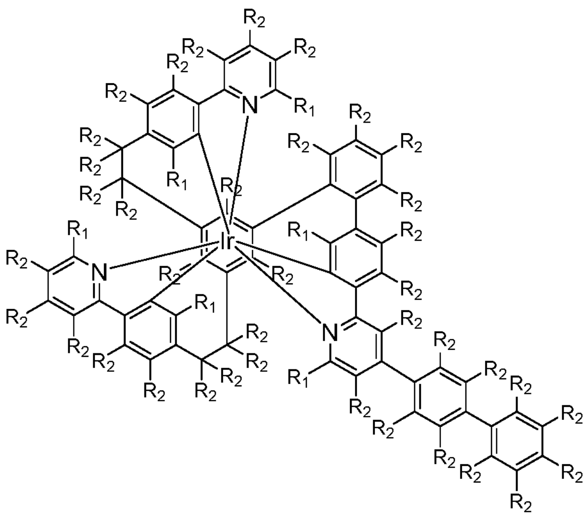

Formel (V) ,

wobei die Symbole und Indizes für diese Formeln (I), (II), (III), (IV) und (V) die Bedeutung haben:
$R_1$ ist H oder D, $R^2$ ist H, D oder eine verzweigte oder lineare Alkylgruppe mit 1 bis 10 C-Atomen oder eine teilweise oder vollständig deuterierte verzweigte oder lineare Alkylgruppe mit 1 bis 10 C-Atomen oder eine Cycloalkylgruppe mit 4 bis 10 C-Atomen, die teilweise oder vollständig mit Deuterium substituiert sein kann.

Bevorzugte phosphoreszierende Emitter gemäß der vorliegenden Erfindung entsprechen den Formeln (VI), (VII) oder (VIII),

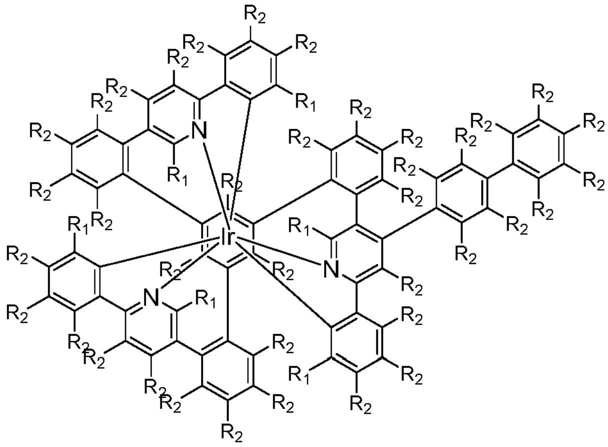

Formel (VI) ,

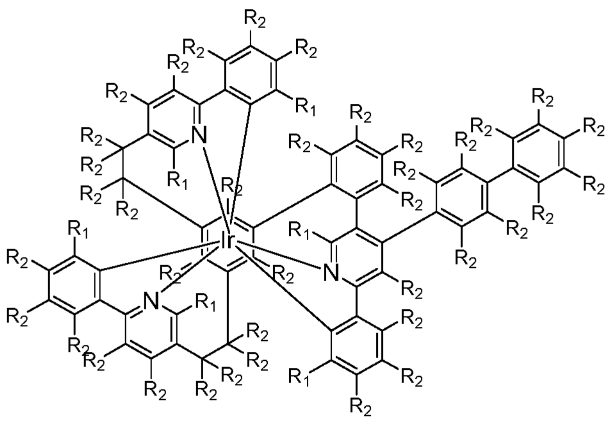

Formel (VII) ,

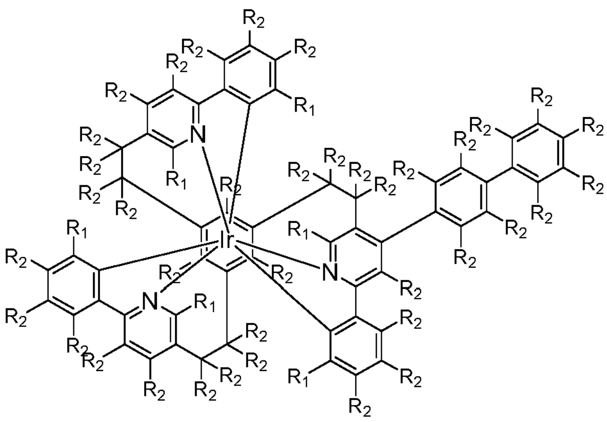

Formel (VIII) ,

wobei die Symbole und Indizes für diese Formeln (VI), (VII) und (VIII) die Bedeutung haben:
$R_1$ ist H oder D, $R^2$ ist H, D, F oder eine verzweigte oder lineare Alkylgruppe mit 1 bis 10 C-Atomen oder eine teilweise oder vollständig deuterierte verzweigte oder lineare Alkylgruppe mit 1 bis 10 C-Atomen oder eine Cycloalkylgruppe mit 4 bis 10 C-Atomen, die teilweise oder vollständig mit Deuterium substituiert sein kann.

Bevorzugte Beispiele von phosphoreszierenden Emittern sind in der folgenden Tabelle 4 aufgeführt.

Tabelle 4:

| | | |
|---|---|---|
| 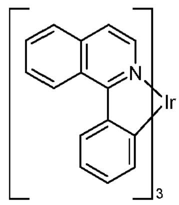 | 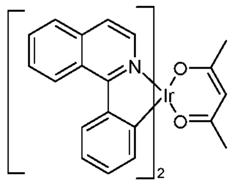 | 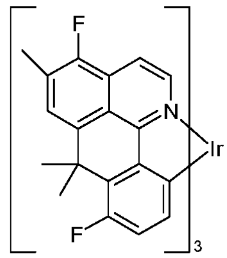 |
| 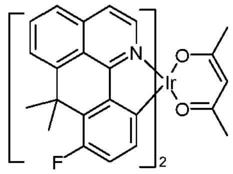 | 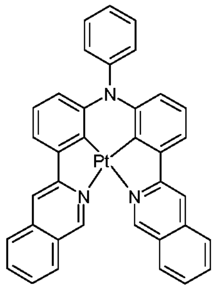 | 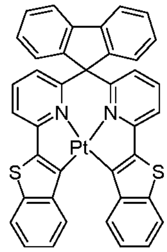 |
| 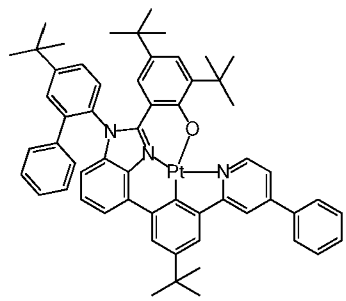 | 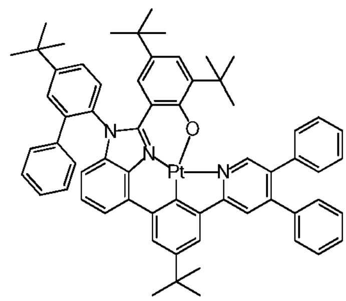 | 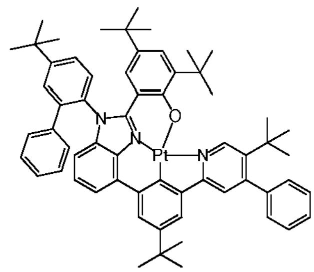 |
| 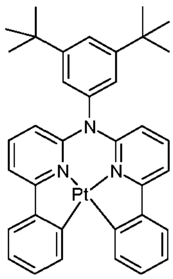 | 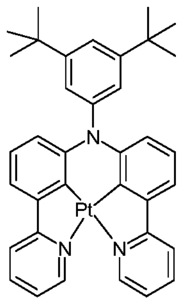 | 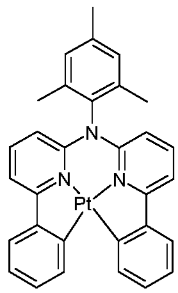 |
| 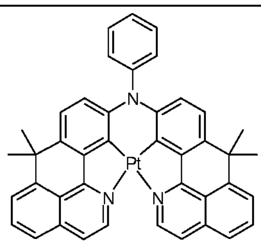 | 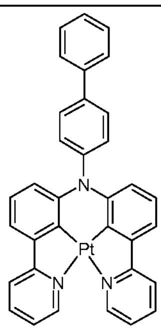 | 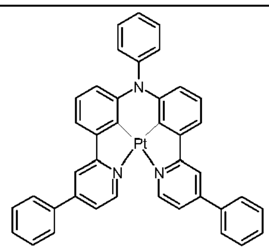 |

| | | |
|---|---|---|
| 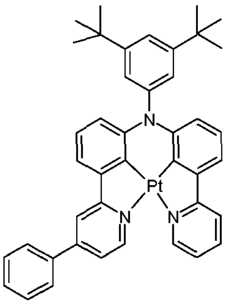 | 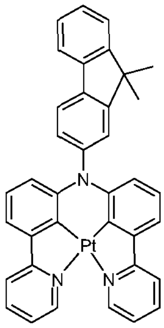 | 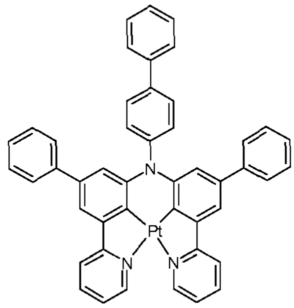 |
| 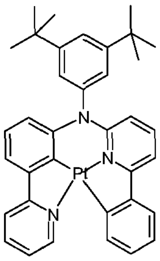 | 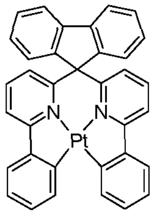 | 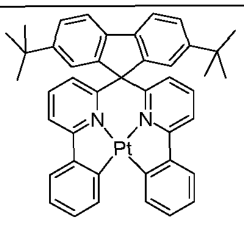 |
| 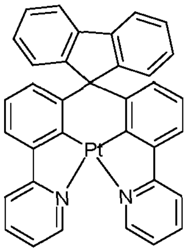 | 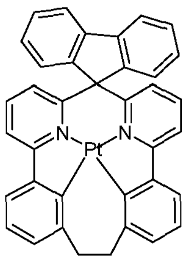 | 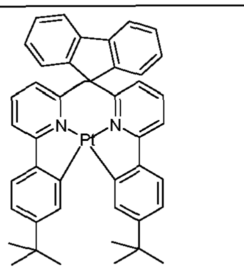 |
| 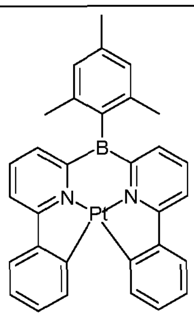 | 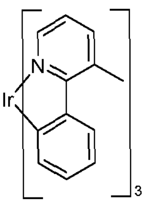 | 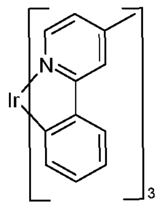 |
| 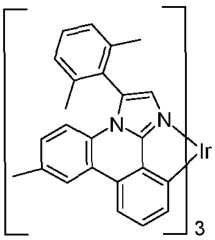 | 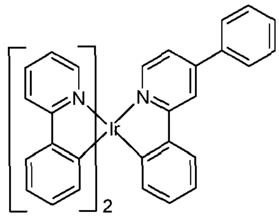 | 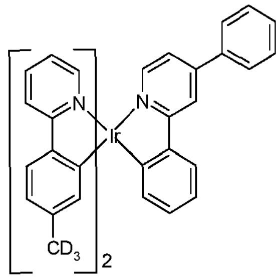 |
| 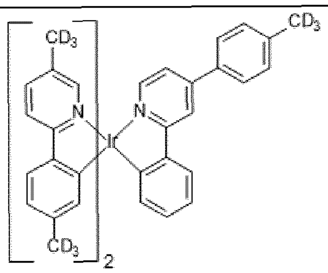 | 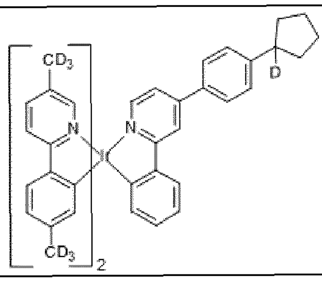 | 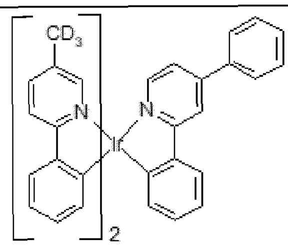 |

| | | |
|---|---|---|
| 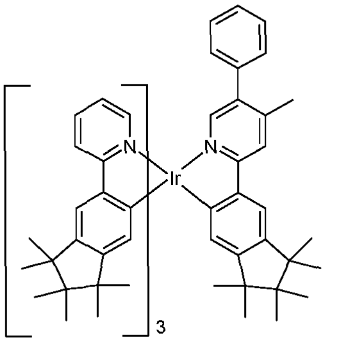 | 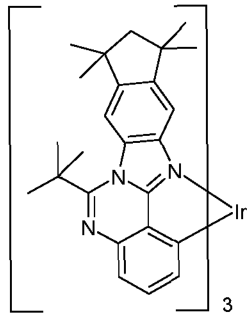 | 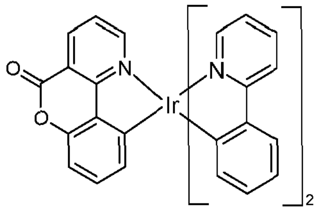 |
| 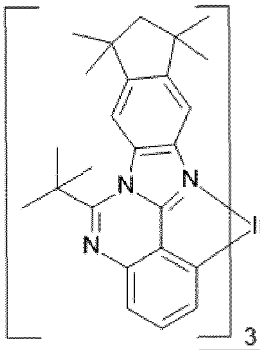 | 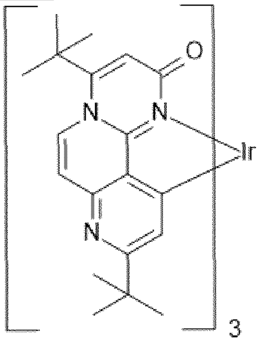 | 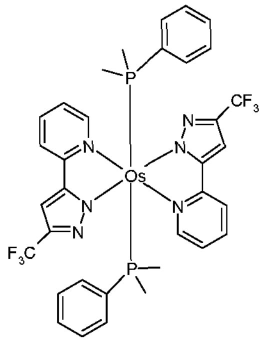 |
| 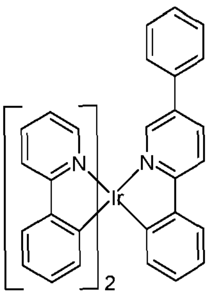 | 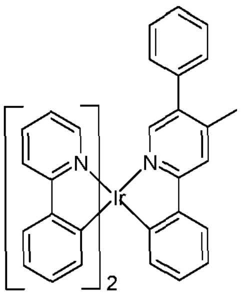 | 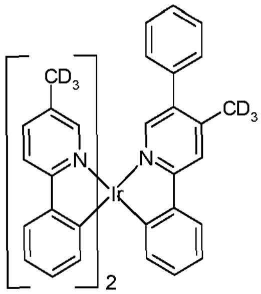 |
| 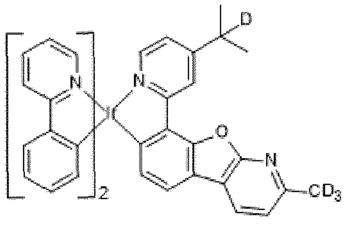 | 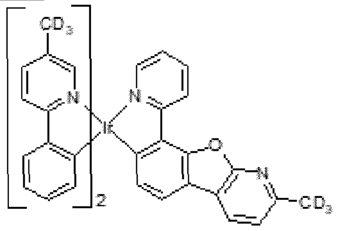 | 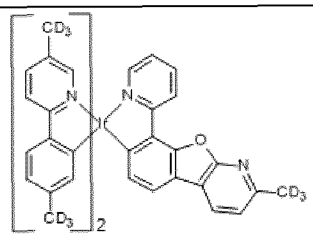 |
| 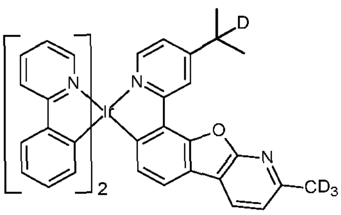 | 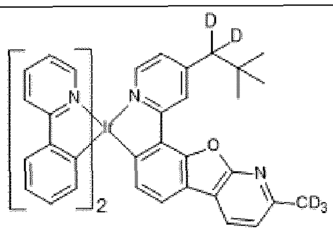 | 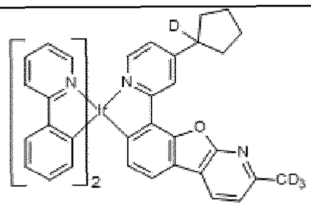 |
| 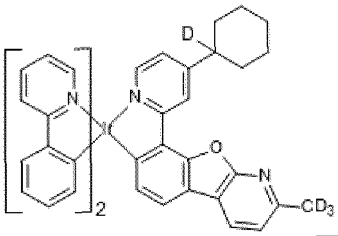 | 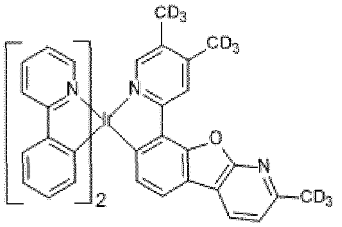 | 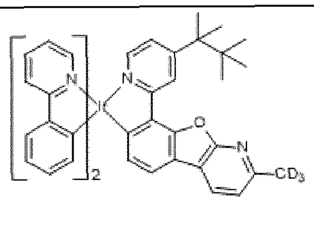 |

| | | |
|---|---|---|
| 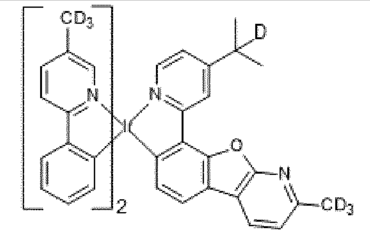 | 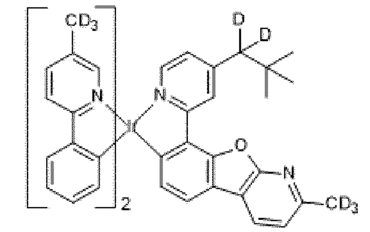 | 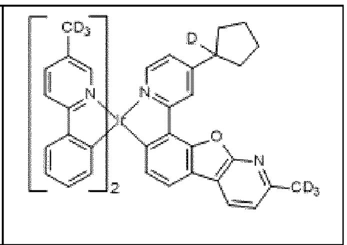 |
| 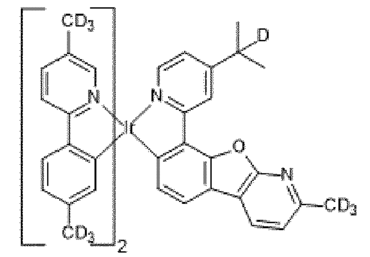 | 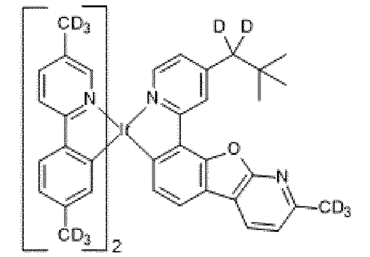 | 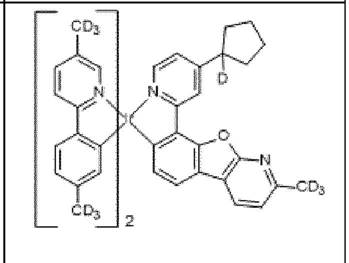 |
| 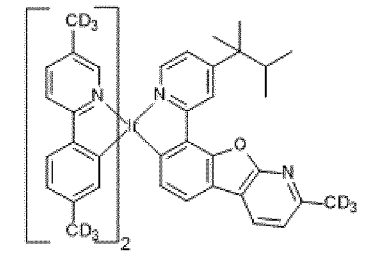 | 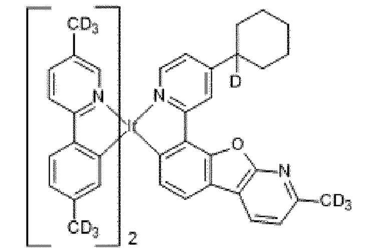 | 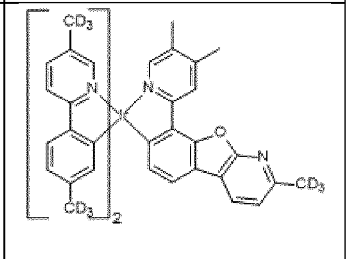 |
| 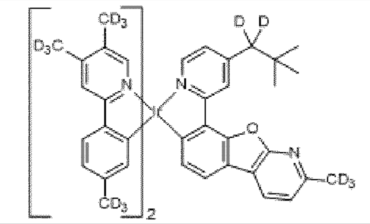 | 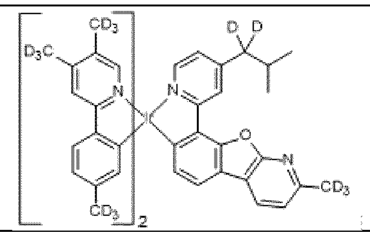 | 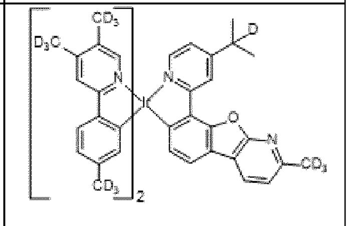 |
| 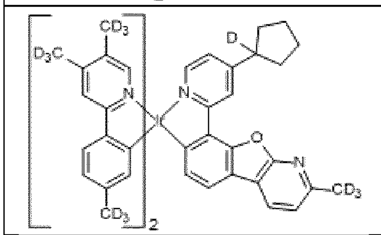 | 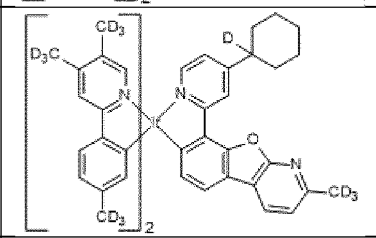 | 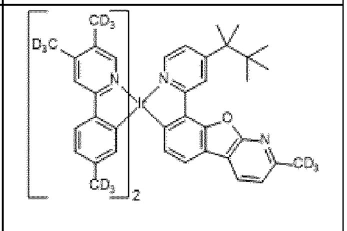 |
| 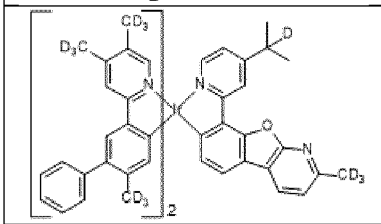 | 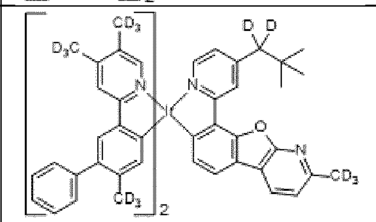 | 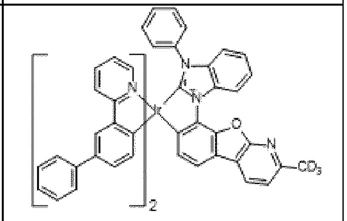 |
| 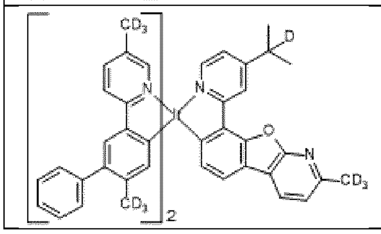 | 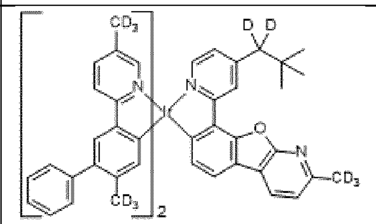 | 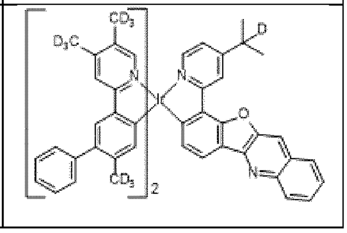 |

| | | |
|---|---|---|
| 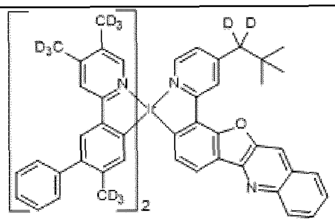 | 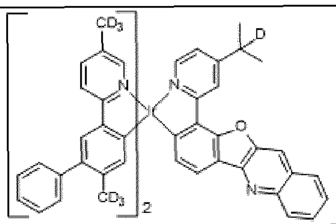 | 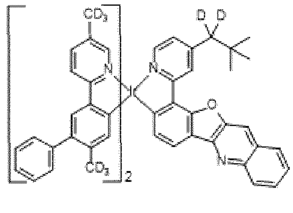 |
| 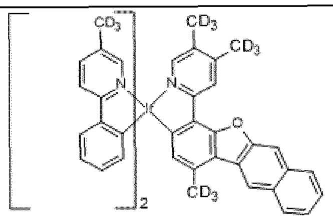 | 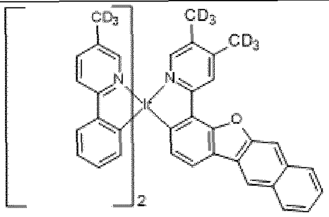 | 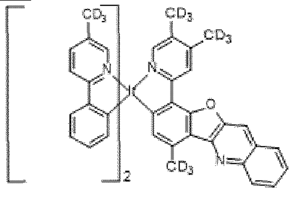 |
| 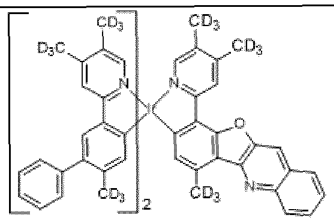 | 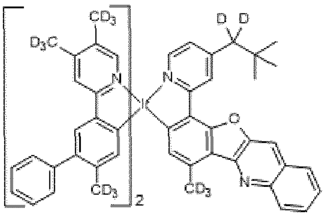 | 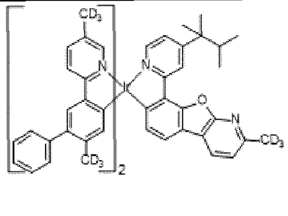 |
| 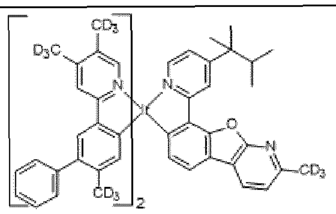 | 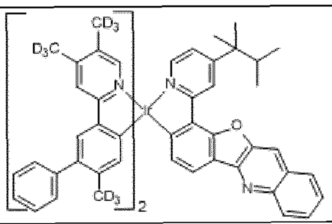 | 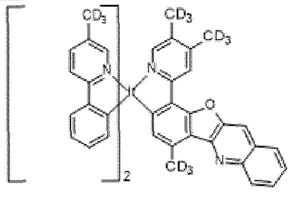 |
| 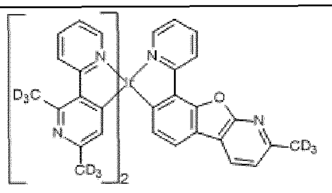 | 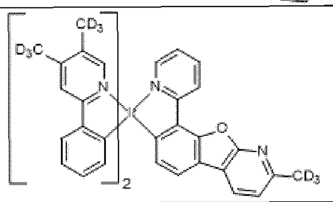 | 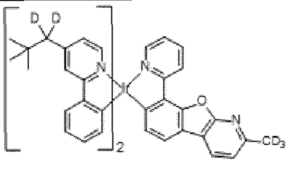 |
| 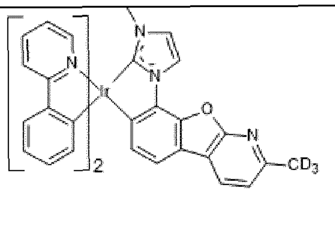 | 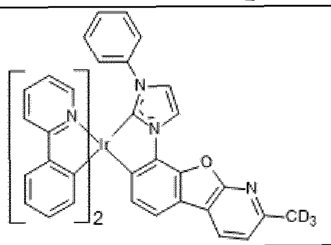 | 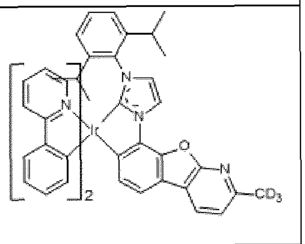 |
| 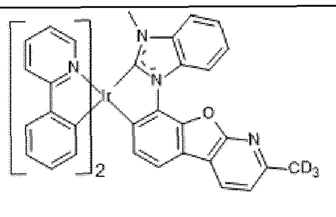 | 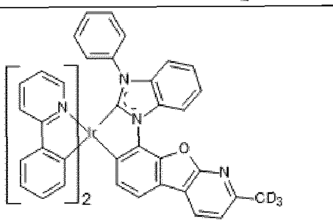 | 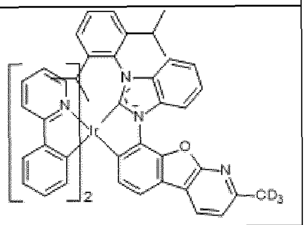 |
| 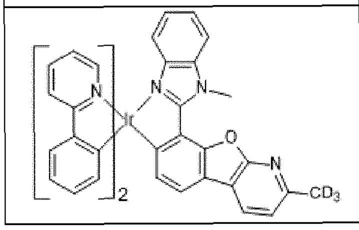 | 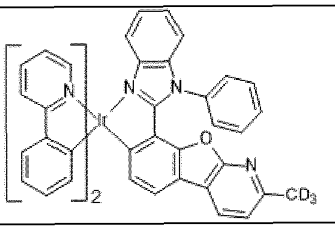 | 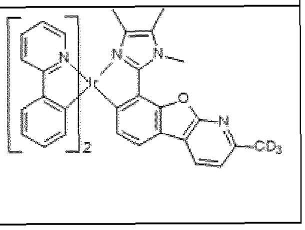 |

| | | |
|---|---|---|
| 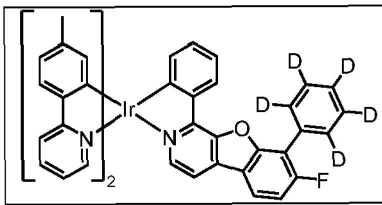 | 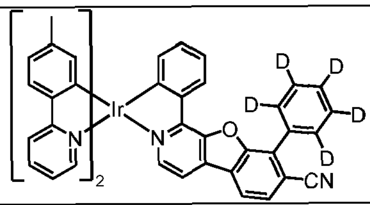 | 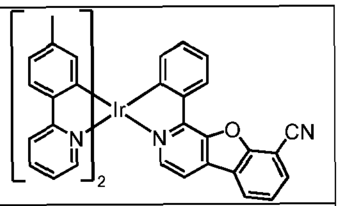 |
| 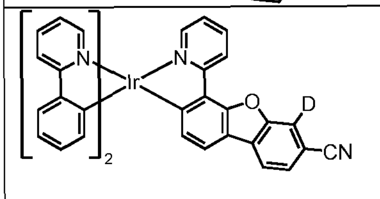 | 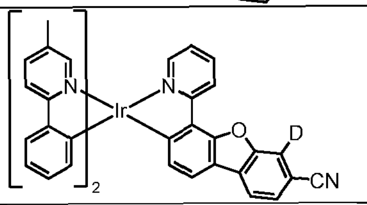 | 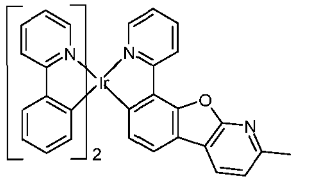 |
| 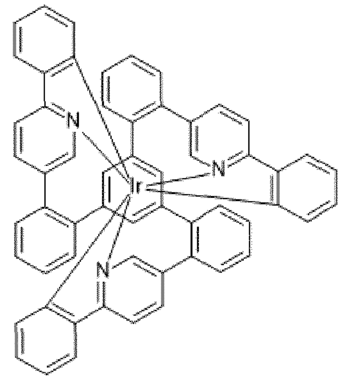 | 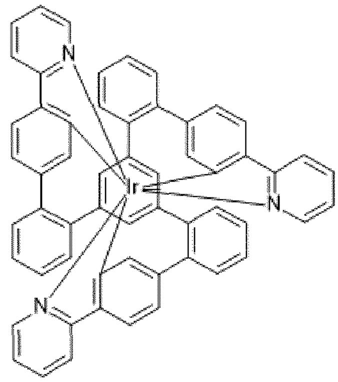 | 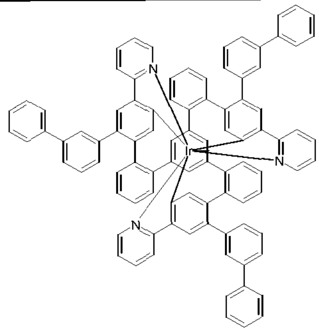 |
| 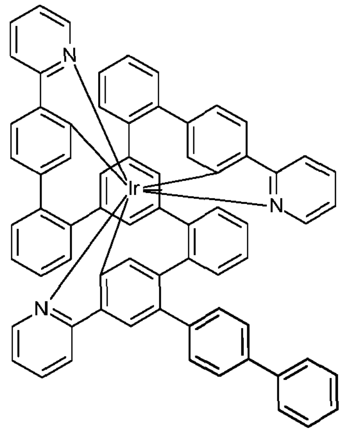 | 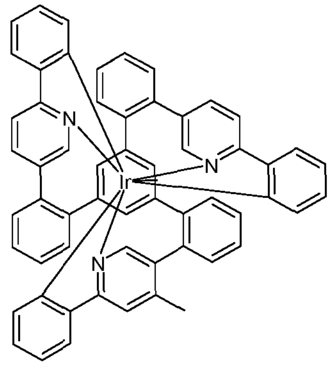 | 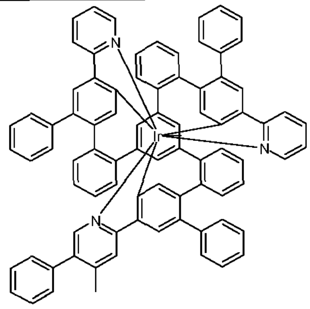 |
| 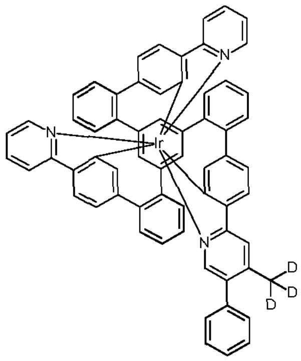 | 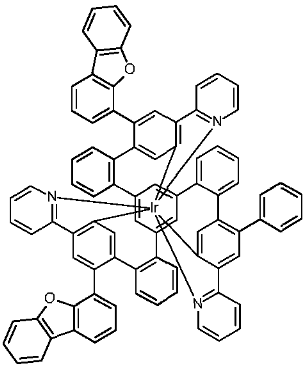 | 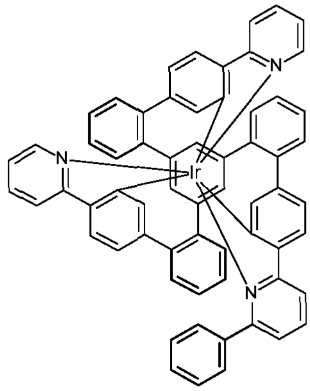 |

| | | |
|---|---|---|
| 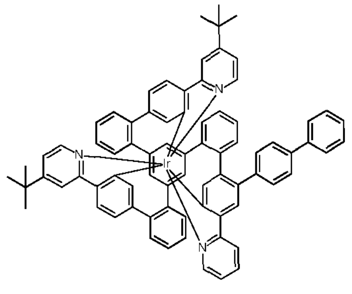 | 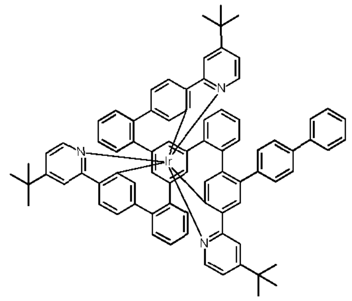 | 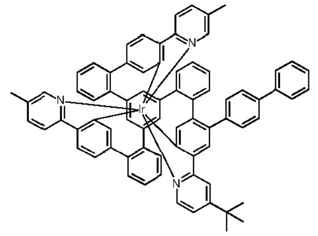 |
| 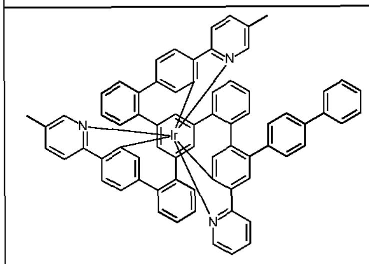 | 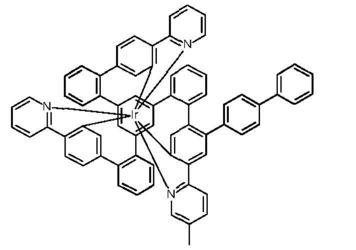 | 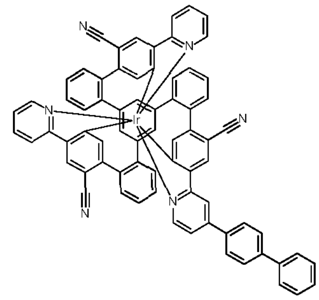 |
| 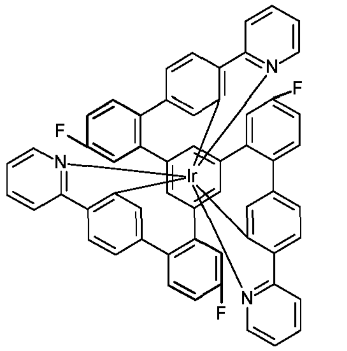 | 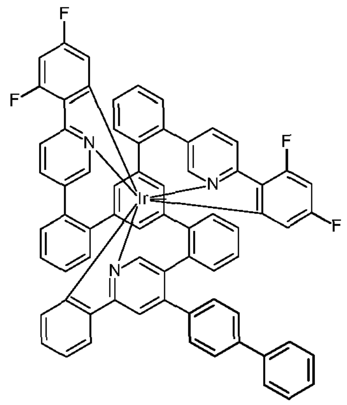 | 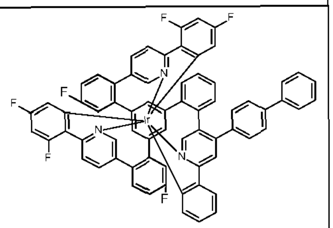 |
| 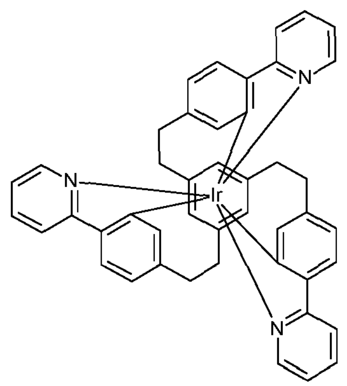 | 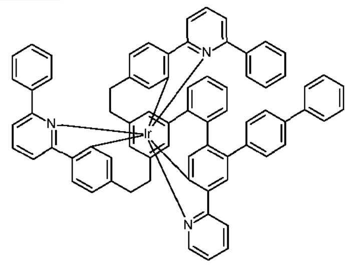 | 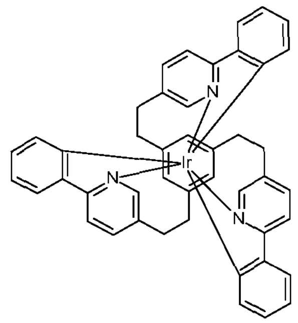 |
| 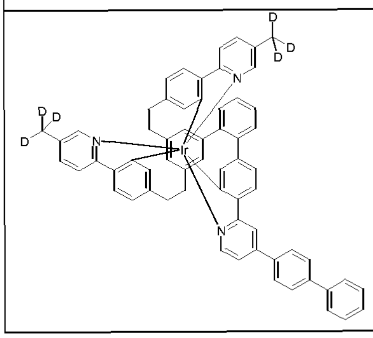 | 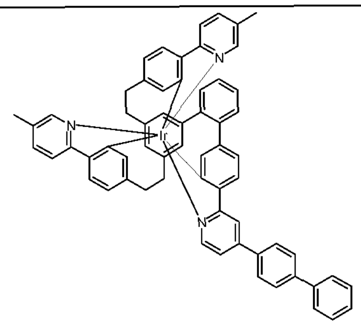 | 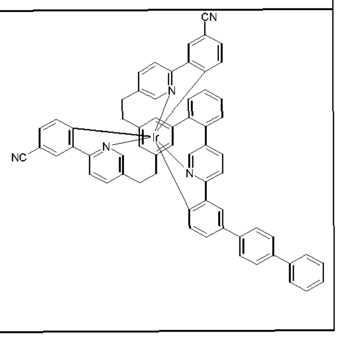 |

| | | |
|---|---|---|
| 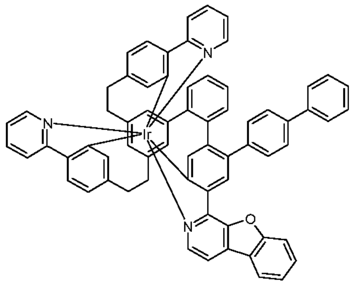 | 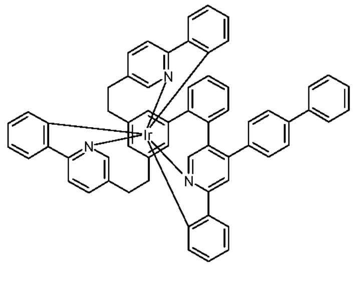 | 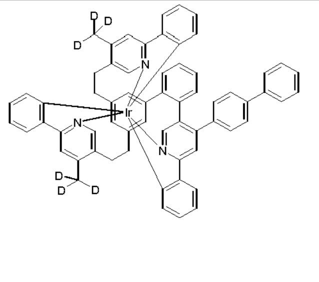 |
| 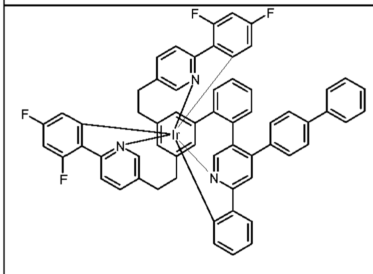 | 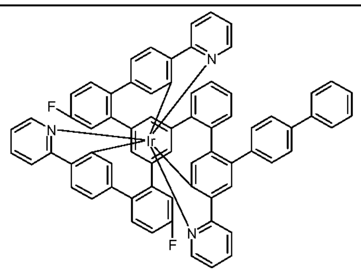 | 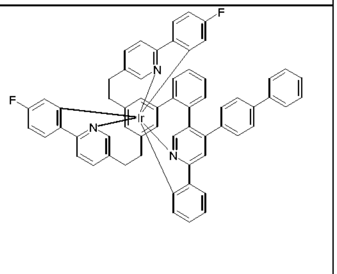 |
| 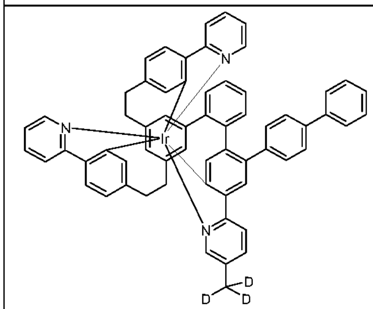 | 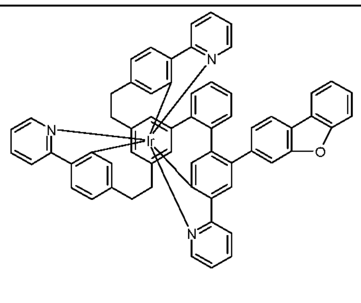 | 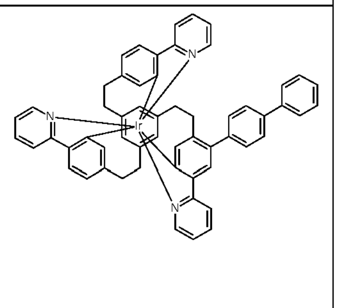 |
| 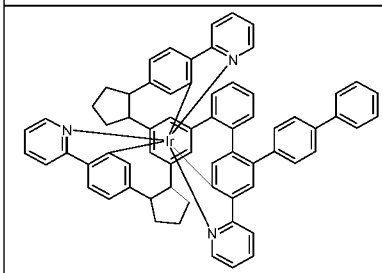 | 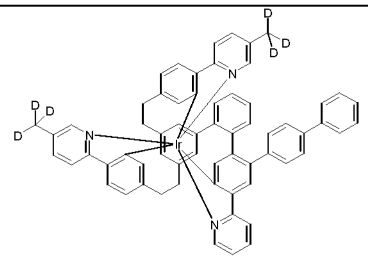 | 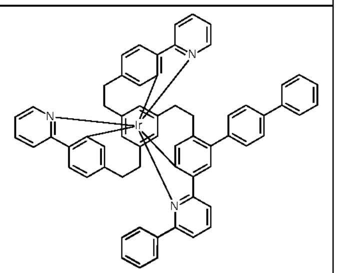 |
| 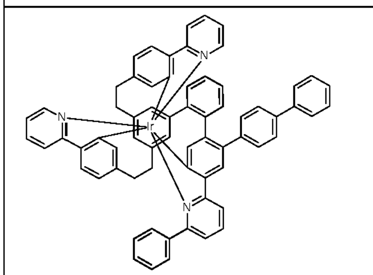 | 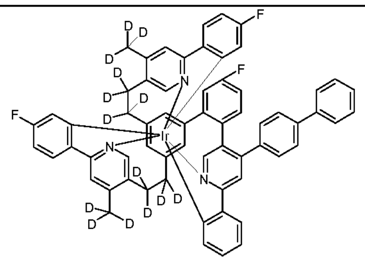 | 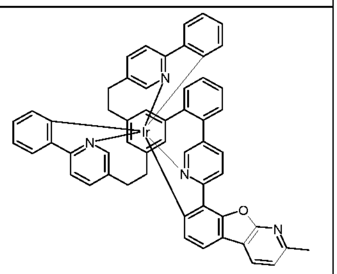 |
| 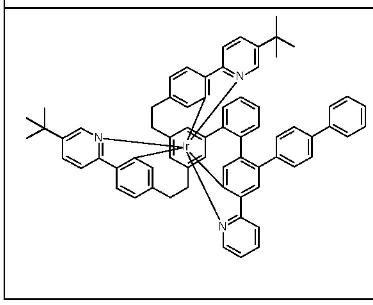 | 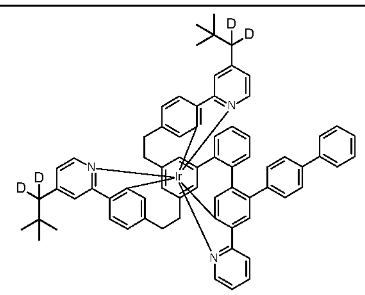 | 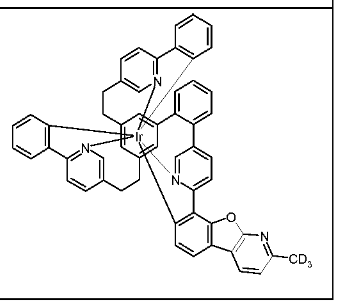 |

| | | |
|---|---|---|
| 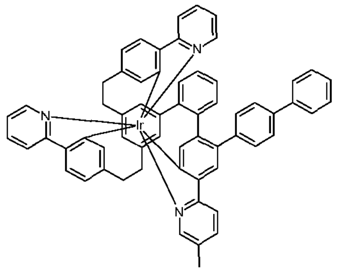 | 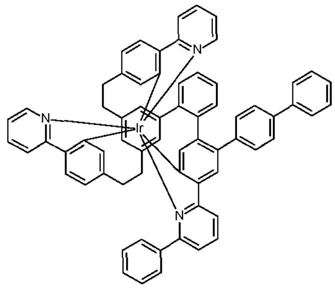 | 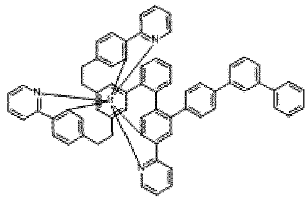 |
| 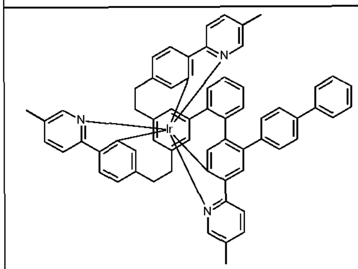 | 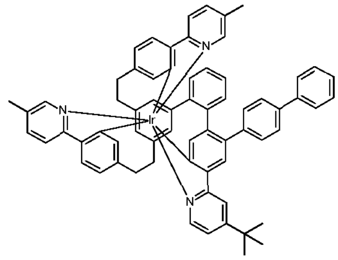 | 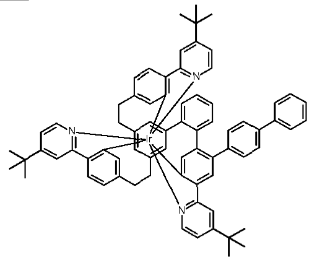 |
| 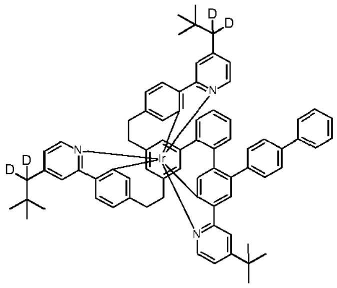 | 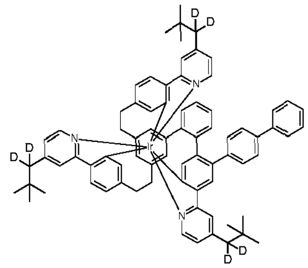 | 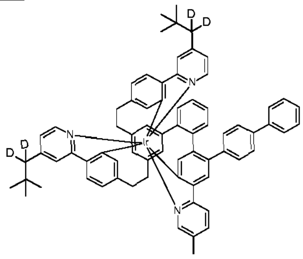 |
| 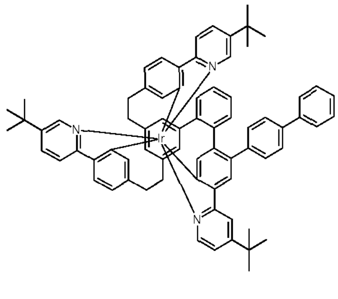 | 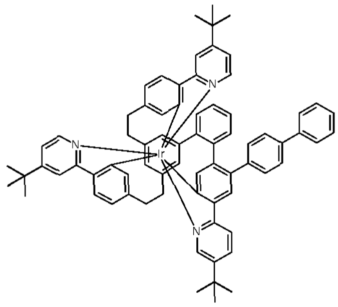 | 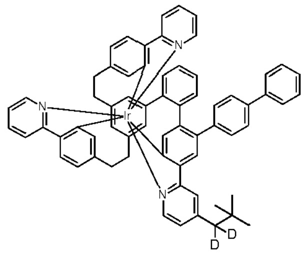 |
| 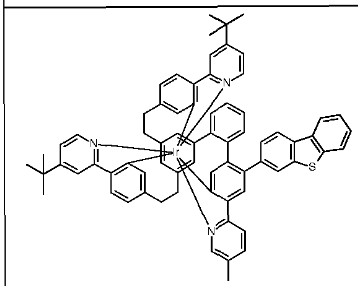 | 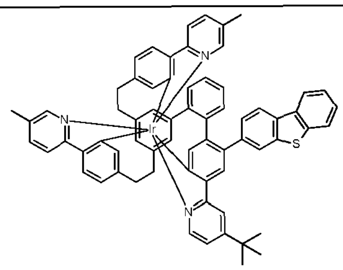 | 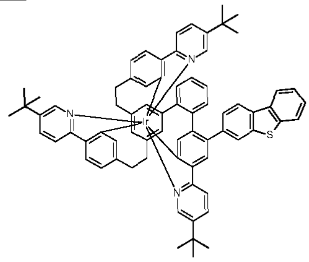 |
| 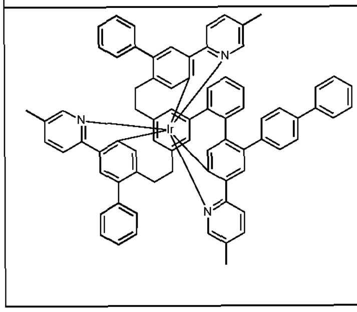 | 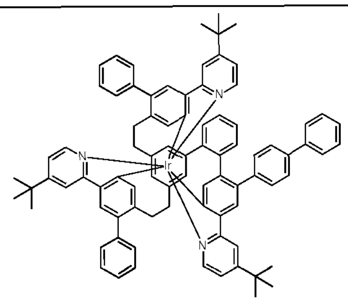 | 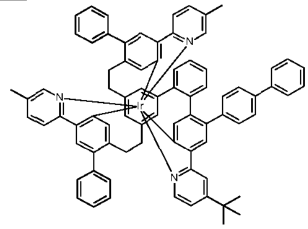 |

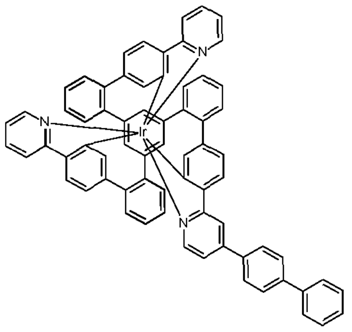

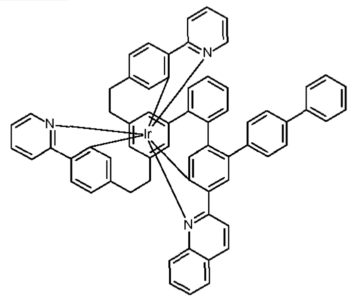

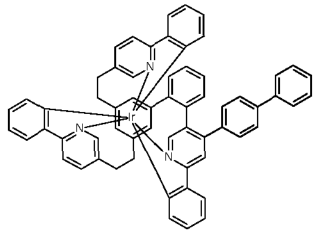

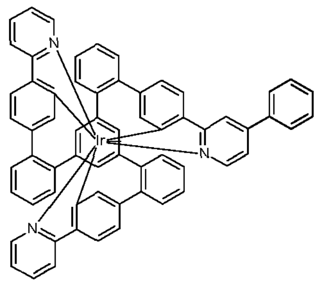

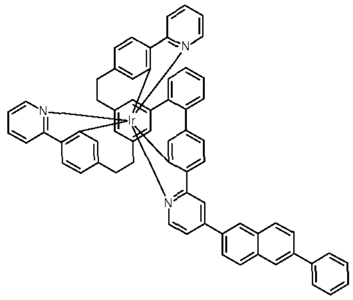

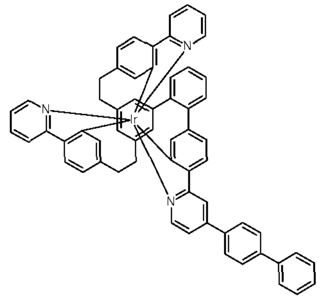

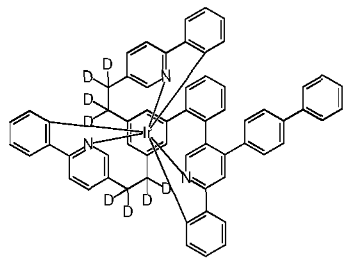

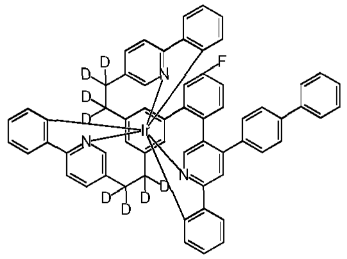

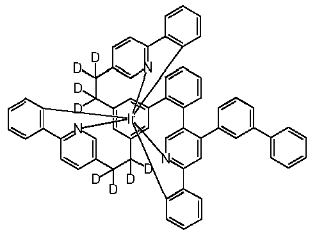

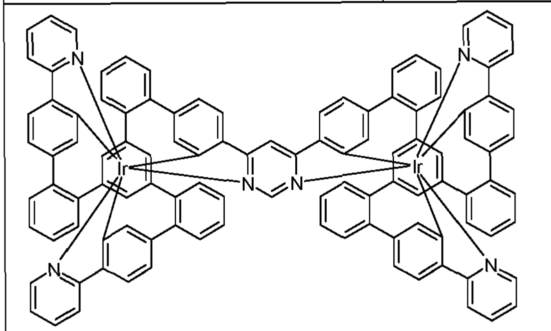

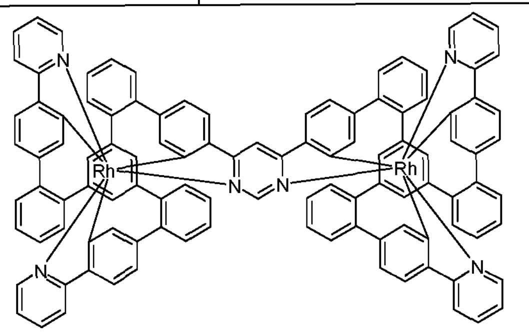

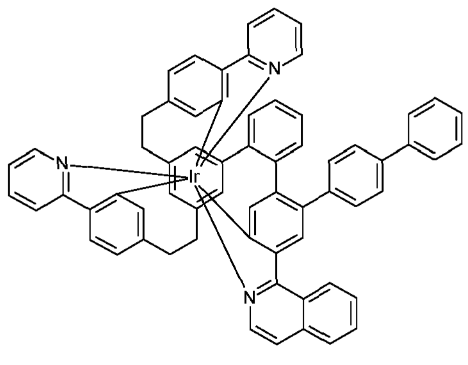

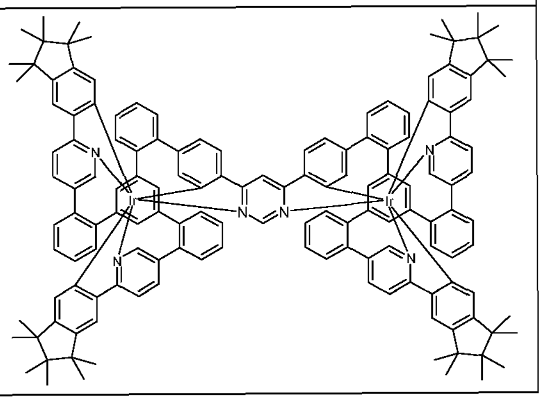

| | |
|---|---|
| 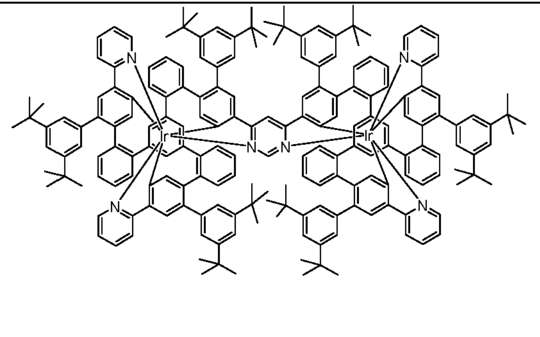 | 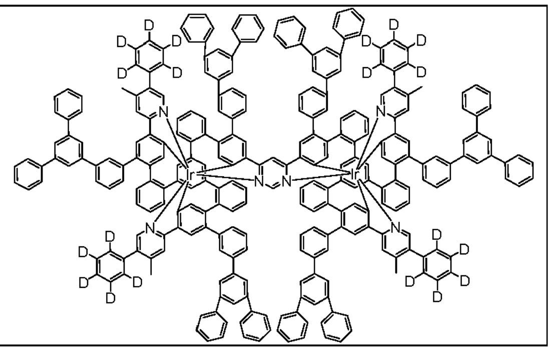 |

In den erfindungsgemäßen Mischungen oder in der lichtemittierenden Schicht der erfindungsgemäßen Vorrichtung wird bevorzugt jede Mischung ausgewählt aus der Summe der Mischungen M1 bis M729 mit einer Verbindung der Formel (3) oder einer Verbindung der Formeln (I) bis (VIII) oder einer Verbindung aus Tabelle 4 kombiniert.

Die lichtemittierende Schicht in der erfindungsgemäßen organischen elektrolumineszierenden Vorrichtung enthaltend mindestens einen phosphoreszierenden Emitter ist bevorzugt eine infra-rot emittierende, gelb, orange, rot, grün, blau oder ultraviolett emittierende Schicht, besonders bevorzugt eine gelb oder grün emittierende Schicht und ganz besonders bevorzugt eine grün emittierende Schicht.

Dabei wird unter einer gelb emittierenden Schicht eine Schicht verstanden, deren Photolumineszenzmaximum im Bereich von 540 bis 570 nm liegt. Unter einer orange emittierenden Schicht wird eine Schicht verstanden, deren Photolumineszenzmaximum im Bereich von 570 bis 600 nm liegt. Unter einer rot emittierenden Schicht wird eine Schicht verstanden, deren Photolumineszenzmaximum im Bereich von 600 bis 750 nm liegt. Unter einer grün emittierenden Schicht wird eine Schicht verstanden, deren Photolumineszenzmaximum im Bereich von 490 bis 540 nm liegt. Unter einer blau emittierenden Schicht wird eine Schicht verstanden, deren Photolumineszenzmaximum im Bereich von 440 bis 490 nm liegt. Dabei wird das Photolumineszenzmaximum der Schicht durch Messung des Photolumineszenzspektrums der Schicht mit einer Schichtdicke von 50 nm bei Raumtemperatur bestimmt, wobei die Schicht die erfindungsgemäße Kombination der Hostmaterialien der Formeln (1) und (2) und den entsprechenden Emitter enthält.

Die Aufnahme des Photolumineszenzspektrums der Schicht erfolgt beispielsweise mit einem handelsüblichen Photolumineszenzspektrometer.

Das Photolumineszenzspektrum des gewählten Emitters wird in der Regel in Sauerstofffreier Lösung, 10-5 molar, gemessen, wobei die Messung bei Raumtemperatur erfolgt und jedes Lösemittel geeignet ist, in dem sich der gewählte Emitter in der genannten Konzentration löst. Besonders geeignete Lösemittel sind üblicherweise Toluol oder 2-Methyl-THF, aber auch Dichlormethan. Gemessen wird mit einem handelsüblichen Photolumineszenzspektrometer. Die Triplettenergie T1 in eV wird aus den Photolumineszenzspektren der Emitter bestimmt. Es wird zunächst das Peakmaximum Plmax. (in nm) des Photolumineszenzspektrums bestimmt. Das Peakmaximum Plmax. (in nm) wird dann in eV umgerechnet gemäß: E(T1 in eV) = 1240/E(T1 in nm) = 1240/PLmax. (in nm).

Bevorzugte phosphoreszierende Emitter sind demzufolge gelbe Emitter, vorzugsweise der Formel (3), der Formeln (I) bis (VIII) oder aus Tabelle 4, deren Triplettenergie $T_1$ bevorzugt bei ~2.3 eV bis ~2.1 eV liegt.

Bevorzugte phosphoreszierende Emitter sind demzufolge grüne Emitter, vorzugsweise der Formel (3), der Formeln (I) bis (VIII) oder aus Tabelle 4, deren Triplettenergie $T_1$ bevorzugt bei ~2.5 eV bis ~2.3 eV liegt.

Besonders bevorzugte phosphoreszierende Emitter sind demzufolge grüne Emitter, vorzugsweise der Formel (3), der Formeln (I) bis (VIII) oder aus Tabelle 4, wie zuvor beschrieben, deren Triplettenergie $T_1$ bevorzugt bei ~2.5 eV bis ~2.3 eV liegt.

Ganz besonders bevorzugt werden grüne Emitter, vorzugsweise der Formel (3), der Formeln (I) bis (VIII) oder aus Tabelle 4, wie zuvor beschrieben, für die erfindungsgemäße Zusammensetzung bzw. erfindungsgemäße emittierende Schicht ausgewählt.

In der lichtemittierenden Schicht der erfindungsgemäßen Vorrichtung können auch fluoreszierende Emitter enthalten sein.

Bevorzugte fluoreszierende Emitter sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält.

In einer weiteren bevorzugten Ausführungsform der Erfindung kann die mindestens eine lichtemittierende Schicht der organischen elektrolumineszierenden Vorrichtung neben den Hostmaterialien 1 und 2, wie zuvor beschrieben oder als bevorzugt beschrieben, weitere Hostmaterialien oder Matrixmaterialien umfassen, sogenannte Mixed-Matrix-Systeme. Die Mixed-Matrix-Systeme umfassen bevorzugt drei oder vier verschiedene Matrixmaterialien, besonders bevorzugt drei verschiedene Matrixmaterialien (das heißt, eine weitere Matrixkomponente zusätzlich zu den Hostmaterialien 1 und 2, wie zuvor beschrieben). Besonders geeignete Matrixmaterialien, welche in Kombination als Matrixkomponente eines Mixed-Matrix-Systems verwendet werden können, sind ausgewählt aus *wide-band*-gap-Materialien, bipolaren Hostmaterialien, Elektronentransportmaterialien (ETM) und Lochtransportmaterialien (HTM).

Unter wide-band-gap-Material wird hierin ein Material im Sinne der Offenbarung von US 7,294,849 verstanden, das durch eine Bandlücke von mindestens 3.5 eV charakterisiert ist, wobei unter Bandlücke der Abstand zwischen HOMO und LUMO-Energie eines Materials verstanden wird.

Genauere Angaben zu Mixed-Matrix-Systemen sind unter anderem in der Anmeldung WO 2010/108579 enthalten. Besonders geeignete Matrixmaterialien, welche in Kombination mit den Hostmaterialien 1 und 2, wie zuvor beschrieben oder bevorzugt beschrieben, als Matrixkomponenten eines Mixed-Matrix-Systems in phosphoreszierenden oder fluoreszierenden organischen Elektrolumineszenzvorrichtungen eingesetzt werden können, sind ausgewählt aus den unten angegebenen bevorzugten Matrixmaterialien für phosphoreszierende Emitter oder den bevorzugten Matrixmaterialien für fluoreszierende Emitter, je nachdem welche Art von Emitter eingesetzt wird. Bevorzugt wird das Mixed-Matrix-System auf einen Emitter der Formel (3), der Formeln (I) bis (VIII) oder aus Tabelle 4 optimiert.

Gemäß einer Ausführungsform der vorliegenden Erfindung enthält die Mischung neben den Bestandteilen elektronentransportierendes Hostmaterial der Formel (1) und lochtransportierendes Hostmaterial der Formel (2) keine weiteren Bestandteile, das heißt, funktionellen Materialien. Es handelt sich um Materialmischungen, die als solche zur Herstellung der lichtemittierenden Schicht, verwendet werden. Man bezeichnet diese Mischungen auch als Premix-Systeme, die als einzige Materialquelle bei der Aufdampfung der Hostmaterialien für die lichtemittierende Schicht verwendet wird und die ein konstantes Mischungsverhältnis bei der Aufdampfung haben. Dadurch lässt sich auf einfache und schnelle Art und Weise das Aufdampfen einer Schicht mit gleichmäßiger Verteilung der Komponenten erreichen, ohne dass eine präzise Ansteuerung einer Vielzahl an Materialquellen notwendig ist.

Gemäß einer alternativen Ausführungsform der vorliegenden Erfindung enthält die Mischung neben den Bestandteilen elektronentransportierendes Hostmaterial der Formel (1) und lochtransportierendes Hostmaterial der Formel (2) noch den phosphoreszierenden Emitter, wie zuvor beschrieben. Bei geeignetem Mischungsverhältnis bei der Aufdampfung kann auch diese Mischung als einzige Materialquelle verwendet werden, wie zuvor beschrieben.

Die Komponenten bzw. Bestandteile der lichtemittierenden Schicht der erfindungsgemäßen Vorrichtung können somit durch Aufdampfen oder aus Lösung prozessiert werden. Die Materialkombination der Hostmaterialien 1 und 2, wie zuvor beschrieben oder bevorzugt beschrieben, gegebenenfalls mit dem phosphoreszierenden Emitter, wie zuvor beschrieben oder bevorzugt beschrieben, werden dazu in einer Formulierung bereitgestellt, die mindestens ein Lösemittel enthält. Diese Formulierungen können beispielsweise Lösungen, Dispersionen oder Emulsionen sein. Es kann bevorzugt sein, hierfür Mischungen aus zwei oder mehr Lösemitteln zu verwenden.

Ein weiterer Gegenstand der vorliegenden Erfindung ist daher eine Formulierung enthaltend eine erfindungsgemäße Mischung an Hostmaterialien 1 und 2, wie zuvor beschrieben, gegebenenfalls in Kombination mit einem phosphoreszierenden Emitter, wie zuvor beschrieben oder bevorzugt beschrieben, und mindestens ein Lösemittel.

Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Die Formulierung kann dabei auch mindestens eine weitere organische oder anorganische Verbindung enthalten, die ebenfalls in der lichtemittierenden Schicht der erfindungsgemäßen Vorrichtung eingesetzt wird, insbesondere eine weitere emittierende Verbindung und/oder ein weiteres Matrixmaterial. Geeignete emittierende Verbindungen und weitere Matrixmaterialien wurden vorstehend bereits aufgeführt.

Die lichtemittierende Schicht in der erfindungsgemäßen Vorrichtung gemäß den bevorzugten Ausführungsformen und der emittierenden Verbindung enthält vorzugsweise zwischen 99,9 und 1 Vol.-%, weiter vorzugsweise zwischen 99 und 10 Vol.-%, besonders bevorzugt zwischen 98 und 60 Vol.-%, ganz besonders bevorzugt zwischen 97 und 80 Vol.-% an Matrixmaterial aus mindestens einer Verbindung der Formel (1) und mindestens einer Verbindung der Formel (2) gemäß den bevorzugten Ausführungsformen, bezogen auf die gesamte Zusammensetzung aus Emitter und Matrixmaterial. Entsprechend enthält die lichtemittierende Schicht in der erfindungsgemäßen Vorrichtung vorzugsweise zwischen 0,1 und 99 Vol.-%, weiter vorzugsweise zwischen 1 und 90 Vol.-%, besonders bevorzugt zwischen 2 und 40 Vol.-%, ganz besonders bevorzugt zwischen 3 und 20 Vol.-% des Emitters bezogen auf die gesamte Zusammensetzung der aus Emitter und Matrixmaterial bestehenden lichtemittierenden Schicht. Werden die Verbindungen aus Lösung verarbeitet, so werden statt der oben angegebenen Mengen in Vol.-% bevorzugt die entsprechenden Mengen in Gew.-%

verwendet.

Die lichtemittierende Schicht in der erfindungsgemäßen Vorrichtung gemäß den bevorzugten Ausführungsformen und der emittierenden Verbindung enthält vorzugsweise das Matrixmaterial der Formel (1) und das Matrixmaterial der Formel (2) in einem Volumenprozentverhältnis zwischen 3:1 und 1:3, bevorzugt zwischen 1:2.5 und 1:1, besonders bevorzugt zwischen 1:2 und 1:1. Werden die Verbindungen aus Lösung verarbeitet, so werden statt des oben angegebenen Verhältnisses in Vol.-% bevorzugt das entsprechende Verhältnis in Gew.-% verwendet.

Die vorliegende Erfindung betrifft auch eine organische elektrolumineszierende Vorrichtung, wie zuvor beschrieben oder bevorzugt beschrieben, wobei die organische Schicht eine Lochinjektionsschicht (HIL) und/oder eine Lochtransportschicht (HTL) enthält, deren lochinjizierendes Material und lochtransportierendes Material ein Monoamin ist, welches keine Carbazoleinheit enthält. Bevorzugt enthält das lochinjizierendes Material und lochtransportierendes Material ein Monoamin enthaltend eine Fluorenyl- oder Bispirofluorenylgrupe, jedoch keine Carbazoleinheit.

Bevorzugte Monoamine, die erfindungsgemäß in der organischen Schicht der erfindungsgemäßen Vorrichtung eingesetzt werden, können durch die Formel (4) beschrieben werden

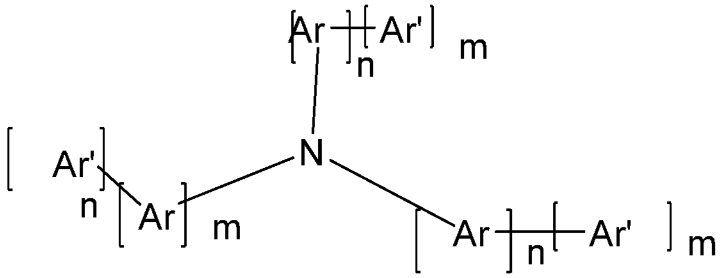

Formel (4),

wobei die Symbole und Indizes für diese Formel (4) die Bedeutung haben:

Ar und Ar' sind jeweils unabhängig bei jedem Auftreten ein aromatisches Ringsystem mit 6 bis 40 Ringatomen oder ein heteroaromatisches Ringsystem mit 7 bis 40 Ringatomen,

wobei Carbazoleinheiten im heteroaromatischen Ringsystem ausgeschlossen sind;
n ist jeweils unabhängig bei jedem Auftreten 0 oder 1;
m ist jeweils unabhängig bei jedem Auftreten 0 oder 1.

Bevorzugt ist mindestens ein Ar' in Formel (4) eine Gruppe der folgenden Formeln (4a) oder (4b)

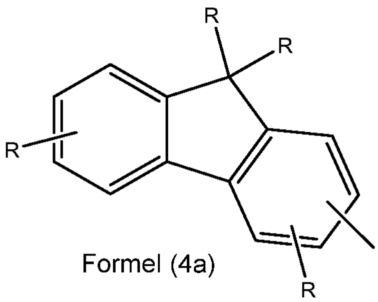

Formel (4a)

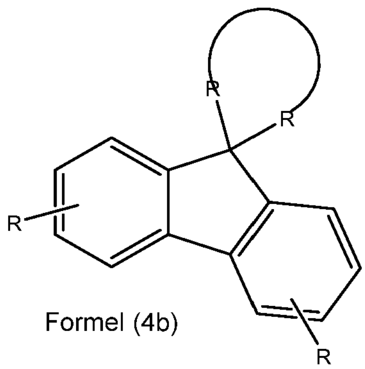

Formel (4b)

wobei R in Formel (4a) und (4b) bei jedem Auftreten gleich oder verschieden ausgewählt sind aus H, D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch $R^2C=CR^2$, O oder S ersetzt sein können und wobei ein oder mehrere H-Atome durch D, F, oder CN ersetzt sein können und wobei zwei R einen cyclischen oder polycyclischen Ring bilden können und * die Anbindung an den Rest der Formel (4) bezeichnet.

Bevorzugte Monoamine, die erfindungsgemäß in der organischen Schicht der erfindungsgemäßen Vorrichtung eingesetzt werden, sind in Tabelle 5 beschrieben.

Tabelle 5:

| | | |
|---|---|---|
| 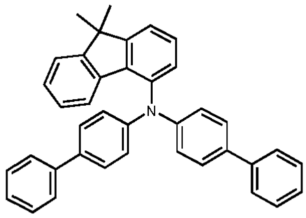 | 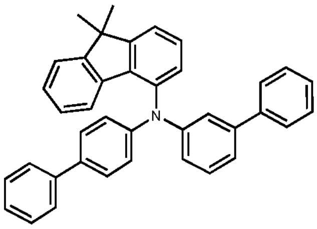 | 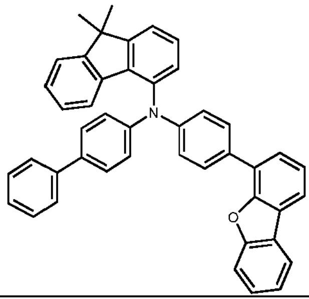 |
| 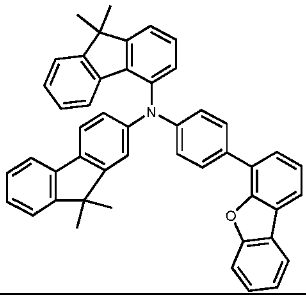 | 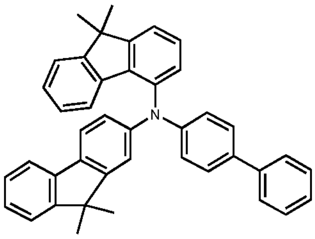 | 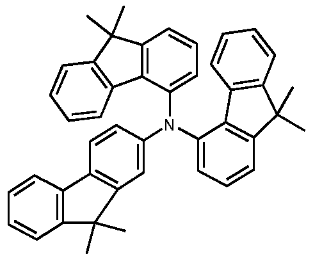 |
| 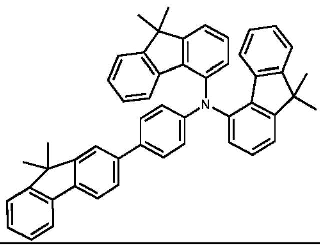 | 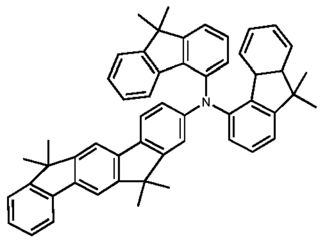 | 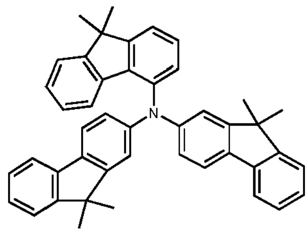 |
| 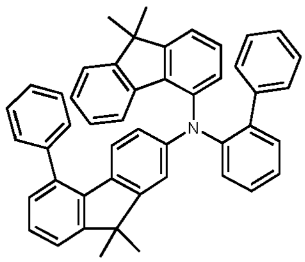 | 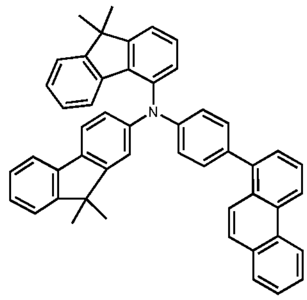 | 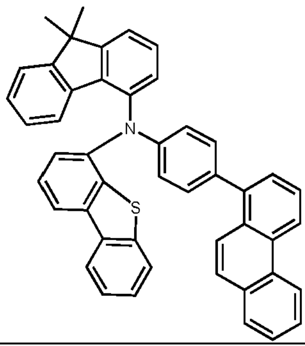 |
| 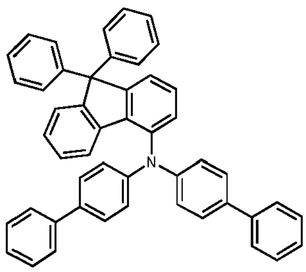 | 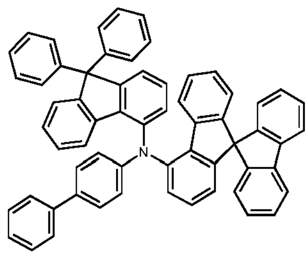 | 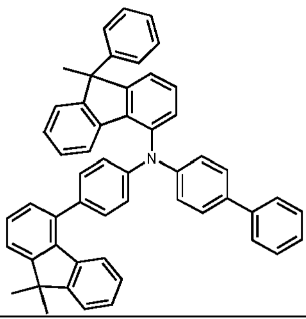 |

| | | |
|---|---|---|
| 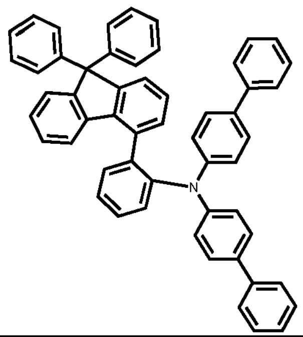 | 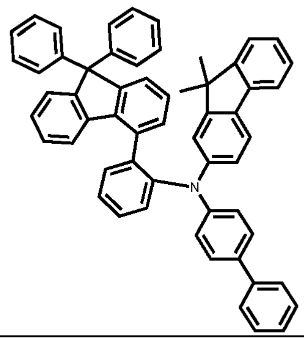 | 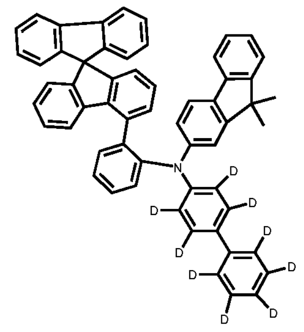 |
| 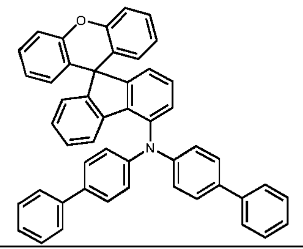 | 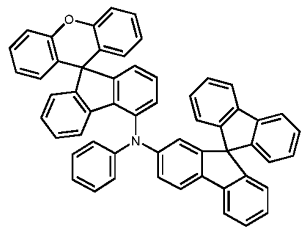 | 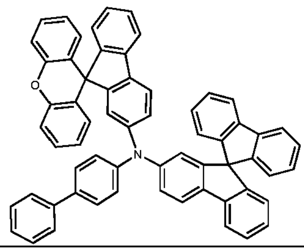 |
| 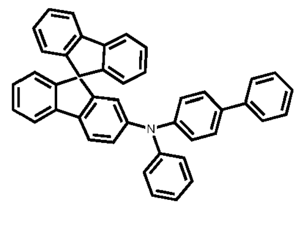 | 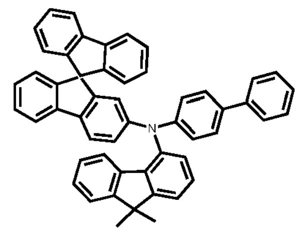 | 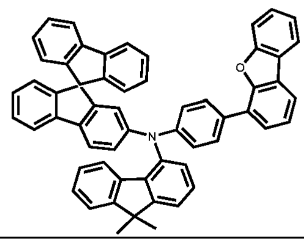 |
| 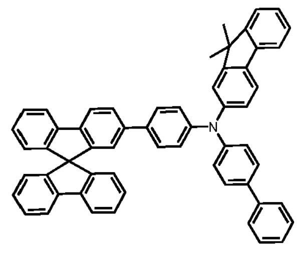 | 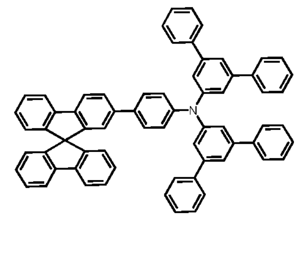 | 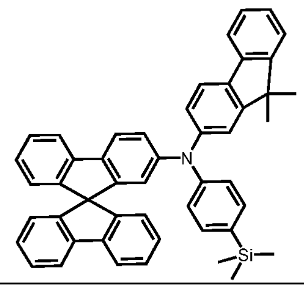 |
| 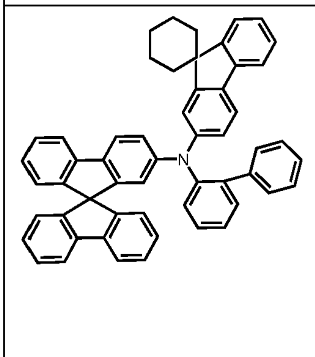 | 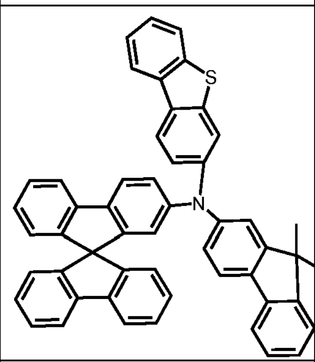 | 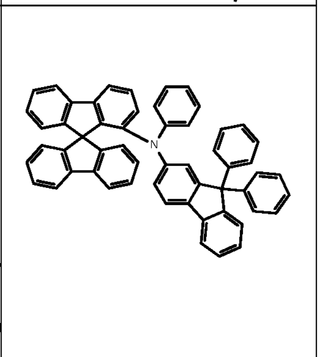 |
| 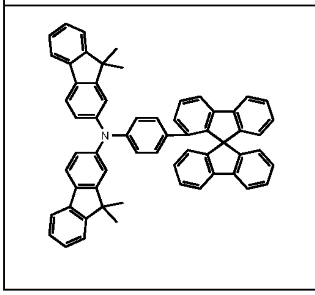 | 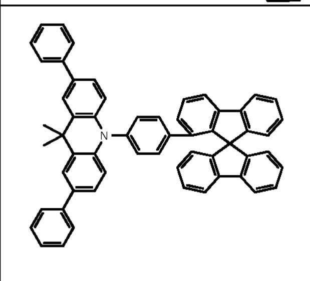 | 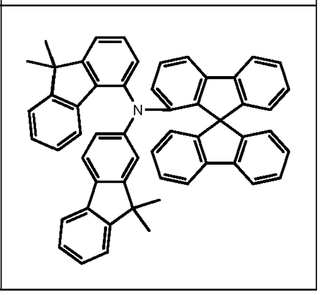 |

| | | |
|---|---|---|
| 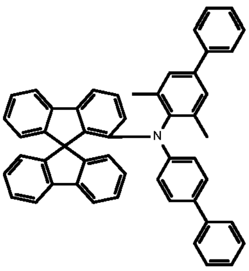 | 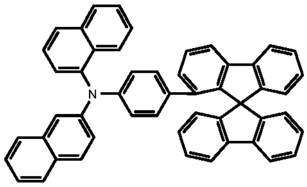 | 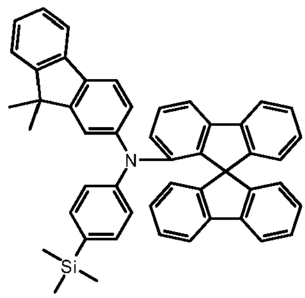 |
| 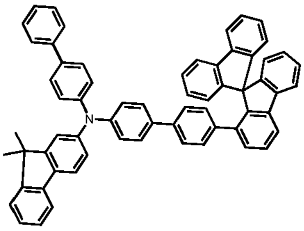 | 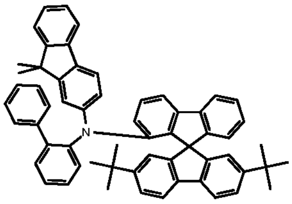 | 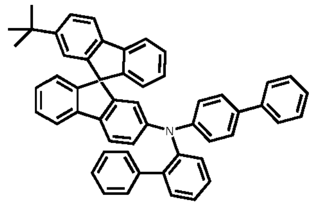 |
| 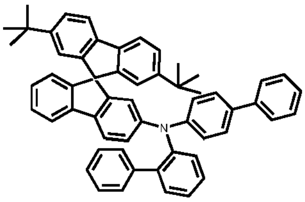 | 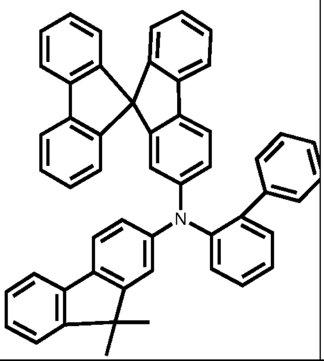 | 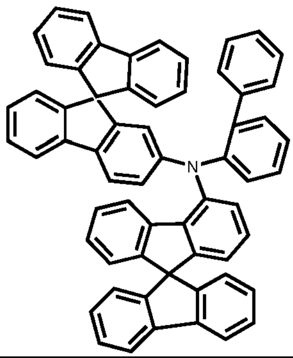 |
| 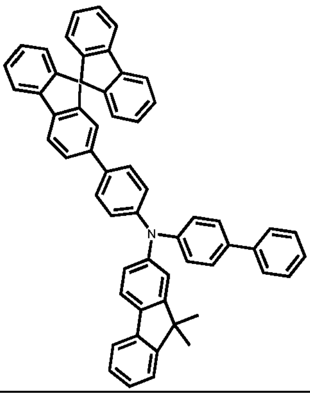 | 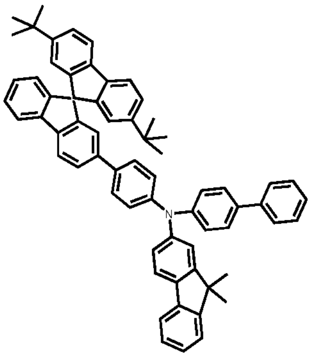 | 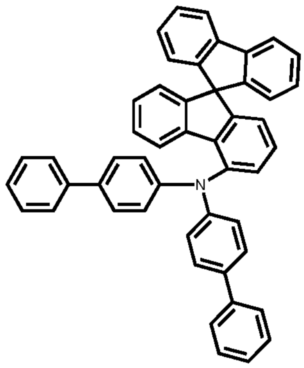 |
| 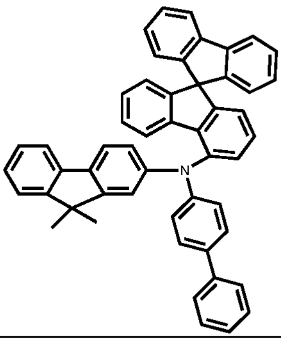 | 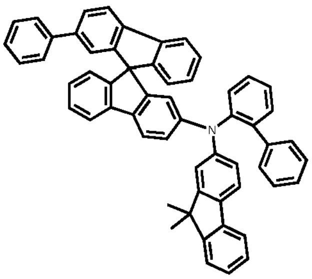 | 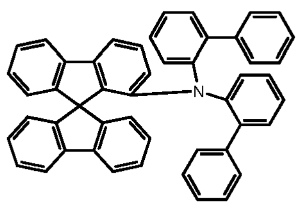 |

| | | |
|---|---|---|
| 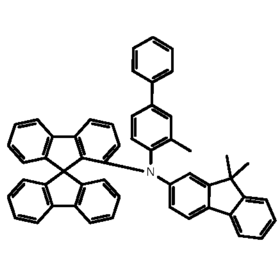 | 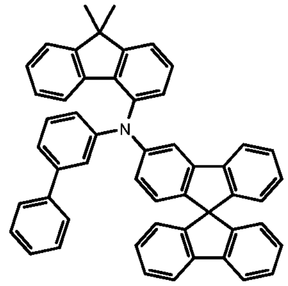 | 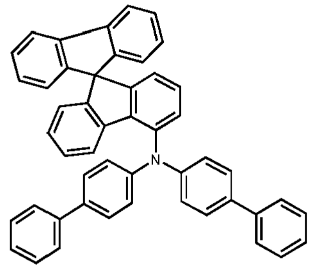 |
| 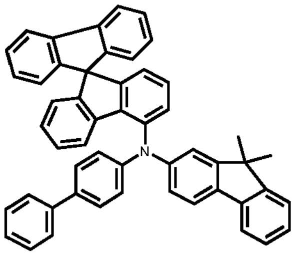 | 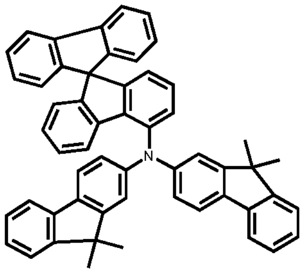 | 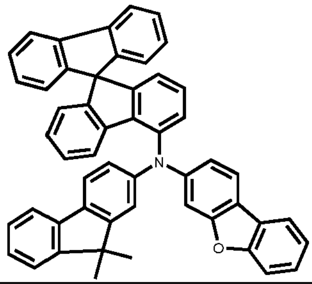 |
| 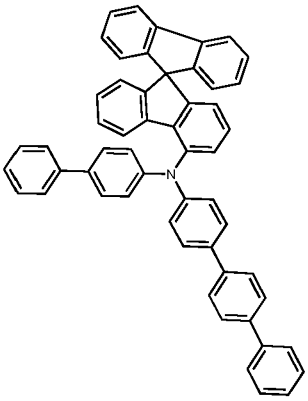 | 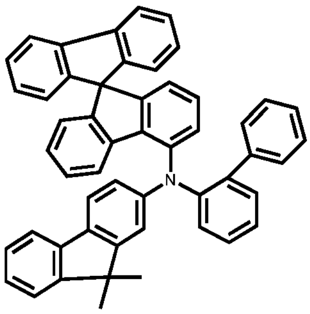 | 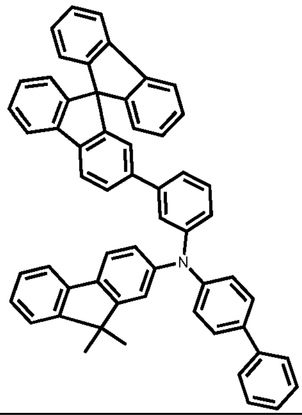 |
| 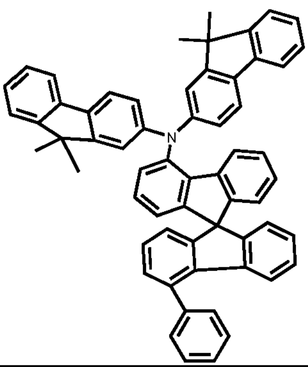 | 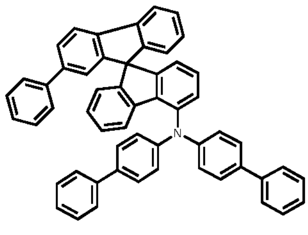 | 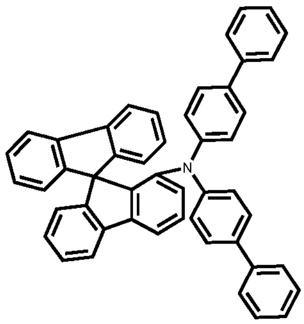 |
| 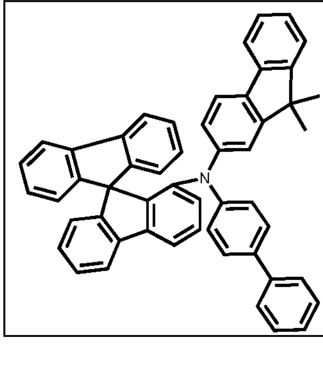 | 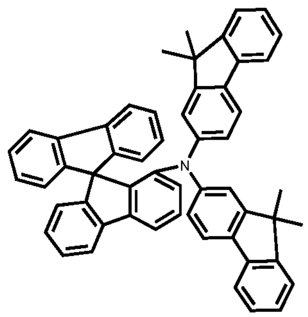 | 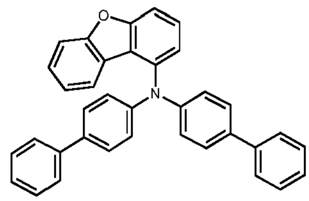 |

| | | |
|---|---|---|
| 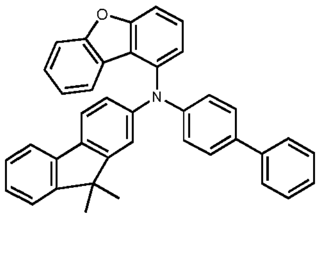 | 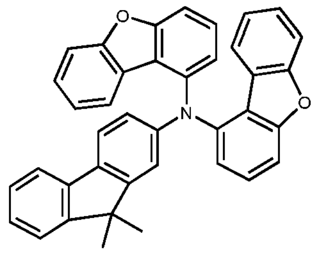 | 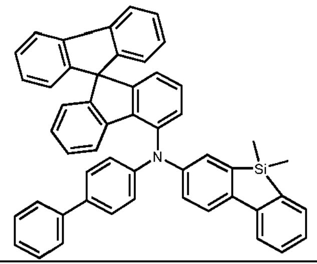 |
| 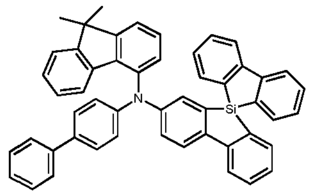 | 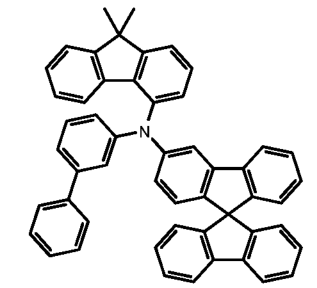 | 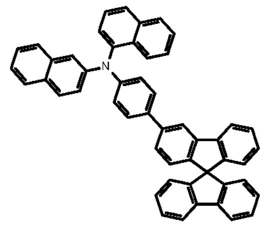 |
| 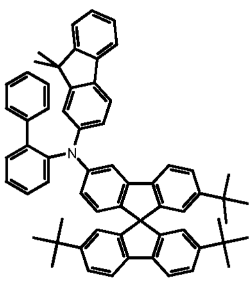 | 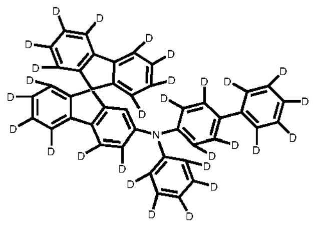 | |

Als Lochtransportmaterialien sind weiterhin in Kombination mit den Verbindungen der Tabelle 5 oder als Alternativen für Verbindungen der Tabelle 5 auch Materialien bevorzugt, die in einer Lochtransport-, Lochinjektions- oder Elektronenblockierschicht verwendet werden können, wie Indenofluorenamin-Derivate, Hexaazatriphenylenderivate, Monobenzoindenofluorenamine, Dibenzoindenofluorenamine, Dihydroacridin-Derivate.

Die Abfolge der Schichten in der erfindungsgemäßen organischen Elektrolumineszenzvorrichtung ist bevorzugt die folgende:

Anode / Lochinjektionsschicht / Lochtransportschicht / emittierende Schicht / Elektronentransportschicht / Elektroneninjektionsschicht / Kathode.

Diese Abfolge der Schichten ist eine bevorzugte Abfolge.

Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Mindestens eine der emittierenden Schichten ist die erfindungsgemäße lichtemittierende Schicht enthaltend mindestens eine Verbindung der Formel (1) als Hostmaterial 1 und mindestens eine Verbindung der Formel (2) als Hostmaterial 2, wie zuvor beschrieben. Besonders bevorzugt weisen diese Emissionsschichten in diesem Fall insgesamt mehrere Emissionsmaxima zwischen 380 nm und 750 nm auf, so dass insgesamt weiße Emission resultiert. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise $Alq_3$, Zirkoniumkomplexe, beispielsweise $Zrq_4$, Benzimidazolderviate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate.

Als Kathode der erfindungsgemäßen Vorrichtung eignen sich Metalle mit geringer Austrittsarbeit, Metalllegierungen oder mehrlagige Strukturen aus verschiedenen Metallen, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. $Al/Ni/NiO_X$, $Al/PtO_X$) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die erfindungsgemäße organische elektrolumineszierende Vorrichtung wird bei der Herstellung entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

Die Herstellung der erfindungsgemäßen Vorrichtung ist hierbei nicht eingeschränkt. Es ist möglich, dass eine oder mehrere organische Schichten, auch die lichtemittierende Schicht, mit einem Sublimationsverfahren beschichtet werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Dabei ist es jedoch auch möglich, dass der Anfangsdruck noch geringer ist, beispielsweise kleiner $10^{-7}$ mbar.

Bevorzugt ist die erfindungsgemäße organische Elektrolumineszenzvorrichtung dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist die erfindungsgemäße organische Elektrolumineszenzvorrichtung dadurch gekennzeichnet, dass eine oder mehrere organische Schichten enthaltend die erfindungsgemäße Zusammensetzung aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Hostmaterialien 1 und 2 und phosphoreszierende Emitter nötig. Das Verarbeiten aus Lösung hat den Vorteil, dass beispielsweise die lichtemittierende Schicht sehr einfach und kostengünstig aufgebracht werden kann. Diese Technik eignet sich insbesondere für die Massenproduktion organischer elektrolumineszierender Vorrichtungen.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können auf organische Elektrolumineszenzvorrichtungen angewandt werden.

Ein weiterer Gegenstand der Erfindung ist daher ein Verfahren zur Herstellung der erfindungsgemäßen organischen elektrolumineszierenden Vorrichtung, wie zuvor beschrieben oder bevorzugt beschrieben, dadurch gekennzeichnet, dass die organische Schicht, vorzugsweise die lichtemittierende Schicht, die Lochinjektionsschicht und/oder Lochtransportschicht, durch Gasphasenabscheidung, insbesondere mit einem Sublimationsverfahren und/oder mit einem OVPD (Organic Vapour Phase Deposition) Verfahren und/oder mit Hilfe einer Trägergassublimation, oder aus Lösung, insbesondere durch Spincoating oder mit einem Druckverfahren, aufgebracht wird.

Bei der Herstellung mittels Gasphasenabscheidung bestehen grundsätzlich zwei Möglichkeiten, wie die erfindungsgemäße organische Schicht, bevorzugt die lichtemittierende Schicht, auf ein beliebiges Substrat bzw. die vorherige Schicht aufgebracht bzw. aufgedampft werden kann. Zum einen können die verwendeten Materialien jeweils in einer Materialquelle vorgelegt und schließlich aus den verschiedenen Materialquellen verdampft werden ("co-evaporation"). Zum anderen können die verschiedenen Materialien vorgemischt ("premixed", Premix-Systeme) und das Gemisch in einer einzigen Materialquelle vorgelegt werden, aus der es schließlich verdampft wird ("premix-evaporation"). Dadurch lässt sich auf einfache und schnelle Art und Weise das Aufdampfen der lichtemittierenden Schicht mit gleichmäßiger Verteilung der Komponenten erreichen, ohne dass eine präzise Ansteuerung einer Vielzahl an Materialquellen notwendig

ist.

Ein weiterer Gegenstand der Erfindung ist demzufolge ein Verfahren zur Herstellung der erfindungsgemäßen Vorrichtung, dadurch gekennzeichnet, dass die mindestens eine Verbindung der Formel (1), wie zuvor beschrieben oder als bevorzugt beschrieben, und die mindestens eine Verbindung der Formel (2), wie zuvor beschrieben oder als bevorzugt beschrieben, nacheinander oder gleichzeitig aus mindestens zwei Materialquellen, gegebenenfalls mit dem mindestens einen phosphoreszierenden Emitter, wie zuvor beschrieben oder bevorzugt beschrieben, aus der Gasphase abgeschieden werden und die lichtemittierende Schicht bilden.

In einer bevorzugten Ausführungsform der vorliegenden Erfindung wird die lichtemittierende Schicht mittels Gasphasenabscheidung aufgebracht, wobei die Bestandeile der Zusammensetzung vorgemischt und aus einer einzigen Materialquelle verdampft werden.

Ein weiterer Gegenstand der Erfindung ist demzufolge ein Verfahren zur Herstellung der erfindungsgemäßen Vorrichtung, dadurch gekennzeichnet, dass die mindestens eine Verbindung der Formel (1) und die mindestens eine Verbindung der Formel (2) als Mischung, nacheinander oder gleichzeitig mit dem mindestens einen phosphoreszierenden Emitter, aus der Gasphase abgeschieden werden und die lichtemittierende Schicht bilden.

Ein weiterer Gegenstand der Erfindung ist ein Verfahren zur Herstellung der erfindungsgemäßen Vorrichtung, wie zuvor beschrieben oder bevorzugt beschrieben, dadurch gekennzeichnet, dass die mindestens eine Verbindung der Formel (1) und die mindestens eine Verbindung der Formel (2), wie zuvor beschrieben oder bevorzugt beschrieben, zusammen mit dem mindestens einen phosphoreszierenden Emitter, aus Lösung aufgebracht werden, um die lichtemittierende Schicht zu bilden.

Die erfindungsgemäßen Vorrichtungen zeichnen sich durch folgende überraschende Vorteile gegenüber dem Stand der Technik aus:
Die Verwendung der beschriebenen Materialkombination der Hostmaterialien 1 und 2, wie zuvor beschrieben, führt insbesondere zu einer Steigerung der Lebensdauer der Vorrichtungen.

Wie im nachfolgend angegebenen Beispiel ersichtlich, lässt sich durch Vergleich der Daten für OLEDs mit Kombinationen aus dem Stand der Technik feststellen, dass die erfindungsgemäßen Kombinationen an Matrixmaterialien in der EML zu Vorrichtungen führen, deren Lebensdauer signifikant erhöht sind, unabhängig von der Emitterkonzentration.

Die Erfindung wird durch die nachfolgenden Beispiele näher erläutert, ohne sie dadurch einschränken zu wollen.

## Beispiele

Allgemeine Methoden:
In allen quantenchemischen Berechnungen wird das Programmpaket Gaussian16 (Rev. B.01) verwendet. Der neutrale Singulettgrundzustand wird auf dem B3LYP/6-31G(d)-Niveau optimiert. HOMO- und LUMO-Werte werden auf dem B3LYP/6-31G(d)-Niveau für die mit B3LYP/6-31G(d) optimierte Grundzustandsenergie bestimmt. Daraufhin werden TD-DFT-Singulett- und Triplettanregungen (vertikale Anregungen) mit der gleichen Methode (B3LYP/6-31G(d)) und der optimierten Grundzustandsgeometrie berechnet. Die Standardeinstellungen für SCF- und Gradientenkonvergenz werden verwendet.

Aus der Energierechnung erhält man das HOMO als das letzte mit zwei Elektronen besetze Orbital (Alpha occ. eigenvalues) und LUMO als das erste unbesetzte Orbital (Alpha virt. eigenvalues) in Hartree-Einheiten, wobei HEh und LEh für die HOMO Energie in Hartree-Einheiten beziehungsweise für die LUMO-Energie in Hartree-Einheiten steht.

Daraus wird der anhand von Cyclovoltammetriemessungen kalibrierte HOMO- und LUMO-Wert in Elektronenvolt wie folgt bestimmt:

$$\text{HOMOcorr} = 0.90603 * \text{HOMO} - 0.84836$$

$$\text{LUMOcorr} = 0.99687 * \text{LUMO} - 0.72445$$

Das Triplett-Niveau T1 eines Materials ist definiert als die relative Anregungsenergie (in eV) des Triplettzustands mit der niedrigsten Energie, der sich aus der quantenchemischen Energierechnung ergibt.

Das Singulett-Niveau S1 eines Materials ist definiert als die relative Anregungsenergie (in eV) des Singulettzustands mit der zweitniedrigsten Energie, der sich aus der quantenchemischen Energierechnung ergibt.

Der energetisch niedrigste Singulettzustand wird als S0 bezeichnet.

Die hierin beschriebene Methode ist unabhängig von dem verwendeten Softwarepaket und liefert immer dieselben Ergebnisse. Beispiele oft benutzter Programme für diesen Zweck sind "Gaussian09" (Gaussian Inc.) und Q-Chem 4.1 (Q-Chem, Inc.). Vorliegend wird zur Berechnung der Energien das Programmpaket "Gaussian16 (Rev. B.01)"

verwendet.

Beispiel 1: **Herstellung der OLEDs**

**Vorbehandlung für die Herstellung der OLEDs:** Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat/Lochinjektionsschicht (HIL)/Lochtransportschicht (HTL)/Elektronenblockierschicht (EBL)/Emissionsschicht (EML)/optionale Lochblockierschicht (HBL)/Elektronentransportschicht (ETL)/optionale Elektroneninjektionsschicht (EIL) und abschließend eine Kathode. Die Kathode wird durch eine 100 nm dicke Aluminiumschicht gebildet.

Alle Materialien werden in einer Vakuumkammer thermisch aufgedampft. Dabei besteht die Emissionsschicht immer aus mindestens einem Matrixmaterial (auch Hostmaterial oder Wirtsmaterial), im Sinn der Erfindung mindestens zwei Matrixmaterialien und einem emittierenden Dotierstoff (Dotand, Emitter), der dem Matrixmaterial bzw. den Matrixmaterialien durch Coverdampfung in einem bestimmten Volumenanteil beigemischt wird. Analog kann auch die Elektronentransportschicht aus einer Mischung von zwei Materialien bestehen.

Die OLEDs werden standardmäßig charakterisiert. Hierfür werden die Elektrolumineszenzspektren und Strom-Spannungs-Leuchtdichte-Kennlinien (IUL-Kennlinien) gemessen. EQE und die Stromeffizienz SE (in cd/A) werden daraus berechnet. Die Berechnung der SE erfolgt unter Annahme einer lambertschen Abstrahlcharakteristik.

Als Lebensdauer LT wird die Zeit definiert, nach der die Leuchtdichte bei Betrieb mit konstanter Stromdichte $j_0$ in mA/cm$^2$ von einer Startleuchtdichte L0 (in cd/m$^2$) auf einen gewissen Anteil L1 (in cd/m$^2$) absinkt. Eine Angabe L1/L0 = 80% in Tabelle 7 bedeutet, dass die in Spalte LT angegebene Lebensdauer der Zeit (in h) entspricht, nach der die Leuchtdichte auf 80% ihres Anfangswertes (L0) absinkt.

**Verwendung von erfindungsgemäßen Mischungen in OLEDs**

In den folgenden Beispielen V1 bis V15, V15a, V16 bis V18 und V18a und B1 bis B30 (siehe Tabellen 6 und 7) wird der Einsatz der erfindungsgemäßen Materialkombinationen in OLEDs in Vergleich zu Materialkombinationen aus dem Stand der Technik vorgestellt. Die erfindungsgemäßen Materialkombinationen können in der Emissionsschicht in phosphoreszierenden grünen OLEDs eingesetzt werden.

Der Aufbau der OLEDs ist Tabelle 6 zu entnehmen. Die zur Herstellung der OLEDs benötigten Materialien sind in Tabelle 8 gezeigt, sofern nicht schon zuvor beschrieben. Die Device Daten der OLEDs sind in Tabelle 7 aufgelistet.

Eine Angabe wie VG1:H2:TEG1 (33%:60%:7%) 30nm bedeutet hierbei, dass das Vergleichsmaterial 1 (VG1) in einem Volumenanteil von 33% als Hostmaterial 1, die Verbindung H2 als Hostmaterial 2 in einem Anteil von 60% und TEG1 in einem Anteil von 7% in einer 30nm dicken Schicht vorliegt.

Die Beispiele V1 bis V15, V17 und V18 sind Vergleichsbeispiele mit einem elektronentransportierenden Host gemäß dem Stand der Technik oder in V15a und V16 mit einem elektronentransportierenden Host gemäß dem Stand der Technik in Kombination mit dem Host H0 und in V18a mit dem Host HA. Die Beispiele B1 bis B30 verwenden erfindungsgemäße Materialkombinationen in der EML.

Beim Vergleich der erfindungsgemäßen Beispiele mit den entsprechenden Vergleichsbeispielen, ist klar erkennbar, dass die erfindungsgemäßen Beispiele jeweils einen deutlichen Vorteil in der Device Lebensdauer aufzeigen.

Tabelle 6:

| Bsp | HIL Dicke | HTL Dicke | EBL Dicke | EML Dicke | HBL Dick e | ETL Dicke | EIL Dicke |
|---|---|---|---|---|---|---|---|
| V1 | SpMA1:P D1 (95%:5%) 20nm | SpMA1 215nm | SpMA2 20nm | **VG1**:**H2**:TEG1 (33%:60%:7%) 30nm | ST2 10n m | ST2:LiQ (50%: 50%) 30nm | LiQ 1nm |
| B1 | SpMA1:P D1 (95%:5%) 20nm | SpMA1 215nm | SpMA2 20nm | **E1**:**H2**:TEG1 (33%: 60%:7%) 30nm | ST2 10n m | ST2:LiQ (50%: 50%) 30nm | LiQ 1nm |
| V2 | SpMA1:P D1 (95%:5%) 20nm | SpMA1 215nm | SpMA2 20nm | **VG2**:**H2**:TEG1 (33%:60%:7%) 30nm | ST2 10n m | ST2:LiQ (50%: 50%) 30nm | LiQ 1nm |

(fortgesetzt)

| Bsp | HIL Dicke | HTL Dicke | EBL Dicke | EML Dicke | HBL Dick e | ETL Dicke | EIL Dicke |
|---|---|---|---|---|---|---|---|
| B2 | SpMA1:P D1 (95%:5%) 20nm | SpMA1 215nm | SpMA2 20nm | **E2**:**H2**:TEG1 (33%: 60%:7%) 30nm | ST2 10n m | ST2:LiQ (50%: 50%) 30nm | LiQ 1nm |
| V3 | SpMA1:P D1 (95%:5%) 20nm | SpMA1 215nm | SpMA2 20nm | **VG3**:**H2**:TEG1 (33%:60%:7%) 30nm | ST2 10n m | ST2:LiQ (50%: 50%) 30nm | LiQ 1nm |
| B3 | SpMA1:P D1 (95%:5%) 20nm | SpMA1 215nm | SpMA2 20nm | **E4**:**H2**:TEG1 (33%: 60%:7%) 30nm | ST2 10n m | ST2:LiQ (50%: 50%) 30nm | LiQ 1nm |
| V4 | SpMA1:P D1 (95%:5%) 20nm | SpMA1 215nm | SpMA2 20nm | **VG4**:**H2**:TEG1 (33%:60%:7%) 30nm | ST2 10n m | ST2:LiQ (50%: 50%) 30nm | LiQ 1nm |
| B4 | SpMA1:P D1 (95%:5%) 20nm | SpMA1 215nm | SpMA2 20nm | **E5**:**H2**:TEG1 (33%: 60%:7%) 30nm | ST2 10n m | ST2:LiQ (50%: 50%) 30nm | LiQ 1nm |
| V5 | SpMA1:P D1 (95%:5%) 20nm | SpMA1 215nm | SpMA2 20nm | **VG5**:**H2**:TEG1 (33%:60%:7%) 30nm | ST2 10n m | ST2:LiQ (50%: 50%) 30nm | LiQ 1nm |
| B5 | SpMA1:P D1 (95%:5%) 20nm | SpMA1 215nm | SpMA2 20nm | **E11**:**H2**:TEG1 (33%: 60%:7%) 30nm | ST2 10n m | ST2:LiQ (50%: 50%) 30nm | LiQ 1nm |
| V6 | SpMA1:P D1 (95%:5%) 20nm | SpMA1 215nm | SpMA2 20nm | **VG6:H1**:TEG1 (33%:60%:7%) 30nm | ST2 10n m | ST2:LiQ (50%: 50%) 30nm | LiQ 1nm |
| B6 | SpMA1:P D1 (95%:5%) 20nm | SpMA1 215nm | SpMA2 20nm | **E19:H1**:TEG1 (33%: 60%:7%) 30nm | ST2 10n m | ST2:LiQ (50%: 50%) 30nm | LiQ 1nm |
| V7 | SpMA1:P D1 (95%:5%) 20nm | SpMA1 215nm | SpMA2 20nm | **VG7:H2**:TEG1 (33%:60%:7%) 30nm | ST2 10n m | ST2:LiQ (50%: 50%) 30nm | LiQ 1nm |
| B7 | SpMA1:P D1 (95%:5%) 20nm | SpMA1 215nm | SpMA2 20nm | **E14**:**H2**:TEG1 (33%: 60%:7%) 30nm | ST2 10n m | ST2:LiQ (50%: 50%) 30nm | LiQ 1nm |
| V8 | SpMA1:P D1 (95%:5%) 20nm | SpMA1 215nm | SpMA2 20nm | **VG8**:**H2**:TEG1 (33%:60%:7%) 30nm | ST2 10n m | ST2:LiQ (50%: 50%) 30nm | LiQ 1nm |
| B8 | SpMA1:P D1 (95%:5%) 20nm | SpMA1 215nm | SpMA2 20nm | **E1**:**H2**:TEG1 (33%: 60%:7%) 30nm | ST2 10n m | ST2:LiQ (50%: 50%) 30nm | LiQ 1nm |
| V9 | SpMA1:P D1 (95%:5%) 20nm | SpMA1 215nm | SpMA2 20nm | **VG9**:**H2**:TEG1 (33%:60%:7%) 30nm | ST2 10n m | ST2:LiQ (50%: 50%) 30nm | LiQ 1nm |
| B9 | SpMA1:P D1 (95%:5%) 20nm | SpMA1 215nm | SpMA2 20nm | **E12**:**H2**:TEG1 (33%: 60%:7%) 30nm | ST2 10n m | ST2:LiQ (50%: 50%) 30nm | LiQ 1nm |

(fortgesetzt)

| Bsp | HIL Dicke | HTL Dicke | EBL Dicke | EML Dicke | HBL Dick e | ETL Dicke | EIL Dicke |
|---|---|---|---|---|---|---|---|
| V1 0 | SpMA1:P D1 (95%:5%) 20nm | SpMA1 215nm | SpMA2 20nm | **VG10**:**H2**:TEG 1 (33%:60%:7%) 30nm | ST2 10n m | ST2:LiQ (50%: 50%) 30nm | LiQ 1nm |
| B1 0 | SpMA1:P D1 (95%:5%) 20nm | SpMA1 215nm | SpMA2 20nm | **E14**:**H2**:TEG1 (33%: 60%:7%) 30nm | ST2 10n m | ST2:LiQ (50%: 50%) 30nm | LiQ 1nm |
| V1 1 | SpMA1:P D1 (95%:5%) 20nm | SpMA1 215nm | SpMA2 20nm | **VG11**:**H1**:TEG 1 (46%:47%:7%) 30nm | ST2 10n m | ST2:LiQ (50%: 50%) 30nm | LiQ 1nm |
| B1 1 | SpMA1:P D1 (95%:5%) 20nm | SpMA1 215nm | SpMA2 20nm | **E1**:**H2**:TEG1 (33%: 60%:7%) 30nm | ST2 10n m | ST2:LiQ (50%: 50%) 30nm | LiQ 1nm |
| V1 2 | SpMA1:P D1 (95%:5%) 20nm | SpMA1 215nm | SpMA2 20nm | **VG12**:**H23**:TE G1 (33%:60%:7%) 30nm | ST2 10n m | ST2:LiQ (50%: 50%) 30nm | LiQ 1nm |
| B1 2 | SpMA1:P D1 (95%:5%) 20nm | SpMA1 215nm | SpMA2 20nm | **E1:H23:**TEG1 (33%:60%:7%) 30nm | ST2 10n m | ST2:LiQ (50%: 50%) 30nm | LiQ 1nm |
| V1 3 | SpMA1:P D1 (95%:5%) 20nm | SpMA1 215nm | SpMA2 20nm | **VG13:H1:**TEG 1 (33%:60%:7%) 30nm | ST2 10n m | ST2:LiQ (50%: 50%) 30nm | LiQ 1nm |
| B1 3 | SpMA1:P D1 (95%:5%) 20nm | SpMA1 215nm | SpMA2 20nm | **E15:H1:**TEG1 (33%:60%:7%) 30nm | ST2 10n m | ST2:LiQ (50%: 50%) 30nm | LiQ 1nm |
| V1 4 | SpMA1:P D1 (95%:5%) 20nm | SpMA1 215nm | SpMA2 20nm | **VG14:H2:**TEG 1 (33%:60%:7%) 30nm | ST2 10n m | ST2:LiQ (50%: 50%) 30nm | LiQ 1nm |
| B1 4 | SpMA1:P D1 (95%:5%) 20nm | SpMA1 215nm | SpMA2 20nm | **E17:H2:**TEG1 (33%:60%:7%) 30nm | ST2 10n m | ST2:LiQ (50%: 50%) 30nm | LiQ 1nm |
| V1 5 | SpMA1:P D1 (95%:5%) 20nm | SpMA1 215nm | SpMA2 20nm | **VG15:H2:**TEG 1 (33%:60%:7%) 30nm | ST2 10n m | ST2:LiQ (50%: 50%) 30nm | LiQ 1nm |
| V1 5a | SpMA1:P D1 (95%:5%) 20nm | SpMA1 215nm | SpMA2 20nm | **VG15:H0:**TEG 1 (33%:60%:7%) 30nm | ST2 10n m | ST2:LiQ (50%: 50%) 30nm | LiQ 1nm |
| B1 5 | SpMA1:P D1 (95%:5%) 20nm | SpMA1 215nm | SpMA2 20nm | **E1**:**H2**:TEG1 (33%: 60%:7%) 30nm | ST2 10n m | ST2:LiQ (50%: 50%) 30nm | LiQ 1nm |
| V1 6 | SpMA1:P D1 (95%:5%) 20nm | SpMA1 215nm | SpMA2 20nm | **VG16:H0:**TEG 1 (33%:60%:7%) 30nm | ST2 10n m | ST2:LiQ (50%: 50%) 30nm | LiQ 1nm |
| B1 6 | SpMA1:P D1 (95%:5%) 20nm | SpMA1 215nm | SpMA2 20nm 2 | **E20**:**H2**:TEG1 (33%: 60%:7%) 30nm | ST2 10n m | ST2:LiQ (50%: 50%) 30nm | LiQ 1nm |

(fortgesetzt)

| Bsp | HIL Dicke | HTL Dicke | EBL Dicke | EML Dicke | HBL Dick e | ETL Dicke | EIL Dicke |
|---|---|---|---|---|---|---|---|
| V1 7 | SpMA1:P D1 (95%:5%) 20nm | SpMA1 215nm | SpMA2 20nm | **VG17:H2:**TEG 1 (33%:60%:7%) 30nm | ST2 10n m | ST2:LiQ (50%: 50%) 30nm | LiQ 1nm |
| B1 7 | SpMA1:P D1 (95%:5%) 20nm | SpMA1 215nm | SpMA2 20nm | **E13**:**H2**:TEG1 (33%: 60%:7%) 30nm | ST2 10n m | ST2:LiQ (50%: 50%) 30nm | LiQ 1nm |
| V1 8 | SpMA1:P D1 (95%:5%) 20nm | SpMA1 215nm | SpMA2 20nm | **VG18**:**H2**:TEG 1 (33%:60%:7%) 30nm | ST2 10n m | ST2:LiQ (50%: 50%) 30nm | LiQ 1nm |
| V1 8a | SpMA1:P D1 (95%:5%) 20nm | SpMA1 215nm | SpMA2 20nm | **VG18**:**HA**:TEG 1 (33%:60%:7%) 30nm | ST2 10n m | ST2:LiQ (50%: 50%) 30nm | LiQ 1nm |
| B1 8 | SpMA1:P D1 (95%:5%) 20nm | SpMA1 215nm | SpMA2 20nm | **E18**:**H2**:TEG1 (33%: 60%:7%) 30nm | ST2 10n m | ST2:LiQ (50%: 50%) 30nm | LiQ 1nm |

Tabelle 7: Daten der OLEDs

| Bsp. | U1000 (V) | SE1000 (cd/A) | EQE1000 (%) | CIE x/y at 1000 cd/m2 | $j_0$ (mA/cm$^2$) | L1 (%) | LT (h) |
|---|---|---|---|---|---|---|---|
| V1 | 3.5 | 67 | 18 | 0.33/0.63 | 20 | 80 | 360 |
| B1 | 3.3 | 76 | 19 | 0.35/0.62 | 20 | 80 | 620 |
| V2 | 3.6 | 71 | 16 | 0.34/0.61 | 20 | 80 | 340 |
| B2 | 3.2 | 72 | 19 | 0.32/0.62 | 20 | 80 | 630 |
| V3 | 3.8 | 69 | 17 | 0.35/0.62 | 20 | 80 | 345 |
| B3 | 3.4 | 72 | 18.5 | 0.33/0.63 | 20 | 80 | 600 |
| V4 | 3.5 | 68 | 18 | 0.32/0.62 | 20 | 80 | 380 |
| B4 | 3.2 | 70 | 18 | 0.34/0.61 | 20 | 80 | 595 |
| V5 | 3.7 | 71 | 17 | 0.34/0.61 | 20 | 80 | 375 |
| B5 | 3.5 | 75 | 18 | 0.33/0.63 | 20 | 80 | 590 |
| V6 | 3.5 | 70 | 17 | 0.35/0.62 | 20 | 80 | 380 |
| B6 | 3.3 | 75 | 18 | 0.33/0.63 | 20 | 80 | 585 |
| V7 | 3.5 | 68 | 18 | 0.33/0.63 | 20 | 80 | 375 |
| B7 | 3.2 | 75 | 18.5 | 0.33/0.63 | 20 | 80 | 615 |
| V8 | 3.4 | 70 | 17 | 0.34/0.61 | 20 | 80 | 325 |
| B8 | 3.3 | 76 | 19 | 0.35/0.62 | 20 | 80 | 620 |
| V9 | 3.7 | 69 | 16.5 | 0.35/0.62 | 20 | 80 | 310 |
| B9 | 3.3 | 75 | 17.5 | 0.33/0.63 | 20 | 80 | 515 |
| V10 | 3.4 | 67 | 18.5 | 0.32/0.62 | 20 | 80 | 395 |
| B10 | 3.2 | 75 | 18.5 | 0.33/0.63 | 20 | 80 | 615 |
| V11 | 3.2 | 74 | 20.1 | 0.32/0.63 | 20 | 80 | 650 |
| B11 | 3.3 | 76 | 19 | 0.35/0.62 | 20 | 80 | 620 |

(fortgesetzt)

| Bsp. | U1000 (V) | SE1000 (cd/A) | EQE1000 (%) | CIE x/y at 1000 cd/m2 | $j_0$ (mA/cm$^2$) | L1 (%) | LT (h) |
|---|---|---|---|---|---|---|---|
| V12 | 3.9 | 67 | 16 | 0.35/0.62 | 20 | 80 | 290 |
| B12 | 3.3 | 76 | 19 | 0.35/0.62 | 20 | 80 | 620 |
| V13 | 3.8 | 70 | 17 | 0.34/0.61 | 20 | 80 | 280 |
| B13 | 3.5 | 76 | 18 | 0.35/0.62 | 20 | 80 | 610 |
| V14 | 4.1 | 69 | 16 | 0.35/0.62 | 20 | 80 | 170 |
| B14 | 3.4 | 76 | 18.5 | 0.34/0.61 | 20 | 80 | 570 |
| V15 | 3.6 | 70 | 16 | 0.34/0.61 | 20 | 80 | 310 |
| V15a | 3.5 | 68 | 18.5 | 0.32/0.62 | 20 | 80 | 390 |
| B15 | 3.3 | 76 | 19 | 0.33/0.63 | 20 | 80 | 620 |
| V16 | 3.4 | 70 | 18 | 0.34/0.61 | 20 | 80 | 394 |
| V16a | 3.6 | 70 | 17 | 0.33/0.63 | 20 | 80 | 315 |
| B16 | 3.3 | 70 | 17.5 | 0.34/0.61 | 20 | 80 | 575 |
| V17 | 3.6 | 73 | 16.5 | 0.35/0.61 | 20 | 80 | 320 |
| 17 | 3.4 | 76 | 18.5 | 0.35/0.62 | 20 | 80 | 575 |
| V18 | 3.5 | 73 | 18.5 | 0.35/0.61 | 20 | 80 | 365 |
| V18a | 3.4 | 68 | 18 | 0.32/0.62 | 20 | 80 | 310 |
| B18 | 3.4 | 76 | 18.5 | 0.33/0.63 | 20 | 80 | 595 |
| | | | | | | | |
| B19 | 3.4 | 76 | 18 | 0.33/0.63 | 20 | 80 | 595 |
| B20 | 3.5 | 67 | 18.5 | 0.34/0.61 | 20 | 80 | 610 |
| B21 | 3.5 | 71 | 17 | 0.35/0.63 | 20 | 80 | 555 |
| B22 | 3.4 | 72 | 17.5 | 0.35/0.61 | 20 | 80 | 545 |
| B23 | 3.5 | 69 | 18 | 0.34/0.62 | 20 | 80 | 615 |
| B24 | 3.4 | 67 | 17 | 0.33/0.63 | 20 | 80 | 545 |
| B25 | 3.6 | 76 | 17.5 | 0.35/0.62 | 20 | 80 | 565 |
| B26 | 3.3 | 72 | 18 | 0.35/0.63 | 20 | 80 | 625 |
| B27 | 3.3 | 71 | 18.5 | 0.34/0.62 | 20 | 80 | 630 |
| B28 | 3.1 | 69 | 19 | 0.34/0.62 | 20 | 80 | 635 |
| B29 | 3.2 | 67 | 19 | 0.34/0.61 | 20 | 80 | 630 |
| B30 | 3.1 | 68 | 18 | 0.34/0.61 | 20 | 80 | 630 |

Tabelle 8: verwendete Materialien, sofern noch nicht zuvor beschrieben:

| | |
|---|---|
| 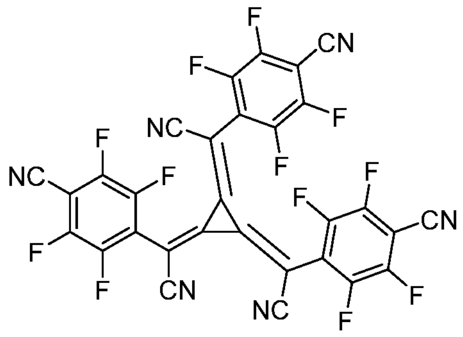 | 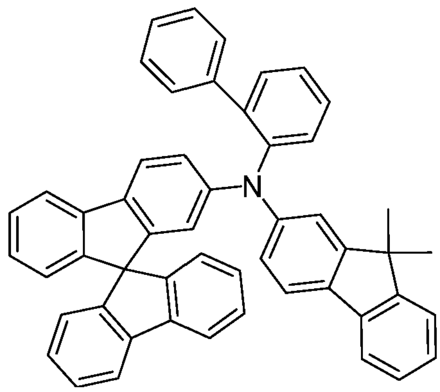 |
| PD1 (1224447-88-4) | SpMA1 |
| 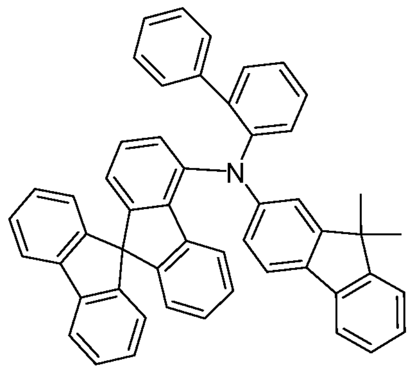 | 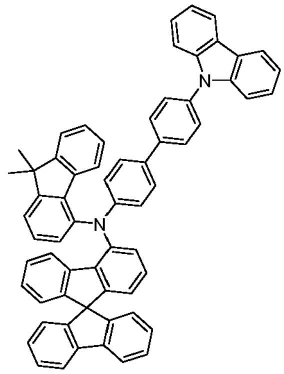 |
| SpMA2 | SpMA5 |
| 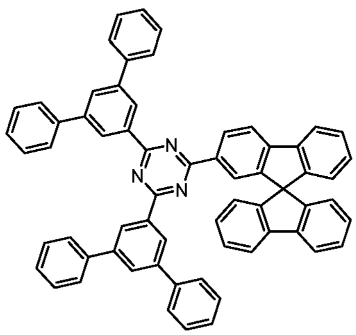 | 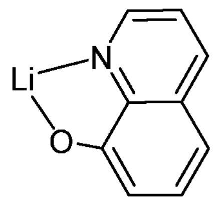 |
| ST2 | LiQ |
| 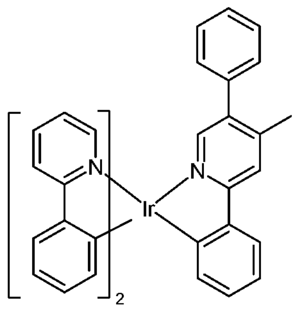 | 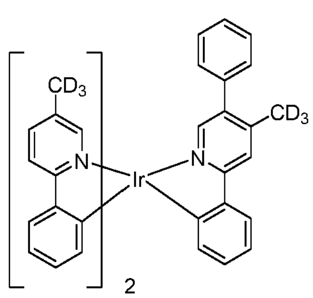 |
| TEG1 | TEG2 |

(fortgesetzt)

| | |
|---|---|
| 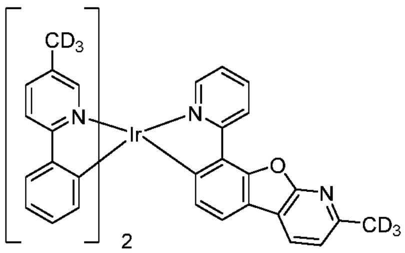 | 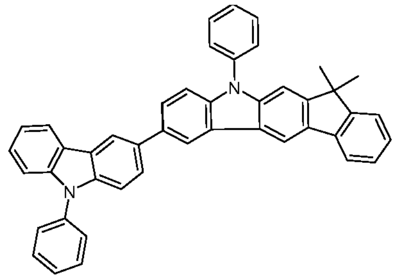 |
| TEG3 | H0 |
| 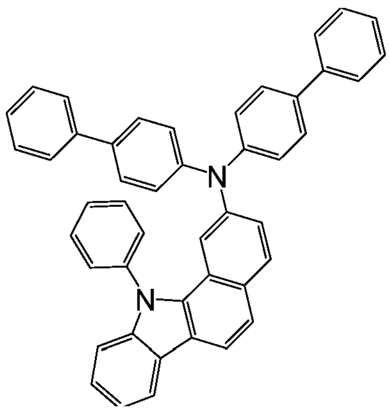 | 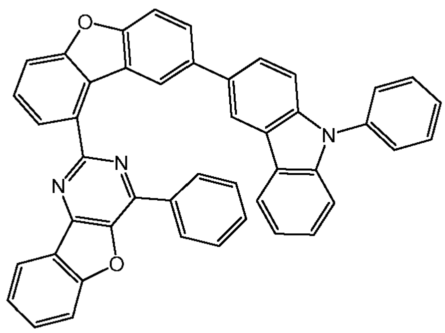 |
| HA [WO20067657] | **VG1** [KR2018010165] |
| 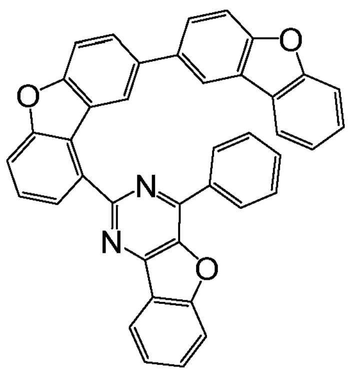 | 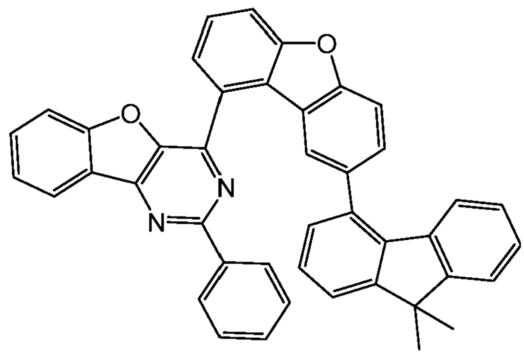 |
| **VG2** [KR2018010165] | **VG3** [KR2018010165] |
| 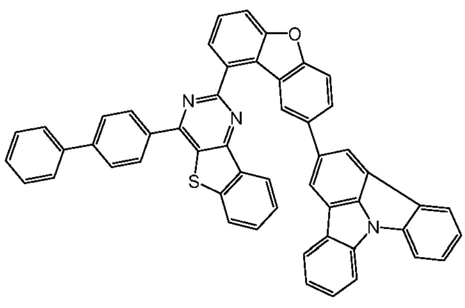 | 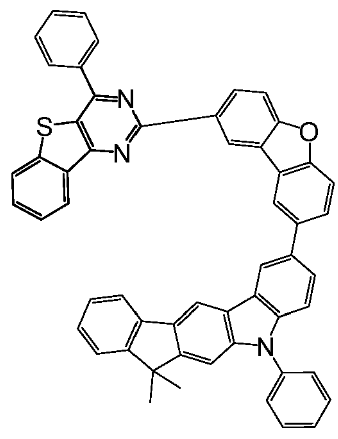 |
| **VG4 [**KR20170113320] | **VG5** [KR2018010165] |

(fortgesetzt)

| | |
|---|---|
| 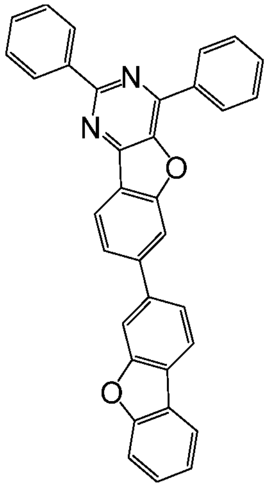 | 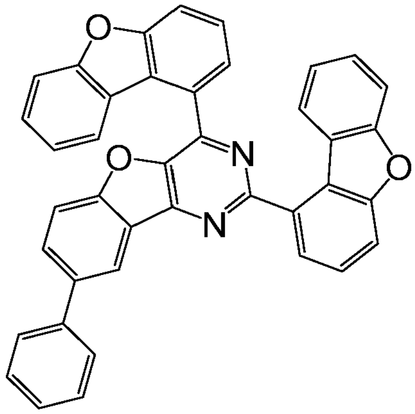 |
| **VG6** [WO19059577] | **VG7** [WO19229584] |
| 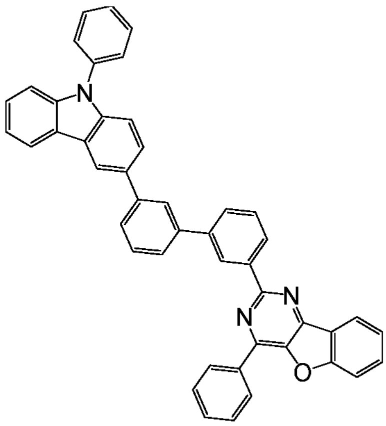 | 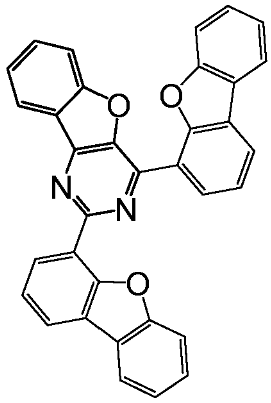 |
| **VG8** [WO15105315] | **VG9** [WO15105315] |
| 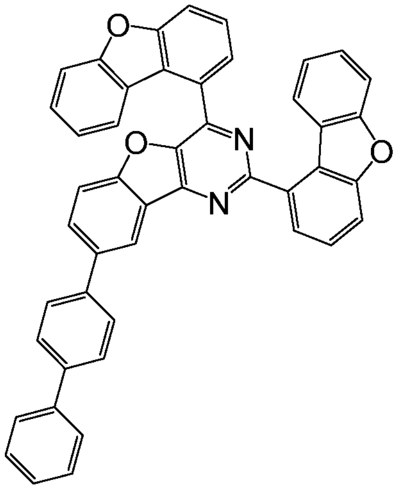 | 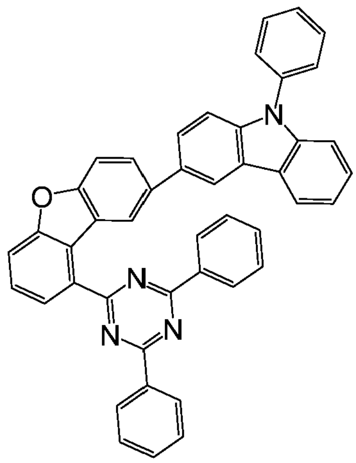 |
| **VG10** [WO19229583] | **VG11** [WO2015169412] |

(fortgesetzt)

| | |
|---|---|
| 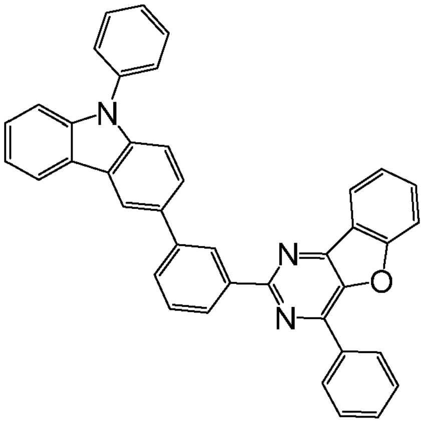 | 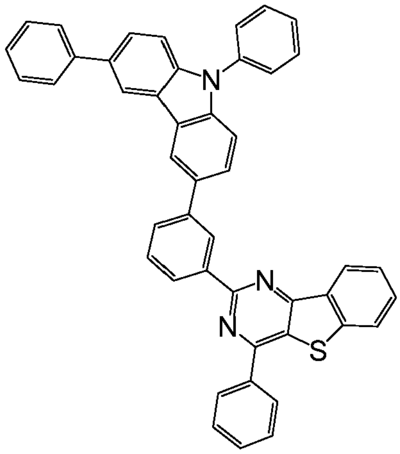 |
| **VG12** [WO15105315] | **VG13** [US2016072078] |
| 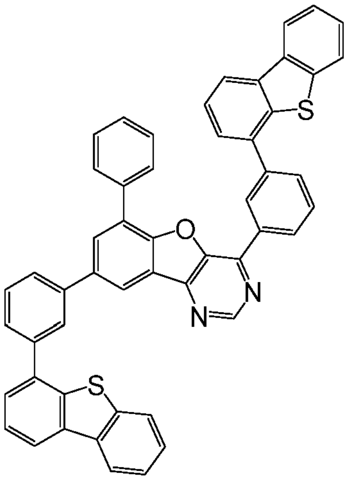 | 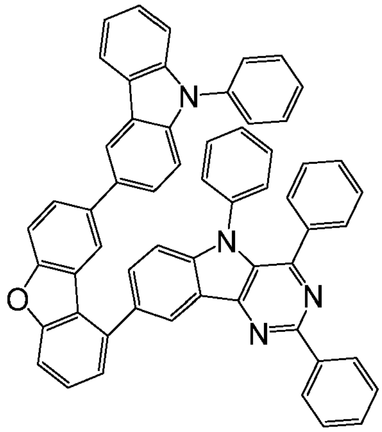 |
| **VG14** [WO17109637] | **VG15** [WO171867601 |
| 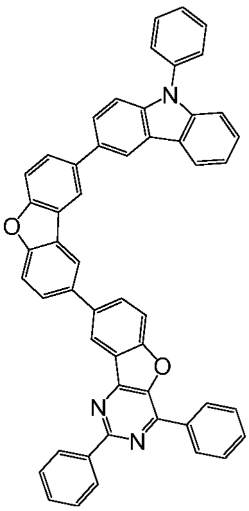 | 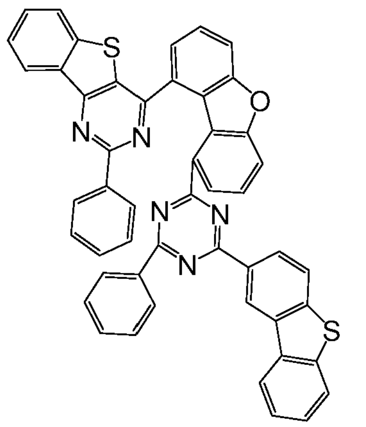 |
| **VG16** [WO18060307] | **VG17** [US20200010476] |

(fortgesetzt)

| | |
|---|---|
| 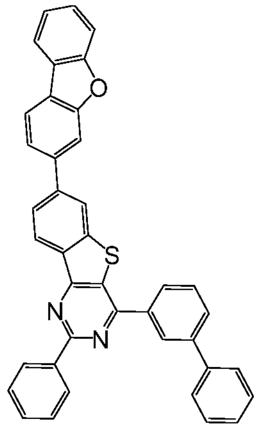 | |
| **VG18** [WO20067657] | |

Beispiel 2: Synthese von Hostmaterialien und deren Vorstufen:

Die nachfolgenden Synthesen werden, sofern nicht anders angegeben, unter einer Schutzgasatmosphäre in getrockneten Lösungsmitteln durchgeführt. Die erfindungsgemäßen Verbindungen können mittels dem Fachmann bekannten Syntheseverfahren dargestellt werden.

**a) 2-Chloro-4,8-diphenyl-benzofuro[3,2-d]pyrimidin**

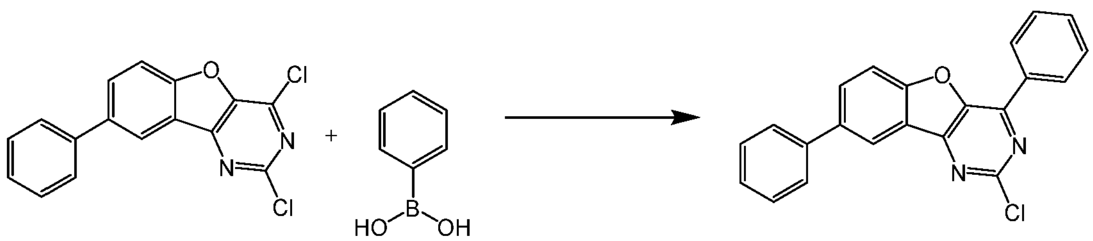

31,4 (100 mmol) 2,4-Dichloro-8-phenyl-benzofuro[3,2-d]pyrimidin,12,2 g (100 mmol) Phenylboronsäure und 11,8 g (111mmol) Natriumcarbonat werden in 800ml 1,4-Dioxan, 800 ml Wasser und 250ml Toluol gelöst, und unter Argonatmosphäre gerührt. 1,2 g (1mmol) Tetrakis (Triphenylphosphin)-Palladium wird dem Kolben dazugegeben. Das Reaktionsgemisch wird unter Rückfluss über Nacht gerührt. Nach dem Abkühlen wird das Gemisch gequenscht. Die organische Phase wird getrennt, mit 300 ml Wasser drei Mal gewaschen, über $MgSO_4$ getrocknet, filtriert und das Lösungsmittel im Vakuum entfernt. Der Rückstand wird durch Säulenchromatographie über Kieselgel (Eluent: DCM/ Heptan (1:10) gereinigt. Die Ausbeute beträgt 28 g (80 mmol), entsprechend 80 % der Theorie.

Analog dazu werden die folgenden Verbindungen hergestellt:

| | Edukt 1 | Edukt 1 | Produkt | Ausbeute |
|---|---|---|---|---|
| **1a** | 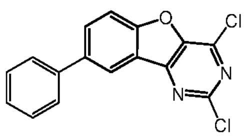 [1835207-37-8] | 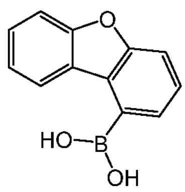 [162607-19-4] | 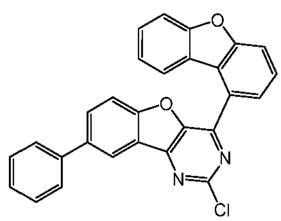 | 75% |

(fortgesetzt)

| | Edukt 1 | Edukt 1 | Produkt | Ausbeute |
|---|---|---|---|---|
| **2a** | 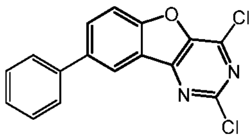 [1835207-37-8] | 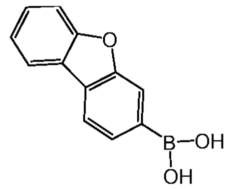 [395087-89-5 ] | 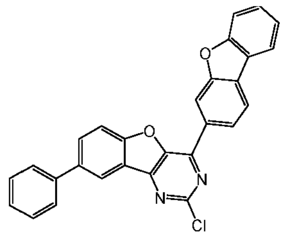 | 73% |
| **3a** | 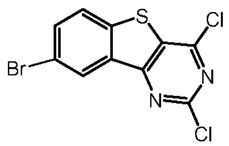 [2201128-35-8] | 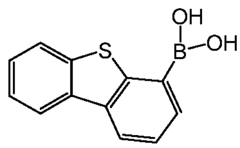 [108847-20-7 ] | 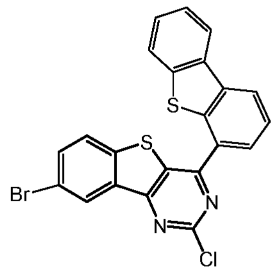 | 67% |
| **4a** | 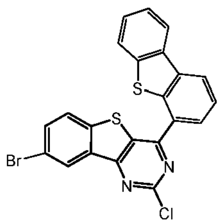 | 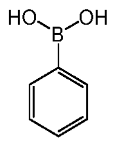 | 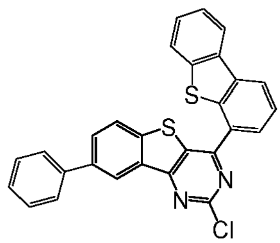 | 80% |
| **5a** | 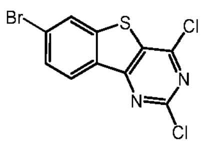 [2201128-36-9] | 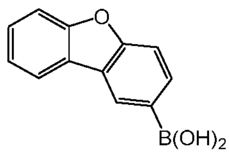 [402936-15-6] | 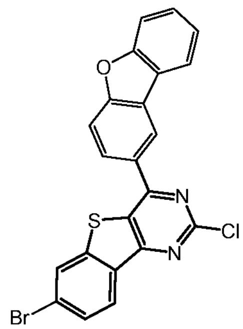 | 73% |
| **6a** | 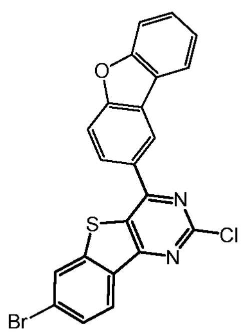 | 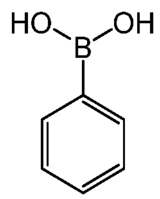 | 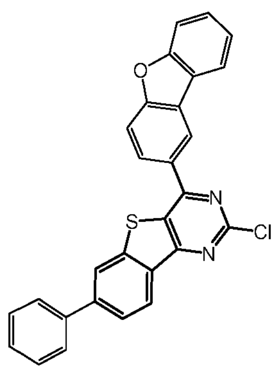 | 86% |

(fortgesetzt)

| | Edukt 1 | Edukt 1 | Produkt | Ausbeute |
|---|---|---|---|---|
| **7a** | 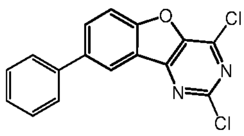 [1835207-37-8] | 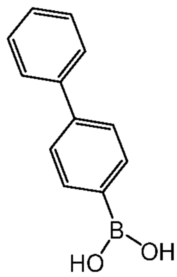 [ 5122-94-1 ] | 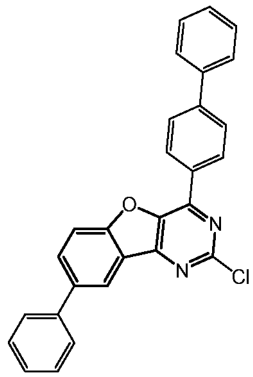 | 84% |
| **8a** | 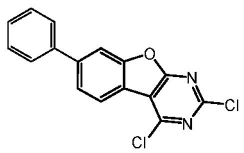 [2201128-28-9] | 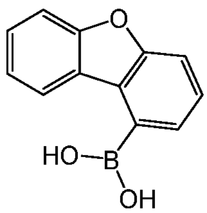 [162607-19-4] | 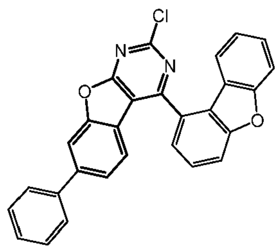 | 76% |
| **9a** | 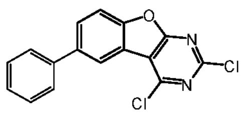 [2201128-33-6] | 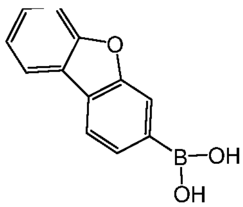 [ 395087-89-5 ] | 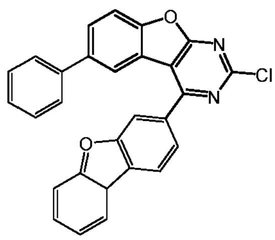 | 73% |
| **10a** | 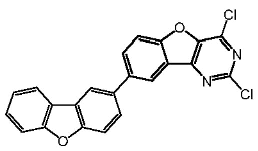 [2219361-31-4] | 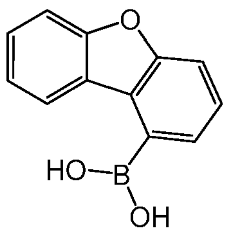 [162607-19-4] | 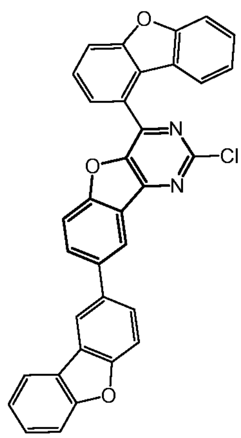 | 79% |

(fortgesetzt)

| | Edukt 1 | Edukt 1 | Produkt | Ausbeute |
|---|---|---|---|---|
| **11a** | 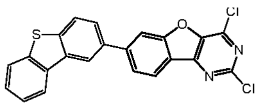 [2201128-2-2] | 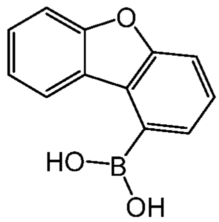 [162607-19-4] | 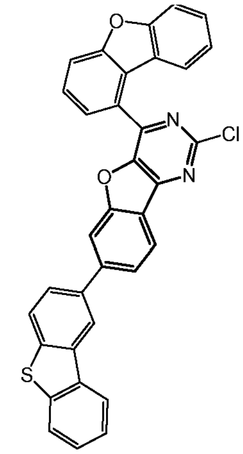 | 81% |
| **12a** | 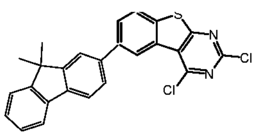 [2219361-27-8] | 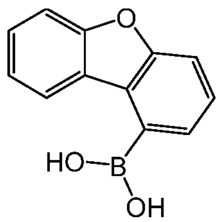 [162607-19-4] | 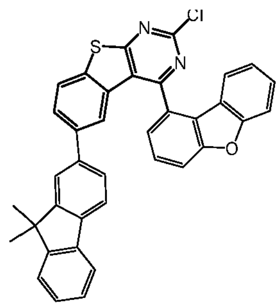 | 76% |
| **13a** | 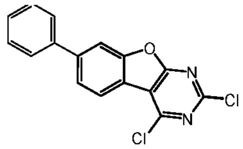 [2201128-28-9] | 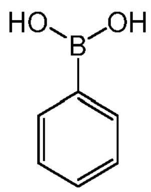 | 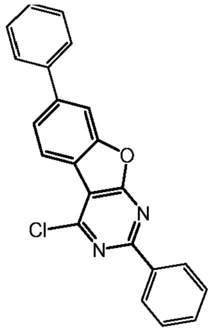 [2201128-28-9] | 75% |
| **14a** | 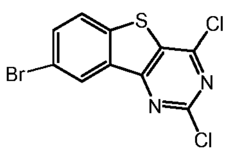 [2201128-35-8] | 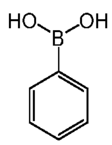 | 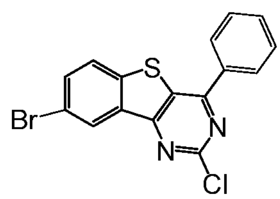 | 77% |
| **15a** | 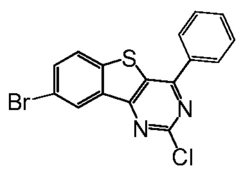 | 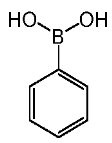 | 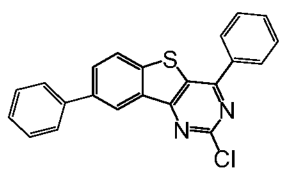 | 81% |

(fortgesetzt)

| | Edukt 1 | Edukt 1 | Produkt | Ausbeute |
|---|---|---|---|---|
| **16a** | 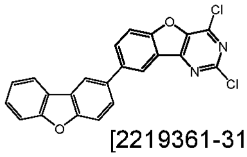 [2219361-31-4] | 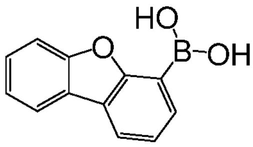 [100124-06-9] | 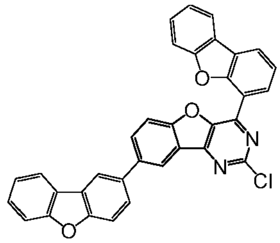 | 63% |
| **17a** | 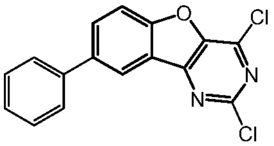 [1835207-37-8] | 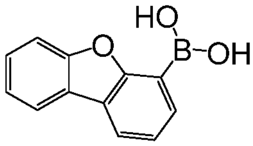 [100124-06-9] | 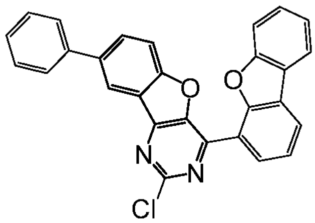 | 82% |
| **18a** | 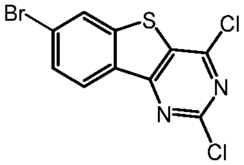 [2201128-36-9] | 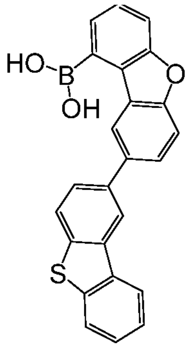 [2412400-98-5] | 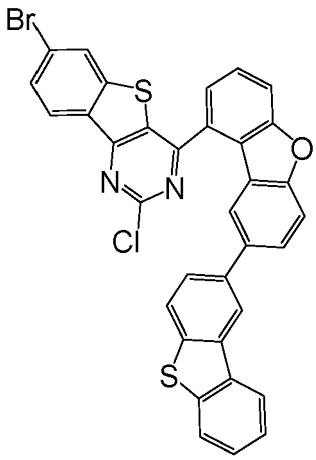 | 64% |
| **19a** | 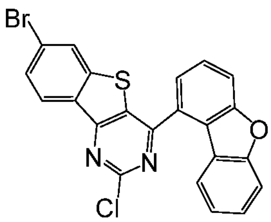 | 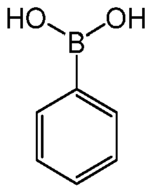 | 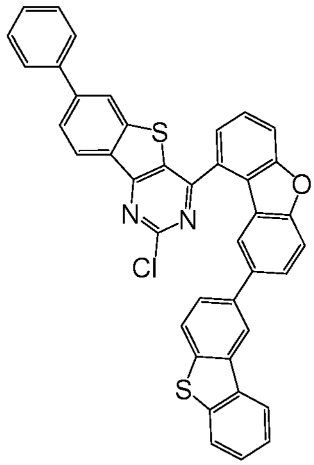 | 84% |

(fortgesetzt)

| | Edukt 1 | Edukt 1 | Produkt | Ausbeute |
|---|---|---|---|---|
| **20a** | 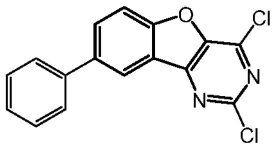 [1835207-37-8] | 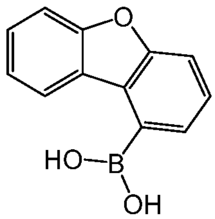 [162607-19-4] | 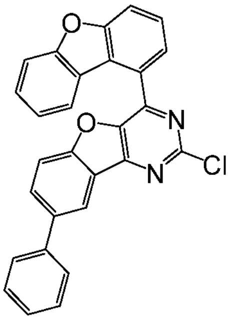 | 74% |
| **21a** | 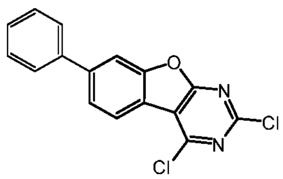 [2201128-28-9] | 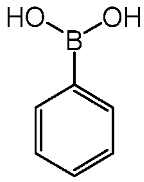 | 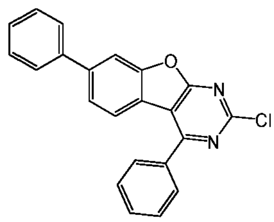 | 62% |
| **22a** | 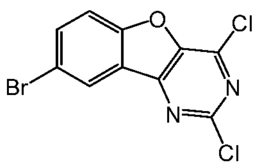 [1169560-03-5 ] | 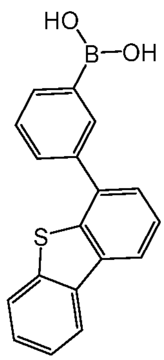 [1307859-67-1 ] | 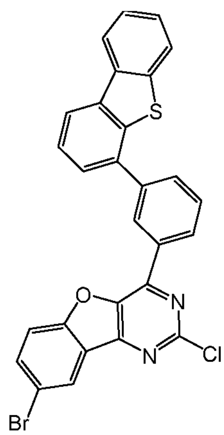 | 65% |
| **23a** | 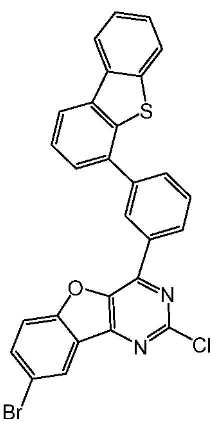 | 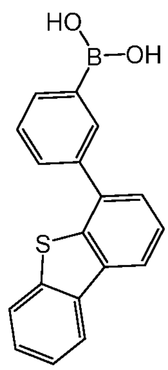 [1307859-67-1 ] | 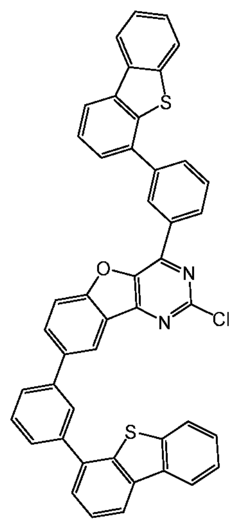 | 72% |

(fortgesetzt)

| | Edukt 1 | Edukt 1 | Produkt | Ausbeute |
|---|---|---|---|---|
| **24a** | 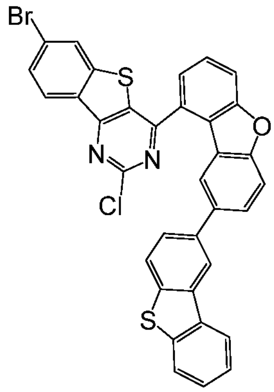 | 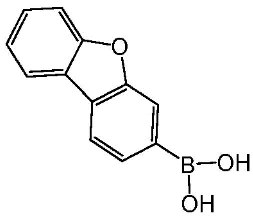 [ 395087-89-5 ] | 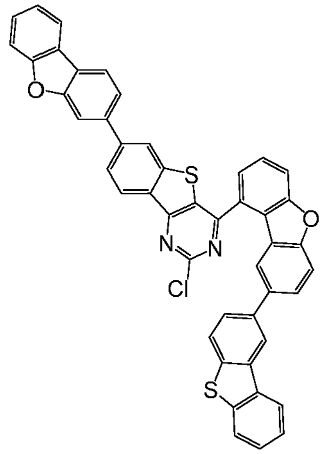 | 65% |
| **25a** | 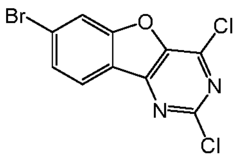 [2201128-38-1 ] | 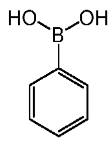 | 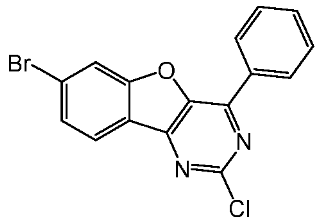 | 56% |
| **26a** | 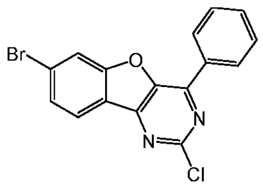 | 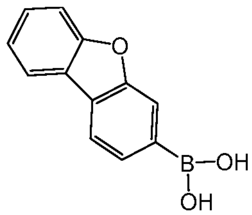 [ 395087-89-5 ] | 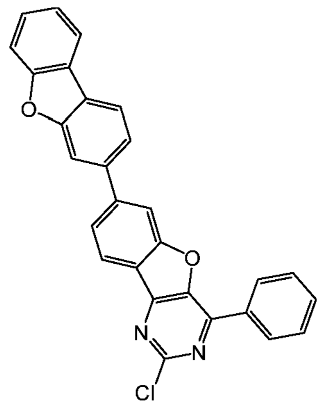 | 77% |
| **27a** | 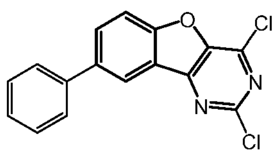 [1835207-37-8] | 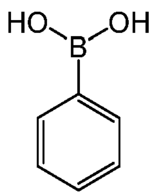 | 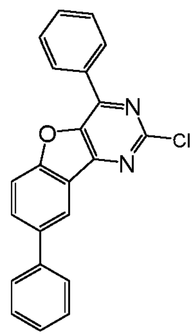 | 65% |

**b) 4,8-Diphenyl-2-[8-(9-phenylcarbazol-3-yl)dibenzofuran-1-yl]benzofuro[3,2-d]pyrimidin**

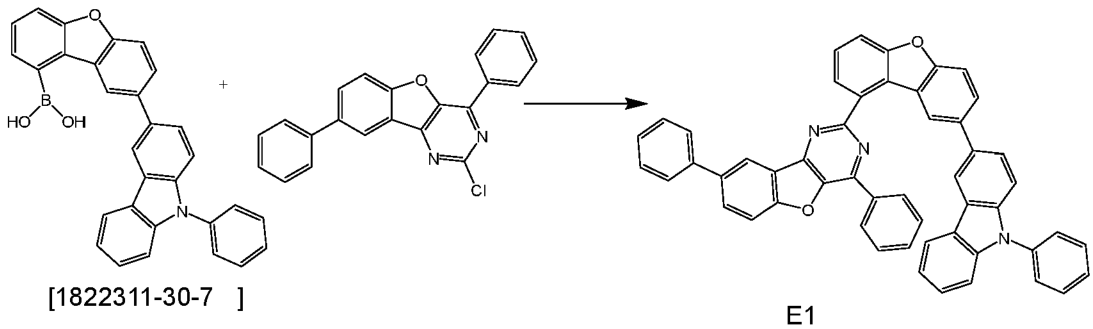

[1822311-30-7 ]

E1

35,6 (100 mmol) 2-Chloro-4,8-diphenyl-benzofuro[3,2-d]pyrimidin, 54,2 g (100 mmol) [8-(9-Phenylcarbazol-3-yl)dibenzofuran-1-yl]boronsäure und 11,8 g (111mmol) Natriumcarbonat werden in 800ml 1,4-Dioxan, 800 ml Wasser und 250ml Toluol gelöst, und unter Argonatmosphäre gerührt. 1,2 g (1mmol) Tetrakis (Triphenylphosphin)-Palladium wird dem Kolben dazugegeben. Das Reaktionsgemisch wird unter Rückfluss über Nacht gerührt. Nach dem Abkühlen wird das Gemisch gequenscht. Die organische Phase wird getrennt, mit 300 ml Wasser drei Mal gewaschen, über $MgSO_4$ getrocknet, filtriert und das Lösungsmittel im Vakuum entfernt. Der Rückstand wird durch Säulenchromatographie über Kieselgel (Eluent: DCM/ Heptan (1:10)) gereinigt. Die Ausbeute beträgt 51 g (71 mmol), entsprechend 71% der Theorie.

Analog dazu werden die folgenden Verbindungen hergestellt:

| | Edukt 1 | Edukt 2 | Produkt | Ausbeute |
|---|---|---|---|---|
| **1b** | 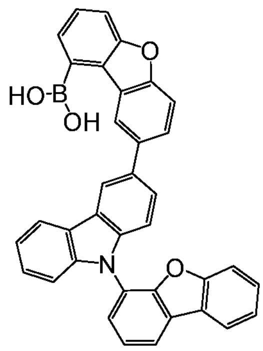 [ 1822311-33-0 ] | 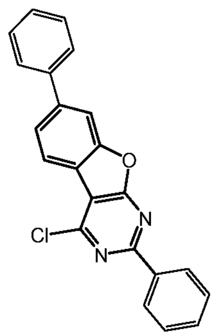 [2201128-28-9] | 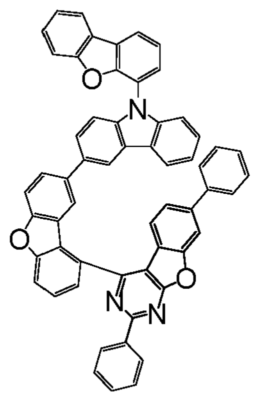 | 72% |
| **2b** | 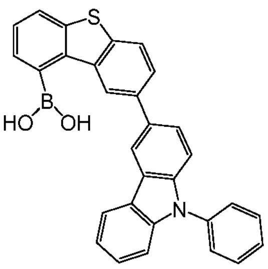 [1822311-32-9] | 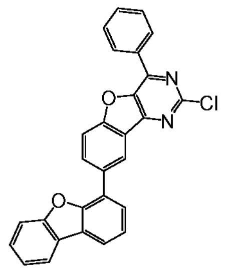 [ 2268732-49-4] | 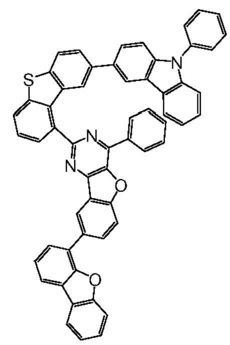 | 75% |

(fortgesetzt)

| | Edukt 1 | Edukt 2 | Produkt | Ausbeute |
|---|---|---|---|---|
| **3b** | 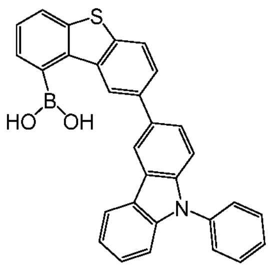 [1822311-32-9] | 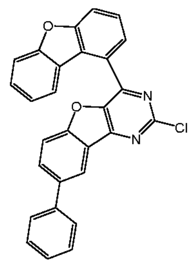 | 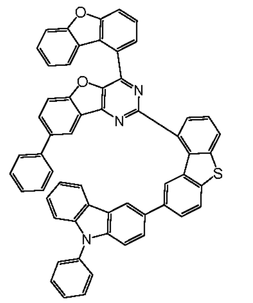 E25 | 70% |
| **4b** | 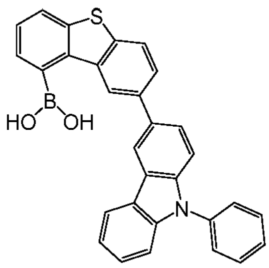 [1822311-32-9] | 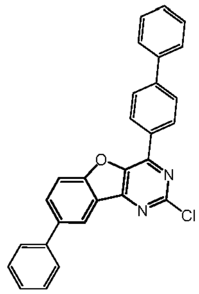 | 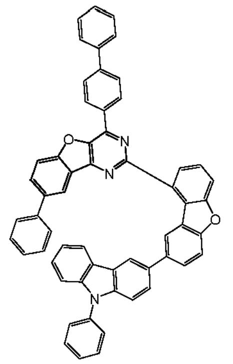 E5 | 68% |
| **5b** | 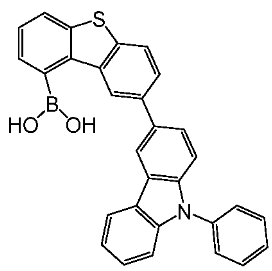 [1822311-32-9] | 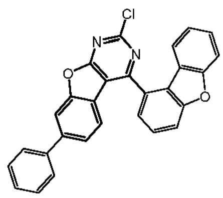 | 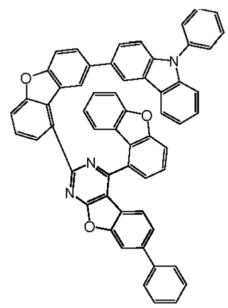 | 67% |
| **6b** | 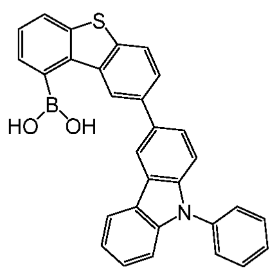 [1822311-32-9] | 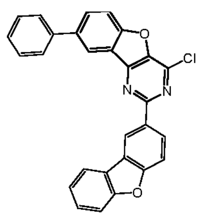 [2268733-22-6 ] | 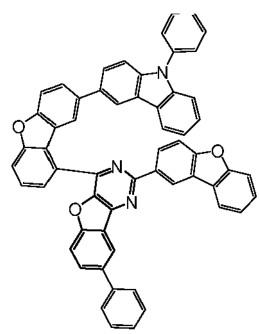 E27 | 82% |

(fortgesetzt)

| | Edukt 1 | Edukt 2 | Produkt | Ausbeute |
|---|---|---|---|---|
| 7b | 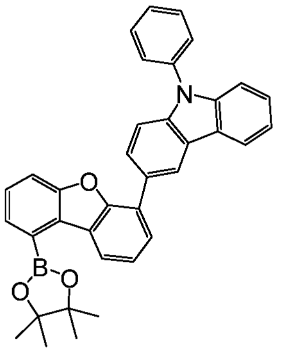 [2096512-72-8 ] | 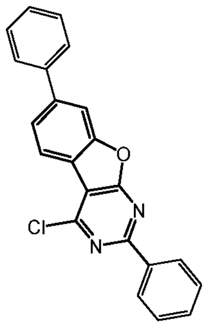 [2201128-28-9] | 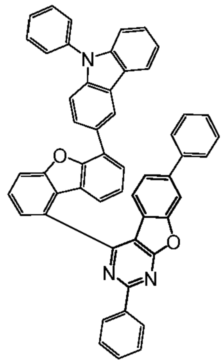 | 73% |
| 8b | 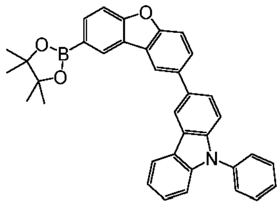 [1582802-05-8 ] | 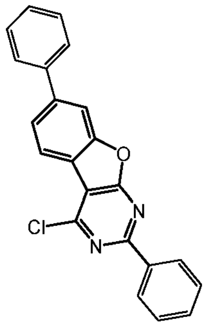 [2201128-28-9] | 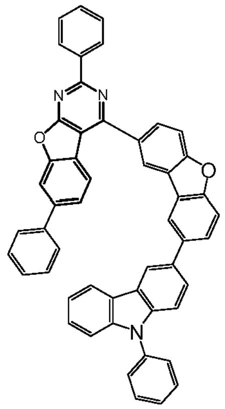 | 79% |
| 9b | 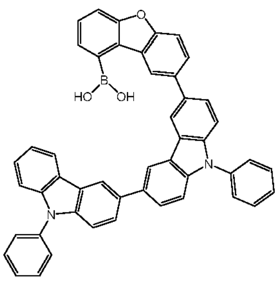 | 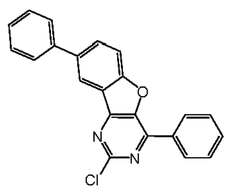 | 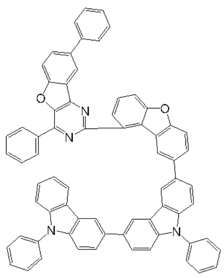 | 69% |
| 11b | 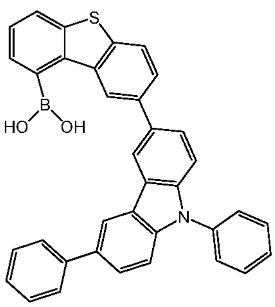 | 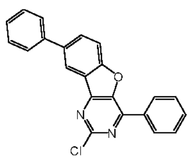 | 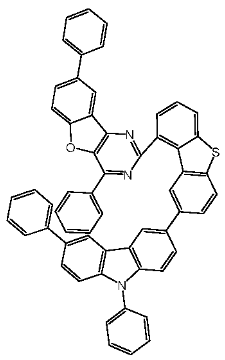 | 76% |

(fortgesetzt)

| | Edukt 1 | Edukt 2 | Produkt | Ausbeute |
|---|---|---|---|---|
| 12b | 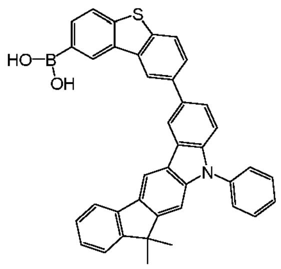 [1307793-77-6 ] | 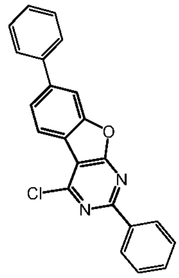 [2201128-28-9] | 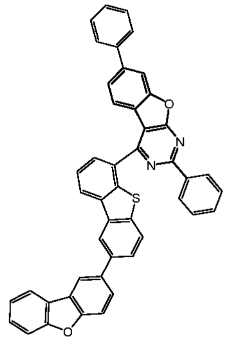 | 74% |
| 13b | 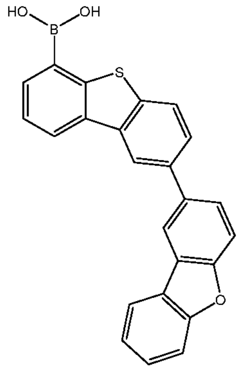 [2411332-76-6 ] | 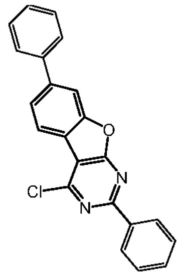 [2201128-28-9] | 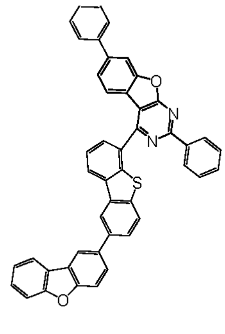 | 77% |
| 14b | 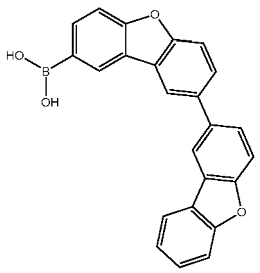 [1427560-52-8 ] | 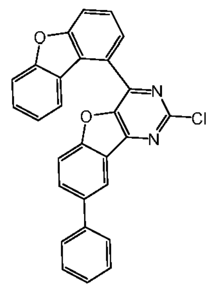 | 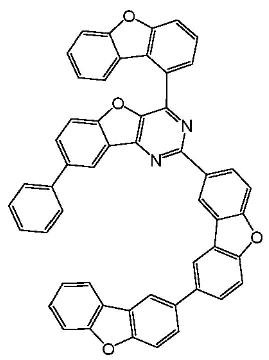 E26 | 81% |
| 15b | 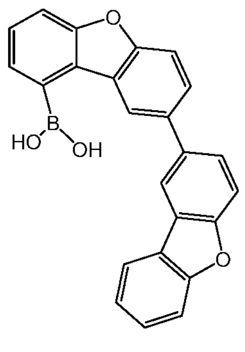 [1822320-55-7 ] | 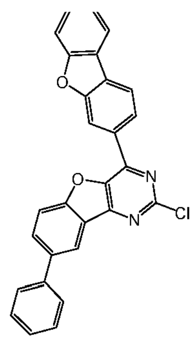 | 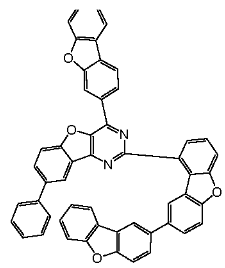 E6 | 80% |

(fortgesetzt)

| | Edukt 1 | Edukt 2 | Produkt | Ausbeute |
|---|---|---|---|---|
| **16b** | 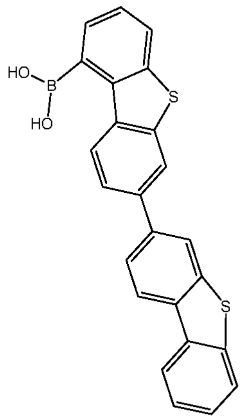 [2412401-01-3 ] | 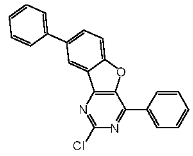 | 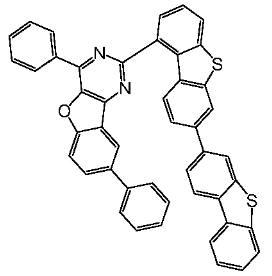 | 83% |
| **17b** | 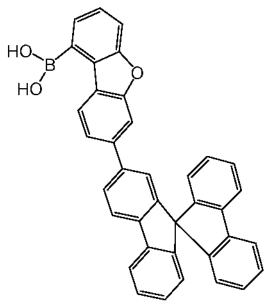 1822311-38-5 ] | 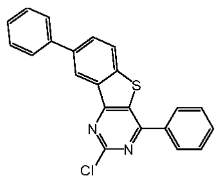 | 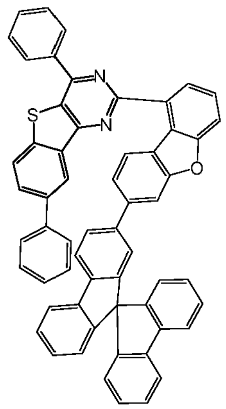 E24 | 78% |
| **18b** | 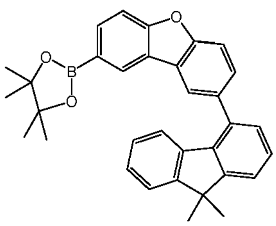 [2392930-01-5 ] | 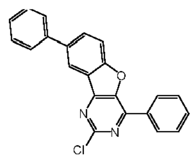 | 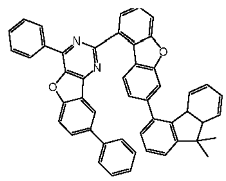 E4 | 81% |
| **19b** | 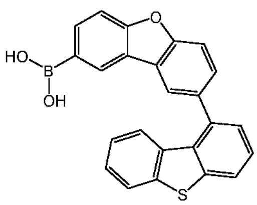 [1779497-51-6 ] | 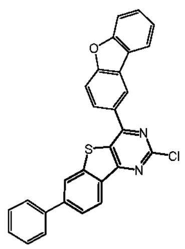 | 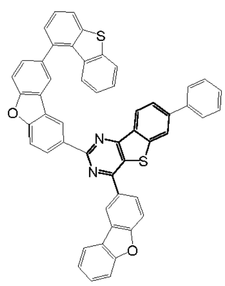 | 80% |

(fortgesetzt)

| | Edukt 1 | Edukt 2 | Produkt | Ausbeute |
|---|---|---|---|---|
| **20b** | 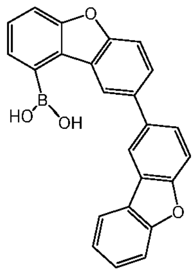 [1822320-55-7 ] | 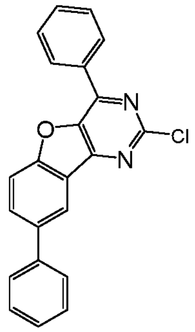 | 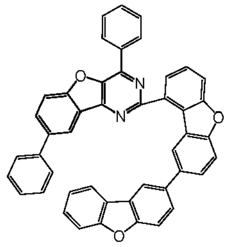 E2 | 79% |
| **21b** | 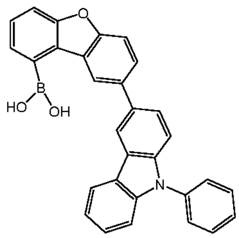 [1822311-30-7 ] | 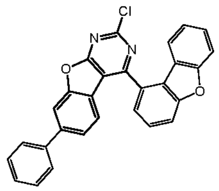 | 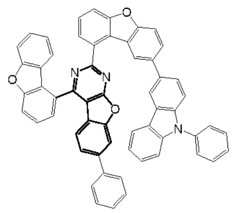 E23 | 76% |
| **22b** | 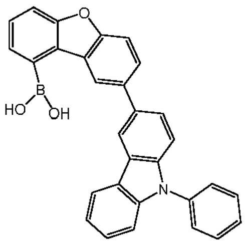 [1822311-30-7 ] | 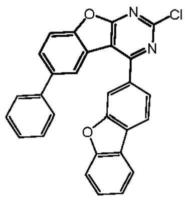 | 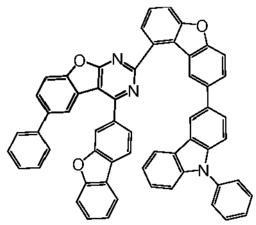 E7 | 65% |
| **23b** | 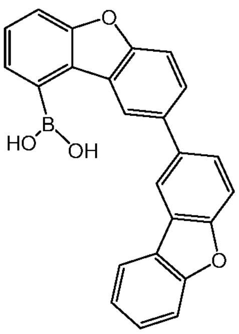 [1822320-55-7 ] | 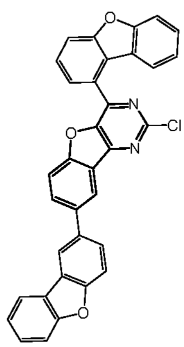 | 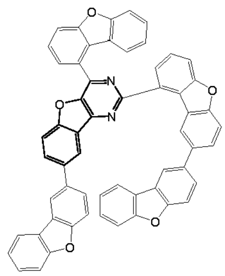 E22 | 78% |

(fortgesetzt)

| | Edukt 1 | Edukt 2 | Produkt | Ausbeute |
|---|---|---|---|---|
| **24b** | 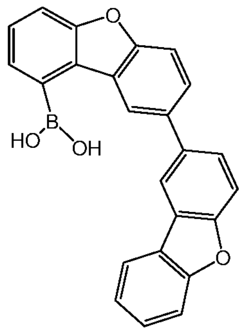 [1822320-55-7 ] | 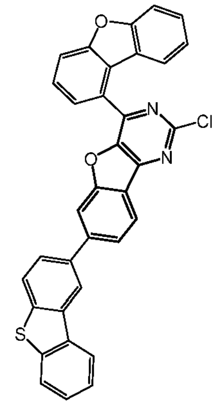 | 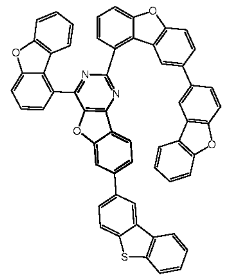 E9 | 68% |
| **25b** | 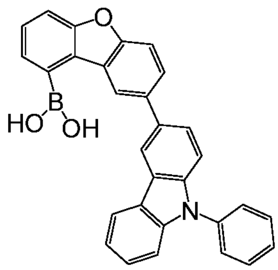 [1822311-30-7 ] | 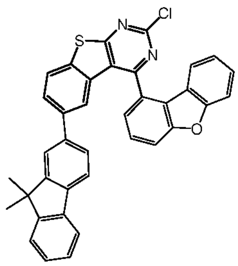 | 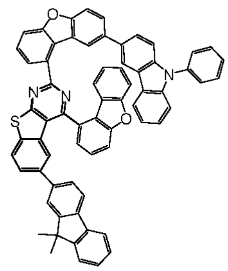 | 67% |
| **26b** | 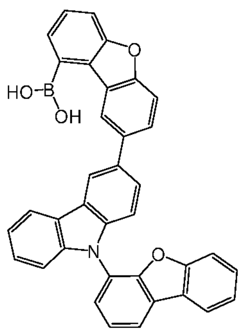 [1822311-33-0] | 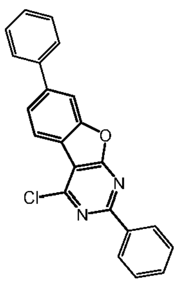 [2201128-28-9] | 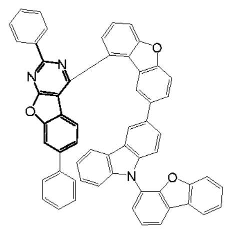 | 81% |
| **27b** | 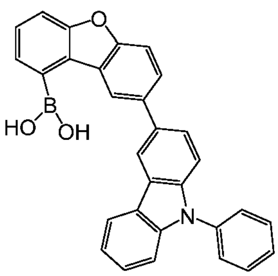 [1822311-30-7] | 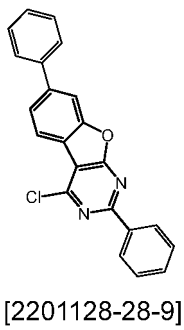 [2201128-28-9] | 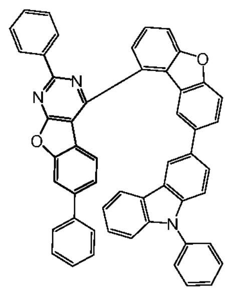 E10 | 76% |

(fortgesetzt)

| | Edukt 1 | Edukt 2 | Produkt | Ausbeute |
|---|---|---|---|---|
| **28b** | 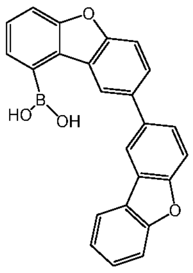 [1822320-55-7 ] | 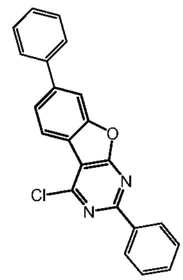 [2201128-28-9] | 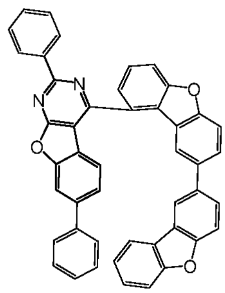 E8 | 79% |
| **29b** | 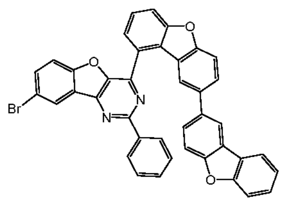 | 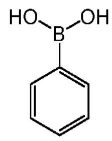 | 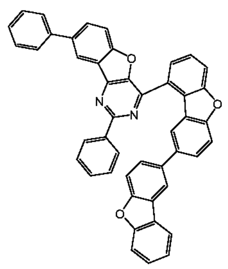 E3 | 81% |
| **30b** | 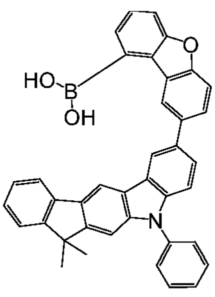 [1822311-35-2] | 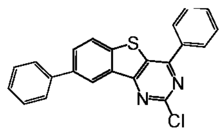 | 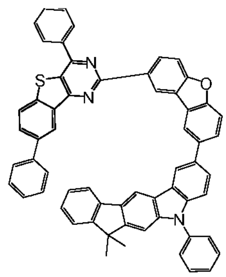 E11 | 70% |
| **31b** | 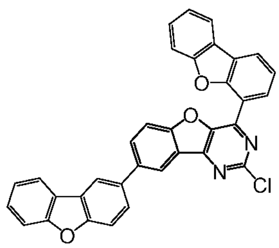 | 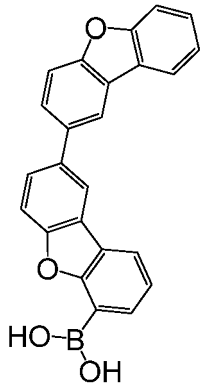 [2411326-31-1 ] | 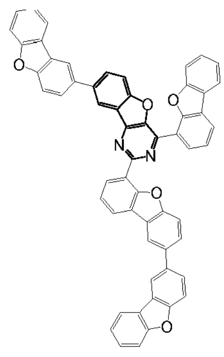 | 75% |

(fortgesetzt)

| | Edukt 1 | Edukt 2 | Produkt | Ausbeute |
|---|---|---|---|---|
| **32b** | 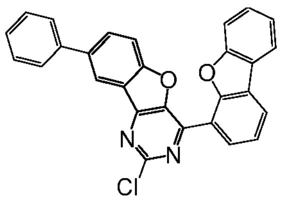 | 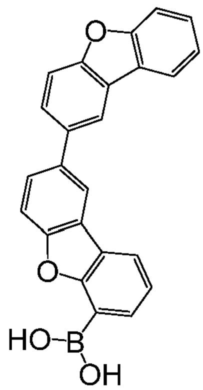 [2411326-31-1 ] | 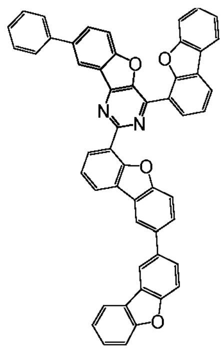 E12 | 66% |
| **33b** | 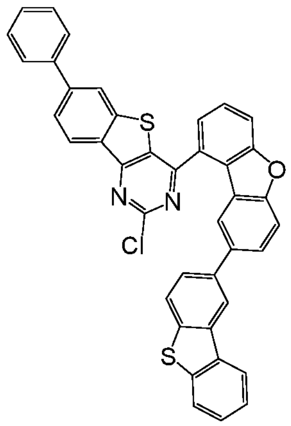 | 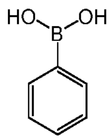 | 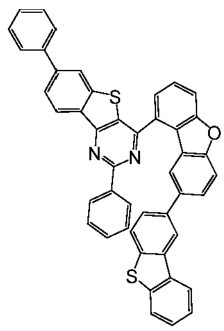 E13 | 69% |
| **34b** | 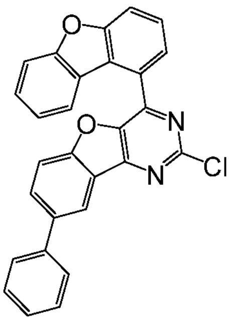 | 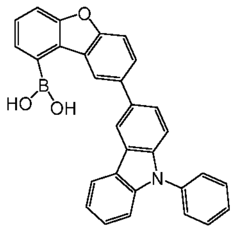 [1822311-30-7 ] | 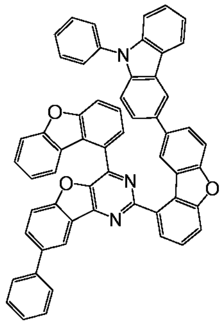 E14 | 68% |
| **35b** | 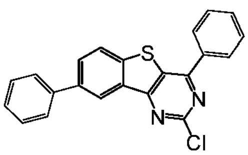 | 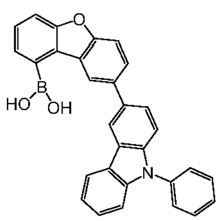 [1822311-30-7 ] | 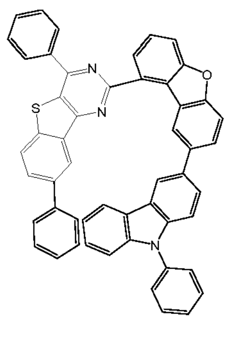 E15 | 64% |

(fortgesetzt)

| | Edukt 1 | Edukt 2 | Produkt | Ausbeute |
|---|---|---|---|---|
| **36b** | 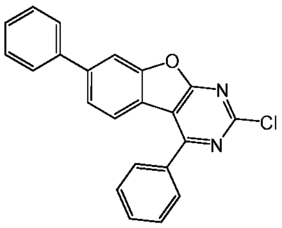 | 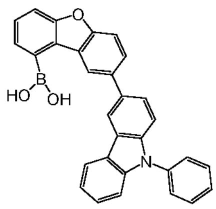 [1822311-30-7 ] | 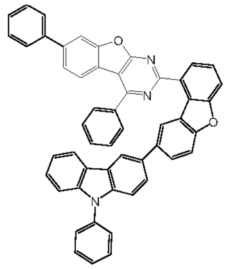 E16 | 63% |
| **37b** | 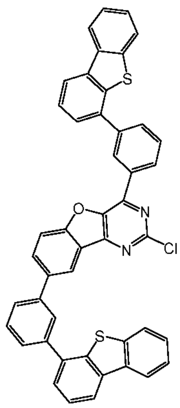 | 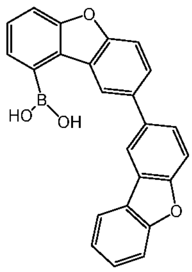 [1822320-55-7 ] | 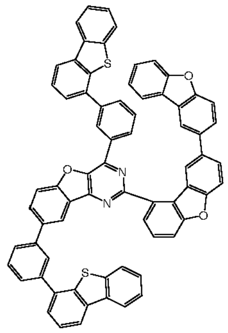 E17 | 72% |
| **38b** | 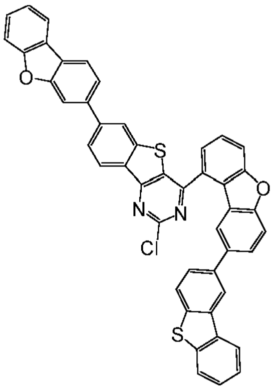 | 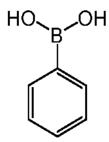 | 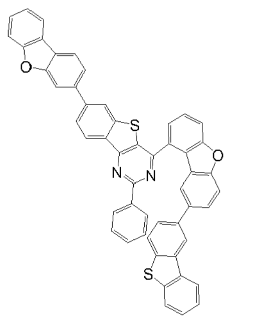 E18 | 70% |
| **39b** | 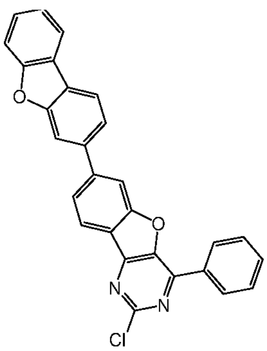 | 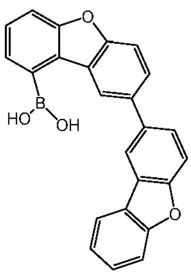 [1822320-55-7 ] | 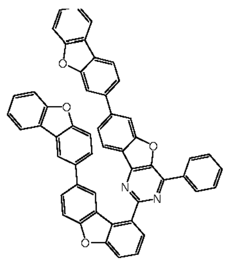 E19 | 73% |

(fortgesetzt)

| | Edukt 1 | Edukt 2 | Produkt | Ausbeute |
|---|---|---|---|---|
| **40b** | 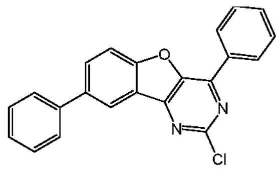 | 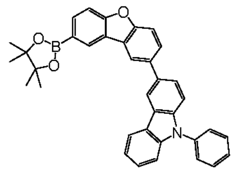 [1582802-05-8 ] | 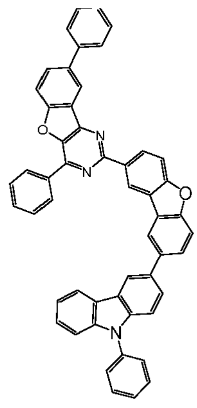 E20 | 76% |

## Patentansprüche

1. Organische elektrolumineszierende Vorrichtung umfassend eine Anode, eine Kathode und mindestens eine organische Schicht, enthaltend mindestens eine lichtemittierende Schicht, wobei die mindestens eine lichtemittierende Schicht mindestens eine Verbindung der Formel (1) als Hostmaterial 1 und mindestens eine Verbindung der Formel (2) als Hostmaterial 2 enthält,

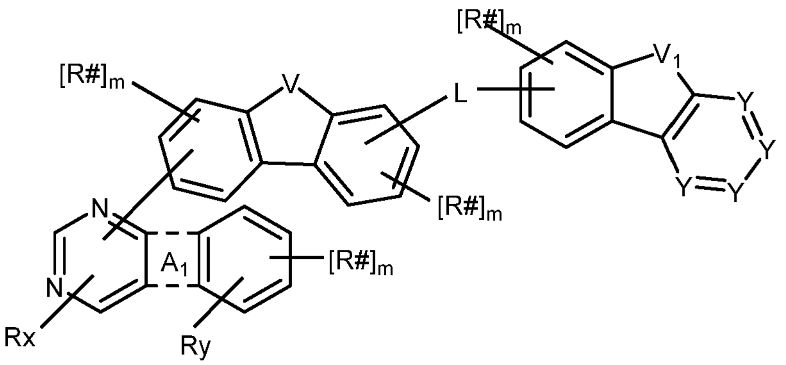

Formel (1) ,

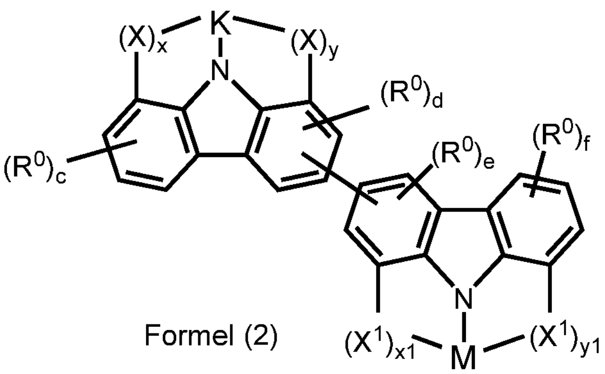

Formel (2) ,

wobei für die verwendeten Symbole und Indizes gilt:
Ring $A_1$ in Formel (1) entspricht der Formel (1A)

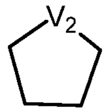

Formel (1A)

V ist O oder S;
$V_1$ ist O, S, $C(R^1)_2$ oder N-A;
$V_2$ ist O oder S;
Y ist bei jedem Auftreten unabhängig voneinander gleich oder verschieden CH, CR oder CA, oder zwei benachbarte Gruppen Y bilden zusammen eine Gruppe der Formel (1B)

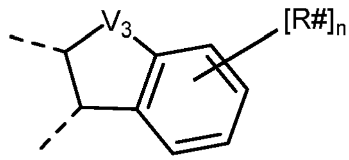

Formel (1B)

und die restlichen Gruppen Y sind unabhängig voneinander CH oder CR, wobei $V_3$ O, S, $C(R^1)_2$ oder N-A bedeutet und die gestrichelten Bindungen die Verknüpfung dieser Gruppe an den Rest der Formel (1) zeigen;
Rx ist $L_1$-R*;
Ry ist $L_2$-R*;
R* ist bei jedem Auftreten unabhängig ein aromatisches oder heteroaromatisches Ringsystem mit 6 bis 14 Ringatomen, das mit einem oder mehreren Resten R substituiert sein kann;
L, $L_1$, $L_2$ sind jeweils unabhängig voneinander eine Bindung oder eine Phenylengruppe, die jeweils mit einem oder mehreren Resten R substituiert sein kann;
A ist bei jedem Auftreten gleich oder verschieden ein aromatisches oder heteroaromatisches Ringsystem mit 6 bis 30 Ringatomen, das mit einem oder mehreren Resten R substituiert sein kann;
n ist 0, 1, 2, 3 oder 4;
m ist 0, 1, 2 oder 3;
R# ist D oder eine Arylgruppe mit 6 bis 12 C-Atomen, die mit einem oder mehreren Resten R substituiert sein kann;
$R^1$ ist bei jedem Auftreten gleich oder verschieden eine Alkylgruppe mit 1 bis 20 C-Atomen, Phenyl, das mit einem oder mehreren Resten R substituiert sein kann oder die zwei $R^1$ bilden zusammen mit dem C-Atom an das sie binden eine Spiroverbindung;
R ist bei jedem Auftreten gleich oder verschieden ausgewählt aus D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch $R^2C=CR^2$, O oder S ersetzt sein können und wobei ein oder mehrere H-Atome durch D, F, oder CN ersetzt sein können;
$R^2$ ist bei jedem Auftreten gleich oder verschieden ausgewählt aus der Gruppe bestehend aus H, D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch O oder S ersetzt sein können und wobei ein oder mehrere H-Atome durch D, F, oder CN ersetzt sein können;
K, M sind jeweils unabhängig voneinander ein unsubstituiertes oder teilweise oder vollständig deuteriertes oder einfach durch R* substituiertes aromatisches Ringsystem mit 6 bis 40 Ringatomen, wenn x und y 0 bedeuten und wenn x1 und y1 0 bedeuten, oder
K, M bilden jeweils unabhängig voneinander zusammen mit X oder $X^1$ ein heteroaromatisches Ringsystem mit 14 bis 40 Ringatomen, sobald der Wert von x, x1, y und/oder y1 1 bedeutet;
x, x1 sind jeweils unabhängig bei jedem Auftreten 0 oder 1;
y, y1 sind jeweils unabhängig bei jedem Auftreten 0 oder 1;
X und $X^1$ sind jeweils unabhängig voneinander bei jedem Auftreten eine Bindung oder $C(R^+)_2$;
$R^0$ ist bei jedem Auftreten unabhängig voneinander ein unsubstituiertes oder teilweise oder vollständig deuteriertes aromatisches Ringsystem mit 6 bis 18 C-Atomen;
$R^+$ ist bei jedem Auftreten unabhängig voneinander eine geradkettige oder verzweigte Alkylgruppe mit 1 bis 4 C-Atomen und
c, d, e und f sind unabhängig voneinander 0 oder 1.

2. Organische elektrolumineszierende Vorrichtung nach Anspruch 1, **dadurch gekennzeichnet, dass** das Hostmaterial 1 der Formel (1a) entspricht,

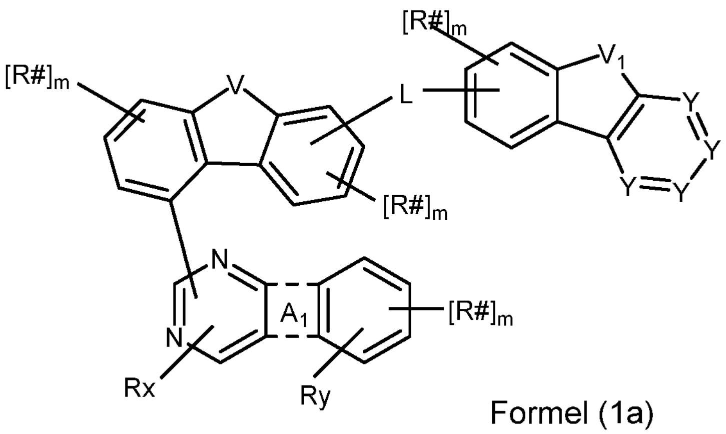

Formel (1a) ,

wobei die verwendeten Symbole Y, V, $V_1$, R#, m, Rx, Ry, L und Ring $A_1$ eine Bedeutung wie in Anspruch 1 haben.

**3.** Organische elektrolumineszierende Vorrichtung nach Anspruch 1 oder 2, **dadurch gekennzeichnet, dass** $V_1$ O oder NA bedeutet.

**4.** Organische elektrolumineszierende Vorrichtung nach einem oder mehreren der Ansprüche 1 bis 3, **dadurch gekennzeichnet, dass** das Hostmaterial 2 einer der Formeln (2a), (2b) oder (2c) entspricht

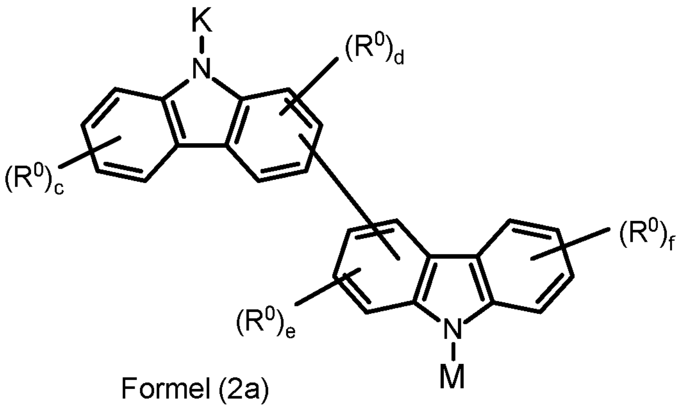

Formel (2a) ,

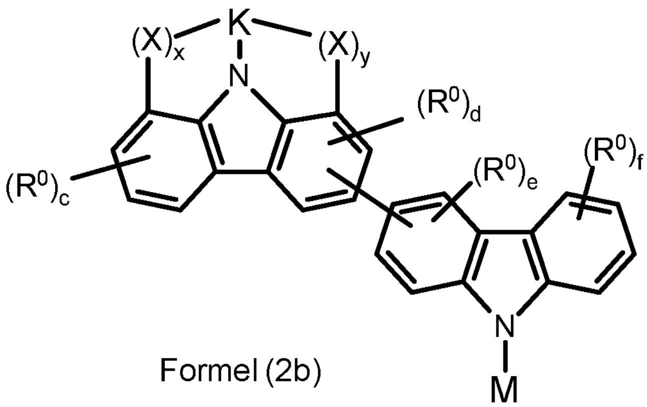

Formel (2b) ,

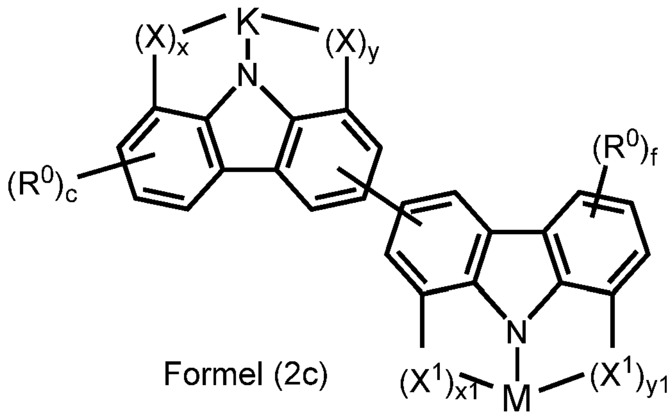

Formel (2c)

wobei die verwendeten Symbole und Indizes X, $X^1$, $R^0$, c, d, e und f eine Bedeutung wie in Anspruch 1 haben und K und M in Verbindungen der Formel (2a) jeweils unabhängig voneinander ein unsubstituiertes oder teilweise oder vollständig deuteriertes oder einfach durch R* substituiertes aromatisches Ringsystem mit 6 bis 40 Ringatomen bedeuten;
M in Verbindungen der Formel (2b) ein unsubstituiertes oder teilweise oder vollständig deuteriertes oder einfach durch R* substituiertes aromatisches Ringsystem mit 6 bis 40 Ringatomen bedeutet;
K in Verbindungen der Formel (2b) zusammen mit X ein heteroaromatisches Ringsystem mit 14 bis 40 Ringatomen bildet und x und y in Verbindungen der Formel (2b) jeweils unabhängig voneinander 0 oder 1 bedeuten und die Summe von x und y mindestens 1 bedeutet; und
K und M in Verbindungen der Formel (2c) jeweils unabhängig voneinander zusammen mit X oder $X^1$ ein heteroaromatisches Ringsystem mit 14 bis 40 Ringatomen bilden; und
x, x1, y und y1 in Verbindungen der Formel (2c) jeweils unabhängig voneinander 0 oder 1 bedeuten und die Summe von x und y mindestens 1 bedeutet und die Summe von x1 und y1 mindestens 1 bedeutet.

5. Organische elektrolumineszierende Vorrichtung nach einem oder mehreren der Ansprüche 1 bis 4, **dadurch gekennzeichnet, dass** es sich um eine Elektrolumineszenzvorrichtung handelt, die ausgewählt ist aus den organischen lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und den organischen lichtemittierenden Dioden (OLEDs).

6. Organische elektrolumineszierende Vorrichtung nach einem oder mehreren der Ansprüche 1 bis 5, **dadurch gekennzeichnet, dass** diese organische Schicht neben der lichtemittierenden Schicht (EML), eine Lochinjektionsschicht (HIL), eine Lochtransportschicht (HTL), eine Elektronentransportschicht (ETL), eine Elektroneninjektionsschicht (EIL) und/oder eine Lochblockierschicht (HBL) enthält.

7. Organische elektrolumineszierende Vorrichtung nach einem oder mehreren der Ansprüche 1 bis 6, **dadurch gekennzeichnet, dass** die lichtemittierende Schicht neben dem mindestens einen Hostmaterial 1 und dem mindestens einen Hostmaterial 2 mindestens einen phosphoreszierenden Emitter enthält.

8. Organische elektrolumineszierende Vorrichtung nach einem oder mehreren der Ansprüche 1 bis 7, **dadurch gekennzeichnet, dass** die organische Schicht eine Lochinjektionsschicht (HIL) und/oder eine Lochtransportschicht (HTL) enthält, deren lochinjizierendes Material und lochtransportierendes Material ein Monoamin ist, welches keine Carbazoleinheit enthält.

9. Verfahren zur Herstellung einer Vorrichtung nach einem oder mehreren der Ansprüche 1 bis 8, **dadurch gekennzeichnet, dass** die organische Schicht durch Gasphasenabscheidung oder aus Lösung aufgebracht wird.

10. Verfahren nach Anspruch 9, **dadurch gekennzeichnet, dass** zur Herstellung der lichtemittierenden Schicht die mindestens eine Verbindung der Formel (1) und die mindestens eine Verbindung der Formel (2), nacheinander oder gleichzeitig aus mindestens zwei Materialquellen, gegebenenfalls mit dem mindestens einen phosphoreszierenden Emitter, aus der Gasphase abgeschieden werden.

11. Verfahren nach Anspruch 9, **dadurch gekennzeichnet, dass** zur Herstellung der lichtemittierenden Schicht die

mindestens eine Verbindung der Formel (1) und die mindestens eine Verbindung der Formel (2) als Mischung, nacheinander oder gleichzeitig mit dem mindestens einen phosphoreszierenden Emitter, aus der Gasphase abgeschieden werden.

**12.** Verfahren nach Anspruch 9, **dadurch gekennzeichnet, dass** zur Herstellung der lichtemittierenden Schicht die mindestens eine Verbindung der Formel (1) und die mindestens eine Verbindung der Formel (2) zusammen mit dem mindestens einen phosphoreszierenden Emitter, aus einer Lösung aufgebracht werden.

**13.** Mischung enthaltend mindestens eine Verbindung der Formel (1) und mindestens eine Verbindung der Formel (2),

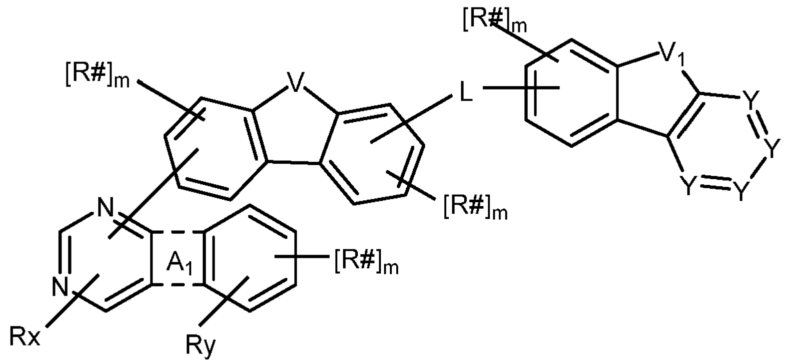

Formel (1)

,

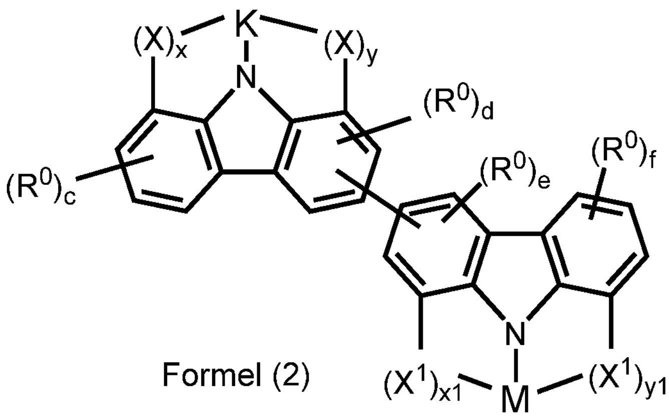

Formel (2)

,

wobei für die verwendeten Symbole und Indizes gilt:
Ring $A_1$ in Formel (1) entspricht der Formel (1A)

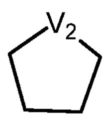

Formel (1A)

V ist O oder S;
$V_1$ ist O, S, $C(R^1)_2$ oder N-A;
$V_2$ ist O oder S;
Y ist bei jedem Auftreten unabhängig voneinander gleich oder verschieden CH, CR oder CA, oder zwei benachbarte Gruppen Y bilden zusammen eine Gruppe der Formel (1B)

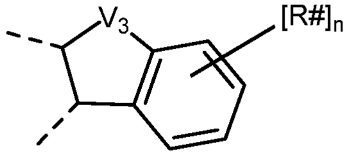

Formel (1B)

und die restlichen Gruppen Y sind unabhängig voneinander CH oder CR, wobei $V_3$ O, S, $C(R^1)_2$ oder N-A bedeutet und die gestrichelten Bindungen die Verknüpfung dieser Gruppe an den Rest der Formel (1) zeigen;
Rx ist $L_1$-R*;
Ry ist $L_2$-R*;
R* ist bei jedem Auftreten unabhängig ein aromatisches oder heteroaromatisches Ringsystem mit 6 bis 14 Ringatomen, das mit einem oder mehreren Resten R substituiert sein kann;
L, $L_1$, $L_2$ sind jeweils unabhängig voneinander eine Bindung oder eine Phenylengruppe, die jeweils mit einem oder mehreren Resten R substituiert sein kann;
A ist bei jedem Auftreten gleich oder verschieden ein aromatisches oder heteroaromatisches Ringsystem mit 6 bis 30 Ringatomen, das mit einem oder mehreren Resten R substituiert sein kann;
n ist 0, 1, 2, 3 oder 4;
m ist 0, 1, 2 oder 3;
R# ist D oder eine Arylgruppe mit 6 bis 12 C-Atomen, die mit einem oder mehreren Resten R substituiert sein kann;
$R^1$ ist bei jedem Auftreten gleich oder verschieden eine Alkylgruppe mit 1 bis 20 C-Atomen, Phenyl, das mit einem oder mehreren Resten R substituiert sein kann oder die zwei $R^1$ bilden zusammen mit dem C-Atom an das sie binden eine Spiroverbindung;
R ist bei jedem Auftreten gleich oder verschieden ausgewählt aus D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch $R^2C=CR^2$, O oder S ersetzt sein können und wobei ein oder mehrere H-Atome durch D, F, oder CN ersetzt sein können;
$R^2$ ist bei jedem Auftreten gleich oder verschieden ausgewählt aus der Gruppe bestehend aus H, D, F, CN, einer geradkettigen Alkylgruppe mit 1 bis 20 C-Atomen oder einer verzweigten oder cyclischen Alkylgruppe mit 3 bis 20 C-Atomen, wobei eine oder mehrere nicht-benachbarte $CH_2$-Gruppen durch O oder S ersetzt sein können und wobei ein oder mehrere H-Atome durch D, F, oder CN ersetzt sein können;
K, M sind jeweils unabhängig voneinander ein unsubstituiertes oder teilweise oder vollständig deuteriertes oder einfach durch R* substituiertes aromatisches Ringsystem mit 6 bis 40 Ringatomen, wenn x und y 0 bedeuten und wenn x1 und y1 0 bedeuten, oder
K, M bilden jeweils unabhängig voneinander zusammen mit X oder $X^1$ ein heteroaromatisches Ringsystem mit 14 bis 40 Ringatomen, sobald der Wert von x, x1, y und/oder y1 1 bedeutet;
x, x1 sind jeweils unabhängig bei jedem Auftreten 0 oder 1;
y, y1 sind jeweils unabhängig bei jedem Auftreten 0 oder 1;
X und $X^1$ sind jeweils unabhängig voneinander bei jedem Auftreten eine Bindung oder $C(R^+)_2$;
$R^0$ ist bei jedem Auftreten unabhängig voneinander ein unsubstituiertes oder teilweise oder vollständig deuteriertes aromatisches Ringsystem mit 6 bis 18 C-Atomen;
$R^+$ ist bei jedem Auftreten unabhängig voneinander eine geradkettige oder verzweigte Alkylgruppe mit 1 bis 4 C-Atomen und
c, d, e und f sind unabhängig voneinander 0 oder 1.

**14.** Mischung nach Anspruch 13, **dadurch gekennzeichnet, dass** die Mischung aus mindestens einer Verbindung der Formel (1), mindestens einer Verbindung der Formel (2) und einem phosphoreszierenden Emitter besteht.

**15.** Formulierung enthaltend eine Mischung nach Anspruch 13 oder 14 sowie mindestens ein Lösemittel.

## Claims

**1.** Organic electroluminescent device comprising an anode, a cathode and at least one organic layer, comprising at least one light-emitting layer, where the at least one light-emitting layer comprises at least one compound of the formula (1) as host material 1 and at least one compound of the formula (2) as host material 2,

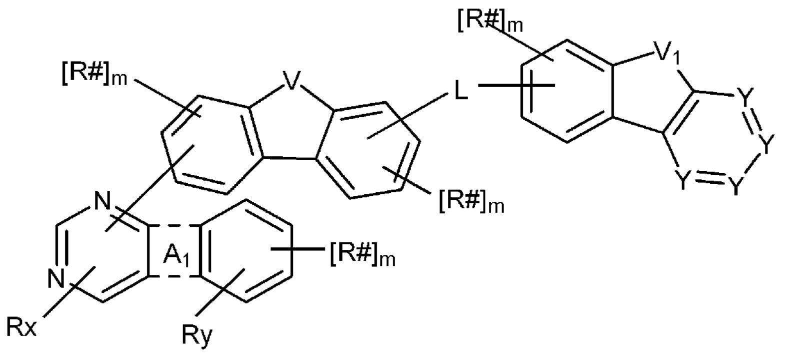

Formula (1)

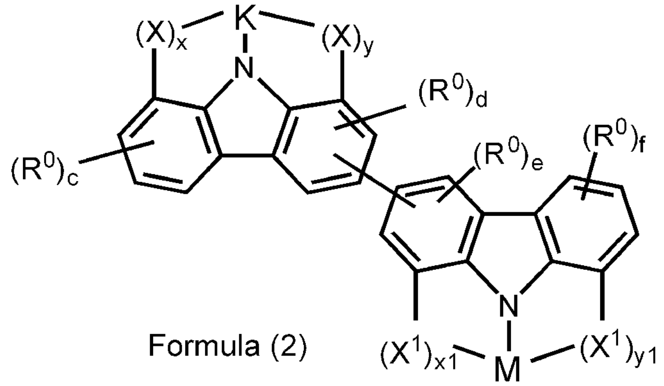

Formula (2)

where the following applies to the symbols and indices used:
ring $A_1$ in formula (1) conforms to the formula (1A)

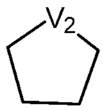

Formula (1A)

V is O or S;
$V_1$ is O, S, $C(R^1)_2$ or N-A;
$V_2$ is O or S;
Y is on each occurrence, independently of one another, identically or differently, CH, CR or CA, or two adjacent groups Y together form a group of the formula (1B)

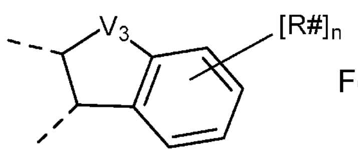

Formula (1B)

and the remaining groups Y are, independently of one another, CH or CR, where $V_3$ denotes O, S, $C(R^1)_2$ or N-A and the dashed bonds show the linking of this group to the remainder of the formula (1);
Rx is $L_1$-R*;
Ry is $L_2$-R*;
R* is on each occurrence, independently, an aromatic or heteroaromatic ring system having 6 to 14 ring atoms, which may be substituted by one or more radicals R;
L, $L_1$, $L_2$ are in each case, independently of one another, a bond or a phenylene group, which may in each case be substituted by one or more radicals R;
A is on each occurrence, identically or differently, an aromatic or heteroaromatic ring system having 6 to 30 ring atoms, which may be substituted by one or more radicals R;

n is 0, 1, 2, 3 or 4;
m is 0, 1, 2 or 3;
R# is D or an aryl group having 6 to 12 C atoms, which may be substituted by one or more radicals R;
$R^1$ is on each occurrence, identically or differently, an alkyl group having 1 to 20 C atoms, phenyl, which may be substituted by one or more radicals R, or the two $R^1$, together with the C atom to which they are bonded, form a spiro compound;
R is selected on each occurrence, identically or differently, from D, F, CN, a straight-chain alkyl group having 1 to 20 C atoms or a branched or cyclic alkyl group having 3 to 20 C atoms, where one or more non-adjacent $CH_2$ groups may be replaced by $R^2C=CR^2$, O or S and where one or more H atoms may be replaced by D, F or CN;
$R^2$ is selected on each occurrence, identically or differently, from the group consisting of H, D, F, CN, a straight-chain alkyl group having 1 to 20 C atoms or a branched or cyclic alkyl group having 3 to 20 C atoms, where one or more non-adjacent $CH_2$ groups may be replaced by O or S and where one or more H atoms may be replaced by D, F or CN;
K, M are in each case, independently of one another, an unsubstituted or partially or fully deuterated or mono-R*-substituted aromatic ring system having 6 to 40 ring atoms if x and y denote 0 and if x1 and y1 denote 0, or K, M in each case, independently of one another, form, together with X or $X^1$, a heteroaromatic ring system having 14 to 40 ring atoms as soon as the value of x, x1, y and/or y1 denotes 1;
x, x1 are in each case, independently on each occurrence, 0 or 1;
y, y1 are in each case, independently on each occurrence, 0 or 1;
X and $X^1$ are in each case, independently on each occurrence, a bond or $C(R^+)_2$;
$R^0$ is on each occurrence, independently of one another, an unsubstituted or partially or fully deuterated aromatic ring system having 6 to 18 C atoms;
$R^+$ is on each occurrence, independently of one another, a straight-chain or branched alkyl group having 1 to 4 C atoms and
c, d, e and f are, independently of one another, 0 or 1.

2. Organic electroluminescent device according to Claim 1, **characterised in that** the host material 1 conforms to the formula (1a)

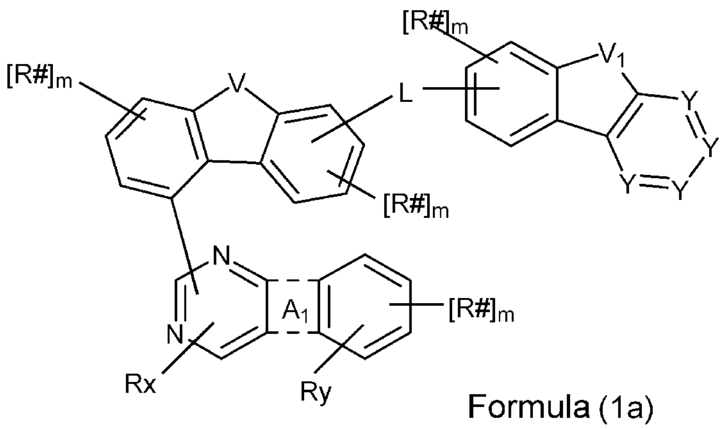

Formula (1a)

where the symbols Y, V, $V_1$, R#, m, Rx, Ry, L and ring $A_1$ used have a meaning as in Claim 1.

3. Organic electroluminescent device according to Claim 1 or 2, **characterised in that** $V_1$ denotes O or NA.

4. Organic electroluminescent device according to one or more of Claims 1 to 3, **characterised in that** the host material 2 conforms to one of the formulae (2a), (2b) or (2c)

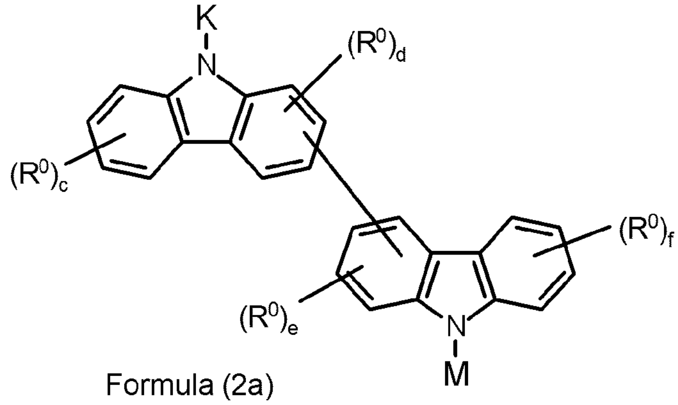

Formula (2a)

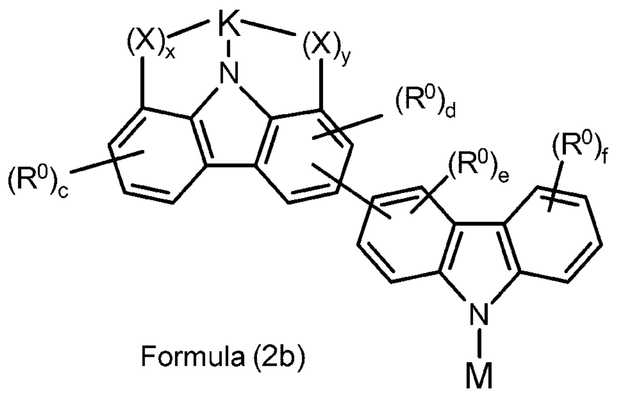

Formula (2b)

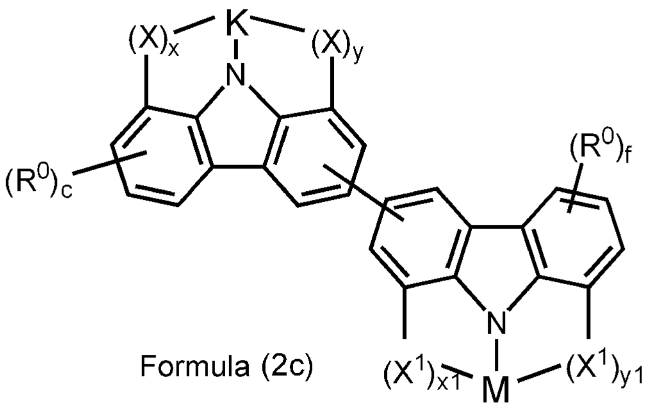

Formula (2c)

where the symbols and indices X, $X^1$, $R^0$, c, d, e and f used have a meaning as in Claim 1 and
K and M in compounds of the formula (2a) in each case, independently of one another, denote an aromatic ring system having 6 to 40 ring atoms which is unsubstituted or partially or fully deuterated or monosubstituted by R*;
M in compounds of the formula (2b) denotes an aromatic ring system having 6 to 40 ring atoms which is unsubstituted or partially or fully deuterated or monosubstituted by R*;
K in compounds of the formula (2b) forms, together with X, a heteroaromatic ring system having 14 to 40 ring atoms and x and y in compounds of the formula (2b) in each case, independently of one another, denote 0 or 1 and the sum of x and y denotes at least 1; and
K and M in compounds of the formula (2c) in each case, independently of one another, form, together with X or $X^1$, a heteroaromatic ring system having 14 to 40 ring atoms; and
x, x1, y and y1 in compounds of the formula (2c) in each case, independently of one another, denote 0 or 1 and the sum of x and y denotes at least 1 and the sum of x1 and y1 denotes at least 1.

**5.** Organic electroluminescent device according to one or more of Claims 1 to 4, **characterised in that** it is an electroluminescent device selected from organic light-emitting transistors (OLETs), organic field-quench devices (OFQDs), organic light-emitting electrochemical cells (OLECs, LECs, LEECs), organic laser diodes (O-lasers) and

organic light-emitting diodes (OLEDs).

6. Organic electroluminescent device according to one or more of Claims 1 to 5, **characterised in that** this organic layer, besides the light-emitting layer (EML), comprises a hole-injection layer (HIL), a hole-transport layer (HTL), an electron-transport layer (ETL), an electron-injection layer (EIL) and/or a hole-blocking layer (HBL).

7. Organic electroluminescent device according to one or more of Claims 1 to 6, **characterised in that** the light-emitting layer, besides the at least one host material 1 and the at least one host material 2, comprises at least one phosphorescent emitter.

8. Organic electroluminescent device according to one or more of Claims 1 to 7, **characterised in that** the organic layer comprises a hole-injection layer (HIL) and/or a hole-transport layer (HTL) whose hole-injecting material and hole-transporting material is a monoamine which does not contain a carbazole unit.

9. Process for the production of a device according to one or more of Claims 1 to 8, **characterised in that** the organic layer is applied by gas-phase deposition or from solution.

10. Process according to Claim 9, **characterised in that**, for the production of the light-emitting layer, the at least one compound of the formula (1) and the at least one compound of the formula (2) are deposited from the gas phase successively or simultaneously from at least two material sources, optionally with the at least one phosphorescent emitter.

11. Process according to Claim 9, **characterised in that**, for the production of the light-emitting layer, the at least one compound of the formula (1) and the at least one compound of the formula (2) are deposited from the gas phase as a mixture, successively or simultaneously with the at least one phosphorescent emitter.

12. Process according to Claim 9, **characterised in that**, for the production of the light-emitting layer, the at least one compound of the formula (1) and the at least one compound of the formula (2) are applied from a solution together with the at least one phosphorescent emitter.

13. Mixture comprising at least one compound of the formula (1) and at least one compound of the formula (2),

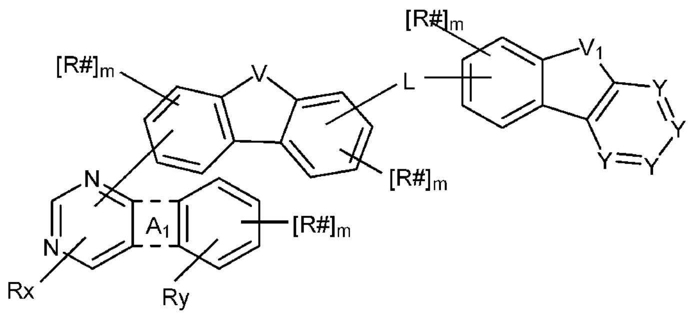

Formula (1)

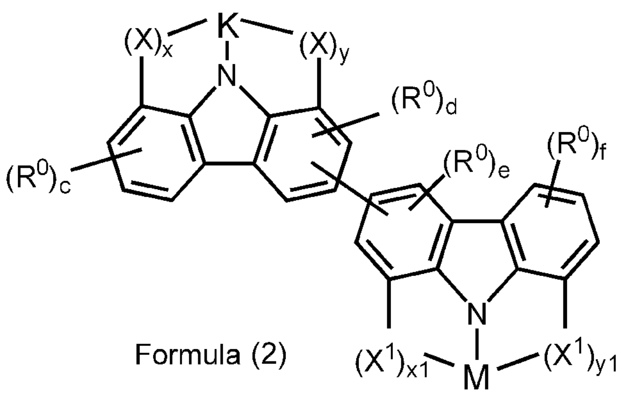

Formula (2)

where the following applies to the symbols and indices used:
ring $A_1$ in formula (1) conforms to the formula (1A)

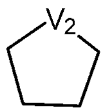
Formula (1A)

V is O or S;
$V_1$ is O, S, $C(R^1)_2$ or N-A;
$V_2$ is O or S;
Y is on each occurrence, independently of one another, identically or differently, CH, CR or CA, or two adjacent groups Y together form a group of the formula (1B)

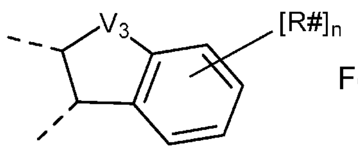
Formula (1B)

and the remaining groups Y are, independently of one another, CH or CR, where $V_3$ denotes O, S, $C(R^1)_2$ or N-A and the dashed bonds show the linking of this group to the remainder of the formula (1);
Rx is $L_1$-R*;
Ry is $L_2$-R*;
R* is on each occurrence, independently, an aromatic or heteroaromatic ring system having 6 to 14 ring atoms, which may be substituted by one or more radicals R;
L, $L_1$, $L_2$ are in each case, independently of one another, a bond or a phenylene group, which may in each case be substituted by one or more radicals R;
A is on each occurrence, identically or differently, an aromatic or heteroaromatic ring system having 6 to 30 ring atoms, which may be substituted by one or more radicals R;
n is 0, 1, 2, 3 or 4;
m is 0, 1, 2 or 3;
R# is D or an aryl group having 6 to 12 C atoms, which may be substituted by one or more radicals R;
$R^1$ is on each occurrence, identically or differently, an alkyl group having 1 to 20 C atoms, phenyl, which may be substituted by one or more radicals R, or the two $R^1$, together with the C atom to which they are bonded, form a spiro compound;
R is selected on each occurrence, identically or differently, from D, F, CN, a straight-chain alkyl group having 1 to 20 C atoms or a branched or cyclic alkyl group having 3 to 20 C atoms, where one or more non-adjacent $CH_2$ groups may be replaced by $R^2C=CR^2$, O or S and where one or more H atoms may be replaced by D, F or CN;
$R^2$ is selected on each occurrence, identically or differently, from the group consisting of H, D, F, CN, a straight-chain alkyl group having 1 to 20 C atoms or a branched or cyclic alkyl group having 3 to 20 C atoms, where one or more non-adjacent $CH_2$ groups may be replaced by O or S and where one or more H atoms may be replaced by D, F or CN;
K, M are in each case, independently of one another, an unsubstituted or partially or fully deuterated or mono-R*-substituted aromatic ring system having 6 to 40 ring atoms if x and y denote 0 and if x1 and y1 denote 0, or K, M in each case, independently of one another, form, together with X or $X^1$, a heteroaromatic ring system having 14 to 40 ring atoms as soon as the value of x, x1, y and/or y1 denotes 1;
x, x1 are in each case, independently on each occurrence, 0 or 1;
y, y1 are in each case, independently on each occurrence, 0 or 1;
X and $X^1$ are in each case, independently on each occurrence, a bond or $C(R^+)_2$;
$R^0$ is on each occurrence, independently of one another, an unsubstituted or partially or fully deuterated aromatic ring system having 6 to 18 C atoms;
$R^+$ is on each occurrence, independently of one another, a straight-chain or branched alkyl group having 1 to 4 C atoms and
c, d, e and f are, independently of one another, 0 or 1.

**14.** Mixture according to Claim 13, **characterised in that** the mixture consists of at least one compound of the formula (1), at least one compound of the formula (2) and a phosphorescent emitter.

**15.** Formulation comprising a mixture according to Claim 13 or 14 and at least one solvent.

## Revendications

**1.** Dispositif électroluminescent organique comprenant une anode, une cathode et au moins une couche organique, comprenant au moins une couche émettrice de lumière, où la au moins une couche émettrice de lumière comprend au moins un composé de formule (1) comme matériau hôte 1 et au moins un composé de formule (2) comme matériau hôte 2,

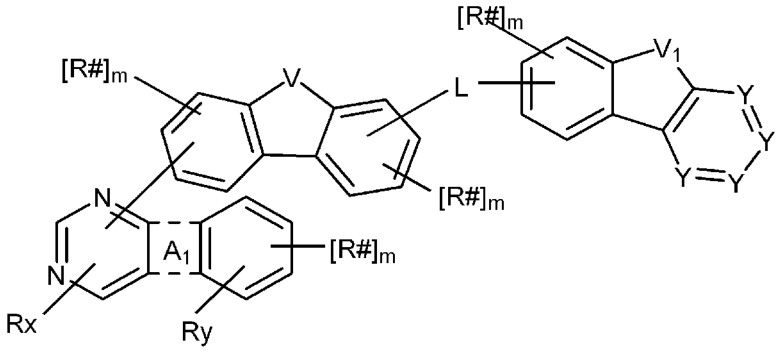

Formule (1)

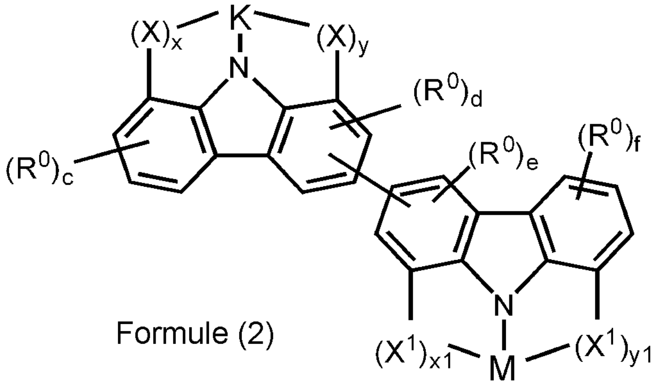

Formule (2)

dans lesquelles ce qui suit s'applique aux symboles et indices utilisés :
le cycle $A_1$ dans la formule (1) répond à la formule (1A)

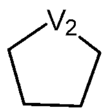

Formule (1A)

V est O ou S ;
$V_1$ est O, S, $C(R^1)_2$ ou N-A ;
$V_2$ est O ou S ;
Y est à chaque occurrence, indépendamment les uns des autres, de manière identique ou différente, CH, CR ou CA, ou deux groupements Y adjacents forment ensemble un groupement de formule (1B)

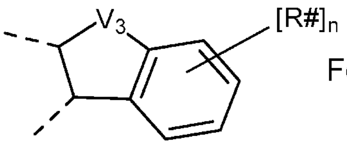

Formule (1B)

et les groupements Y restants sont, indépendamment les uns des autres, CH ou CR, où $V_3$ désigne O, S, $C(R^1)_2$ ou N-A et les liaisons en pointillés illustrent la liaison de ce groupement au reste de la formule (1) ;
Rx est $L_1$-R* ;
Ry est $L_2$-R* ;
R* est à chaque occurrence, indépendamment, un noyau aromatique ou hétéroaromatique ayant de 6 à 14 atomes de cycle, qui peut être substitué par un ou plusieurs radicaux R ;
L, $L_1$, $L_2$ sont dans chaque cas, indépendamment les uns des autres, une liaison ou un groupement phénylène, qui peut dans chaque cas être substitué par un ou plusieurs radicaux R ;
A est à chaque occurrence, de manière identique ou différente, un noyau aromatique ou hétéroaromatique ayant de 6 à 30 atomes de cycle, qui peut être substitué par un ou plusieurs radicaux R ;
n vaut 0, 1, 2, 3 ou 4 ;
m vaut 0, 1, 2 ou 3 ;
R# est D ou un groupement aryle ayant de 6 à 12 atomes de C, qui peut être substitué par un ou plusieurs radicaux R ;
$R^1$ est à chaque occurrence, de manière identique ou différente, un groupement alkyle ayant de 1 à 20 atomes de C, phényle, qui peut être substitué par un ou plusieurs radicaux R, ou les deux $R^1$, conjointement avec l'atome de C auquel ils sont liés, forment un composé spiro ;
R est choisi à chaque occurrence, de manière identique ou différente, parmi D, F, CN, un groupement alkyle à chaîne linéaire ayant de 1 à 20 atomes de C ou un groupement alkyle ramifié ou cyclique ayant de 3 à 20 atomes de C, où un ou plusieurs groupements $CH_2$ non adjacents peuvent être remplacés par $R^2C=CR^2$, O ou S et où un ou plusieurs atomes de H peuvent être remplacés par D, F ou CN ;
$R^2$ est choisi à chaque occurrence, de manière identique ou différente, dans le groupe constitué par H, D, F, CN, un groupement alkyle à chaîne linéaire ayant de 1 à 20 atomes de C ou un groupement alkyle ramifié ou cyclique ayant de 3 à 20 atomes de C, où un ou plusieurs groupements $CH_2$ non adjacents peuvent être remplacés par O ou S et où un ou plusieurs atomes de H peuvent être remplacés par D, F ou CN ;
K, M sont dans chaque cas, indépendamment l'un de l'autre, un noyau aromatique qui est non substitué ou partiellement ou totalement deutérié ou monosubstitué par R* ayant de 6 à 40 atomes de cycle si x et y dénotent 0 et si x1 et y1 dénotent 0, ou
K, M dans chaque cas, indépendamment l'un de l'autre, forment, conjointement avec X ou $X^1$, un noyau hétéroaromatique ayant de 14 à 40 atomes de cycle dès que la valeur de x, x1, y et/ou y1 désigne 1 ;
x, x1 valent dans chaque cas, indépendamment à chaque occurrence, 0 ou 1 ;
y, y1 valent dans chaque cas, indépendamment à chaque occurrence, 0 ou 1 ;
X et $X^1$ sont dans chaque cas, indépendamment à chaque occurrence, une liaison ou $C(R^+)_2$ ;
R° est à chaque occurrence, indépendamment les uns des autres, un noyau aromatique non substitué ou partiellement ou totalement deutérié ayant de 6 à 18 atomes de C ;
$R^+$ est à chaque occurrence, indépendamment les uns des autres, un groupement alkyle à chaîne linéaire ou ramifiée ayant de 1 à 4 atomes de C et
c, d, e et f valent, indépendamment les uns des autres, 0 ou 1.

2. Dispositif électroluminescent organique selon la revendication 1, **caractérisé en ce que** le matériau hôte 1 répond à la formule (1a)

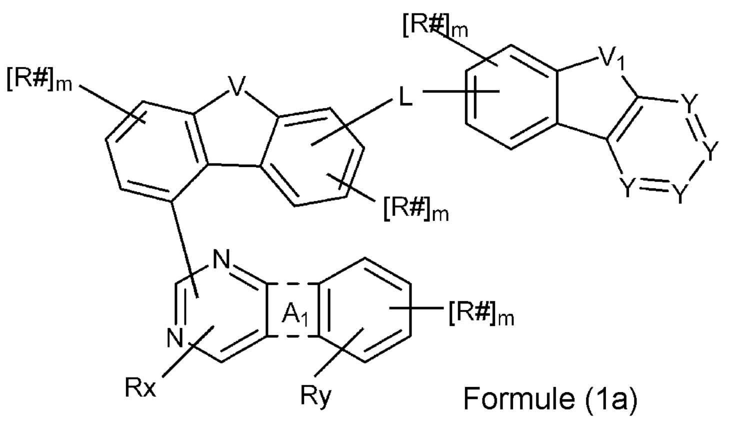

Formule (1a)

dans laquelle les symboles Y, V, $V_1$, R#, m, Rx, Ry, L et le cycle $A_1$ utilisés revêtent une signification telle que selon

la revendication 1.

**3.** Dispositif électroluminescent organique selon la revendication 1 ou 2, **caractérisé en ce que** $V_1$ désigne O ou NA.

**4.** Dispositif électroluminescent organique selon l'une ou plusieurs parmi les revendications 1 à 3, **caractérisé en ce que** le matériau hôte 2 répond à l'une des formules (2a), (2b) ou (2c)

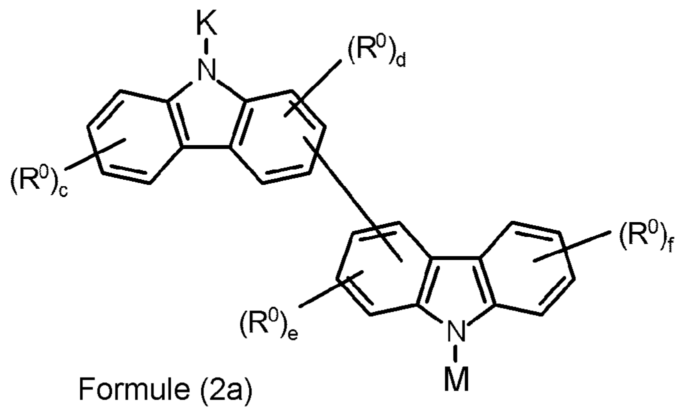

Formule (2a)

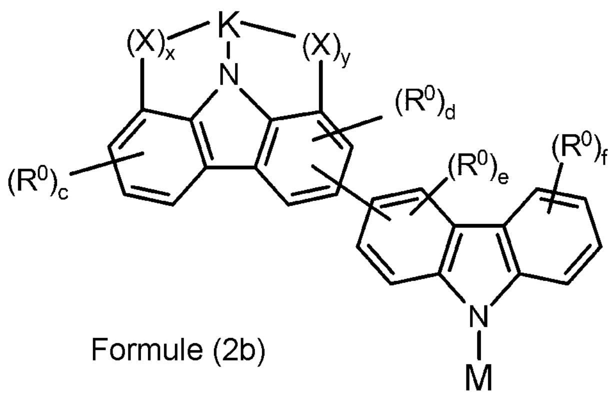

Formule (2b)

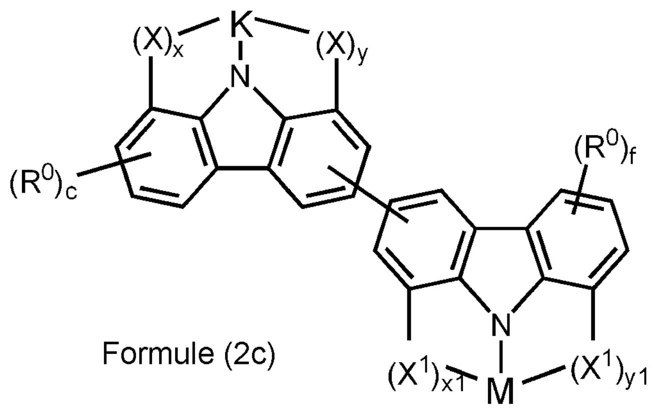

Formule (2c)

dans lesquelles les symboles et indices X, $X^1$, $R^0$, c, d, e et f utilisés revêtent une signification telle que selon la revendication 1 et
K et M dans les composés de formule (2a) dans chaque cas, indépendamment l'un de l'autre, dénotent un noyau aromatique ayant de 6 à 40 atomes de cycle qui est non substitué ou partiellement ou totalement deutérié ou monosubstitué par R* ; M dans les composés de formule (2b) désigne un noyau aromatique ayant de 6 à 40 atomes de cycle qui est non substitué ou partiellement ou totalement deutérié ou monosubstitué par R* ;
K dans les composés de formule (2b) forme, conjointement avec X, un noyau hétéroaromatique ayant de 14 à 40 atomes de cycle et x et y dans les composés de formule (2b) dans chaque cas, indépendamment l'un de l'autre, dénotent 0 ou 1 et la somme de x et y désigne au moins 1 ; et

K et M dans les composés de formule (2c) dans chaque cas, indépendamment l'un de l'autre, forment, conjointement avec X ou $X^1$, un noyau hétéroaromatique ayant de 14 à 40 atomes de cycle ; et

x, x1, y et y1 dans les composés de formule (2c) dans chaque cas, indépendamment les uns des autres, dénotent 0 ou 1 et la somme de x et y désigne au moins 1 et la somme de x1 et y1 désigne au moins 1.

5. Dispositif électroluminescent organique selon l'une ou plusieurs parmi les revendications 1 à 4, **caractérisé en ce qu'**il s'agit d'un dispositif électroluminescent choisi parmi les transistors organiques émetteurs de lumière (OLET), les dispositifs organiques à extinction de champ (OFQD), les cellules électrochimiques organiques émettrices de lumière (OLEC, LEC, LEEC), les diodes laser organiques (O-lasers) et les diodes électroluminescentes organiques (OLED).

6. Dispositif électroluminescent organique selon l'une ou plusieurs parmi les revendications 1 à 5, **caractérisé en ce que** cette couche organique, outre la couche émettrice de lumière (EML), comprend une couche d'injection de trous (HIL), une couche de transport de trous (HTL), une couche de transport d'électrons (ETL), une couche d'injection d'électrons (EIL) et/ou une couche de blocage de trous (HBL).

7. Dispositif électroluminescent organique selon l'une ou plusieurs parmi les revendications 1 à 6, **caractérisé en ce que**, outre le au moins un matériau hôte 1 et le au moins un matériau hôte 2, la couche émettrice de lumière comprend au moins un émetteur phosphorescent.

8. Dispositif électroluminescent organique selon l'une ou plusieurs parmi les revendications 1 à 7, **caractérisé en ce que** la couche organique comprend une couche d'injection de trous (HIL) et/ou une couche de transport de trous (HTL) dont le matériau d'injection de trous et le matériau de transport de trous est une monoamine qui ne contient pas de motif carbazole.

9. Procédé de production d'un dispositif selon l'une ou plusieurs parmi les revendications 1 à 8, **caractérisé en ce que** la couche organique est appliquée par dépôt en phase gazeuse ou à partir d'une solution.

10. Procédé selon la revendication 9, **caractérisé en ce que**, pour la production de la couche émettrice de lumière, le au moins un composé de formule (1) et le au moins un composé de formule (2) sont déposés à partir de la phase gazeuse successivement ou simultanément à partir d'au moins deux sources de matériau, éventuellement avec le au moins un émetteur phosphorescent.

11. Procédé selon la revendication 9, **caractérisé en ce que**, pour la production de la couche émettrice de lumière, le au moins un composé de formule (1) et le au moins un composé de formule (2) sont déposés à partir de la phase gazeuse sous forme de mélange, successivement ou simultanément avec le au moins un émetteur phosphorescent.

12. Procédé selon la revendication 9, **caractérisé en ce que**, pour la production de la couche émettrice de lumière, le au moins un composé de formule (1) et le au moins un composé de formule (2) sont appliqués à partir d'une solution conjointement avec le au moins un émetteur phosphorescent.

13. Mélange comprenant au moins un composé de formule (1) et au moins un composé de formule (2),

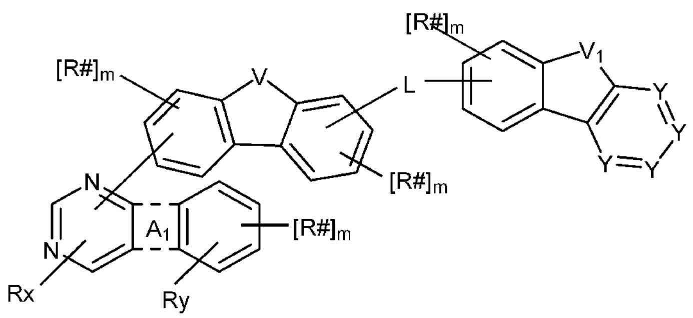

Formule (1)

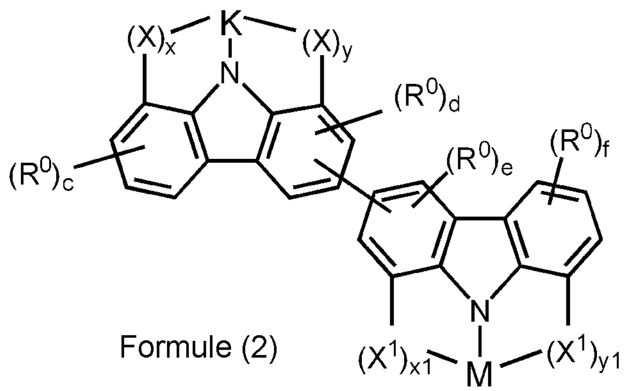

Formule (2)

dans lesquelles ce qui suit s'applique aux symboles et indices utilisés :
le cycle $A_1$ dans la formule (1) répond à la formule (1A)

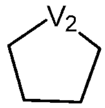

Formule (1A)

V est O ou S ;
$V_1$ est O, S, $C(R^1)_2$ ou N-A ;
$V_2$ est O ou S ;
Y est à chaque occurrence, indépendamment les uns des autres, de manière identique ou différente, CH, CR ou CA, ou deux groupements Y adjacents forment ensemble un groupement de formule (1B)

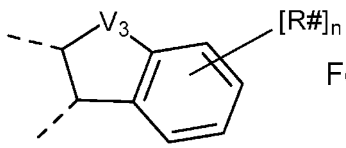

Formule (1B)

et les groupements Y restants sont, indépendamment les uns des autres, CH ou CR, où $V_3$ désigne O, S, $C(R^1)_2$ ou N-A et les liaisons en pointillés illustrent la liaison de ce groupement au reste de la formule (1) ;
Rx est $L_1$-R* ;
Ry est $L_2$-R* ;
R* est à chaque occurrence, indépendamment, un noyau aromatique ou hétéroaromatique ayant de 6 à 14 atomes de cycle, qui peut être substitué par un ou plusieurs radicaux R ;
L, $L_1$, $L_2$ sont dans chaque cas, indépendamment les uns des autres, une liaison ou un groupement phénylène, qui peut dans chaque cas être substitué par un ou plusieurs radicaux R ;
A est à chaque occurrence, de manière identique ou différente, un noyau aromatique ou hétéroaromatique ayant de 6 à 30 atomes de cycle, qui peut être substitué par un ou plusieurs radicaux R ;
n vaut 0, 1, 2, 3 ou 4 ;
m vaut 0, 1, 2 ou 3 ;
R# est D ou un groupement aryle ayant de 6 à 12 atomes de C, qui peut être substitué par un ou plusieurs radicaux R ;
$R^1$ est à chaque occurrence, de manière identique ou différente, un groupement alkyle ayant de 1 à 20 atomes de C, phényle, qui peut être substitué par un ou plusieurs radicaux R, ou les deux $R^1$, conjointement avec l'atome de C auquel ils sont liés, forment un composé spiro ;
R est choisi à chaque occurrence, de manière identique ou différente, parmi D, F, CN, un groupement alkyle à chaîne linéaire ayant de 1 à 20 atomes de C ou un groupement alkyle ramifié ou cyclique ayant de 3 à 20 atomes de C, où un ou plusieurs groupements $CH_2$ non adjacents peuvent être remplacés par $R^2C=CR^2$, O ou S et où un ou plusieurs atomes de H peuvent être remplacés par D, F ou CN ;
$R^2$ est choisi à chaque occurrence, de manière identique ou différente, dans le groupe constitué par H, D, F, CN, un groupement alkyle à chaîne linéaire ayant de 1 à 20 atomes de C ou un groupement alkyle ramifié ou cyclique ayant de 3 à 20 atomes de C, où un ou plusieurs groupements $CH_2$ non adjacents peuvent être

remplacés par O ou S et où un ou plusieurs atomes de H peuvent être remplacés par D, F ou CN ;
K, M sont dans chaque cas, indépendamment l'un de l'autre, un noyau aromatique qui est non substitué ou partiellement ou totalement deutérié ou monosubstitué par R* ayant de 6 à 40 atomes de cycle si x et y dénotent 0 et si x1 et y1 dénotent 0, ou
K, M dans chaque cas, indépendamment l'un de l'autre, forment, conjointement avec X ou $X^1$, un noyau hétéroaromatique ayant de 14 à 40 atomes de cycle dès que la valeur de x, x1, y et/ou y1 désigne 1 ;
x, x1 valent dans chaque cas, indépendamment à chaque occurrence, 0 ou 1 ;
y, y1 valent dans chaque cas, indépendamment à chaque occurrence, 0 ou 1 ;
X et $X^1$ sont dans chaque cas, indépendamment à chaque occurrence, une liaison ou $C(R^+)_2$ ;
$R^0$ est à chaque occurrence, indépendamment les uns des autres, un noyau aromatique non substitué ou partiellement ou totalement deutérié ayant de 6 à 18 atomes de C ;
$R^+$ est à chaque occurrence, indépendamment les uns des autres, un groupement alkyle à chaîne linéaire ou ramifiée ayant de 1 à 4 atomes de C et
c, d, e et f valent, indépendamment les uns des autres, 0 ou 1.

**14.** Mélange selon la revendication 13, **caractérisé en ce que** le mélange est constitué d'au moins un composé de formule (1), d'au moins un composé de formule (2) et d'un émetteur phosphorescent.

**15.** Formulation comprenant un mélange selon la revendication 13 ou 14 et au moins un solvant.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6392250 B1
- US 6803720 B1
- WO 2015169412 A
- WO 2015105315 A
- WO 2015105316 A
- US 2016013421 A
- US 2016072078 A
- US 2017200903 A
- KR 20160046077
- KR 20160046078
- KR 20170113320
- WO 17109637 A
- WO 17186760 A
- WO 18060218 A
- KR 20180010165
- WO 18060307 A
- WO 18234932 A
- WO 19059577 A
- WO 19229583 A
- WO 19229584 A
- US 2020161564 A
- US 20200010476 A
- WO 20067657 A
- US 7294849 B
- WO 2010108579 A
- KR 2018010165
- WO 15105315 A
- WO 171867601 A


### In der Beschreibung aufgeführte Nicht-Patentliteratur


- **M. S. ARNOLD et al.** *Appl. Phys. Lett.,* 2008, vol. 92, 053301